(19) Europäisches Patentamt
European Patent Office
Office européen des brevets

(11) EP 4 050 002 A1

(12) **EUROPEAN PATENT APPLICATION**

(43) Date of publication:
**31.08.2022 Bulletin 2022/35**

(21) Application number: **21159563.2**

(22) Date of filing: **26.02.2021**

(51) International Patent Classification (IPC):
***C07D 271/107*** *(2006.01)* ***C07D 413/10*** *(2006.01)*
***C07D 413/14*** *(2006.01)* ***A61P 35/00*** *(2006.01)*
***A61P 1/08*** *(2006.01)* ***A61K 31/4245*** *(2006.01)*

(52) Cooperative Patent Classification (CPC):
(C-Sets available)
**C07D 271/107; A61K 31/4245; A61K 31/4439; A61K 31/454; A61K 31/4725; A61K 31/497; A61K 31/506; A61K 45/06; A61P 1/08; A61P 35/00; C07D 413/10; C07D 413/14;**
A61K 9/0019; A61K 47/10; A61K 47/14; (Cont.)

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicant: **First Health Pharmaceuticals B.V.**
**1098 XH Amsterdam (NL)**

(72) Inventors:
- **Andreini, Matteo**
  **51013 Pistoia (IT)**
- **Bakker, Jan Willem**
  **53034 Casole d'Elsa (IT)**
- **Tarditi, Alessia**
  **1091 RP Amsterdam (NL)**

(74) Representative: **Pace Napoleone, Maria et al**
**De Simone & Partners S.r.l.**
**Via Vincenzo Bellini, 20**
**00198 Roma (IT)**

(54) **ANTI-PROLIFERATIVE AGENTS**

(57) The present invention relates to compounds of formula (I) and to salts, solvates, stereoisomers and tautomers thereof useful as anti-proliferative agents and/or as inhibitors of DEAD-box (DDX) RNA helicases. The present invention also provides: uses of such compounds, particularly for the prevention and/or treatment of hyperproliferative disorders, pharmaceutical compositions comprising such compounds and processes for the manufacture thereof.

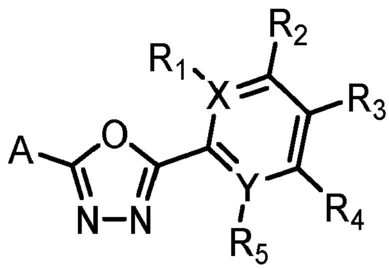

(I)

EP 4 050 002 A1

(52) Cooperative Patent Classification (CPC): (Cont.)
A61K 47/20; A61K 47/26; A61K 47/44

C-Sets
**A61K 31/4245, A61K 2300/00;**
**A61K 31/4439, A61K 2300/00;**
**A61K 31/454, A61K 2300/00;**
**A61K 31/4725, A61K 2300/00;**
**A61K 31/497, A61K 2300/00;**
**A61K 31/506, A61K 2300/00**

## Description

### TECHNICAL FIELD OF THE INVENTION

The present invention relates to compounds of formula (I) useful as anti-proliferative agents and/or as inhibitors of DEAD-box (DDX) RNA helicases. Such compounds are thus suitable for pharmaceutical applications, in particular for the prevention and/or treatment of hyperproliferative disorders. The present invention further concerns uses of such compounds, pharmaceutical compositions comprising them and processes for their manufacture.

### BACKGROUND ART

RNA Helicases are versatile enzymes involved in both nuclear and cytoplasmic functions. Through their main tasks of RNA binding, transport and unwinding and protein translation, they initiate and modulate many cellular pathways. Protein translation is at the heart of all biological life, however, with respect to healthy normally proliferating cells, cancer cells have an increased metabolism with significantly higher levels of protein synthesis needed to sustain their up-regulated proliferation. Cancer cells have been described to be dependent on various DDX helicases, as reported by the wide body of literature available that investigates the overexpression and the role of various RNA Helicases in cancer (Wanpei Cai, et al., Wanted DEAD/H or Alive: Helicases Winding Up in Cancer. Journal of the National Cancer Institute 2017, 109(6)). For instance the overexpression of DDX5 is correlated with worse outcome including lower disease free survival and lower overall survival; moreover, the knockdown of DDX5 suppress hepatocellular carcinoma migration, invasion and epithelial to mesenchymal transition (Xue, Y. et al., DDX5 promotes hepatocellular carcinoma tumorigenesis via Akt signaling pathway. Biochemical and biophysical research communications, 2018, 503, 2885; Ma, Z. et al., Knockdown of DDX5 Inhibits the Proliferation and Tumorigenesis in Esophageal Cancer. Oncology research, 2017, 25, 887; Guturi, K. K. N. et al., DEAD-box protein p68 is regulated by β-catenin/transcription factor 4 to maintain a positive feedback loop in control of breast cancer progression. Breast cancer research, 2014, 16, 496; Choi, Y.-J. & Lee, S.-G. The DEAD-box RNA helicase DDX3 interacts with DDX5, co-localizes with it in the cytoplasm during the G2/M phase of the cycle, and affects its shuttling during mRNP export. J. Cell. Biochem., 2012, 113, 985; Mazurek, A. et al., DDX5 regulates DNA replication and is required for cell proliferation in a subset of breast cancer cells. Cancer discovery, 2012, 2, 812; Wang, D. et al., RNA helicase DDX5 regulates microRNA expression and contributes to cytoskeletal reorganization in basal breast cancer cells. Molecular & cellular proteomics, 2012, 11, M111.011932).

Another DDX helicase, DDX3X is under evaluation as a prognostic marker in many cancers and is reported to play an oncogenic role in breast cancer by cell cycle modulation, by inhibiting the expression of "Kruppel-like factor 4" - a transcription factor and cell cycle repressor -, or by promoting the expression of various cyclins (Dutertre, M. et al., Estrogen regulation and physiopathologic significance of alternative promoters in breast cancer. Cancer research, 2010, 70, 3760; Vellky, J. E. et al., Expression and Localization of DDX3 in Prostate Cancer Progression and Metastasis. The American journal of pathology, 2019, 189, 1256; Wang, Z. et al., Rottlerin upregulates DDX3 expression in hepatocellular carcinoma. Biochemical and biophysical research communications, 2018, 495, 1503; Chen, H.-H. et al., DDX3 activates CBC-eIF3-mediated translation of uORF-containing oncogenic mRNAs to promote metastasis in HNSCC. Cancer research, 2018, 78, 4512; Tantravedi, S. et al., Targeting DDX3 in Medulloblastoma Using the Small Molecule Inhibitor RK-33. Translational oncology, 2018, 12, 96; Cannizzaro, E. et al., DDX3X RNA helicase affects breast cancer cell cycle progression by regulating expression of KLF4. FEBS letters, 2018, 592, 2308; Heerma van Voss, M. R. et al., Combination treatment using DDX3 and PARP inhibitors induces synthetic lethality in BRCA1-proficient breast cancer. Medical oncology, 2017, 34, 33; Heerma van Voss, M. R. et al., The prognostic effect of DDX3 upregulation in distant breast cancer metastases. Clinical & experimental metastasis, 2017, 34, 85; van Voss, M. R. H. et al., Targeting mitochondrial translation by inhibiting DDX3: a novel radiosensitization strategy for cancer treatment Oncogene, Springer Nature, 2017, 37, 63; Heerma van Voss, M. R.; et al., Nuclear DDX3 expression predicts poor outcome in colorectal and breast cancer. OncoTargets and therapy, 2017, 10, 3501; Xie, M. et al., RK-33 radiosensitizes prostate cancer cells by blocking the RNA helicase DDX3. Cancer Res., 2016; He, T.-Y. et al., DDX3 promotes tumor invasion in colorectal cancer via the CK1ε/Dv12 axis. Sci. Rep., 2016, 6, 21483; Wilky, B. et al., RNA helicase DDX3: a novel therapeutic target in Ewing sarcoma. Oncogene, 2016, 35, 2574; Xie, M. et al., RK-33 Radiosensitizes Prostate Cancer Cells by Blocking the RNA Helicase DDX3. Cancer research, 2016, 76, 6340; Bol, G. M. et al., DDX3, a potential target for cancer treatment. Mol. Cancer, 2015, 14, 188).

DDX21 is highly expressed in colon cancer, lymphomas, and some breast cancers, where it promotes rRNA processing. A significant reduction of tumorigenicity in vitro an in vivo have been obtained after DDX21 knockdown in tumor cells expressing high levels of this protein. Studies on the mechanism of tumorigenic functions of DDX21 found that this protein supports tumorigenesis by two independent mechanisms; promoting AP-1 activity and rRNA processing. (Cao, J. et al., DDX21 promotes gastric cancer proliferation by regulating cell cycle. Biochemical and biophysical research communications, 2018, 505, 1189; Zhang, Y. et al., Elevated DDX21 regulates c-Jun activity and rRNA processing in

human breast cancers. Breast cancer research, 2014, 16, 449).

DDX23 overexpression is correlated with poor survival prognosis in glioma patients. Glioma cell proliferation and invasion is suppressed by knockdown of DDX23 expression in vitro and in vivo. It is demonstrated that DDX23 modulates ribonuclease Drosha microprocessor activity through the formation a DDX23-pri-miR-21-Drosha complex. Mutagenesis experiments demonstrated that the helicase activity of DDX23 is essential for the maturation of miR-21. miR-21 is considered a typical "onco-miR"; most of the targets of this micro RNA are tumor suppressors, its overexpression is associated with a wide variety of cancers including breast, liver, colon, ovaries, lung, prostate, thyroid, esophagus, pancreas and brain (Mao, G. et al., DDX23-Linc00630-HDAC1 axis activates the Notch pathway to promote metastasis. Oncotarget, 2017, 8, 38937-38949; Yin, J., et al., DEAD-box RNA helicase DDX23 modulates glioma malignancy via elevating miR-21 biogenesis. Brain: a journal of neurology, 2015, 138, 2553).

The tumorigenic mechanisms which involve the DDX helicases are not yet completely understood but it is clear that the functions, or the overexpression of these proteins, are tightly correlated with the pathogenesis of several types of cancers.

Genetic mutations induced by both inherited and environmental factors drive cancer development. Mutations leading to over-activation of the proteins and related pathways have been exploited by targeted therapy with success, but not all the mutated genes or proteins are druggable, thus some mutations still lead an unfavorable prognosis.

So far some DDX3 inactivating mutations have been described in cases of medulloblastoma, but not in other cancer types. Indeed the DDX3-mediated mechanisms converge with pathways associated to several cancer driven known mutations, such as the cell cycle, WNT pathways and apoptosis induction.

TP53 and CDKN2A are the genes most commonly inactivated in cancer and code for proteins modulating cell cycle progression, apoptosis and cell divisions. Melanoma, leukemia, glioblastoma, breast, head and neck, pancreas, prostate, liver and lung cancer have high frequency of mutations of CDKN2A and TP53 and some mutations have been associated to lower susceptibility to other treatments (Xande, J.G., et al. Bicistronic transfer of CDKN2A and p53 culminates in collaborative killing of human lung cancer cells in vitro and in vivo. Gene Ther. 2020, 27, 51; Cicenas J, et al., KRAS, TP53, CDKN2A, SMAD4, BRCA1, and BRCA2 Mutations in Pancreatic Cancer. Cancers. 2017; 9 (5), 42; Hodis E., et al., A Landscape of Driver Mutations in Melanoma. Cell, 2012, 150 (2), 251).

For lung cancer, such as adenocarcinoma and squamous cell carcinomas, several mutations have been described for K-RAS, EGFR, BRAF, MET, TP53 and CDKN2A. Targeted therapies have been developed that significantly improved the prognosis of this type of cancer, as Gefitinib targeting EGFR, but cases of resistance and of low susceptibility due to other mutations are frequent (Greulich H. The Genomics of Lung Adenocarcinoma. Opportunities for Targeted Therapies. Genes Cancer, 2010 Dec; 1 (12), 1200). Breast cancer is also associated with several mutations, some of which represent targets for successful therapy as Her2-neu, whereas their absence represents an unfavorable diagnosis as in the Triple Negative Breast Cancer type (TBNC). BRCA, BRAF and K-RAS are among the mutations frequently associated to incurable breast cancer or with resistance to treatments (Harbeck, N., et al. Breast cancer. Nat. Rev. Dis. Primers, 2019, 5, 66; Peshkin BN et al., BRCA1/2 mutations and triple negative breast cancers. Breast Dis., 2010, 32(1-2), 25).

Therefore, there is still the need to provide inhibitors of DDX RNA helicases and/or agents endowed with anti-hyperproliferative activity.

Within the present invention a novel class of oxadiazoles derivatives has been identified characterized by strong anti-proliferative effect and also having optimized pharmacological properties, including high solubility, stability in human plasma, low potential for drug-drug interaction and clean on the hERG mediated cardiac toxicity. They also show a good pharmacokinetic profile and distribute to relevant organs while not inducing systemic or organ toxicity.

## SUMMARY OF THE INVENTION

The present invention concerns a class of human DDX RNA Helicase inhibitors which specifically target the site where the RNA molecules bind to the enzyme, thus interfering with the enzyme's main tasks of unwinding and transport of RNA molecules and as such affecting the entire process of protein translation.

In particular, the approach underlying the invention is focused on the identification of small molecule inhibitors with a polypharmacology profile toward a subset of DDX helicases, DDX3X, DDX4, DDX5, DDX17, DDX21 and DDX23, involved in hyper-proliferative diseases. Moreover, the small molecule inhibitors identified are specifically designed to directly block the interaction of RNA with the DDX helicases in question, blocking all the functions of DDX helicases that involve RNA processing and leaving untouched all the functions that involve ATP hydrolysis, reducing the toxicity and potential collateral effects.

Advantageously, the compounds of the present invention exert a strong anti-proliferative activity and cytotoxicity in a variety of cancers *in vitro* while having negligible toxicity in non-cancer cell lines. In particular the compounds have a strong anti-proliferative effect against triple negative breast cancer, lung adenocarcinoma, hepatocellular carcinoma, prostate cancer, malignant melanoma, pancreas epithelioid carcinoma, chronic myelogenous leukemia, T-lymphocyte

lymphoma and glioblastoma. Moreover, they can counteract tumor growth *in vivo.* As such, they may be employed as anti-proliferative agents with a broad activity spectrum.

The compounds of the present invention also possess a desirable pharmacological profile. In fact the compounds of the invention are soluble up to high concentrations. They display stability in human plasma. They do not inhibit cytochrome P450. The compounds of the invention do not induce hERG mediated cardiac toxicity. They also show a good pharmacokinetic profile and distribute to relevant organs while not inducing systemic or organ toxicity.

In clinical practice, it has been observed that the presence of one or more mutations might affect the results of treatments, being targeted therapies or chemotherapy, and as such the compounds of the present invention have been tested on a panel of cancer derived cells bearing different mutations, in particular those reported to affect the success of the existing therapeutic approaches such as those at the BRCA, KRAS and CDKN2A genes (Table 4).

## DETAILED DESCRIPTION OF THE INVENTION

Therefore, it is an object of the present invention a compound of formula (I):

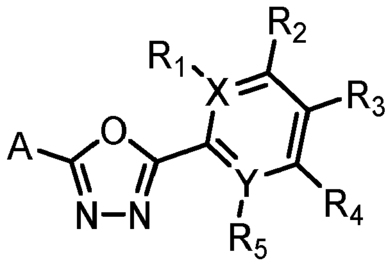

(I)

or a salt, solvate, stereoisomer or tautomer thereof, wherein:

A is substituted or unsubstituted aryl or substituted or unsubstituted heteroaryl;
wherein the substituted aryl and the substituted heteroaryl are independently substituted with one or more substituents each independently selected from the group consisting of: unsubstituted or substituted $C_1$-$C_6$ alkyl, unsubstituted or substituted $C_2$-$C_6$ alkenyl, unsubstituted or substituted $C_2$-$C_6$ alkynyl, unsubstituted or substituted cyclic $C_3$-$C_8$ alkyl, unsubstituted or substituted cyclic $C_3$-$C_8$ alkenyl, a cyclic amine selected from the group of pyrrolidine, piperidine, morpholine, piperazine, halogen, $-OR_A$, $-SR_A$, $-NR_AR_B$, $-S(O)R_A$, $-S(O)_2R_A$, $-S(O)_2OR_A$, $-S(O)_2NR_AR_B$,$-NR_AS(O)_2R_B$, $-COOR_A$, $-C(O)NR_AR_B$, $-C(O)R_A$, $-OC(O)R_A$, $-NR_BC(O)R_A$, $-NR_CC(O)NR_AR_B$,$-OP(O)(OR_A)_2$, $-OP(O)(NR_AR_B)_2$, $-NO_2$, -NO, -C≡N, $-N^+{\equiv}C^-$, -SCN, -OCN and 5-membered heteroaromatic ring optionally substituted with $C_1$-$C_6$ alkyl;

**X** and **Y** are each independently selected from the group consisting of: C and N.
**$R_1$, $R_2$, $R_4$** and **$R_5$** are each independently selected from the group consisting of: H, unsubstituted or substituted $C_1$-$C_6$ alkyl, unsubstituted or substituted $C_2$-$C_6$ alkenyl, unsubstituted or substituted $C_2$-$C_6$ alkynyl, unsubstituted or substituted cyclic $C_3$-$C_8$ alkyl, unsubstituted or substituted cyclic $C_3$-$C_8$ alkenyl, halogen, $-OR_A$, $-SR_A$, $-NR_AR_B$, $-S(O)R_A$, $-S(O)_2R_A$, $-S(O)_2OR_A$, $-S(O)_2NR_AR_B$,$-NR_AS(O)_2R_B$, $-COOR_A$, $-C(O)NR_AR_B$, $-C(O)R_A$, $-OC(O)R_A$, $-NR_AC(O)R_B$, $-NR_CC(O)NR_AR_B$,$-OP(O)(OR_A)_2$, $-OP(O)(NR_AR_B)_2$, $-NO_2$, -NO, -C≡N, $-N^+{\equiv}C^-$, -SCN and -OCN;
$R_3$ is a heteroaryl group selected from the group consisting of:

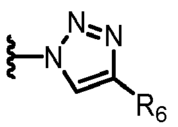 , 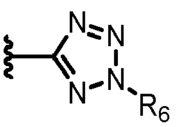 , 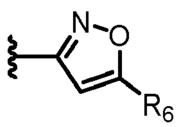 , 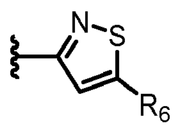 , 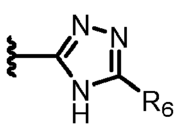 , 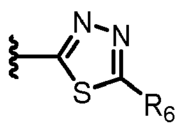 ,

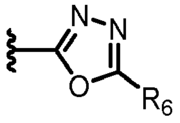 , 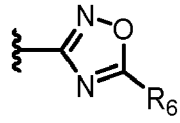 , 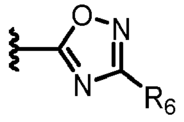 , 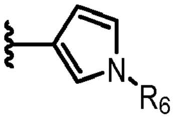 , 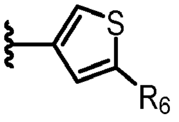 , 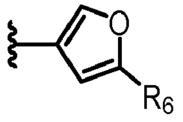 ,

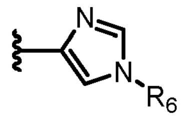 , 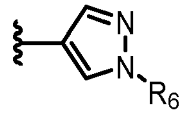 , 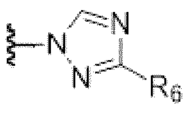 , 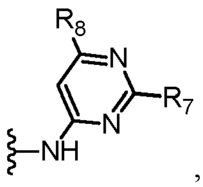 , 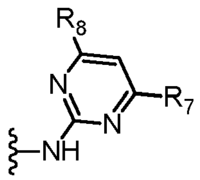

and

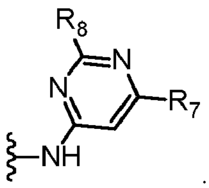.

$R_6$ is unsubstituted or substituted $C_1$-$C_8$ alkyl;

wherein the substituted $C_1$-$C_8$ alkyl is substituted with one or more substituents each independently selected from the group consisting of: unsubstituted or substituted $C_1$-$C_6$ alkyl, unsubstituted or substituted $C_2$-$C_6$ alkenyl, unsubstituted or substituted $C_2$-$C_6$ alkynyl, unsubstituted or substituted cyclic $C_3$-$C_8$ alkyl, unsubstituted or substituted cyclic $C_3$-$C_8$ alkenyl, halogen, $-OR_A$, $-SR_A$, $-NR_AR_B$, $-S(O)R_A$, $-S(O)_2R_A$, $-S(O)_2OR_A$, $-S(O)_2NR_AR_B$, $-NR_AS(O)_2R_B$, - $COOR_A$, $-C(O)NR_AR_B$, $-C(O)R_A$, $-OC(O)R_A$, $-NR_BC(O)R_A$, $-NR_CC(O)NR_AR_B$, $-OP(O)(OR_A)_2$, $-OP(O)(NR_AR_B)_2$, $-NO_2$, -NO, -C≡N, $-N^+{\equiv}C^-$, -SCN and -OCN;

wherein any one or more of said substituted $C_1$-$C_6$ alkyl, substituted $C_2$-$C_6$ alkenyl, substituted $C_2$-$C_6$ alkynyl, substituted cyclic $C_3$-$C_8$ alkyl and substituted cyclic $C_3$-$C_8$ alkenyl are each independently substituted with one or more substituents each independently selected from the group consisting of: halogen, $-OR_A$, $-SR_A$, $-NR_AR_B$, $-S(O)R_A$, $-S(O)_2R_A$, $-S(O)_2OR_A$,$-S(O)_2NR_AR_B$, $-NR_AS(O)_2R_B$, $-COOR_A$, $-C(O)NR_AR_B$, $-C(O)R_A$, $-OC(O)R_A$, $-NR_BC(O)R_A$,$-NR_CC(O)NR_AR_B$, $-OP(O)(OR_A)_2$, $-OP(O)(NR_AR_B)_2$, $-NO_2$, -NO, -C≡N, $-N^+{\equiv}C^-$, -SCN and-OCN;

$\mathbf{R_7}$ is N-piperidinyl, N-pyrrolidinyl or N-morpholinyl;

$\mathbf{R_8}$ is H or unsubstituted or substituted $C_1$-$C_8$ alkyl;

wherein the substituted $C_1$-$C_8$ alkyl is substituted with one or more substituents each independently selected from the group consisting of: unsubstituted or substituted $C_1$-$C_6$ alkyl, unsubstituted or substituted $C_2$-$C_6$ alkenyl, unsubstituted or substituted $C_2$-$C_6$ alkynyl, unsubstituted or substituted cyclic $C_3$-$C_8$ alkyl, unsubstituted or substituted cyclic $C_3$-$C_8$ alkenyl, halogen, $-OR_A$, $-SR_A$, $-NR_AR_B$, $-S(O)R_A$, $-S(O)_2R_A$, $-S(O)_2OR_A$, $-S(O)_2NR_AR_B$, $-NR_AS(O)_2R_B$,$-COOR_A$, $-C(O)NR_AR_B$, $-C(O)R_A$, $-OC(O)R_A$, $-NR_BC(O)R_A$, $-NR_CC(O)NR_AR_B$, $-OP(O)(OR_A)_2$, $-OP(O)(NR_AR_B)_2$, $-NO_2$, -NO, -C≡N, $-N^+{\equiv}C^-$, -SCN and -OCN;

wherein any one or more of said substituted $C_1$-$C_6$ alkyl, substituted $C_2$-$C_6$ alkenyl, substituted $C_2$-$C_6$ alkynyl, substituted cyclic $C_3$-$C_8$ alkyl and substituted cyclic $C_3$-$C_8$ alkenyl are each independently substituted with one or more substituents each independently selected from the group consisting of: halogen, $-OR_A$, $-SR_A$, $-NR_AR_B$, $-S(O)R_A$, $-S(O)_2R_A$, $-S(O)_2OR_A$,$-S(O)_2NR_AR_B$, $-NR_AS(O)_2R_B$, $-COOR_A$, $-C(O)NR_AR_B$, $-C(O)R_A$, $-OC(O)R_A$, $-NR_BC(O)R_A$,$-NR_CC(O)NR_AR_B$, $-OP(O)(OR_A)_2$, $-OP(O)(NR_AR_B)_2$, $-NO_2$, -NO, -C≡N, $-N^+{\equiv}C^-$, -SCN and-OCN;

$\mathbf{R_A}$, $\mathbf{R_B}$ and $\mathbf{R_C}$ are each independently selected from the group consisting of: H, unsubstituted or substituted $C_1$-$C_6$ alkyl, unsubstituted or substituted $C_2$-$C_6$ alkenyl, unsubstituted or substituted $C_2$-$C_6$ alkynyl, unsubstituted or substituted cyclic $C_3$-$C_8$ alkyl, unsubstituted or substituted cyclic $C_3$-$C_8$ alkenyl,

or $\mathbf{R_A}$ and $\mathbf{R_B}$ together with the nitrogen to which they are attached, form a 4-7 membered saturated or partially unsaturated ring optionally containing one or more additional heteroatoms independently selected from N, S and O, wherein the substituted $C_1$-$C_6$ alkyl, the substituted $C_2$-$C_6$ alkenyl, the substituted $C_2$-$C_6$ alkynyl, the substituted cyclic $C_3$-$C_8$ alkyl and the substituted cyclic $C_3$-$C_8$ alkenyl in position $\mathbf{R_A}$, $\mathbf{R_B}$ and/or $\mathbf{R_C}$ and the 4-7 membered saturated or partially unsaturated ring are each independently substituted with one or more substituents each independently selected from the group consisting of: $C_1$-$C_6$ alkyl, $C_2$-$C_6$ alkenyl, $C_2$-$C_6$ alkynyl, cyclic $C_3$-$C_8$alkyl, cyclic $C_3$-$C_8$ alkenyl, $C_1$-$C_6$ alkoxy, halogen, $C_1$-$C_6$haloalkyl, OH, $-NO_2$, -NO, -C≡N, $-N^+{\equiv}C^-$, -SCN and -OCN;

with the proviso that if $R_3$ is

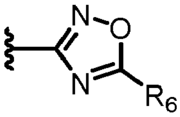

then A is not

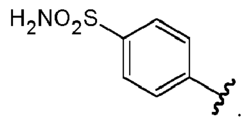.

In a preferred embodiment in A the aryl is phenyl or the heteroaryl is selected from the group consisting of: isoquinoline, quinoline, pyridine, pyrimidine, pyrazine and pyridazine; still preferably the substituted aryl and the substituted heteroaryl are independently substituted with one or more substituents each independently selected from the group consisting of: unsubstituted or substituted $C_1$-$C_6$ alkyl, halogen, -$OR_A$, -$NR_AS(O)_2R_B$, -$NR_BC(O)R_A$ and -$C(O)N(R_A)R_B$; wherein $\mathbf{R_A}$ is selected from the group consisting of: H, unsubstituted or substituted $C_1$-$C_6$ alkyl and unsubstituted or substituted cyclic $C_3$-$C_8$ alkyl;
wherein $\mathbf{R_B}$ is unsubstituted or substituted $C_1$-$C_6$ alkyl or unsubstituted or substituted cyclic $C_3$-$C_8$ alkyl;
wherein any one of said substituted $C_1$-$C_6$ alkyl and cyclic $C_3$-$C_8$ alkyl is independently substituted with one or more halogens.

Still preferably $\mathbf{R_1}$, $\mathbf{R_2}$, $\mathbf{R_4}$ and $\mathbf{R_5}$ are each independently selected from the group consisting of: H, OMe, OEt, F, Cl and NHMe.

Preferably $\mathbf{R_3}$ is selected from the group consisting of:

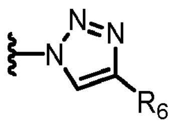, 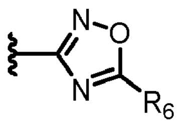,

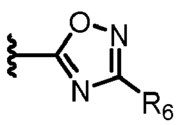 and 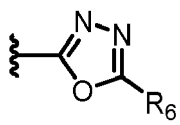.

Preferably, any one of said unsubstituted or substituted $C_1$-$C_8$ alkyl or said unsubstituted or substituted $C_1$-$C_6$ alkyl is an unsubstituted or substituted $C_1$-$C_3$ alkyl or an unsubstituted or substituted $C_1$-$C_4$ alkyl.

In a further preferred embodiment $\mathbf{R_6}$ is unsubstituted or substituted $C_1$-$C_5$ alkyl, preferably selected from the group consisting of: substituted or unsubstituted n-butyl, substituted or unsubstituted n-pentyl, substituted or unsubstituted n-propyl, substituted or unsubstituted ethyl, substituted or unsubstituted methyl, substituted or unsubstituted tert-butyl and substituted or unsubstituted isopropyl, wherein any one of said substituted $C_1$-$C_5$ alkyl is independently substituted with one or more halogens.

Preferably, any one of said cyclic $C_3$-$C_8$ alkyl is cyclopentyl; still preferably any one of said cyclic $C_3$-$C_8$ alkyl is cyclohexyl.

Preferably, the substituted aryl and the substituted heteroaryl are independently substituted with one or more substituents each independently selected from the group consisting of: unsubstituted or substituted $C_1$-$C_6$ alkyl, halogen and -$OR_A$;
wherein $R_A$ is unsubstituted or substituted $C_1$-$C_6$ alkyl or cyclic $C_3$-$C_8$ alkyl;
wherein any one of said substituted $C_1$-$C_6$ alkyl is independently substituted with one or more halogens.
Preferably, the substituted aryl and the substituted heteroaryl are independently substituted with one or more substituents each independently selected from the group consisting of: fluorine, chlorine, methyl, -O-cyclopentyl, trifluoromethyl, isopropyl, -$OCHF_2$.

It is a further object of the invention a compound, salt, solvate, stereoisomer or tautomer as defined above being selected from the group consisting of:

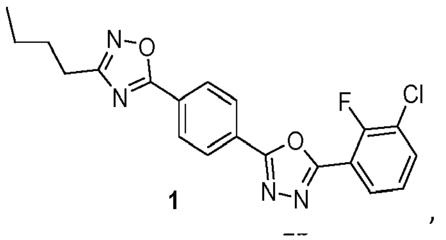

**1** ,

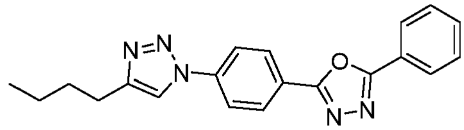

**2** ,

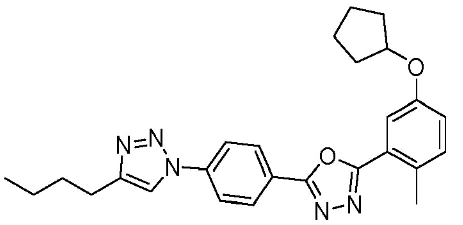

**3** ,

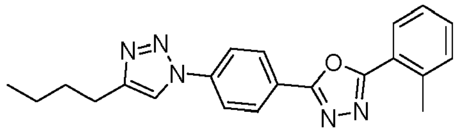

**4** ,

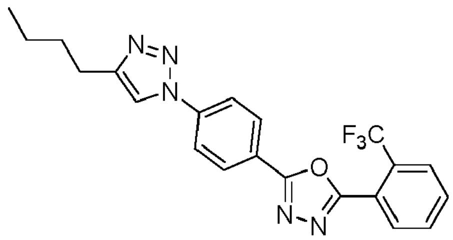

**5** ,

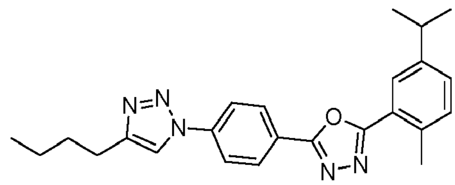

**6** ,

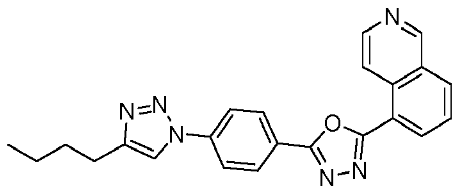

**7** ,

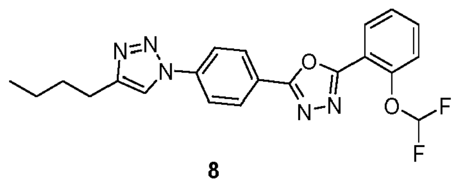

**8** ,

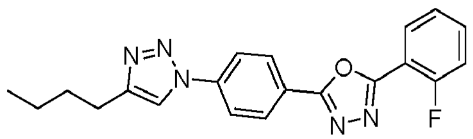

**9** ,

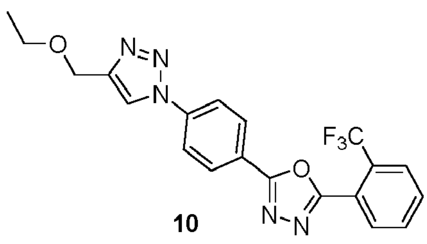

**10** ,

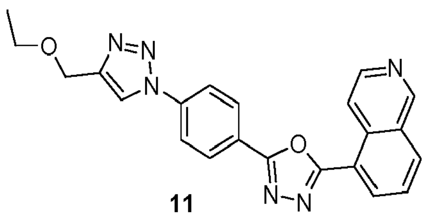

**11** ,

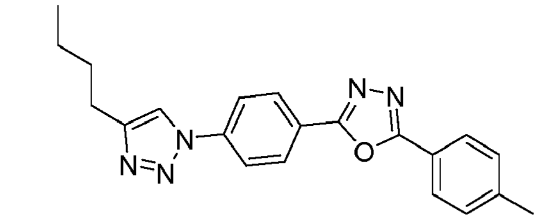

**12** ,

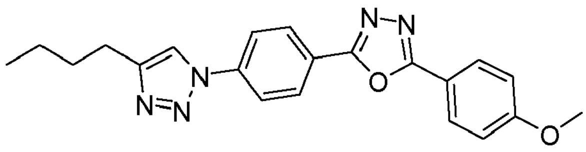

**13** ,

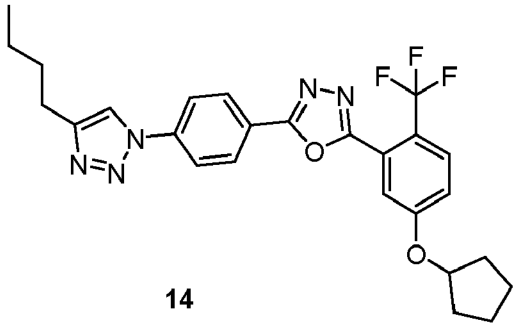

**14** ,

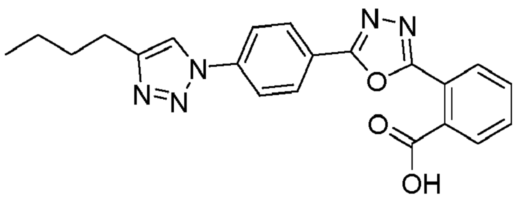

**15** ,

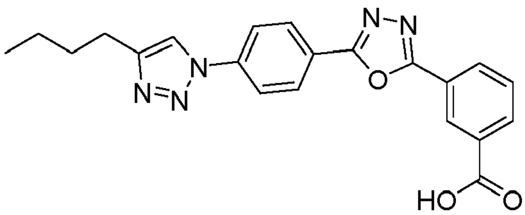

**16** ,

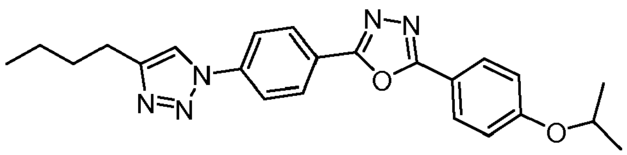

**17** ,

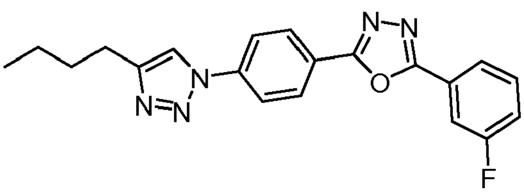

**18** ,

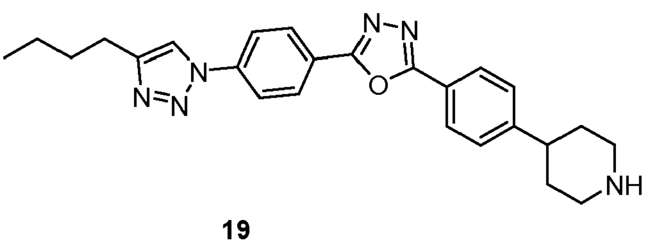

**19** ,

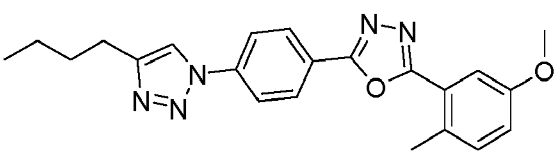

**20** ,

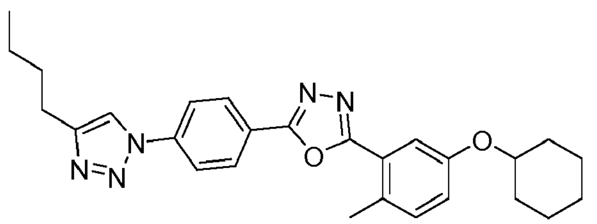

**21** ,

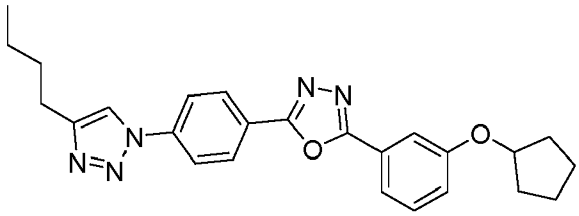

**22** ,

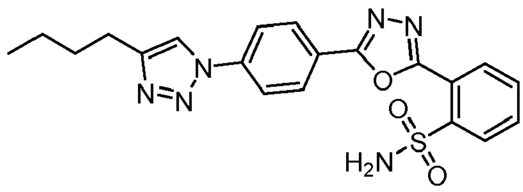

**23** ,

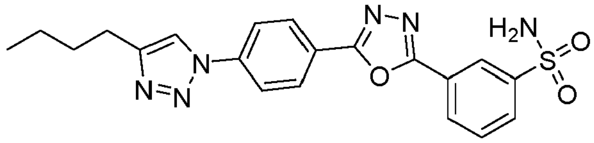

**24** ,

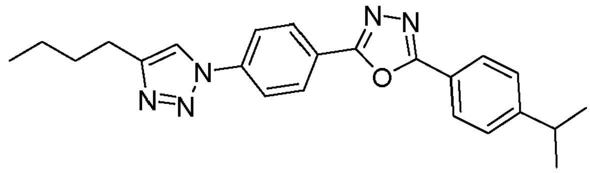

**25** ,

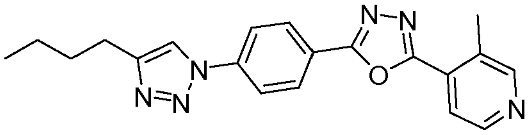

**26** ,

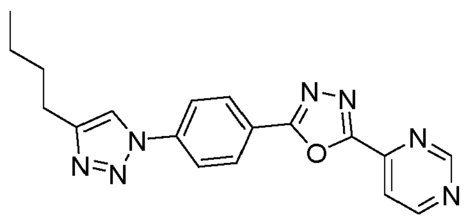

**27** ,

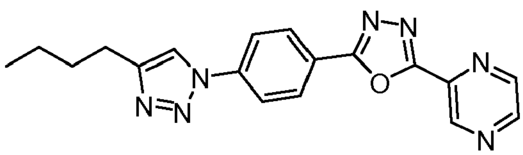

**28** ,

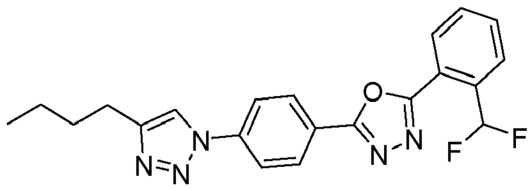

**29** ,

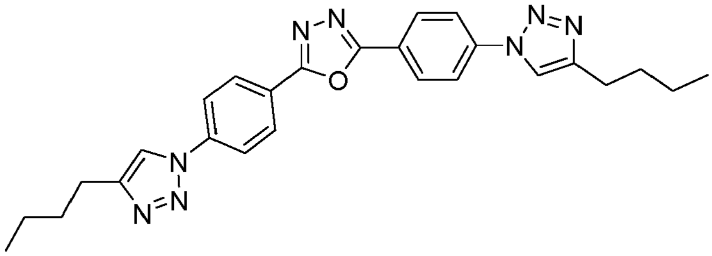

**30** ,

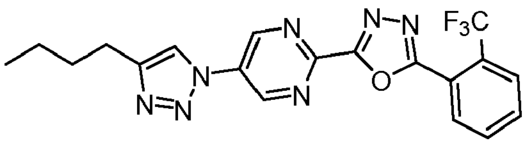

**31**

and

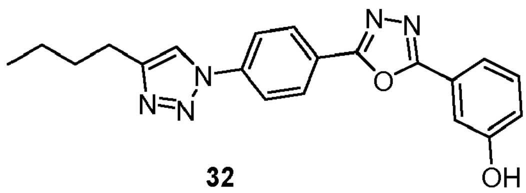

**32**

or salt, solvate, stereoisomer or tautomer thereof.

Preferably, said salt, solvate, stereoisomer or tautomer thereof is pharmaceutically acceptable.

The compound, salt, solvate, stereoisomer or tautomer of the invention is inhibitor of at least one DDX helicase, preferably being an inhibitor of at least two DDX helicases, preferably said DDX helicase is selected from the group consisting of DDX3X, DDX4, DDX5, DDX17, DDX21, DDX23, DDX18, DDX24, DDX48, DDX1, DDX2A, DDX2B..

The compound, salt, solvate, stereoisomer or tautomer as defined above is for use as a medicament, preferably said medicament is an anti-proliferative medicament; preferably for use in a method for the prevention and/or treatment of a hyperproliferative disorder; preferably said hyperproliferative disorder is dependent on a DDX helicase, preferably said hyperproliferative disorder is dependent on at least two DDX helicases, preferably said DDX helicase is selected from the group consisting of: DDX3X, DDX4, DDX5, DDX17, DDX21, DDX23, DDX18, DDX24, DDX48, DDX1, DDX2A and DDX2B.

In a preferred embodiment of the invention the hyperproliferative disorder is selected from the group consisting of: cancer, neurofibromatosis, hemangiomas, hyperproliferative arterial stenosis, a myelodysplastic disorder, arthritis, inflammatory arthritis, familial intestinal polyposes, histiocytosis, hyperkeratosis, including keloids, hypertrophic scars, oral leukoplakias and actinic keratosis, and papulosquamous eruptions including, pityriasis rosea, lichen planus and psoriasis; preferably said cancer is primary or metastatic; preferably said cancer is selected from the group consisting of: hepatocellular carcinoma, lung cancer, melanoma, pancreatic cancer, prostate cancer, cutaneous T lymphocyte lymphoma, breast cancer, colon cancer, colorectal cancer, lymphoma, liver cancer, ovarian cancer, thyroid cancer, esophagus cancer, stomach cancer, gastrointestinal cancer, brain or central and peripheral nervous system cancer, testis cancer, bone cancer, retinoblastoma, cervical carcinoma, kidney cancer, myeloma, leukemia, endocrine cancer, sarcoma, skin cancer, sinus cancer, urethral cancer, bladder cancer, genitourinary cancer, oral cancer, head and neck squamous carcinoma, pontine tumors, hepatoblastoma, gallbladder cancer; preferably said familial intestinal polyposes is Gardner syndrome; preferably said lung cancer is lung adenocarcinoma; preferably said pancreatic cancer is epithelioid pancreas carcinoma; preferably said breast cancer is triple negative breast cancer; preferably said brain or central and peripheral nervous system cancer is selected from the group consisting of: glioblastoma, glioma, medulloblastoma and neuroblastoma; preferably said cervical carcinoma is cervical carcinoma-in-situ; preferably said leukemia is acute lymphocytic leukemia, acute myelogenous leukemia, chronic lymphocytic leukemia and chronic myelogenous leukemia; preferably said sarcoma is selected from the group consisting of: angiosarcomas, osteosarcomas, fibrosarcomas, muscle rhabdomyosarcoma and ewing sarcoma; preferably said cancer is resistant to one or more of: gefitinib, erlotinib, olaparib, an anthracycline, temozolomide, Paclitaxel, Trastuzumab, Epirubicin, Doxorubicin, Adriamycin, Vincristine, cisplatin, 5-fluorouracil, oxaliplatin, mitomycin C, Bortezomib, Thalidomide, Dexamethasone, Tamoxifen, Etoposide, Lapatinib, Melphalan, Adriamycin, an EGFR inhibitor, apro-apoptotic agent, a monoclonal antibody, an interleukin or interferon; preferably said cancer has a mutation in one or more of the following genes: CDKN2A, PTEN, CDKN2C, N-RAS, TERT, TP53, K-RAS, HER2neu, LKB1, BRAF, p53, ARID1A, RB1, BRCA1, BRCA2, Wnt7b, MDR1, ABCG2, PDCD4, P-gp, MRP1, ABCB1, BAFF, CCND1, GRB2, ERK2, RSK1, RSK2, AKT, MET, ACVR2A, EP300, PI3KCA, TGFBR2, KDR, APC, ALK, EGFR, ROS1.

Preferably, said glioblastoma is glioblastoma multiforme. Preferably, the cancer is benign or malignant.

Preferably the compound, salt, solvate, stereoisomer or tautomer of the invention is combined with a further therapeutic intervention, preferably said further therapeutic intervention is: surgery, radiotherapy or chemotherapy.

It is a further object of the invention a pharmaceutical composition comprising:

- the compound, salt, solvate, stereoisomer or tautomer as defined in any one of claims 1 to 8,
- a pharmaceutically acceptable vehicle, and
- optionally a further therapeutic agent,

preferably said further therapeutic agent is an anti-proliferative agent, anti-pain agent, anti-emetic agent (such as Aprepitant, Fosaprepitant, Dolasetron, Granisetron, Ondansetron, Palonosetron, Tropisetron, Ramosetron or Dexamethasone), preferably said further therapeutic agent is selected from the group consisting of: Gefitinib, Erlotinib, Olaparib, an Anthracycline, Temozolomide, Paclitaxel, Trastuzumab, Epirubicin, Doxorubicin, Adriamycin, Vincristine, Cisplatin, 5-fluorouracil, Oxaliplatin, Mitomycin C, Bortezomib, Thalidomide, Dexamethasone, Tamoxifen, Etoposide, Lapatinib,

Melphalan, Adriamycin and EGFR inhibitors.

Preferably the pharmaceutical composition defined above is for the use in the prevention and/or treatment of a hyperproliferative disorder; preferably said hyperproliferative disorder is dependent on a DDX helicase, preferably said hyperproliferative disorder is dependent on at least two DDX helicases, preferably said DDX helicase is selected from the group consisting of: DDX3X, DDX4, DDX5, DDX17, DDX21, DDX23, DDX18, DDX24, DDX48, DDX1, DDX2A and DDX2B; still preferably the hyperproliferative disorder is selected from the group consisting of: cancer, neurofibromatosis, hemangiomas, hyperproliferative arterial stenosis, a myelodysplastic disorder, arthritis, inflammatory arthritis, familial intestinal polyposes, histiocytosis, hyperkeratosis, including keloids, hypertrophic scars, oral leukoplakias and actinic keratosis, and papulosquamous eruptions including, pityriasis rosea, lichen planus and psoriasis; preferably said cancer is primary or metastatic; preferably said cancer is selected from the group consisting of: hepatocellular carcinoma, lung cancer, melanoma, pancreatic cancer, prostate cancer, cutaneous T lymphocyte lymphoma, breast cancer, colon cancer, colorectal cancer, lymphoma, liver cancer, ovarian cancer, thyroid cancer, esophagus cancer, stomach cancer, gastrointestinal cancer, brain or central and peripheral nervous system cancer, testis cancer, bone cancer, retinoblastoma, cervical carcinoma, kidney cancer, myeloma, leukemia, endocrine cancer, sarcoma, skin cancer, sinus cancer, urethral cancer, bladder cancer, genitourinary cancer, oral cancer, head and neck squamous carcinoma, pontine tumors, hepatoblastoma, gallbladder cancer; preferably said familial intestinal polyposes is Gardner syndrome; preferably said lung cancer is lung adenocarcinoma; preferably said pancreatic cancer is epithelioid pancreas carcinoma; preferably said breast cancer is triple negative breast cancer; preferably said brain or central and peripheral nervous system cancer is selected from the group consisting of: glioblastoma, glioma, medulloblastoma and neuroblastoma; preferably said cervical carcinoma is cervical carcinoma-in-situ; preferably said leukemia is acute lymphocytic leukemia, acute myelogenous leukemia, chronic lymphocytic leukemia and chronic myelogenous leukemia; preferably said sarcoma is selected from the group consisting of: angiosarcomas, osteosarcomas, fibrosarcomas, muscle rhabdomyosarcoma and ewing sarcoma; preferably said cancer is resistant to one or more of: gefitinib, erlotinib, olaparib, an anthracycline, temozolomide, Paclitaxel, Trastuzumab, Epirubicin, Doxorubicin, Adriamycin, Vincristine, cisplatin, 5-fluorouracil, oxaliplatin, mitomycin C, Bortezomib, Thalidomide, Dexamethasone, Tamoxifen, Etoposide, Lapatinib, Melphalan, Adriamycin, an EGFR inhibitor, a pro-apoptotic agent, a monoclonal antibody, an interleukin or interferon; preferably said cancer has a mutation in one or more of the following genes: CDKN2A, PTEN, CDKN2C, N-RAS, TERT, TP53, K-RAS, HER2neu, LKB1, BRAF, p53, ARID1A, RB1, BRCA1, BRCA2, Wnt7b, MDR1, ABCG2, PDCD4, P-gp, MRP1, ABCB1, BAFF, CCND1, GRB2, ERK2, RSK1, RSK2, AKT, MET, ACVR2A, EP300, PI3KCA, TGFBR2, KDR, APC, ALK, EGFR, ROS1.

It is a further object of the invention a process of making the compound, salt, solvate, stereoisomer or tautomer as defined in any one of claims 1-8, comprising the step of reacting a compound of formula (II):

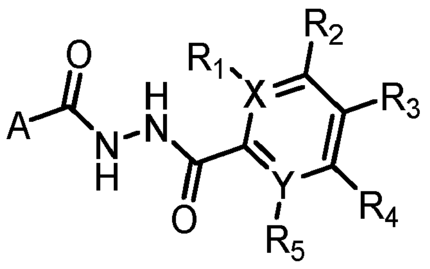

(II)

with Burgess Reagent or with $POCl_3$ thus obtaining cyclisation of the -C(O)-NH-NH-C(O)-moiety.

When any variable (e.g. $R_1$ and $R_2$, etc.) occurs more than one time in any constituent, its definition on each occurrence is independent at every other occurrence. Also, combinations of substituents and variables are permissible only if such combinations result in stable compounds. It is understood that substituents and substitution patterns on the compounds of the instant invention can be selected by one of ordinary skill in the art to provide compounds that are chemically stable and that can be readily synthesized by techniques known in the art, as well as those methods set forth below, from readily available starting materials. If a substituent is itself substituted with more than one group, it is understood that these multiple groups may be on the same carbon or on different carbons, so long as a stable structure results.

As used herein, "substituted or unsubstituted" referred to a given moiety preferably means that, in such moiety, at least one hydrogen atom is optionally replaced by any other atom or group of atoms. Any one, two or more chemically-feasible positions for substitution on the moiety may be substituted. When a moiety or group of moieties is substituted, the identity and positioning of the substituent is independent at each occurrence (i.e. they may be the same or different).

As used herein, "halogen" refers to any atom commonly referred to as halogen, such as fluorine, chlorine, bromine, iodine and astatine. Preferred examples of "halogen" include fluorine and chlorine.

As used herein, "Aryl" refers to an aromatic ring where all the atoms are carbon. Suitable examples of such an aryl

include but are not limited to phenyl, indenyl, indanyl, pentalenyl, cyclobutadienyl, cyclooctatetraenyl, cyclodecapentaenyl, naphthyl, anthracenyl, phenanthrenyl and pyrenyl.

As used herein, "Heteroaryl" refers to an aromatic ring containing at least one heteroatom. Suitable examples of such a heteroaryl include but are not limited to isoquinoline, quinoline, pyridine, pyrimidine, pyrazine, pyridazine, pyrrole, pyrazole, isoindole, indole, indolizine, quinazoline, cinnoline, pteridine, phalazine, 1,2,4-triazine, 1,3,5-triazine, quinoxaline, phenazine, indazole, furan, benzofuran, isobenzofuran, thiophene, benzothiophene, 4H-quinolizine, acridine, 1,8-naphtiridine, oxazole, isoxazole, benzoxazole, benzthiazole, isothiazole, thiazole, imidazole, 1,3,4-thiadiazole, tetrazole, 1,2,3-triazole, 1,2,3-oxadiazole, benzimidazole, 1,2,4-triazole, purine, phenothiazine and phenoxazine.

As used herein, "$C_1$-$C_6$ alkyl" and "$C_1$-$C_8$ alkyl" refer to a linear or branched chain of saturated carbon atoms, counting respectively by a number of carbon atoms between one and six or between one and eight. Suitable examples of said alkyl include but are not limited to methyl, ethyl, n-propyl, isopropyl, butyl, iso-butyl, sec-butyl, tert-butyl, pentyl, isopentyl, sec-pentyl, tert-pentyl, hexyl, isohexyl, sec-hexyl, tert-hexyl, heptyl, isoheptyl, sec-heptyl, tert-heptyl, octyl, isooctyl, sec-octyl, tert-octyl, etc.

As used herein, "$C_2$-$C_6$ alkenyl" refers to a linear or branched chain of carbon atoms containing one or more double bonds, and counting a number of carbon atoms between two and six. Suitable examples of $C_2$-$C_6$ alkenyl include but are not limited to ethenyl, propenyl, allyl, butenyl, isobuthenyl, pentenyl, penta-1,3-dienyl, isopentenyl, 3-methylbut-2-enyl, prenyl, hexenyl, hexa-2,4-dienyl, hexa-1,3-dienyl, hexa-1,4-dienyl, isohexenyl, 2-methylpent-1-ene, 4-methylpent-2-ene, 4-methylpent-1-ene, etc.

As used herein, "$C_2$-$C_6$ alkynyl" refers to a linear or branched chain of carbon atoms containing one or more triple bonds, and counting a number of carbon atoms between two and six. Suitable examples of $C_2$-$C_6$ alkynyl but are not limited to acetylenyl, ethynyl, propynyl, but-2-ynyl, but-1-ynyl, pent-3-ynyl, pent-2-ynyl, pent-1-ynyl, 4-metylpent-2-ynyl, 4-metylpent-lynyl, 3-metylpent-lynyl etc.

The term "haloalkyl" refers to a linear or branched alkyl group wherein at least one hydrogen atom on a carbon atom is replaced by a halogen atom. Suitable examples of haloalkyl include but are not limited to $CHFCH(CH_3)(CH_2CH_2CH_3)$, $CH_2CH_2CH_2F$, $C_4F_9$, $CF_3$, $CHF_2$, $CH_2F$, $CH_2CH_2CHF_2$, $CH_2Cl$, $CH_2CH_2Cl$, $CH_2$ $CH_2CH_2Cl$, $CH_2Br$, $CH_2$ $CH_2Br$, $CH_2$ $CH_2CH_2Br$, etc. The term "alkoxy" refers to an organic unit having the general formula -OR, wherein R is an aliphatic chain. Suitable examples of alkoxy groups include, but are not limited to methoxy, ethoxy, propoxy, isopropoxy, butoxy, isobutoxy, sec-butoxy, tert-butoxy, pentoxy, isopentoxy, sec-pentoxy, tert-pentoxy, hexoxy, isohexoxy, sec-hexoxy, tert-hexoxy, heptoxy, isoheptoxy, sec-heptoxy, tert-heptoxy, octoxy, isooctoxy, sec-octoxy, tert-octoxy, etc.

As used herein, the terms N-piperidinyl, N-pyrrolidinyl or N-morpholinyl indicate that each of said cyclic amine is linked through its nitrogen atom.

As used herein, "hyperproliferative disorder" refers to a condition which involves unregulated or uncontrolled cellular proliferation, including neoplastic and non-neoplastic hyperproliferative disorders. The neoplastic disorders include but are not limited to malignant disorders such as cancers or tumors. The non-neoplastic hyperproliferative disorders include but are not limited to hyperproliferative arterial stenosis, a myelodysplastic disorder; arthritis; inflammatory arthritis; familial intestinal polyposes such as Gardner syndrome; histiocytosis; hyperkeratosis, including keloids, hypertrophic scars, oral leukoplakias and actinic keratosis; papulosquamous eruptions including, pityriasis rosea, lichen planus and psoriasis; cervical carcinoma-in-situ.

The term "cancer" or "tumor" includes, but is not limited to, solid tumors and bloodborne tumors. The cancer may be primary or metastatic. The term "tumor" may refer to any mass of tissue that results from excessive cell growth or proliferation, either benign (noncancerous) or malignant (cancerous).

As used herein, "hyperproliferative disorder dependent on" refers to any type of cancer or hyperproliferative disorder which is characterized by overexpression of one or more DDX helicases, or is proven a specific cause-effect relationship between the helicase function and/or levels of expression and the hyperproliferative state of the cells.

As used herein, "anti-proliferative agent" or "anti-proliferative medicament" refers to an agent that slows down, prevents, or inhibits the growth of neoplastic tissue (e.g. cancer) and other non-neoplastic hyperproliferative disorders. The agent can be used to manipulate cellular metabolism and/or cellular control RNA and/or induce cytotoxicity and/or to inhibit growth by apoptosis and/or necrosis in the target cells, which are generally hyperproliferative cells (including neoplastic tissue and cancers, along with non-neoplastic or pre-malignant and non-malignant hyperproliferative disorders).

The activity and expression of DDX helicases can be measured by techniques known in the art for instance by measuring enzymatic activity of the protein. Any of those techniques may be used within the present invention. Methods to measure enzymatic inhibition of DDX helicases can be found for example in the papers of Jankowsky et al. (Jankowsky, Duplex unwinding with DDX proteins. Methods in molecular biology, 2010, 587, 245) and Bizebard et al. (Bizebard, A FRET-based, continuous assay for the helicase activity of DDX proteins. Methods in molecular biology, 2015, 1259, 199).

It is a further object of the invention a method for inhibiting a DDX helicases as defined above comprising contacting the compound, salt, solvate, stereoisomer, tautomer or the composition as defined above with said DDX helicase, thereby inhibiting the activity of DDX3X, DDX5 or other selected DDX helicases.

Preferably, the aminoacid sequences of the cited DDX helicases are as defined in the UniProtKB database (https://www.uniprot.org/uniprot/) using the following entry number and version: DDX3X (000571 version 3), DDX4 (Q9NQI0 version 2), DDX5 (P17844 version 1), DDX17 (Q92841 version 2), DDX21 (Q9NR30 version 5), DDX23 (Q9BUQ8 version 3).

In the present invention, any reference to a protein includes the respective gene, mRNA, cDNA and the protein by them codified, including fragments (in particular those including the binding site), derivatives, variants, isoforms, etc. thereof.

Salts of the compounds of the present invention are also encompassed within the scope of the invention. Because of their potential use in medicine, the salts of the compounds of formula (I) are preferably pharmaceutically acceptable. The pharmaceutically acceptable salts of the instant compounds can be synthesized from the compounds of this invention which contain a basic or acidic moiety by conventional chemical methods. Generally, the salts of the basic compounds are prepared either by ion exchange chromatography or by reaction of the free base with stoichiometric amounts or with an excess of the desired salt-forming inorganic or organic acid in a suitable solvent or various combinations of solvents. Similarly, the salts of the acidic compounds are formed by reactions with the appropriate inorganic or organic base. In a preferred embodiment, the compounds of the invention have at least one acidic proton and the corresponding sodium or potassium salt can be formed, for example, by reaction with the appropriate base.

Suitable pharmaceutically acceptable salts comprise conventional non-toxic salts obtained by salification with inorganic acids (e.g. hydrochloric, hydrobromic, sulphuric, or phosphoric acids), or with organic acids (e.g. acetic, propionic, succinic, benzoic, sulfanilic, 2-acetoxy-benzoic, cinnamic, mandelic, salicylic, glycolic, lactic, oxalic, malic, maleic, malonic, fumaric, tartaric, citric, p-toluenesulfonic, methanesulfonic, ethanesulfonic or naphthalensulfonic acids).

In addition, pharmaceutically acceptable base addition salts can be formed with a suitable inorganic or organic base such as triethylamine, ethanolamine, triethanolamine, dicyclohexylamine, ammonium hydroxide, pyridine, etc. The term "inorganic base," as used herein, has its ordinary meaning as understood by one of ordinary skill in the art and broadly refers to an inorganic compound that can act as a proton acceptor. The term "organic base," as used herein, also has its ordinary meaning as understood by one of ordinary skill in the art and broadly refers to an organic compound that can act as a proton acceptor.

Other suitable pharmaceutically acceptable salts include pharmaceutically acceptable alkalimetal or alkaline-earth-metal salts such as sodium, potassium, calcium or magnesium salts; in particular pharmaceutically acceptable salts of one or more carboxylic acid moieties that may be present in the compound of formula (I).

Other salts, which are not pharmaceutically acceptable may be useful in the preparation of compounds of this invention (i.e. the trifluoroacetate salt), and these form a further aspect of the invention. The invention includes within its scope all possible stoichiometric and nonstoichiometric forms of the salts of the compounds of formula (I).

The free form of the specific salt compounds described may be isolated using techniques known in the art. For example, the free form may be regenerated by treating the salt with a suitable dilute aqueous base solution such as dilute aqueous NaOH, potassium carbonate, ammonia and sodium bicarbonate. The free forms may differ from their respective salt forms somewhat in certain physical properties, such as solubility in polar solvents, but the acid and base salts are otherwise pharmaceutically equivalent to their respective free forms for purposes of the invention.

The preparation of the pharmaceutically acceptable salts described above and other typical pharmaceutically acceptable salts is more fully described by Berg et al., "Pharmaceutical Salts," J. Pharm. Sci., 1977, 66, 1.

In addition, the compounds of formula (I) and salts thereof may exist in unsolvated as well as in solvated forms with pharmaceutically acceptable solvents such as water, EtOH and the like. Such solvates are also included within the scope of the present invention.

Certain compounds of formula (I) may exist in stereoisomer forms (e.g. they may contain one or more asymmetric carbon atoms, chiral axes and/or chiral planes). The individual stereoisomers (enantiomers and diastereomers) as well as all mixtures of these are included within the scope of the present invention. Racemic mixtures may be separated to give their individual enantiomer using preparative HPLC using a column with chiral stationary phase or resolved to yield individual enantiomers utilizing methods known to those skilled in the art. Additionally or alternatively, enantiomers may be separated from each other by the selective crystallization of their diastereomeric salts. In addition, chiral intermediate compounds may be resolved and used to prepare individual enantiomers.

The compounds of the invention may exist in one or more polymorphic forms. The invention extends to all such forms whether in a pure polymorphic form or when admixed with any other material, such as another polymorphic form.

The compounds of formula (I) may also exist in zwitterionic form, all of which are included within the scope of the present invention.

Likewise, it is understood that compounds of formula (I) may exist in tautomeric forms other than the one depicted and all of these are also included within the scope of the present invention. It will be appreciated by those skilled in the art that certain protected derivatives of the compounds of the invention, which may be made prior to a final deprotection stage, may not possess pharmacological activity as such, but may, in certain instances, be administered to a subject and thereafter metabolized in the body to form compounds defined in the first aspect which are pharmacologically active, optionally with improved delivery properties over the parent molecule. The transformation in vivo may be, for example, as the result of some metabolic process, such as chemical or enzymatic hydrolysis of a carboxylic, phosphoric or sulphate

ester, or reduction or oxidation of a susceptible functionality. Such derivatives may therefore be described as "prodrugs". All protected derivatives and prodrugs of compounds defined above are included within the scope of the invention. Examples of suitable prodrugs for the compounds of the present invention are described in Drugs of Today, Volume 19, Number 9, 1983, pp. 499 and in Topics in Chemistry, Chapter 31, pp. 306 and in "Design of Prodrugs" by H. Bundgaard, Elsevier, 1985, Chapter 1 (the disclosure in which document is incorporated herein by reference). It will be further appreciated by those skilled in the art that certain moieties, known to those skilled in the art as "pro-moieties", for described by H. Bundgaard, in "Design of Prodrugs" (the disclosure in which document is incorporated herein by reference) may be placed on appropriate functionalities when such functionalities are present within the compound defined in the first aspect.

The invention also includes all suitable isotopic variations of a compound of the invention. An isotopic variation is defined as one in which at least one atom is replaced by an atom having the same atomic number but an atomic mass different from the atomic mass usually found in nature. Examples of isotopes that can be incorporated into compounds of the invention include isotopes such as $^{2}H$, $^{3}H$, $^{13}C$, $^{14}C$, $^{15}N$, $^{17}O$, $^{18}O$, $^{31}P$, $^{32}P$, $^{35}S$, $^{18}F$ and $^{36}Cl$, respectively. Certain isotopic variations of the invention, for example, those in which a radioactive isotope such as $^{3}H$ or $^{14}C$ is incorporated, are useful in drug and/or substrate tissue distribution studies. Further, substitution with isotopes such as deuterium $^{2}H$, may afford certain therapeutic advantages resulting from greater metabolic stability. Isotopic variations of the compounds of the invention can generally be prepared by conventional procedures such as by the illustrative methods or by the preparations described in the examples hereafter using appropriate isotopic variations of suitable reagents. In the present invention, the compounds may be administered as pure or as pharmaceutical formulations/compositions. Then the invention also provides pharmaceutical compositions comprising at least one compound of this invention or a pharmaceutical acceptable salt, solvate, tautomer or stereoisomer thereof and one or more pharmaceutically acceptable vehicle, carrier, excipient and/or diluent. The pharmaceutical composition may comprise two or more compounds of the invention or pharmaceutical acceptable salt, solvate, tautomer or stereoisomer thereof and may be administered in combination with approved drugs for the treatment of cancers and/or with pharmaceutical compositions comprising such drugs as part of combinatorial multidrug cancer therapy.

The pharmaceutical formulations may be suitable for parenteral, oral, or rectal administrations. Each of said formulations may contain excipients and/or diluents and/or fillers and/or additives and/or binders, coatings and/or suspending agents and/or emulsifying agents, preserving and/or control release agents suitable for the selected pharmaceutical form.

The pharmaceutical compositions can be chosen based on the treatment requirements. Such compositions are prepared by blending and are suitably adapted for oral or parenteral administration, and as such can be administered in the form of tablets, capsules, oral preparations, powders, granules, pills, injectable, or infusible liquid solutions, suspensions, suppositories, preparation for inhalation. Tablets and capsules for oral administration are normally presented in unit dose form and contain conventional excipients such as binders, fillers (including cellulose, mannitol, lactose), diluents, tableting agents, lubricants (including magnesium stearate), detergents, disintegrants (e.g. polyvinylpyrrolidone and starch derivatives such as sodium glycolate starch), coloring agents, flavoring agents, and wetting agents (for example sodium lauryl sulfate).

The oral solid compositions can be prepared by conventional methods of blending, filling or tableting. The blending operation can be repeated to distribute the active principle throughout compositions containing large quantities of fillers. Such operations are conventional. Oral liquid preparations can be in the form of, for example, aqueous or oily suspensions, solutions, emulsions, syrups or elixirs, or can be presented as a dry product for reconstitution with water or with a suitable vehicle before use. Such liquid preparations can contain conventional additives such as suspending agents, for example sorbitol, syrup, methyl cellulose, gelatin, hydroxyethyl cellulose, carboxymethyl cellulose, aluminium stearate gel, or hydrogenated edible fats; emulsifying agents, such as lecithin, sorbitan monooleate, or acacia; non-aqueous vehicles (which can include edible oils), such as almond oil, fractionated coconut oil, oily esters such as esters of glycerine, propylene glycol, or ethyl alcohol; preservatives, such as methyl or propyl p-hydroxybenzoate or sorbic acid, and if desired, conventional flavoring or coloring agents. Oral formulations also include conventional slow-release formulations such as enterically coated tablets or granules.

Pharmaceutical preparation for administration by inhalation can be delivered from an insufflator or a nebulizer pressurized pack.

For parenteral administration fluid unit dosages can be prepared, containing the compound and a sterile vehicle. The compound can be either suspended or dissolved, depending on the vehicle and concentration. The parenteral solutions are normally prepared by dissolving the compound in a vehicle, sterilising by filtration, filling suitable vials and sealing. Advantageously, adjuvants such as local anaesthetics, preservatives and buffering agents can also be dissolved in the vehicle. To increase the stability, the composition can be frozen after having filled the vials and removed the water under vacuum. Parenteral suspensions are prepared in substantially the same manner, except that the compound can be suspended in the vehicle instead of being dissolved, and sterilized by exposure to ethylene oxide before suspending in the sterile vehicle. Advantageously, a surfactant or wetting agent can be included in the composition to facilitate uniform distribution of the compound of the invention.

For buccal or sublingual administration the compositions may be tablets, lozenges, pastilles, or gel.
The compounds can be pharmaceutically formulated as suppositories or retention enemas, e.g. containing conventional suppositories bases such as cocoa butter, polyethylene glycol, or other glycerides, for a rectal administration.
Another means of administering the compounds of the invention regards topical treatment. Topical formulations can contain for example ointments, creams, lotions, gels, solutions, pastes and/or can contain liposomes, micelles and/or microspheres. Examples of ointments include oleaginous ointments such as vegetable oils, animal fats, semisolid hydrocarbons, emulsifiable ointments such as hydroxystearin sulfate, anhydrous lanolin, hydrophilic petrolatum, cetyl alcohol, glycerol monostearate, stearic acid, water soluble ointments containing polyethylene glycols of various molecular weights. Creams, as known to formulation experts, are viscous liquids or semisolid emulsions, and contain an oil phase, an emulsifier and an aqueous phase. The oil phase generally contains petrolatum and an alcohol such as cetyl or stearic alcohol. Formulations suitable for topical administration to the eye also include eye drops, wherein the active ingredient is dissolved or suspended in a suitable carrier, especially an aqueous solvent for the active ingredient.
A further method of administering the compounds of the invention regards transdermal delivery. Topical transdermal formulations comprise conventional aqueous and non-aqueous vectors, such as creams, oils, lotions or pastes or can be in the form of membranes or medicated patches.
To allow access of the active ingredients of the composition to deeper-lying skin cells, vehicles which improve penetration through outer layers of the skin, e. g., the stratum corneum, are useful. Vehicle constituents for this purpose include, but are not limited to, ethanol, isopropanol, diethylene glycol ethers such as diethylene glycol monoethyl ether, azone (1-dodecylazacycloheptan-2-one), oleic acid, linoleic acid, propylene glycol, hypertonic concentrations of glycerol, lactic acid, glycolic acid, citric acid, and malic acid. In one embodiment, propylene glycol is used as a delivery vehicle. In a preferred embodiment, a mixture of propylene glycol:ethanol:isopropyl myristate (1:2.7:1) containing 3% benzensulfonic acid and 5% oleyl alcohol is used.
In another embodiment, a liposome preparation can be used. The liposome preparation can comprise liposomes which penetrate the cells of interest or the stratum corneum, and fuse with the cell membrane, resulting in delivery of the contents of the liposome into the cell. For example, liposomes such as those described in U. S. Patent No. 5,077, 211 of Yarosh, U. S. Patent No. 4,621, 023 of Redziniak et al. or U. S. Patent No. 4,508, 703 of Redziniak et al. can be used.
The compositions of the invention intended to target skin conditions can be administered before, during, or after exposure of the skin of the mammal to UV or agents causing oxidative damage. Other suitable formulations can employ niosomes. Niosomes are lipid vesicles similar to liposomes, with membranes consisting largely of non-ionic lipids, some forms of which are effective for transporting compounds across the stratum corneum.
In order to increase bioavailability the compounds can be pharmaceutically formulated in nanoparticles. Acceptable nanoparticles include albumin nanoparticles and gold nanoparticles.
Other suitable delivery methods intended primarily for skin include use of a hydrogel formulation, comprising an aqueous or aqueous-alcoholic medium and a gelling agent in addition to the oligonucleotide (s). Suitable gelling agents include methylcellulose, carboxymethyl cellulose, hydroxypropylmethyl cellulose, carbomer (carbopol), hypan, polyacrylate, and glycerol polyacrylate. A reference for the formulations is the book by Remington (The Science and Practice of Pharmacy, Academic Press, ISBN: 9780128200070).
The compounds of the present invention may be employed for use in the treatment and/or prevention of the above mentioned conditions alone as a sole therapy or in combination with other therapeutic agents either by separate administrations, or by including the two or more active principles in the same pharmaceutical formulation. The compounds may be administered simultaneously or sequentially.
Still further aspects include combining the compounds or compositions described herein with other anticancer therapies for synergistic or additive benefit.
The other therapeutic agents may be any approved drugs for the treatment of hyperproliferative disorder, in particular cancer. Non-exhaustive examples of suitable additional agents include in particular drugs belonging to the group of: a pro-apoptotic agent, a monoclonal antibody, interleukins, interferons, an anthracycline and an EGFR inhibitor. In particular the compounds of the invention may be used together with Abitrexate (Methotrexate Injection), Abraxane (Paclitaxel Injection), Adcetris (Brentuximab Vedotin Injection), Adriamycin (Doxorubicin), Adrucil Injection (5-Fluorouracil), Afinitor (Everolimus), Afinitor Disperz (Everolimus), Alimta (PEMETREXED), Alkeran Injection (Melphalan Injection), Alkeran Tablets (Melphalan), Aredia (Pamidronate), Arimidex (Anastrozole), Aromasin (Exemestane), Arranon (Nelarabine), Arzerra (Ofatumumab Injection), Avastin (Bevacizumab), Bexxar (Tositumomab), BiCNU (Carmustine), Blenoxane (Bleomycin), Bosulif (Bosutinib), Busulfex Injection (Busulfan Injection), Campath (Alemtuzumab), Camptosar (Irinotecan), Caprelsa (Vandetanib), Casodex (Bicalutamide), CeeNU (Lomustine), CeeNU Dose Pack (Lomustine), Cerubidine (Daunorubicin), Clolar (Clofarabine Injection), Cometriq (Cabozantinib), Cosmegen (Dactinomycin), CytosarU (Cytarabine), Cytoxan (Cytoxan), Cytoxan Injection (Cyclophosphamide Injection), Dacogen (Decitabine), DaunoXome (Daunorubicin Lipid Complex Injection), Decadron (Dexamethasone), DepoCyt (Cytarabine Lipid Complex Injection), Dexamethasone Intensol (Dexamethasone), Dexpak Taperpak (Dexamethasone), Docefrez (Docetaxel), Doxil (Doxorubicin Lipid Complex Injection), Droxia (Hydroxyurea), DTIC (Decarbazine), Eligard (Leuprolide), Ellence (Epirubicin), Eloxatin (Oxaliplatin), Elspar (Asparaginase), Emcyt (Estramustine), Erbitux (Cetuximab), Erivedge (Vismodegib), Erwinaze (Asparaginase Erwinia chrysanthemi), Ethyol (Amifostine), Etopophos (Etoposide Injection), Eulexin (Flutamide), Fareston (Toremifene), Faslodex (Fulvestrant), Femara (Letrozole), Firmagon (Degarelix Injection), Fludara (Fludarabine), Folex (Methotrexate Injection), Folotyn (Pralatrexate Injection), FUDR (Floxuridine), Gemzar (Gemcitabine), Gilotrif (Afatinib), Gleevec (Imatinib Mesylate), Gliadel Wafer (Carmustine wafer), Halaven (Eribulin Injection), Herceptin (Trastuzumab), Hexalen (Altretamine), Hycamtin (Topotecan), Hycamtin (Topotecan), Hydrea (Hydroxyurea), Iclusig (Ponatinib), Idamycin PFS (Idarubicin), Ifex (Ifosfamide), Inlyta (Axitinib), Intron A alfab (Interferon alfa-2a), Iressa (Gefitinib), Istodax (Romidepsin Injection), Ixempra (Ixabepilone Injection), Jakafi (Ruxolitinib), Jevtana (Cabazitaxel Injection), Kadcyla (Ado-trastuzumab Emtansine), Kyprolis (Carfilzomib), Leukeran (Chlorambucil), Leukine (Sargramostim), Leustatin (Cladribine), Lupron (Leuprolide), Lupron Depot (Leuprolide), Lupron DepotPED (Leuprolide), Lysodren (Mitotane), Marqibo Kit (Vincristine Lipid Complex Injection), Matulane (Procarbazine), Megace (Megestrol), Mekinist (Trametinib), Mesnex (Mesna), Mesnex (Mesna Injection), Metastron (Strontium-89 Chloride), Mexate (Methotrexate Injection), Mustargen (Mechlorethamine), Mutamycin (Mitomycin), Myleran (Busulfan), Mylotarg (Gemtuzumab Ozogamicin), Navelbine (Vinorelbine), Neosar Injection (Cyclophosphamide Injection), Neulasta (filgrastim), Neulasta (pegfilgrastim), Neupogen (filgrastim), Nexavar (Sorafenib), Nilandron (Nilutamide), Nipent (Pentostatin), Nolvadex (Tamoxifen), Novantrone (Mitoxantrone), Olaparib, Oncaspar (Pegaspargase), Oncovin (Vincristine), Ontak (Denileukin Diftitox), Onxol (Paclitaxel Injection), Panretin (Alitretinoin), Paraplatin (Carboplatin), Perjeta (Pertuzumab Injection), Platinol (Cisplatin), Platinol (Cisplatin Injection), PlatinolAQ (Cisplatin), PlatinolAQ (Cisplatin Injection), Pomalyst (Pomalidomide), Prednisone Intensol (Prednisone), Proleukin (Aldesleukin), Purinethol (Mercaptopurine), Reclast (Zoledronic acid), Revlimid (Lenalidomide), Rheumatrex (Methotrexate), Rituxan (Rituximab), RoferonA alfaa (Interferon alfa-2a), Rubex (Doxorubicin), Sandostatin (Octreotide), Sandostatin LAR Depot (Octreotide), Soltamox (Tamoxifen), Sprycel (Dasatinib), Sterapred (Prednisone), Sterapred DS (Prednisone), Stivarga (Regorafenib), Supprelin LA (Histrelin Implant), Sutent (Sunitinib), Sylatron (Peginterferon Alfa-2b Injection), Synribo (Omacetaxine Injection), Tabloid (Thioguanine), Taflinar (Dabrafenib), Tarceva (Erlotinib), Targretin Capsules (Bexarotene), Tasigna (Decarbazine), Taxol (Paclitaxel Injection), Taxotere (Docetaxel), Temodar (Temozolomide), Temodar (Temozolomide Injection), Tepadina (Thiotepa), Thalomid (Thalidomide), TheraCys (BCG), Thioplex (Thiotepa), TICE BCG (BCG), Toposar (Etoposide Injection), Torisel (Temsirolimus), Treanda (Bendamustine hydrochloride), Trelstar (Triptorelin Injection), Trexall (Methotrexate), Trisenox (Arsenic trioxide), Tykerb (Lapatinib), Valstar (Valrubicin Intravesical), Vantas (Histrelin Implant), Vectibix (Panitumumab), Velban (Vinblastine), Velcade (Bortezomib), Vepesid (Etoposide), Vepesid (Etoposide Injection), Vesanoid (Tretinoin), Vidaza (Azacitidine), Vincasar PFS (Vincristine), Vincrex (Vincristine), Votrient (Pazopanib), Vumon (Teniposide), Wellcovorin IV (Leucovorin Injection), Xalkori (Crizotinib), Xeloda (Capecitabine), Xtandi (Enzalutamide), Yervoy (Ipilimumab Injection), Zaltrap (Ziv-aflibercept Injection), Zanosar (Streptozocin), Zelboraf (Vemurafenib), Zevalin (Ibritumomab Tiuxetan), Zoladex (Goserelin), Zolinza (Vorinostat), Zometa (Zoledronic acid), Zortress (Everolimus), Zytiga (Abiraterone), Nimotuzumab and immune checkpoint inhibitors such as Nivolumab, Pembrolizumab/MK-3475, Pidilizumab and AMP-224 targeting PD-1; and BMS-935559, MEDI4736, MPDL3280A and MSB0010718C targeting PD-L1 and those targeting CTLA-4 such as Ipilimumab, Mitomycin C, Cisplatin, Etoposide, Vincristine, Doxorubicin, Isotretinoin and Cyclophosphamide.

Radiotherapy refers to the use of radiation, usually X-rays, to treat a condition. Radiotherapy may be from outside the body as external radiotherapy, using X-rays, cobalt irradiation, electrons, and more rarely other particles such as protons. It may also be from within the body as internal radiotherapy, which uses radioactive metals or liquids (isotopes) to treat cancer.

Compounds of general formula (I) may be administered to a patient in a total daily dose of, for example, from 0.1 to 100 mg/kg body weight daily. Dosage unit compositions may contain such amounts of submultiples thereof to make up the daily dose. The compound may also be administered weekly or any other day. The determination of optimum dosages for a particular patient is well known to one skilled in the art. As is common practice, the compositions are normally accompanied by written or printed instructions for use in the treatment in question.

Based on pharmacokinetic studies and efficacy in vivo, it is possible to estimate a dosage in humans by allometric relationship (USFDA. Guidance for Industry: Estimating the Maximum Safe Starting Dose in Adult Healthy Volunteer. Rockville, MD: US Food and Drug Administration; 2005). Considering the oral drug bioavailability and the drug levels obtained by IP and PO administration and the efficacy in mouse xenograft models of breast and lung cancer reported in this embodiment for compound 5, the pharmacologically active dose (PAD) of 22.5 mg·kg-1·day-1 can be determined for mouse models. The human equivalent dose (HED) can be estimated according with the FDA guidelines by means of the following formula:

$$HED = animal\ dose \times \left(\frac{animal\ weight}{human\ weight}\right)^{(1-0.67)}$$

Based on PAD in mouse models, the HED can be estimated as 1.8 mg/(Kg·day) considering a mouse average weight of 30 g and an adult human body weight of 60 Kg.

Compounds of the invention may be prepared in a variety of ways. These processes form further aspects of the invention.

The present invention will now be described with reference to the following non-limiting figures and examples. They are provided for illustration purposes only and should not be considered as limiting the scope of the invention but merely as representative thereof.

**Figure 1.** Multi-dimensional scaling (MDS) in two dimensions of the distance matrix, obtained by alignment of complete proteins sequences. The "MDS Col 1" and "MDS Col 2" axes are arbitrary values functional to the representation of the relative distances between the proteins in a two-dimensional space.

**Figure 2.** Multi-dimensional scaling (MDS) in two dimensions of the distance matrix, obtained by alignment of double stranded RNA binding site proteins sequences. The "MDS Col 1" and "MDS Col 2" axes are arbitrary values functional to the representation of the relative distances between the proteins in a two-dimensional space.

**Figure 3.** a) Global Model Quality Estimation (GMQE) index per residue for the DDX3X homology model. b) Comparison of normalized QMEAN score (star) of the DDX3X homology model with the distribution of normalized QMEAN score for a non-redundant set of PDB structures.

**Figure 4.** a) GMQE index per residue for the DDX4 homology model. b) Comparison of normalized QMEAN score (star) of the DDX4 homology model with the distribution of normalized QMEAN score for a non-redundant set of PDB structures.

**Figure 5.** a) GMQE index per residue for the DDX5 homology model. b) Comparison of normalized QMEAN score (star) of the DDX5 homology model with the distribution of normalized QMEAN score for a non-redundant set of PDB structures.

**Figure 6.** a) GMQE index per residue for the DDX17 homology model. b) Comparison of normalized QMEAN score (star) of the DDX17 homology model with the distribution of normalized QMEAN score for a non-redundant set of PDB structures.

**Figure 7.** a) GMQE index per residue for the DDX21 homology model. b) Comparison of normalized QMEAN score (star) of the DDX21 homology model with the distribution of normalized QMEAN score for a non-redundant set of PDB structures.

**Figure 8.** a) GMQE index per residue for the DDX23 homology model. b) Comparison of normalized QMEAN score (star) of the DDX23 homology model with the distribution of normalized QMEAN score for a non-redundant set of PDB structures.

**Figure 9.** RMSD variation of carbon alpha trace during the Molecular dynamics simulation of DDX3X (a), DDX4 (b), DDX5 (c), DDX21 (d) and DDX23 (e) homology models.

**Figure 10.** Sorted RMSD distance matrix between binding site residues of DDX helicases, and clustering of residue conformations: DDX3X (a), DDX4 (b), DDX5 (c), DDX21 (d) and DDX23 (e).

**Figure 11.** Best scoring docking conformation of compound 3 in DDX3X, DDX4, DDX5, DDX21 and DDX23.

**Figure 12.** Best scoring docking conformation of compound 5 in DDX3X, DDX4, DDX5, DDX21 and DDX23.

**Figure 13.** Enzymatic assay performed with immunoprecipitated DDX3X expressed in 293T cells. Measurement of dsRNA probe unwinding in presence of compound 5 at 50, 250 and 500 μM concentration.

**Figure 14.** Enzymatic assay performed with immunoprecipitated DDX3X expressed in 293T cells. Measurement of dsRNA probe unwinding in presence of compound 16 at 50, 250 and 500 μM concentration.

**Figure 15.** Kinetic solubility, turbidimetry absorbance plot. Absorbance at 620nm vs. Concentration in μM. Compound 5 vs. control compounds: Miconazole, Reserpine, Propanolol.

**Figure 16.** Inhibition of hERG channel activity at different concentrations of the compound 5 (triangles). Quinidine (circles) is used as reference compound.

**Figure 17.** Plasma concentration (ng/ml) during the 24 hours following single administration of compound **5,** via intravenous (dose 1 mg/kg, triangles), intraperitoneal (dose 45mg/Kg, squares) and oral administration (dose 45mg/Kg, X marks) to male Swiss Albino Mice. Mean value and standard deviation (S.D.) of each time point are reported. For the intravenous administration, values were below quantitation limits after 8 hours (last time point reported).

**Figure 18.** Representative images of tissue from animals (Athymic, immunodeficient mice, Nude Foxnlnu/nu, Envigo spa) untreated (a, c, e and g) and treated with Compound 5 (b, d, f and h) for 6 weeks (via I.P. 45mg/kg every 48 hours). Brain (a and b); liver (c and d); kidney (e and f); spleen (g and h). Scale bars are equal to 100 μm.

**Figure 19.** In vivo efficacy in mouse subcutaneous xenograft models (Athymic, immunodeficient mice, strain: Nude Foxn1 nu/nu, Envigo spa); two tumors for each mouse, five animals for each group. The vehicle employed was water, DMSO 10%, Tween 80 5%; 100 microliters administered via I.P. In all the experiments the treated group was administered with compound 5 at 45 mg/Kg dose (circle), and the control group with vehicle (square), every Monday,

Wednesday and Friday for 5 weeks; a) Triple negative breast cancer (cell line MDA-MB-231). Treatments started when the tumors had an average volume of 10 $mm^3$. b) Non-small cell lung cancer (cell line A549). Treatments started when the tumor had an average volume of 80 $mm^3$. Mean values and Standard Error of Mean (SEM) are reported for each time point; each time point corresponds to the day of tumor volume measurement after the first administration. c) Non-small cell lung cancer engineered with a luciferase reporter (cell line A549 Red-FLuc). Treatments started when the tumor had an average volume of 50 $mm^3$. Mean values and Standard Error of Mean (SEM) are reported for each time point; each time point corresponds to the day of tumor volume measurement after the first administration.

## EXAMPLES

The following table lists abbreviations used within the present document.

Table 1. Abbreviations used within the present document

| | |
|---|---|
| $\mu g$ | Microgram |
| $\mu M$ | Micromolar |
| $^{13}C$ | Carbon isotope 13 |
| $^{19}F$ | Fluorine isotope 19 |
| $^{1}H$ | Proton |
| 1A2 | Cytochrome P450 subtype 1A2 |
| 2C9 | Cytochrome P450 subtype 2C9 |
| 2B6 | Cytochrome P450 subtype 2B6 |
| 2D6 | Cytochrome P450 subtype 2D6 |
| 3A4 | Cytochrome P450 subtype 3A4 |
| ACN | Acetonitrile |
| AcOH | Acetic acid |
| ADP | Adenosine diphosphate |
| ATP | Adenosine triphosphate |
| atm | Pressure in atmospheres |
| AUC | Area under the concentration-time curve |
| AUCExtra | Area under the concentration-time curve extrapolated from time 0 to infinite in % of the total AUC |
| AUC0-last | Area under the concentration-time curve from time 0 to last measurement |
| $AUC_{0-inf}$ | Area under the concentration-time curve from time 0 to infinite |
| BOC or boc | Tert-butyloxycarbonyl |
| BW | Body weight |
| C0 | Plasma concentration at time 0 |
| Cmax | Maximum plasma concentration |
| $CDCl_3$ | Deuterated chloroform |
| CL | Clearance |
| CL int | Intrinsic Clearance |
| Cmpd. | Compound |
| $D_2O$ | Deuterated water |
| DCM | Dichloromethane |

(continued)

| | |
|---|---|
| DDX | DEAD Box RNA helicases |
| DEADCAT | Diethyl azodicarboxylate |
| DMF | Dimethylformamide |
| DMSO | Dimethyl sulfoxide |
| DMEM | Dulbecco's Modified Eagle's Medium |
| DMEM/F-12 | Gibco Dulbecco's Modified Eagle Medium: Nutrient Mixture F-12 |
| DMSO d-6 | Deuterated dimethyl sulfoxide |
| EDC.HCl | N-(3-Dimethylaminopropyl)-N'-ethylcarbodiimide hydrochloride |
| EMEM | Eagle's Minimum Essential Medium |
| $Et_2O$ | Diethyl ether |
| EtOAc or EA | Ethyl acetate |
| EtOH | Ethanol |
| ext | Temperature external |
| F | Bioavailability |
| fs | Femto second |
| g | Grams |
| h | Hours |
| HBA | Hydrogen bond acceptor interaction |
| HBD | Hydrogen bond donor interaction |
| HED | Human equivalent dose: A dose in humans anticipated to provide the same degree of effect as that observed in animals at a given dose |
| HLM | Human liver microsome |
| HPLC | High Performance Liquid Chromatography |
| Hz | Hertz |
| iPrOH | Isopropanol |
| I.P. | Intra peritoneal administration |
| I.T. | Intra tracheal administration |
| I.V. | Intra venous administration |
| KHz | Kilohertz |
| LC-MS | Liquid Chromatography Mass Spectrum |
| M | Molarity |
| MDS | Multi-dimensional scaling based on Sammons mapping |
| MeOD-d4 | Deuterated methanol |
| MeOH | Methanol |
| mg | Milligrams |
| MHz | Megahertz |
| min | Minutes |
| ml | Milliliters |
| MLM | Mouse liver microsome |

(continued)

| | |
|---|---|
| mmol | Millimoles |
| MRTO-last | Mean resident time from time 0 to last measure |
| MTS | 3-(4,5-Dimethylthiazol-2-yl)-5-(3-carboxymethoxyphenyl)-2-(4-sulfophenyl)-2H-tetrazolium, inner salt |
| MTT | 3-(4,5-dimethylthiazol-2-yl)-2,5-diphenyltetrazolium |
| nm | Nanometers |
| μL | Microliters |
| nL | Nanoliters |
| NMR | Nuclear Magnetic Resonance |
| ns | Nano second |
| o.n. | Overnight |
| PAD | Pharmacologically Active Dose: The lowest dose tested in an animal species with the intended pharmacologic activity |
| PBS | Phosphate-buffered saline |
| PE | Petroleum ether |
| P.O. | Per os |
| Pyr | Pyridine |
| RCF | Relative centrifugal force |
| Rem | Remaining |
| Rf | Retention factor |
| RLM | Rat liver microsome |
| rpm | Rotation per minute |
| RPMI 1640 | Roswell Park Memorial Institute (RPMI) 1640 Medium |
| Rsq | R squared |
| RT or rt or r.t. | Room temperature |
| QH | Hepatic blood flow |
| s | Seconds |
| TBME | Tert-Butyl-methyl ether |
| t-Bu | Tert-butyl |
| t-BuOH | Tert-butyl alcohol |
| t-BuONO | Tert-butyl nitrite |
| $T_{1/2}$ | Half-life (min) |
| TEA | Triethylamine |
| THF | Tetrahydrofuran |
| TIP3P | transferable intermolecular potential with 3 points |
| TLC | Thin-layer chromatography |
| $TMSN_3$ | Trimethylsilyl Azide |
| $T_{max}$ | Time of maximum concentration |
| Tm | Protein melting temperature |

(continued)

| | |
|---|---|
| tR | Retention time |
| UHPLC | Ultra High Performance Liquid Chromatography |
| v:v | Volume to volume ratio |
| Vd | Volume of distribution |
| VdW | Van der Waals contact interaction |
| WFI | Water for injection |
| wt | Wild type |

All temperatures are expressed in °C (degrees centigrade) or K (Kelvin). The yields were calculated assuming that products were 100% pure if not stated otherwise.

**EXAMPLE 1: IN SILICO DATA**

**Computer Aided Drug Design**

The small molecules of the invention are inhibitors with polypharmacology profile for a subfamily of human DDX helicases, acting by competitive inhibition of RNA unwinding. On the basis of RNA binding sites sequence similarity, this subfamily of DDX helicases includes at least DDX3X, DDX4, DDX5, DDX17, DDX21 and DDX23. The molecules those are able to bind to the RNA site of these helicases will be referred to as "MasterKey" inhibitors. The inhibitors show different selectivity profiles against DDX helicases depending on the different molecules decorations.

The inhibitors of this invention have been found using the computational and biological methods described below. In particular, the scaffold described in the current invention has been identified by computational ranking of a library of about 7 million compounds (Molport library year 2017), subsequent optimization of first identified commercially available compounds based on molecular docking calculations. The protein 3D homology models of the six DDX helicases used in docking calculations were optimized by molecular dynamics simulations.

**3D-Similarity analysis of human DDX helicases**

The subfamily of human DDX helicases has been selected on the basis of similarity between the aminoacid residues composing the RNA binding site with the computational methods reported as follows.

The objective of this novel approach is the interference in different cancer proliferation pathways through the inhibition of multiple human DDX helicases by means of a "MasterKey" inhibitor, leading to a synergistic inhibition effect, of a group of DDX helicases. A subset of human DDX helicases involved in cancer pathogenesis was selected with the intent to assess the possibility of optimizing molecules for the inhibition of multiple human DDX helicases enzymes selected from: DDX1, DDX2A, DDX2B, DDX3X, DDX4, DDX5, DDX6, DDX17, DDX18 DDX19B, DDX20, DDX21, DDX23, DDX24, DDX25, DDX39B, DDX48 and DDX51. Both the complete sequence as well as the sequence of the double stranded RNA binding site were superimposed and the distance between the sequences was calculated with CLUSTAL-Omega (Sievers F. et al., Fast, scalable generation of high-quality protein multiple sequence alignments using Clustal Omega. Mol. Syst. Biol. 2011 Oct 11,7, 539) generating two pairwise distance matrices, the first from the pairwise alignment of the sequences of the dsRNA binding site and the second for the complete sequences. The sequences of proteins have been retrieved from UniProtKB database (https://www.uniprot.org/uniprot/) in Fasta format with the following accession codes: DDX1 (Q92499 version 2), DDX2A (P60842 version 1), DDX2B (Q14240 version 2), DDX3X (O00571 version 3), DDX4 (Q9NQI0 version 2), DDX5 (P17844 version 1), DDX6 (P26196 version 2), DDX17 (Q92841 version 2), DDX18 (Q9NVP1 version 2), DDX19B (Q9UMR2 version 1), DDX20 (Q9UHI6 version 2), DDX21 (Q9NR30 version 5), DDX23 (Q9BUQ8 version 3), DDX24 (Q9GZR7 version 1), DDX25 (Q9UBL0 version 2), DDX39B (Q13838 version 1), DDX48 (P38919 version 4), DDX51 (Q8N8A6 version 3).

The Multi-Dimensional Scaling algorithm implemented in KNIME analytic platform (version 3.1; Michael R. Berthold, et al., KNIME: The {K}onstanz {I}nformation {M}iner, Studies in Classification, Data Analysis, and Knowledge Organization (GfKL 2007), Springer, ISBN 978-3-540-78239-1, year 2007) was subsequently applied on the distance matrices, representing the high dimensional space of the relation between the protein sequences onto a two-dimensional space representation. The Sammons mapping was applied to the distance matrix (Sammon JW, A nonlinear mapping for data structure analysis, IEEE Transactions on Computers, 1969, 18, 403); this algorithm iteratively decreases the difference of the distances of high and low dimensional data for 50 epochs achieving the best mapping of data point

distances in the two-dimensional space.

Despite the complete sequence comparison of helicases showing low similarity, indicating the involvement of the various DDX helicases in different physiological/carcinogenic pathways (Figure 1), comparing exclusively the residues that correspond to the double stranded RNA binding sites (Table 2), it is clear that some helicases with different functions can be clustered on the basis of their similarity in the interaction with RNA (Figure 2). Contemporary inhibition of multiple helicases can interfere in multiple pathways, leading to increase the in vitro and in vivo potency of the inhibitors and extend the efficacy spectrum toward multiple types of cancer.

Table 2. Sequence alignment of RNA Binding site residues for helicases analyzed

| Protein ID | Residue ID and sequence | | | | | | | | | | | | | | | | | | | | | | | | | | | | | | | | | | | | | | | | | | | | | | | | | | | | | | |
|---|---|---|---|---|---|---|---|---|---|---|---|---|---|---|---|---|---|---|---|---|---|---|---|---|---|---|---|---|---|---|---|---|---|---|---|---|---|---|---|---|---|---|---|---|---|---|---|---|---|---|---|---|---|---|---|
| DDX1 (Q92499 version 2) | 293 | 294 | 295 | 296 | 297 | 298 | 324 | 325 | 326 | 327 | 329 | 346 | 347 | 348 | 349 | 350 | 351 | 352 | 353 | 371 | 373 | 374 | 375 | 377 | 378 | | 379 | 380 | 381 | 383 | 409 | 513 | 514 | 515 | 516 | 546 | 547 | 554 | 572 | 573 | 574 | 575 | 576 | 577 | 578 | 579 | 592 | 593 | 594 | | | 595 | 597 | 598 | 601 | 602 |
| | E | P | S | R | E | L | I | G | G | V | A | T | P | G | R | L | D | D | L | E | D | G | L | S | Q | - | G | Y | S | F | T | C | R | T | K | H | G | R | T | D | V | A | A | R | G | I | L | P | D | - | - | E | Q | N | H | R |
| DDX2A (P60842 version 1) | 107 | 108 | 109 | 110 | 111 | 112 | 135 | 136 | 137 | 138 | 140 | 158 | 159 | 160 | 161 | 162 | 163 | 164 | 165 | 183 | 185 | 186 | 187 | 189 | 190 | | 191 | 192 | 193 | 195 | 215 | 279 | 280 | 281 | 282 | 303 | 304 | 311 | 329 | 330 | 331 | 332 | 333 | 334 | 335 | 336 | 349 | 350 | 351 | | | 352 | 354 | 355 | 358 | 359 |
| | A | P | T | R | E | L | I | G | G | T | V | T | P | G | R | V | F | D | M | E | D | E | M | S | R | - | G | F | K | Q | T | I | N | T | R | H | G | R | T | D | L | L | A | R | G | I | L | P | T | - | - | N | E | N | H | R |
| DDX2B (Q14240 version 2) | 108 | 109 | 110 | 111 | 112 | 113 | 136 | 137 | 138 | 139 | 141 | 159 | 160 | 161 | 162 | 163 | 164 | 165 | 166 | 184 | 186 | 187 | 188 | 190 | 191 | | 192 | 193 | 194 | 196 | 216 | 280 | 281 | 282 | 283 | 304 | 305 | 312 | 330 | 331 | 332 | 333 | 334 | 335 | 336 | 337 | 350 | 351 | 352 | | | 353 | 355 | 356 | 359 | 360 |
| | A | P | T | R | E | L | I | G | G | T | V | T | P | G | R | V | F | D | M | E | D | E | M | S | R | - | G | F | K | Q | T | L | N | T | R | H | G | R | T | D | L | L | A | R | G | I | L | P | T | - | - | N | E | N | H | R |
| DDX3X (O00571 version 3) | 273 | 274 | 275 | 276 | 277 | 278 | 301 | 302 | 303 | 304 | 306 | 323 | 324 | 325 | 326 | 327 | 328 | 329 | 330 | 348 | 350 | 351 | 352 | 354 | 355 | | 356 | 357 | 358 | 360 | 384 | 448 | 449 | 450 | 451 | 472 | 473 | 480 | 498 | 499 | 500 | 501 | 502 | 503 | 504 | 505 | 518 | 519 | 520 | | | 521 | 523 | 524 | 527 | 528 |
| | A | P | T | R | E | L | Y | G | G | A | I | T | P | G | R | L | V | D | M | E | D | R | M | D | M | - | G | F | E | Q | T | V | E | T | K | H | G | R | T | A | V | A | A | R | G | L | L | P | S | - | - | D | E | E | H | R |
| DDX4 (Q9NQI0 version 2) | 372 | 373 | 374 | 375 | 376 | 377 | 400 | 401 | 402 | 403 | 405 | 422 | 423 | 424 | 425 | 426 | 427 | 428 | 429 | 447 | 449 | 450 | 451 | 453 | 454 | | 455 | 456 | 457 | 459 | 483 | 547 | 548 | 549 | 550 | 571 | 572 | 579 | 597 | 598 | 599 | 600 | 601 | 602 | 603 | 604 | 617 | 618 | 619 | | | 620 | 622 | 623 | 626 | 627 |
| | A | P | T | R | E | L | Y | G | G | T | L | T | P | G | R | L | M | D | I | E | D | R | M | D | M | - | G | F | G | E | T | V | E | T | K | H | G | R | T | S | V | A | A | R | G | L | L | P | S | - | - | T | D | E | H | R |
| DDX5 (P17844 version 1) | 174 | 175 | 176 | 177 | 178 | 179 | 202 | 203 | 204 | 205 | 207 | 224 | 225 | 226 | 227 | 228 | 229 | 230 | 231 | 249 | 251 | 252 | 253 | 255 | 256 | | 257 | 258 | 259 | 261 | 281 | 348 | 349 | 350 | 351 | 372 | 373 | 380 | 398 | 399 | 400 | 401 | 402 | 403 | 404 | 405 | 418 | 419 | 420 | | | 421 | 423 | 424 | 427 | 428 |
| | A | P | T | R | E | L | Y | G | G | A | K | T | P | G | R | L | I | D | F | E | D | R | M | D | M | - | G | F | E | Q | T | V | E | T | K | H | G | R | T | D | V | A | S | R | G | L | Y | P | N | - | - | S | E | D | H | R |
| DDX6 (P26196 version 2) | 171 | 172 | 173 | 174 | 175 | 176 | 200 | 201 | 202 | 203 | 205 | 222 | 223 | 224 | 225 | 226 | 227 | 228 | 229 | 247 | 249 | 250 | 251 | 253 | 254 | | 255 | 256 | 257 | 259 | 279 | 341 | 342 | 343 | 344 | 365 | 366 | 373 | 391 | 392 | 393 | 394 | 395 | 396 | 397 | 398 | 411 | 412 | 413 | | | 414 | 416 | 417 | 420 | 421 |
| | V | P | T | R | E | L | T | G | G | T | L | T | P | G | R | I | L | D | L | E | D | K | L | S | Q | - | D | F | V | I | T | C | N | S | S | H | A | R | T | D | L | F | T | R | G | I | F | P | K | - | - | L | E | T | H | R |
| DDX17 (Q92841 version 2) | 251 | 252 | 253 | 254 | 255 | 256 | 279 | 280 | 281 | 282 | 284 | 301 | 302 | 303 | 304 | 305 | 306 | 307 | 308 | 326 | 328 | 329 | 330 | 332 | 333 | | 334 | 335 | 336 | 338 | 358 | 425 | 426 | 427 | 428 | 449 | 450 | 457 | 475 | 476 | 477 | 478 | 479 | 480 | 481 | 482 | 495 | 496 | 497 | | | 498 | 500 | 501 | 504 | 505 |
| | A | P | T | R | E | L | Y | G | G | A | K | T | P | G | R | L | I | D | F | E | D | R | M | D | M | - | G | F | E | Q | T | V | E | T | K | H | G | R | T | D | V | A | S | R | G | L | Y | P | N | - | - | S | E | D | H | R |
| DDX18 (Q9NVP1 version 2) | 258 | 259 | 260 | 261 | 262 | 263 | 286 | 287 | 288 | 289 | 291 | 308 | 309 | 310 | 311 | 312 | 313 | 314 | 315 | 334 | 336 | 337 | 338 | 340 | 341 | | 342 | 343 | 344 | 346 | 366 | 432 | 433 | 434 | 435 | 456 | 457 | 464 | 482 | 483 | 484 | 485 | 486 | 487 | 488 | 489 | 502 | 503 | 504 | | | 505 | 507 | 508 | 511 | 512 |
| | S | P | T | R | E | L | M | G | G | S | R | T | P | G | R | L | L | D | H | E | D | R | I | D | V | - | G | F | E | E | T | F | S | S | C | H | G | R | T | D | V | A | A | R | G | L | P | P | D | - | - | D | K | E | H | R |
| DDX19B (Q9UMR2 version 1) | 169 | 170 | 171 | 172 | 173 | 174 | 198 | 199 | 200 | | 202 | 217 | 218 | 219 | 220 | 221 | 222 | 223 | 224 | 243 | 245 | 246 | 247 | 249 | 250 | 251 | 252 | 253 | 254 | 256 | 276 | 340 | 341 | 342 | 343 | 364 | 365 | 372 | 390 | 391 | 392 | 393 | 394 | 395 | 396 | 397 | 410 | 411 | 412 | 413 | 414 | 419 | 421 | 422 | 425 | 426 |
| | S | P | T | Y | E | L | V | R | G | - | K | T | P | G | T | V | L | D | W | E | D | V | M | A | T | Q | G | H | Q | Q | T | C | H | T | R | S | G | R | T | N | V | C | A | R | G | I | L | P | V | D | K | D | E | T | H | R |
| DDX20 (Q9UHI6 version 2) | 137 | 138 | 139 | 140 | 141 | 142 | 166 | 167 | 168 | 169 | 171 | 187 | 188 | 189 | 190 | 191 | 192 | 193 | 194 | 212 | 214 | 215 | 216 | 218 | 219 | | 221 | 222 | 223 | 225 | 245 | 316 | 317 | 318 | 319 | 340 | 341 | 348 | 366 | 367 | 368 | 369 | 370 | 371 | 372 | 373 | 386 | 387 | 388 | | | 389 | 391 | 392 | 395 | 396 |
| | A | P | T | R | E | I | I | G | G | T | L | S | P | G | R | I | K | Q | L | E | D | K | L | E | E | - | S | F | Q | Q | T | S | N | L | H | S | G | R | T | D | L | T | S | R | G | I | V | P | L | - | - | D | E | T | H | R |
| DDX21 (Q9NR30 version 5) | 267 | 268 | 269 | 270 | 271 | 272 | 293 | 294 | 295 | 296 | 298 | 315 | 316 | 317 | 318 | 319 | 320 | 321 | 322 | 340 | 342 | 343 | 344 | 346 | 347 | | 348 | 349 | 350 | 352 | 377 | 445 | 446 | 447 | 448 | 468 | 469 | 476 | 494 | 495 | 496 | 497 | 498 | 499 | 500 | 501 | 514 | 515 | 516 | | | 517 | 519 | 520 | 523 | 524 |
| | A | P | T | R | E | L | Y | G | G | T | Y | T | P | G | R | I | K | D | H | E | D | Q | M | D | M | - | G | F | A | Q | T | C | E | T | K | H | G | R | T | N | V | A | A | R | G | L | P | P | K | - | - | D | E | S | H | R |
| DDX23 (Q9BUQ8 version 3) | 475 | 476 | 477 | 478 | 479 | 480 | 503 | 504 | 505 | 506 | 508 | 525 | 526 | 527 | 528 | 529 | 530 | 531 | 532 | 550 | 552 | 553 | 554 | 556 | 557 | | 558 | 559 | 560 | 562 | 608 | 671 | 672 | 673 | 674 | 695 | 696 | 703 | 721 | 722 | 723 | 724 | 725 | 726 | 727 | 728 | 741 | 742 | 743 | | | 744 | 746 | 747 | 750 | 751 |
| | A | P | T | R | E | L | I | G | G | I | R | T | F | G | R | L | I | D | V | E | D | R | M | D | M | - | G | F | E | D | T | V | N | Q | K | H | G | R | T | D | V | A | G | R | G | I | M | A | K | - | - | N | E | D | H | R |
| DDX24 (Q9GZR7 version 1) | 394 | 395 | 396 | 397 | 398 | 399 | 422 | 423 | 424 | 425 | 427 | 444 | 445 | 446 | 447 | 448 | 449 | 450 | 451 | 472 | 474 | 475 | 476 | 478 | 479 | | 480 | 481 | 482 | 484 | 509 | 534 | 535 | 536 | 597 | 618 | 619 | 626 | 644 | 645 | 646 | 647 | 648 | 649 | 650 | 651 | 664 | 665 | 666 | | | 667 | 669 | 670 | 673 | 674 |
| | T | P | T | R | E | L | V | G | G | M | T | T | P | G | R | L | W | E | L | E | D | R | M | E | K | - | G | H | F | E | T | A | N | S | I | H | A | R | T | D | V | A | A | R | G | L | V | P | R | - | - | T | E | I | H | R |
| DDX25 (Q9UHL0 version 2) | 174 | 175 | 176 | 177 | 178 | 179 | 203 | 204 | 205 | | 207 | 222 | 223 | 224 | 225 | 226 | 227 | 228 | 229 | 248 | 250 | 251 | 252 | 254 | 255 | 256 | 257 | 258 | 259 | 261 | 281 | 345 | 346 | 347 | 348 | 369 | 370 | 377 | 395 | 396 | 397 | 398 | 399 | 400 | 401 | 402 | 415 | 416 | 417 | 418 | 419 | 424 | 426 | 427 | 430 | 431 |
| | A | P | T | Y | E | L | I | R | G | - | R | T | P | G | T | V | L | D | W | E | D | V | M | D | T | Q | G | F | S | H | T | C | Q | T | R | S | G | R | T | N | V | C | A | R | G | I | L | P | V | K | Q | D | E | T | H | R |
| DDX39B (Q13838 version 1) | 120 | 121 | 122 | 123 | 124 | 125 | 149 | 150 | 151 | 152 | 154 | 172 | 173 | 174 | 175 | 176 | 177 | 178 | 179 | 197 | 199 | 200 | 201 | 203 | 204 | 205 | 206 | 207 | 208 | 210 | 230 | 294 | 295 | 296 | 297 | 318 | 319 | 326 | 344 | 345 | 346 | 347 | 348 | 349 | 350 | 351 | 364 | 365 | 366 | | | 367 | 369 | 370 | 373 | 374 |
| | C | H | T | R | E | L | F | G | G | L | I | T | P | G | R | I | L | A | L | E | D | K | M | E | Q | L | D | M | R | D | T | V | K | S | V | H | R | R | T | N | L | F | G | R | G | M | M | P | E | - | - | D | D | T | H | R |
| DDX48 (P38919 version 4) | 113 | 114 | 115 | 116 | 117 | 118 | 141 | 142 | 143 | 144 | 146 | 163 | 164 | 165 | 166 | 167 | 168 | 169 | 170 | 188 | 190 | 191 | 192 | 194 | 195 | | 196 | 197 | 198 | 200 | 220 | | 285 | 286 | 287 | 308 | 309 | 316 | 334 | 335 | 336 | 337 | 338 | 339 | 340 | 341 | 354 | 355 | 356 | | | 357 | 359 | 360 | 363 | 364 |
| | A | P | T | R | E | L | I | G | G | T | V | T | P | G | R | V | F | D | M | E | D | E | M | N | K | - | G | F | K | Q | T | - | N | T | K | H | G | R | T | D | V | W | A | R | G | L | L | P | N | - | - | N | E | L | H | R |
| DDX51 (Q8N8A6 version 3) | 288 | 289 | 290 | 291 | 292 | 293 | 316 | 317 | 318 | 319 | 321 | 346 | 347 | 348 | 349 | 350 | 351 | 352 | 353 | 372 | 374 | 375 | 376 | 378 | 379 | | 380 | 381 | 382 | 384 | 433 | 511 | 512 | 513 | 514 | 536 | 537 | 544 | 562 | 563 | 564 | 565 | 566 | 567 | 568 | 569 | 582 | 583 | 584 | | | 585 | 587 | 588 | 591 | 592 |
| | L | P | T | K | E | L | T | G | Q | K | L | T | P | G | R | L | V | D | H | E | D | R | M | D | S | - | M | H | Q | W | T | T | N | S | R | S | S | R | T | D | A | T | A | R | G | I | A | P | Q | - | - | Y | R | T | H | R |

DDX5, DDX17, DDX3X, DDX4, DDX23 and DDX21 form a well-defined cluster (Figure 2), and have been selected as protein targets because of their documented involvement in cancer pathogenesis.

The DDX helicases consist of two main domains that can change their reciprocal position upon the binding of an ATP/ADP co-factor and/or double stranded RNA molecule. The reciprocal positions of the two domains are usually indicated as "open" and "closed" conformations. The closed conformation is identified with the binding of double stranded RNA and ATP/ADP, while the open conformation refers to the binding of only ATP/ADP. Most of the available X-Ray structures of the human DDX helicases were obtained in the open conformation. For the present computational studies, the closed conformation that binds double stranded RNA is essential, and the models of the closed conformations of DDX5, DDX17, DDX3X, DDX4, DDX23 and DDX21 have been modeled by homology modeling as described below.

**Homology modeling of selected helicases**

For each of the six DDX helicases selected, a homology model of the closed conformation has been prepared as described below.

The SWISS-MODEL template library (SMTL version 2017-05-03, PDB release 2017-04-28) was searched with Blast (Altschul et al., 1997) and HHBlits (Remmert, et al., 2011) for evolutionary related structures matching the targets sequence. Template search with Blast and HHBlits has been performed against the SWISS-MODEL template library (SMTL, update: 2017-05-03, included PDB release: 2017-04-28). The target sequence was searched with BLAST (Altschul et al., 1997) against the primary amino acid sequence contained in the SMTL. An initial HHblits profile has been built using the procedure outlined in (Remmert, et al., 2011), followed by 1 iteration of HHblits against NR20. The obtained profile was subsequently searched against all profiles of the SMTL.

Models were built based on target-template alignment using ProMod3 (Biasini M et al., OpenStructure: an integrated software framework for computational structural biology. Acta Cryst, 2013). Coordinates which were conserved between the target and the template were copied from the template to the model. Insertions and deletions were remodeled using a peptide fragment library. Side chains were subsequently rebuilt. Finally, the geometry of the resulting model was regularized by using a force field. Where case loop modeling with ProMod3 failed, an alternative model was built with PROMOD-II (Guex, et al., 1997).

The global and per-residue model quality was assessed using the QMEAN scoring function (Benkert, et al., 2011), using weights of the individual QMEAN terms trained specifically for SWISS-MODEL. The models were assessed for quality by two indexes, the Global Model Quality Estimation (GMQE) and QMEAN scoring function. GMQE is a quality estimation which combines properties from the target-template alignment and the template search method. The resulting GMQE score is expressed as a number between 0 and 1, reflecting the expected accuracy of a model built with that alignment and template and the coverage of the target. Higher numbers indicate higher reliability.

The homology model for DDX3X was built with ProMod3 (version 1.0.2), the protein was modeled as monomer without ligands with a GMQE value of 0.51 (per residue local quality estimation, Figure 3a) and a QMEAN value of -1.80 (Figure 3b). The template structure selected was the ATP dependent RNA helicase vasa, the X-Ray structure with PDB id 2DB3 (chain B), resolution 2.20 Å, residue range 136-671, coverage of target sequence 63%, with a sequence identity of 51.32%, and sequence similarity 0.44, identified by BLAST. Sequence alignment of the DDX3X sequence (SEQ. ID No. 1) with the template sequence (SEQ. ID No. 2):

```
Target     MSHVAVENALGLDQQFAGLDLNSSDNQSGGSTASKGRYIPPHLRNREATKGFYDKDSSGWSSSKDKDAYSSFGSRSDSRG
2db3.1.B   --------------------------------------------------------------------------------
Target     KSSFFSDRGSGSRGRFDDRGRSDYDGIGSRGDRSGFGKFERGGNSRWCDKSDEDDWSKPLPPSERLEQELFSGG-NTGIN
2db3.1.B   -------------------------------------------------------YIPPEPSNDAIE--IFSSGIASGIH
Target     FEKYDDIPVEATGNNCPPHIESFSDVEMGEIIMGNIELTRYTRPTPVQKHAIPIIKEKRDLMACAQTGSGKTAAFLLPIL
2db3.1.B   FSKYNNIPVKVTGSDVPQPIQHFTSADLRDIIIDNVNKSGYKIPTPIQKCSIPVISSGRDLMACAQTGSGKTAAFLLPIL
Target     SQIYSDGPGEALRAMKENGRYGRRKQYPISLVLAPTRELAVQIYEEARKFSYRSRVRPCVVYGGADIGQQIRDLERGCHL
2db3.1.B   SKLLED-PHEL-----ELGR-------PQVVIVSPTRELAIQIFNEARKFAFESYLKIGIVYGGTSFRHQNECITRGCHV
Target     LVATPGRLVDMMERGKIGLDFCKYLVLDEADRMLDMGFEPQIRRIVEQDTMPPKGVRHTMMFSATFPKEIQMLARDFLDE
2db3.1.B   VIATPGRLLDFVDRTFITFEDTRFVVLDEADRMLDMGFSEDMRRIMTHVTMRPE--HQTLMFSATFPEEIQRMAGEFLKN
Target     YIFLAVGRVGSTSENITQKVVWVEESDKRSFLLDLLNATGKDSLTLVFVETKKGADSLEDFLYHEGYACTSIHGDRSQRD
2db3.1.B   YVFVAIGIVGGACSDVKQTIYEVNKYAKRSKLIEILSEQADG--TIVFVETKRGADFLASFLSEKEFPTTSIHGDRLQSQ
Target     REEALHQFRSGKSPILVATAVAARGLDISNVKHVINFDLPSDIEEYVHRIGRTGRVGNLGLATSFFN-ERNINITKDLLD
2db3.1.B   REQALRDFKNGSMKVLIATSVASRGLDIKNIKHVINYDMPSKIDDYVHRIGRTGRVGNNGRATSFFDPEKDRAIAADLVK
Target     LLVEAKQEVPSWLENMAYEHHYKGSSRGRSKSSRFSGGFGARDYRQSSGASSSSFSSSRASSSRSGGGGHGSSRGFGGGG
2db3.1.B   ILEGSGQTVPDFL-------------------------------------------------------------------
Target     YGGFYNSDGYGGNYNSQGVDWWGN
2db3.1.B   ------------------------
```

The homology model for DDX4 was built with ProMod3 (version 1.0.2), the protein was modeled as monomer without ligands with a GMQE value of 0.48 (per residue local quality estimation, Figure 4a) and a QMEAN value of -0.87 (Figure 4b). The template structure selected was the ATP dependent RNA helicase vasa, the X-Ray structure with PDB id 2DB3 (chain B), resolution 2.20 Å, residue range 244-675, coverage of target sequence 58%, with a sequence identity of 55.08%, and sequence similarity 0.45, identified by BLAST. Sequence alignment of the DDX4 sequence (SEQ. ID No. 3) with the template sequence (SEQ. ID No. 4):

```
Target     MGDEDWEAEINPHMSSYVPIFEKDRYSGENGDNFNRTPASSSEMDDGPSRRDHFMKSGFASGRNFGNRDAGECNKRDNTS
2db3.1.B   --------------------------------------------------------------------------------
Target     TMGGFGVGKSFGNRGFSNSRFEDGDSSGFWRESSNDCEDNPTRNRGFSKRGGYRDGNNSEASGPYRRGGRGSFRGCRGGF
2db3.1.B   --------------------------------------------------------------------------------
Target     GLGSPNNDLDPDECMQRTGGLFGSRRPVLSGTGNGDTSQSRSGSGSERGGYKGLNEEVITGSGKNSWKSEAEGGESSDTQ
2db3.1.B   --------------------------------------------------------------------------------
Target     GPKVTYIPPPPPEDEDSIFAH-YQTGINFDKYDTILVEVSGHDAPPAILTFEEANLCQTLNNNIAKAGYTKLTPVQKYSI
2db3.1.B   ---EFYIPPEPSNDAIEIFSSGIASGIHFSKYNNIPVKVTGSDVPQPIQHFTSADLRDIIIDNVNKSGYKIPTPIQKCSI
Target     PIILAGRDLMACAQTGSGKTAAFLLPILAHMMHDGITASRFKELQEPECIIVAPTRELVNQIYLEARKFSFGTCVRAVVI
2db3.1.B   PVISSGRDLMACAQTGSGKTAAFLLPILSKLLEDPHEL----ELGRPQVVIVSPTRELAIQIFNEARKFAFESYLKIGIV
Target     YGGTQLGHSIRQIVQGCNILCATPGRLMDIIGKEKIGLKQIKYLVLDEADRMLDMGFGPEMKKLISCPGMPSKEQRQTLM
2db3.1.B   YGGTSFRHQNECITRGCHVVIATPGRLLDFVDRTFITFEDTRFVVLDEADRMLDMGFSEDMRRIMTHVTM--RPEHQTLM
Target     FSATFPEEIQRLAAEFLKSNYLFVAVGQVGGACRDVQQTVLQVGQFSKREKLVEILRNIGDERTMVFVETKKKADFIATF
2db3.1.B   FSATFPEEIQRMAGEFLK-NYVFVAIGIVGGACSDVKQTIYEVNKYAKRSKLIEILSEQA-DGTIVFVETKRGADFLASF
Target     LCQEKISTTSIHGDREQREREQALGDFRFGKCPVLVATSVAARGLDIENVQHVINFDLPSTIDEYVHRIGRTGRCGNTGR
2db3.1.B   LSEKEFPTTSIHGDRLQSQREQALRDFKNGSMKVLIATSVASRGLDIKNIKHVINYDMPSKIDDYVHRIGRTGRVGNNGR
Target     AISFFDLESDNHLAQPLVKVLTDAQQDVPAWLEEIAFSTYIPGFSGSTRGNVFASVDTRKGKSTLNTAGFSSSQAPNPVD
2db3.1.B   ATSFFDPEKDRAIAADLVKILEGSGQTVPDFLRTC---------------------------------------------
Target     DESWD
2db3.1.B   -----
```

The homology model for DDX5 was built with ProMod3 (version 1.0.2), the protein was modeled as monomer without ligands with a GMQE value of 0.48 (per residue local quality estimation, Figure 5a) and a QMEAN value of -0.54 (Figure 5b). The template structure selected was the Bombyx mori Vasa helicase, the X-Ray structure with PDB id 4D25

(chain A), resolution 1.90 Å, residue range 80-472, coverage of target sequence 63%, with a sequence identity of 38.82%, and sequence similarity 0.39, identified by HHblits. Sequence alignment of the DDX5 sequence (SEQ. ID No. 5) with the template sequence (SEQ. ID No. 6):

```
Target     MSGYSSDRDRGRDRGFGAPRFGGSRAGPLSGKKFGNPGEKLVKKKWNLDELPKFEKNFYQEHPDLARRTAQEVETYRRSK
4d25.1.A   -------------------------------------------------------------------------------I
Target     EITVRGHNCPKPVLNFYEANFPANVMDVIARQNFTEPTAIQAQGWPVALSGLDMVGVAQTGSGKTLSYLLPAIVHINHQP
4d25.1.A   AVKVSGENPPRPIESFETANLRKYVLDNVLKAGYRKPTPIQKNAIPIIMSGRDLMGCAQTGSGKTAAFLVPIINMLLQDP
Target     FL----ERGDGPICLVLAPTRELAQQVQQVAAEYCRACRLKSTCIYGGAPKGPQIRDLERGVEICIATPGRLIDFLECGK
4d25.1.A   KDLISENGCAQPQVIIVSPTRELTLQIFNEARKFSYGSVLKVAVAYGGTAVRHQGDNIARGCHILVATPGRLHDFVERNR
Target     TNLRRTTYLVLDEADRMLDMGFEPQIRKIVDQI--R--PDRQTLMWSATWPKEVRQLAEDFLKDYIHINIGALELSANHN
4d25.1.A   VSFGSVRFVVLDQADCMLDMGFMPSIEKMMLHPTMVETTKRQTLMFSATFPEDIQHLAGRFLNNYLFVAVGIVG-GASTD
Target     ILQIVDVCHDVEKDEKLIRLMEEIMSEKENKTIVFVETKRRCDELTRKMRRDGWPAMGIHGDKSQQERDWVLNEFKHGKA
4d25.1.A   VEQIFIEVTKYEKRNSLKQLIEE---NDGKRILVFVETKRNADFIAAMLSEQQLLTSSIHGDRMQREREEALQNFKSGKH
Target     PILIATDVASRGLDVEDVKFVINYDYPNSSEDYIHRIGRTARSTKTGTAYTFFTPN-NIKQVSDLISVLREANQAINPKL
4d25.1.A   CILVATAVAARGLDIKNVDIVVNYDLPKSIDEYVHRIGRTGRVGNRGKAVSFYDSDQDLALVADLSKILRQADQSVPDFL
Target     LQLVEDRGSGRSRGRGGMKDDRRDRYSAGKRGGFNTFRDRENYDRGYSSLLKRDFGAKTQNGVYSAANYTNGSFGSNFVS
4d25.1.A   K-------------------------------------------------------------------------------
Target     AGIQTSFRTGNPTGTYQNGYDSTQQYGSNVPNMHNGMNQQAYAYPATAAAPMIGYPMPTGYSQ
4d25.1.A   ---------------------------------------------------------------
```

The homology model for DDX17 was built with ProMod3 (version 1.0.2), the protein was modeled as monomer without ligands with a GMQE value of 0.48 (per residue local quality estimation, Figure 6a) and a QMEAN value of -0.54 (Figure 6b). The template structure selected was the Bombyx mori Vasa helicase, the X-Ray structure with PDB id 4D25 (chain A), resolution 1.90 Å, residue range 163-544, coverage of target sequence 52%, with a sequence identity of 42.02%, and sequence similarity 0.40, identified by BLAST. Sequence alignment of the DDX17 sequence (SEQ. ID No. 7) with the template sequence (SEQ. ID No. 8):

```
Target     MPTGFVAPILCVLLPSPTREAATVASATGDSASERESAAPAAAPTAEAPPPSVVTRPEPQALPSPAIRAPLPDLYPFGTM
4d25.1.A   --------------------------------------------------------------------------------
Target     RGGGFGDRDRDRDRGGFGARGGGGLPPKKFGNPGERLRKKKWDLSELPKFEKNFYVEHPEVARLTPYEVDELRRKKEITV
4d25.1.A   --------------------------------------------------------------------------------
Target     RGGDVCPKPVFAFHHANFPQYVMDVLMDQHFTEPTPIQCQGFPLALSGRDMVGIAQTGSGKTLAYLLPAIVHINHQP---
4d25.1.A   --GENPPRPIESFETANLRKYVLDNVLKAGYRKPTPIQKNAIPIIMSGRDLMGCAQTGSGKTAAFLVPIINMLLQDPKDL
Target     YLERGDG-PICLVLAPTRELAQQVQQVADDYGKCSRLKSTCIYGGAPKGPQIRDLERGVEICIATPGRLIDFLESGKTNL
4d25.1.A   ISENGCAQPQVIIVSPTRELTLQIFNEARKFSYGSVLKVAVAYGGTAVRHQGDNIARGCHILVATPGRLHDFVERNRVSF
Target     RRCTYLVLDEADRMLDMGFEPQIRKIVDQIRP------DRQTLMWSATWPKEVRQLAEDFLRDYTQINVGNLELSANHNI
4d25.1.A   GSVRFVVLDQADCMLDMGFMPSIEKMM--LHPTMVETTKRQTLMFSATFPEDIQHLAGRFLNNYLFVAVGIVG-GASTDV
Target     LQIVDVCMESEKDHKLIQLMEEIMAEKENKTIIFVETKRRCDDLTRRMRRDGWPAMCIHGDKSQPERDWVLNEFRSGKAP
4d25.1.A   EQIFIEVTKYEKRNSLKQLIEE---NDGKRILVFVETKRNADFIAAMLSEQQLLTSSIHGDRMQREREEALQNFKSGKHC
Target     ILIATDVASRGLDVEDVKFVINYDYPNSSEDYVHRIGRTARSTNKGTAYTFF-TPGNLKQARELIKVLEEANQAINPKLM
4d25.1.A   ILVATAVAARGLDIKNVDIVVNYDLPKSIDEYVHRIGRTGRVGNRGKAVSFYDSDQDLALVADLSKILRQADQSV-----
Target     QLVDHRGGGGGGGGRSRYRTTSSANNPNLMYQDECDRRLRGVKDGGRRDSASYRDRSETDRAGYANGSGYGSPNSAFGAQ
4d25.1.A   --------------------------------------------------------------------------------
Target     AGQYTYGQGTYGAAAYGTSSYTAQEYGAGTYGASSTTSTGRSSQSSSQQFSGIGRSGQQPQPLMSQQFAQPPGATNMIGY
4d25.1.A   --------------------------------------------------------------------------------
Target     MGQTAYQYPPPPPPPPPSRK
4d25.1.A   --------------------
```

The homology model for DDX21 was built with ProMod3 (version 1.0.2), the protein was modeled as monomer without ligands with a GMQE value of 0.31 (per residue local quality estimation, Figure 7a) and a QMEAN value of -2.59

(Figure 7b). The template structure selected was the Bombyx mori Vasa helicase, the X-Ray structure with PDB id 4D25 (chain A), resolution 1.90 Å, residue range 184-558, coverage of target sequence 47%, with a sequence identity of 31.98%, and sequence similarity 0.36, identified by HHblits. Sequence alignment of the DDX21 sequence (SEQ. ID No. 9) with the template sequence (SEQ. ID No. 10):

```
Target     MPGKLRSDAGLESDTAMKKGETLRKQTEEKEKKEKPKSDKTEEIAEEEETVFPKAKQVKKKAEPSEVDMNSPKSKKAKKK
4d25.1.A   --------------------------------------------------------------------------------
Target     EEPSQNDISPKTKSLRKKKEPIEKKVVSSKTKKVTKNEEPSEEEIDAPKPKKMKKEKEMNGETREKSPKLKNGFPHPEPD
4d25.1.A   --------------------------------------------------------------------------------
Target     CNPSEAASEESNSEIEQEIPVEQKEGAFSNFPISEETIKLLKGRGVTFLFPIQAKTFHHVYSGKDLIAQARTGTGKTFSF
4d25.1.A   -----------------------PIESFETANLRKYVLDNVLKAGYRKPTPIQKNAIPIIMSGRDLMGCAQTGSGKTAAF
Target     AIPLIEKLHGELQD---RKRGRAPQVLVLAPTRELANQVSKDFSDITK--KLSVACFYGGTPYGGQFERMRNGIDILVGT

4d25.1.A   LVPIINMLLQDPKDLISENGCAQPQVIIVSPTRELTLQIFNEARKFSYGSVLKVAVAYGGTAVRHQGDNIARGCHILVAT
Target     PGRIKDHIQNGKLDLTKLKHVVLDEVDQMLDMGFADQVEEILSVAYKKDSEDNPQTLLFSATCPHWVFNVAKKYMKSTYE
4d25.1.A   PGRLHDFVERNRVSFGSVRFVVLDQADCMLDMGFMPSIEKMMLHPTMV-ETTKRQTLMFSATFPEDIQHLAGRFLNNY-L
Target     QVDLIGKKTQKTAITVEHLAIKCHWTQRAAVIGDVIRVYSGHQGRTIIFCETKKEAQELSQN-SAIKQDAQSLHGDIPQK
4d25.1.A   FVAVG--IVGGASTDVEQIFIEVTKYEKRNSLKQLIEEND--GKRILVFVETKRNADFIAAMLSEQQLLTSSIHGDRMQR
Target     QREITLKGFRNGSFGVLVATNVAARGLDIPEVDLVIQSSPPKDVESYIHRSGRTGRAGRTGVCICFYQHKE-EYQLVQVE
4d25.1.A   EREEALQNFKSGKHCILVATAVAARGLDIKNVDIVVNYDLPKSIDEYVHRIGRTGRVGNRGKAVSFYDSDQDLALVADLS
Target     QKAGIKFKRIGVPSATEIIKASSKDAIRLLDSVPPTAISHFKQSAEKLIEEKGAVEALAAALAHISGATSVDQRSLINSN
4d25.1.A   KILRQ---------------------------------------------------------------------------
Target     VGFVTMILQCSIEMPNISYAWKELKEQLGEEIDSKVKGMVFLKGKLGVCFDVPTASVTEIQEKWHDSRRWQLSVATEQPE
4d25.1.A   --------------------------------------------------------------------------------
Target     LEGPREGYGGFRGQREGSRGFRGQRDGNRRFRGQREGSRGPRGQRSGGGNKSNRSQNKGQKRSFSKAFGQ
4d25.1.A   ----------------------------------------------------------------------
```

The homology model for DDX23 was built with ProMod3 (version 1.0.2), the protein was modeled as monomer without ligands with a GMQE value of 0.32 (per residue local quality estimation, Figure 8a) and a QMEAN value of -2.92 (Figure 8b). The template structure selected was the ATP dependent RNA helicase vasa, the X-Ray structure with PDB id 2DB3 (chain B), resolution 2.20 Å, residue range 299-704, coverage of target sequence 47%, with a sequence identity of 41.78%, and sequence similarity 0.41, identified by BLAST. Sequence alignment of the DDX23 sequence (SEQ. ID No. 11) with the template sequence (SEQ. ID No. 12):

```
Target     MAGELADKKDRDASPSKEERKRSRTPDRERDRDRDRKSSPSKDRKRHRSRDRRRGGSRSRSRSRSKSAERERRHKERERD
2db3.1.B   --------------------------------------------------------------------------------
Target     KERDRNKKDRDRDKDGHRRDKDRKRSSLSPGRGKDFKSRKDRDSKKDEEDEHGDKKPKAQPLSLEELLAKKKAEEEAEAK
2db3.1.B   --------------------------------------------------------------------------------
Target     PKFLSKAEREAEALKRRQQEVEERQRMLEEERKKRKQFQDLGRKMLEDPQERERRERRERMERETNGNEDEEGRQKIREE
2db3.1.B   --------------------------------------------------------------------------------
Target     KDKSKELHAIKERYLGGIKKRRRTRHLNDRKFVFEWDASEDTSIDYNPLYKERHQVQLLGRGFIAGIDLKQQKREQSRFY
2db3.1.B   --------------------------------------------------------------------------------
Target     GDLMEKRRTLEEKEQEEARLRKLRKKEAKQRWDDRHWSQKKLDEMTDRDWRIFREDYSITTKGGKIPNPIRSWKDSSLPP
2db3.1.B   ----------------------------------------------------------VKVTGSDVPQPIQHFTSADLRD
Target     HILEVIDKCGYKEPTPIQRQAIPIGLQNRDIIGVAETGSGKTAAFLIPLLVWITTLPKIDRIEESDQG-PYAIILAPTRE
2db3.1.B   IIIDNVNKSGYKIPTPIQKCSIPVISSGRDLMACAQTGSGKTAAFLLPILSKLLEDP-----HELELGRPQVVIVSPTRE
Target     LAQQIEEETIKFGKPLGIRTVAVIGGISREDQGFRLRMGCEIVIATPGRLIDVLENRYLVLSRCTYVVLDEADRMIDMGF
2db3.1.B   LAIQIFNEARKFAFESYLKIGIVYGGTSFRHQNECITRGCHVVIATPGRLLDFVDRTFITFEDTRFVVLDEADRMLDMGF
Target     EPDVQKILEHMPVSNQKPDTDEAEDPEKMLANFESGKHKYRQTVMFTATMPPAVERLARSYLRRPAVVYIGSAGKPHERV
2db3.1.B   SEDMRRIMTHV---TMRPE---------------------HQTLMFSATFPEEIQRMAGEFLKNYVFVAIGIVGGACSDV
Target     EQKVFLMSESEKRKKLLAILEQGFDPPIIIFVNQKKGCDVLAKSLEKMGYNACTLHGGKGQEQREFALSNLKAGAKDILV
2db3.1.B   KQTIYEVNKYAKRSKLIEILSEQADG-TIVFVETKRGADFLASFLSEKEFPTTSIHGDRLQSQREQALRDFKNGSMKVLI
Target     ATDVAGRGIDIQDVSMVVNYDMAKNIEDYIHRIGRTGRAGKSGVAITFLTKE-DSAVFYELKQAILESPVSSCPPELANH
2db3.1.B   ATSVASRGLDIKNIKHVINYDMPSKIDDYVHRIGRTGRVGNNGRATSFFDPEKDRAIAADLVK-ILEGSGQTVP------
Target     PDAQHKPGTILTKKRREETIFA
2db3.1.B   ----------------------
```

### Pharmacophores generation - virtual screening

A series of 3D-pharmacophore models has been generated, starting from the homology models of the human DDX helicases, DDX5, DDX3, DDX4, DDX23 and DDX21. These 3D-pharmacophore models were prepared as described below (DDX17 was excluded because of its high sequence identity with DDX5).

The 3D-pharmacophore models of the RNA binding sites were developed by starting from the conformation of RNA bound to each human DEAD box helicase using the software ALIGN-IT version 1.0.4 (SILICOS-IT; Taminau, J. et al., Pharao: pharmacophore alignment and optimization, J Mol Graph Model, 2008, 27, 161) and refining each of the 3D-pharmacophore models by removing the redundant pharmacophoric features. The pharmacophoric models were used to screen a multi-conformer database of commercially available compounds, a previously filtered version of Molport database 2017 (available at ftp://molport.com/) as described below. The complete MOLPORT compound database was filtered using a set of substructure SMARTS definitions (SMARTS Theory Manual, Daylight Chemical Information Systems, Santa Fe, New Mexico) described in literature to remove reactive species (Bruns, R. F. & Watson, I. A., Rules for identifying potentially reactive or promiscuous compounds. J Med Chem, 2012, 55, 9763) and PAINS (Baell, J. B. Screening-based translation of public research encounters painful problems. ACS Med Chem Lett, 2015, 6, 229; Dahlin, J. L. et al., PAINS in the assay: chemical mechanisms of assay interference and promiscuous enzymatic inhibition observed during a sulfhydryl-scavenging HTS. J Med Chem, 2015, 58, 2091). Applying these filters, the number of commercial compounds to be screened was reduced from about 7 million of compounds to about 5.5 million of compounds. The previously obtained 5.5 million compounds database was subsequently processed to create a tridimensional multi-conformer database with the program BALLOON version 1.6.4.1258 (Copyright © 2006-2016 Mikko J. Vainio and J. Santeri Puranen; Copyright © 2010 Visipoint Ltd.) using the following procedure.

The conformational analyses have been performed using the program BALLOON (Mikko J. Vainio and Mark S. Johnson, Generating Conformer Ensembles Using a Multiobjective Genetic Algorithm, Journal of Chemical Information and Modeling, 2007, 47, 2462; J. Santeri Puranen et al., Accurate conformation-dependent molecular electrostatic potentials for high-throughput in-silico drug discovery, Journal of Computational Chemistry, 2010, 31, 1722), using the following parameters: Merk Molecular force field version 94 (MMFF94) as force field, generating all the conformations within 10 Kcal/mol from the energy minima conformation, root mean squared distance (RMSD) between conformation higher than 1.2 Å and 1000 iteration of simplex minimization. For each of the conformations a 3D-pharmacophore model using the software ALIGN-IT version 1.0.4 was prepared (SILICOS-IT; Taminau, J.; Thijs, G.; De Winter, H., Pharao: pharmacophore alignment and optimization, J Mol Graph Model, 2008, 27, 161). The virtual 3D-pharmacophore screening

has been performed in the servers of First Health Pharmaceutical B.V. in Amsterdam, consisting of three multiprocessor servers with 88 CPU thread and 64GB of RAM each.

The final selection of compounds obtained with the 3D-Pharmacophore virtual screening was around 2500 compounds, that selection was further filtered by molecular docking ranking using the homology models of DDX3X, DDX4, DDX5, DDX17, DDX21 and DDX23, obtaining a final list of 200 compounds (see "In-Silico Molecular docking refinement of selections" section).

**Molecular dynamics Simulations**

Each homology model of the human DDX helicases has been prepared for a series of explicit solvated molecular dynamics simulations, except DDX17, because the percentage of identity with DDX5 for the aminoacids of binding site is 100%. The homology models were refined adding the ATP cofactor and the magnesium 2+ ion with coordination water molecules, the protonation state and orientation of histidine, asparagine and glutamine residues have been optimized with PDB2PQR version 2.1.0 (Dolinsky TJ et al., PDB2PQR: Expanding and upgrading automated preparation of biomolecular structures for molecular simulations. Nucleic Acids Res, 2007, 35, W522; Dolinsky TJ et al., PDB2PQR: an automated pipeline for the setup, execution, and analysis of Poisson-Boltzmann electrostatics calculations. Nucleic Acids Res, 2004, 32, W665). All the missing hydrogen atoms were added according to the AMBER 14 topology parameters using the LEAP module within AMBERTOOLS program suite. ATP was parameterized using the parameters set as described from Meagher, Redman and Carlson (Meagher KL, Redman LT, Carlson HA Development of polyphosphate parameters for use with the AMBER force field; J Comp Chem, 2003, 24, 1016). The protein was solvated in a cubic water box of TIP3P waters, water constituting 10 Å buffer form the protein to the periodic boundary. The system was then neutralized adding the appropriate number of sodium and chloride ions to simulate a concentration of 0.1M. Topology files were parameterized using AMBER14SB forcefield.

All the molecular dynamics simulations were performed with NAMD (version 2.12; Phillips et al., J Comp Chem, 2005, 26, 1781). A total of 5,000 steps of energy minimization have been carried out to remove artificial contacts, without constraint. The system was then equilibrated for 1 ns with a 2 fs time step at 1 atm pressure following a two-step protocol: the first step consist of 0.1 ns simulation where the system was heated form 0 °K to 310 °K with a Berendsen thermostat simulation; the second step is a 0.9 ns Langevin dynamics simulation at 310 °K temperature. Finally, a 20 ns simulation was carried out. The simulations have been run with GPU accelerated molecular dynamics in an 88 CPUs system with two GPU (GeForce GTX 1080) with an average speed of 0.2 days/ns of simulation. Simulation snapshots have been collected every 10000 steps resulting in 1,000 frames, which represent a conformational sampling of the aminoacids side chains degree of freedom. In Figure 9, the RMSD fluctuation of the carbon alpha trace of the proteins respect to the average structure is reported, indicating that the helicases modeled are well equilibrated with an average RMSD fluctuation below 2.5 Å.

The pairwise RMSD matrix between the frames has been calculated with VMD (Humphrey, W. et al., VMD - Visual Molecular Dynamics, J Molec Graphics, 1996, 14, 33). The alignment and RMSD calculation were performed between the residues that contribute to the helicase binding site surface of each molecular dynamic frame. The clustering was performed by hierarchical clustering with average linkage at RMSD 2.0 Å with amaximum number of 20 clusters (Figure 10). For each protein the dominant representative structures of each cluster were selected for the ensemble molecular docking procedure.

**In-Silico Molecular docking refinement of selections**

The molecular docking experiments were performed using two different programs SMINA (O. Trott, A. J. Olson, AutoDock Vina: improving the speed and accuracy of docking with a new scoring function, efficient optimization and multithreading, J Comp Chem, 2010, 31, 455; David Ryan Koes et al., Lessons Learned in Empirical Scoring with Smina from the CSAR 2011 Benchmarking Exercise, J Chem Inf Model, 2013, 538 1893) and RBDOCK (Ruiz-Carmona S et al., rDock: A Fast, Versatile and Open Source Program for Docking Ligands to Proteins and Nucleic Acids. PLoS Comput Biol, 2014, 10: e1003571; Morley, S.D. and Afshar, M., Validation of an empirical RNA-ligand scoring function for fast flexible docking using Ribodock. J Comput Aided Mol Des, 2004, 18, 189). The molecular docking protocol consisted in the docking in parallel with both SMINA and RBDOCK, in all the homology models of the human DDX helicases selected. For each of the 2500 molecules selected in the 3D-pharmacophore screening previously described the docking have been performed, and the docking poses obtained from the two docking programs were rescored using an external scoring function, RF-SCORE-VS (Wójcikowski, M. et al., Performance of machine-learning scoring functions in structure-based virtual screening, Scientific reports, 2017, 7, 46710), and finally the poses were clustered by hierarchical clustering with average linkage at RMSD 2.5 Å. The 200 top ranked compounds were visually inspected and selected for the biological screening.

The criteria applied for the selection were the occupation of the region of binding site that in the physiological

conditions interact with the phosphate backbone of RNA. Specifically the compounds are considered to bind the proteins when the docking procedure find that at least 3 of the interactions shown in table 3a and optionally the interactions of table 3b are present between ligand and protein residues. The hydrogen bond donor (HBD) and hydrogen bond acceptor (HBA) interaction are considered present when the distance between the heavy atoms is between 2 and 3.5 Å, the Van der Waals interactions (VdW) are considered present when any of the atoms of the ligand are at a distance between 3 and 4.5 Å from the atom of the protein residues.

Table 3a. Interactions between ligands and proteins considered indicative of protein binding

| | | Binding site residue - index | | | | | |
|---|---|---|---|---|---|---|---|
| Interaction type - index | Residue interacting part | DDX3X | DDX4 | DDX5 | DDX17 | DDX21 | DDX23 |
| HBD - 1 | Side chain | Arg 276 | Arg 375 | Arg 177 | Arg 254 | Arg 270 | Arg 478 |
| HBD - 2 | Side chain | Arg 480 | Arg 579 | Arg 380 | Arg 457 | Arg 476 | Arg 703 |
| HBD-3 | Side chain | Thr 498 | Thr 597 | Thr 398 | Thr 475 | Thr 494 | Thr 721 |
| VdW/HBA - 4 | Backbone | Pro 274 | Pro 373 | Pro 175 | Pro 252 | Pro 268 | Pro 476 |
| VdW-5 | Side chain | Phe 357 | Phe 456 | Phe 258 | Phe 335 | Phe 349 | Phe 559 |
| VdW - 6 | Side chain | Val 500 | Val 599 | Val 400 | Val 477 | Val 496 | Val 723 |

Table 3b. Optional interactions between ligands and proteins binding site residues

| | | Binding site residue - index | | | | | |
|---|---|---|---|---|---|---|---|
| Interaction type | Residue interactin g part | DDX3X | DDX4 | DDX5 | DDX17 | DDX21 | DDX23 |
| VdW - 7 | Side chain | Gly 473 | Gly 572 | Gly 373 | Gly 450 | Gly 469 | Gly 696 |
| HBA/HBD - 8 | Side chain | Thr 323 | Thr 422 | Thr 224 | Thr 301 | Thr 315 | Thr 525 |
| HBD - 9 | Side chain | Arg 351 | Arg 450 | Arg 252 | Arg 329 | Gln 343 | Arg 553 |
| HBA/HBD/ VdW - 10 | Side chain | Ala 499 | Ser 598 | Asp 399 | Asp 476 | Asn 495 | Asp 722 |

**Biological screening of selected compound library**

The triple negative breast cancer cell line MDA-MB-231 has been selected as prescreening benchmark because of the reported involvement of various DDX helicases in breast cancer progression (Guturi, K. K. N. et al., DEAD-box protein p68 is regulated by β-catenin/transcription factor 4 to maintain a positive feedback loop in control of breast cancer progression, Breast cancer research, 2014, 16, 496; Cannizzaro, E et al., DDX3X RNA helicase affects breast cancer cell cycle progression by regulating expression of KLF4. FEBS letters, 2018, 592, 2308; Hashemi, V. et al., The role of DEAD-box RNA helicase p68 (DDX5) in the development and treatment of breast cancer. Journal of cellular physiology, 2019, 234, 5478; Mazurek, A. et al., DDX5 regulates DNA replication and is required for cell proliferation in a subset of breast cancer cells. Cancer discovery, 2012, 2, 812; Wang, D. et al., RNA helicase DDX5 regulates microRNA expression and contributes to cytoskeletal reorganization in basal breast cancer cells. Molecular & cellular proteomics, 2012, 11, M111.011932; Dutertre, M. et al., Estrogen regulation and physiopathologic significance of alternative promoters in breast cancer. Cancer research, 2010, 70, 3760; Alqahtani, H. et al., DDX17 (P72), a Sox2 binding partner, promotes stem-like features conferred by Sox2 in a small cell population in estrogen receptor-positive breast cancer. Cellular signalling, 2016, 28, 42).

The selected compound library of 200 compounds was screened in duplicate at single concentration 10 μM against the cell line MDA-MB-231 with MTS colorimetric assay. The 16 compounds with residual cell viability below 80% than untreated cells used as control have been selected for further characterization.

The 16 compounds were subsequently tested in MDA-MB-231 cells in a dose response assay at 5 concentrations (0.1 μM, 1.0 μM, 10 μM, 50 μM and 100 μM) in a cell count assay. This assay confirmed 11 compounds as active in dose-response, showing an antiproliferative effect between 1 and 33 μM.

**In-Silico Molecular docking guided optimization**

These 11 compounds were further optimized through an iterative process based on ensemble molecular docking in the human DDX helicases (DDX3X, DDX4, DDX5, DDX21 and DDX23), followed by design of new compounds, synthesis and routine biological evaluation in the MDA-MB-231 cell line. In the optimization phase, the previously defined molecular docking protocol was modified by adding an ensemble of 20 representative conformations for each human DDX helicase under investigation, obtained from the previously described molecular dynamics simulation and clustering of conformations. As for the previously described molecular docking protocol, the docking programs employed were SMINA and RBDOCK, for each ensemble of protein conformations the docking poses were rescored using an external scoring function, RF-SCORE-VS and clustered by hierarchical clustering with average linkage at RMSD 2.5Å. The docking results for each new compound were evaluated by consensus of the docking poses obtained for each human DDX helicases: DDX3X, DDX4, DDX5, DDX21, DDX23 (DDX17 was excluded because the RNA binding site has 100% identity with DDX5).

All the 3D structure models of DDX helicases were aligned together by the alpha carbon of binding site residues, as defined in table 2. Consensus binding for two or more conformations of the same molecule was defined when two conformation of the same molecule docked in different DDX shows an RMDS lower than 3.0 Å.

The small molecules with consensus binding between at least two of the DDX helicases were considered potential "MasterKey" compounds. Further optimization of compounds was carried out in silico, designing compounds able to maximize the number of interaction listed in tables 3a and 3b. The best compounds were then synthesized and tested (compounds 1-32).

**Binding mode analysis**

Only as an example, the binding mode analyses of compounds 3 and 5 are reported below. The ensemble docking of compound 3 on DDX3X, DDX4, DDX5, DDX21 and DDX23 was done following the protocol described above. The best binding mode in all the proteins was reported (Figure 11). For all the five helicases the binding mode of the compound 3 is very similar, with the same orientation of the ligand within the binding sites of DDX3X, DDX4, DDX5, DDX21 and DDX23 aligned in the same frame of reference. The interactions are reported using the interaction "type - index" shown in tables 3a and 3b. The mainly conserved interaction of compound 3 is between the triazole nitrogen atoms and HBD - 1 and HBD - 2; HBD - 1 is present in DDX3X, DDX4 and DDX5, the interaction HBD - 2 can be found in DDX3X, DDX4, DDX5 and DDX23; hydrogen bond interaction between the oxadiazole moiety and HBD - 3 can be identified for DDX4, DDX5, DDX21 and DDX23; In all the helicases, interaction with VdW/HBA - 4 are only Van der Waals contact between central phenyl ring and the backbone of the residue, such as in DDX3X, DDX4 and DDX21; the interaction of compound 3 with VdW - 5 is present in all the helicases but the part of the molecule that interact with this residue it is not clearly defined; interaction between compound 3 and VdW - 6, is clearly identified with the central phenyl ring of compound 3 in all the helicases, it can be defined as a methylene-pi interaction with an energy of interaction higher than the usual Van der Waals interactions (Wang and Yao, Scientific Reports, 2019, 9, 20149); central phenyl ring of compound 3 shows Van der Waals contacts with VdW - 7 residue in DDX4, DDX5, DDX21 and DDX23; in none of the helicases compound 3 shows hydrogen bond interactions with HBA/HBD - 8 and HBD - 9; the moiety 5-(cyclopentyloxy)-2-methylphenyl-lyl of compound 3 interact with HBA/HBD/VdW - 10 in a sub pocket with variable residue composition along the five DDX helicases. Considering the pattern of interaction of compound 3 across all the five helicases examined this compound can be considered a "MasterKey" compound able to interfere with the RNA binding in all the helicases considered for this study.

The ensemble docking of compound 5 on DDX3X, DDX4, DDX5, DDX21 and DDX23 was done as previously described. The best binding mode in all was reported (Figure 12). The binding mode of compound 5 shows a common orientation only in DDX3X and DDX5, while in DDX4 it is flipped and in DDX21 and DDX23 is shifted respect to the other two helicases. Considering the binding mode identified in DDX3X and DDX5 is the most efficient in term of ligand protein interactions, compound five can be considered as selective for these two helicases. As already found in compound 3, the triazole nitrogen atoms of compound 5 interact with HBD -1 and HBD -2; HBD - 1 is present in DDX5, the interaction HBD - 2 can be found in DDX3X and DDX5; hydrogen bond interaction between oxadiazole moiety and HBD - 3 can be identified for DDX5 only; in all the helicases there are Van der Waals contact with VdW/HBA - 4, with different regions of the compound 5; also for compound 5 the interaction with VdW - 5 is present in all the helicases but it is not clearly defined the part of the molecule that interact with this residue; interaction between compound 5 and VdW - 6, is clearly identified between the central phenyl ring of compound 5 and DDX3X, DDX4, DDX5 and DDX21, also in that case the interaction is a methylene-pi contact; docking of compound 5 shows that the VdW - 7 residue is unable to efficiently interact with compound 5; in none of the helicases compound 5 shows hydrogen bond interactions with HBA/HBD - 8 and HBD - 9; the moiety 2-trifluoromethylphenyl-1-yl of compound 5 interact with HBA/HBD/VdW - 10 in a sub pocket with variable residue composition only in DDX3 and DDX5. Considering the pattern of interactions of compound 5 across

all the helicases examined, our model predict that it can interact efficiently only with DDX3 and DDX5. Despite the fact that compound 5 is predicted to interact with only two helicases, it is within the definition of "MasterKey" compound and it can efficiently interfere with RNA metabolism mediated by at least DDX3X and DDX5.

In general compounds 1-32 are all able to interact with the residues of table 3a at least in two of the helicases considered for this study. The polypharmacological profile involving DDX3X, DDX4, DDX5, DDX17, DDX21 and DDX23 helicases, all with a well characterized role in cancer pathogenesis, is considered an advantage to achieve activity against a wide range different cancer types.

## EXAMPLE 2: SYNTHESIS

### General materials and methods

Reagents were obtained from commercial suppliers (for example Sigma-Aldrich). All commercially available chemicals were used as purchased without further purification. TLC was carried out using Merck TLC plates, silica gel 60 F254. Chromatographic purifications were performed via normal phase column chromatography using silica gel 100-200 mesh (W. R. Grace & Co.-Conn) or using Waters Acquity UPLC with Single Quadrupole Detector with ESI source, and XBridge BEH C18 XP column. $^1$H-NMR spectra were recorded at 300 MHz, on a Mercury 300 NMR spectrometer, or at 400 MHz, on a BRUKER 400MHz, TOPSPIN 4.0.7. Chemical shifts are reported relative to tetramethylsilane at 0.00 ppm. $^1$H patterns are described using the following abbreviations: s = singlet, d = doublet, dd = double doublet, dt = double triplet, t = triplet, q = quartet, quin = quintet, sx = sextet, sept = septet, m = multiplet, br = broad signal, br s = broad singlet.

Mass spectra (MS) data were obtained using an Agilent 6100 LC/MS (G6130B) spectrometer with a 0.4 ml/min flow rate using a binary solvent system of 95:5 methanol/water. UV detection was monitored at 215 nm and 276 nm. Mass spectra were acquired in positive and negative mode scanning over the mass range.

The following schemes are examples of synthetic strategies that may be employed to produce the compounds of the invention. They are provided for illustration purposes only and are not to be considered as limiting the scope of the invention, but merely as being representative thereof. In addition to what is depicted in the schemes, reactive groups can be masked with suitable protecting groups and/or deprotected according to well established techniques. Moreover, it will be understood that certain compounds of the invention can be converted into other compounds of the invention according to standard chemical methods.

Compound 1

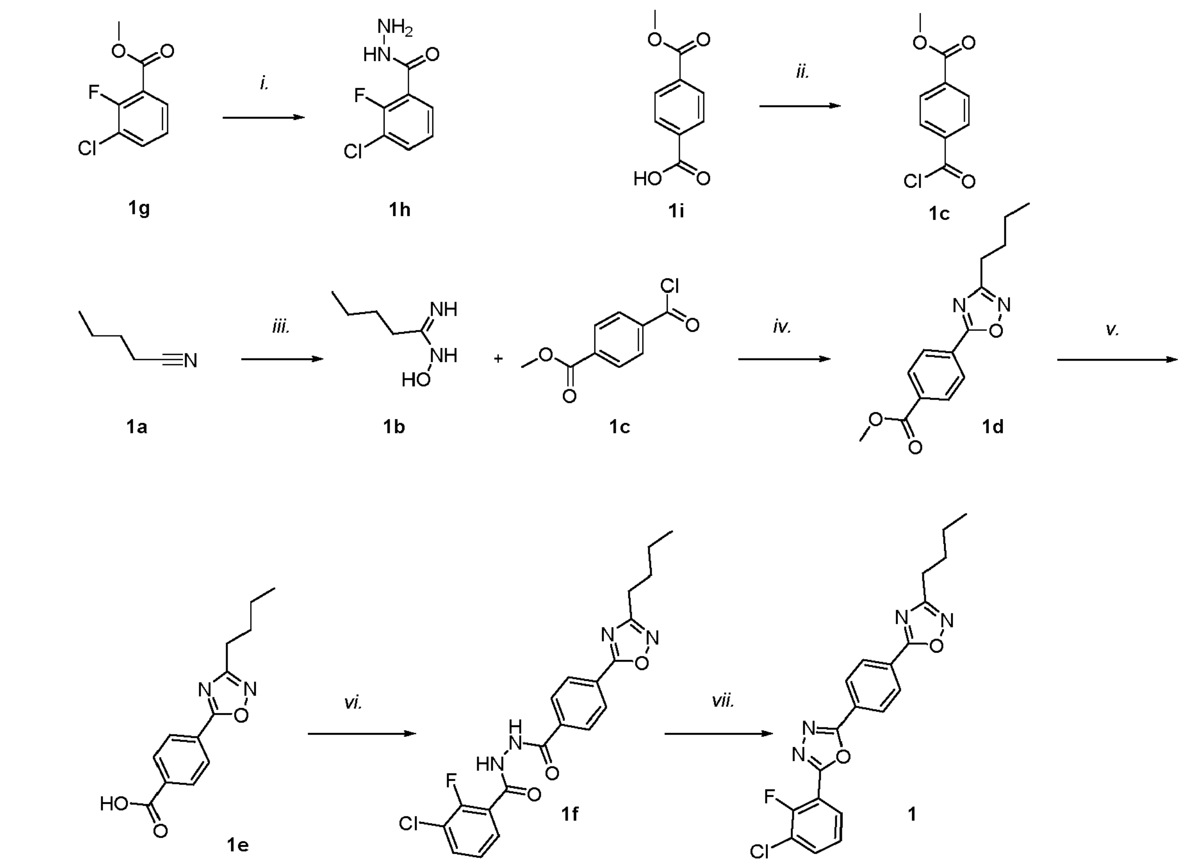


1g 1h 1i 1c

1a 1b 1c 1d

1e 1f 1

**Reagents and conditions:** *i.* Hydrazine hydrate (2 eq.), stirring, RT, overnight, methanol; ii. Oxalyl chloride (5 eq.), DCM, DMF, RT, 3.5h; *iii.* $NH_2OH.HCl$ (1.5 eq.), $Na_2CO_3$ (2 eq.) in isopropyl alcohol, 3h, 80°C, iv. Pyridine (2.1 eq.), Toluene, 115°C, overnight; v. NaOH (2.74 eq.), water, 2-Propanol, stirred at RT, overnight; vi. oxalyl chloride (22 eq.), DCM, 0°C, stirring 4h, followed by 1h (1 eq.), pyridine (2.5 eq.), DCM, from 0°C to RT, overnight; *vii.* $POCl_3$ (30 eq.), toluene, reflux, 3.5h.

**Procedure for the synthesis of compound 1:**

Compound **1h** was obtained after treatment of **1g** (100 mg, 1 Eq, 530 μmol) and hydrazine hydrate 0.05 ml (2 Eq, 0.9 mmol) were dissolved in MeOH (10 ml). The reaction mixture was stirred at room temperature overnight. The reaction mixture was diluted with water (25 ml), extracted with DCM (2 x 25 ml) and concentrated (96 mg, 96% yield) as a white solid.

**Methyl 3-chloro-2-fluorobenzoate (1g).** $^1H$ NMR (300 MHz, $CDCl_3$): δ 7.85 (d, 1H), 7.58 (d, 1H), 7.18 (t, 1H), 3.97 (t, 3H) ppm.

**3-Chloro-2-fluorobenzohydrazide (1h).** $^1H$ NMR (300 MHz, $CDCl_3$): δ 7.98 (d, 1H), 7.86 (s, 1H), 7.56 (d, 1H), 7.25 (t, 1H), 4.17 (s, 2H) ppm.

Compound **1c** (Methyl 4-(chlorocarbonyl)benzoate) was obtained adding oxalyl chloride (7 g, 5 ml, 5 Eq, 60 mmol) to **1i** (4-(methoxycarbonyl)benzoic acid) (2.0 g, 1 Eq, 11.10 mmol) in DCM (50 ml). DMF was added (0.1 ml) and the reaction mixture was stirred for 3.5 hours, then concentrated at 60 °C (Crude was directly taken to next step without further purification).

Compound **1b** was obtained adding a solution of (4 g, 5.0 ml, 1 Eq, 48 mmol) of **1a** (pentanenitrile, Sigma Aldrich catalogue code 155098) in EtOH (25 ml), to a solution of sodium carbonate (5.491 g, 1.1 Eq, 51.81 mmol) and hydroxylamine hydrochloride (3.342 g, 1.0 Eq, 48.09 mmol) in water (25 ml). The reaction mixture was heated at 70°C external. After 4 hours a sample was concentrated. Full conversion was observed. The reaction mixture was concentrated at 60°C and the residue was stirred in DCM (150 ml). The mixture was filtered and the filtrate was concentrated (791 mg, 14% yield)

**Pentanenitrile (1a).** $^1H$ NMR (300 MHz, $CDCl_3$): δ 2.58 (t, 2H), 1.82 (quin, 2H), 1.64 (sx, 2H), 1.05 (t, 3H) ppm.

**N-Hydroxypentanimidamide (1b).** $^1H$ NMR (300 MHz, $CDCl_3$): δ 4.50 (s, 2H), 2.12 (t, 2H), 1.53 (quin, 2H), 1.36 (sx, 2H), 0.91 (t, 3H) ppm.

Subsequent one pot reaction of (589 mg, 1 Eq, 5.07 mmol) of **1b,** pyridine (0.83 g, 0.85 ml, 2.1 Eq, 11 mmol) and compound **1c** (methyl 4-(chlorocarbonyl)benzoate), (906 mg, 0.9 Eq, 4.56 mmol) in toluene (5 ml), was heated at 115°C external overnight. The stirring malfunctioned and insoluble materials were stuck to the flask. Water (25 ml) was added to the reaction mixture and the layers were separated. The aqueous phase was extracted with toluene (10 ml). The combined organic layers were dried with $Na_2SO_4$, filtered and concentrated (1.273 g, 90.2% yield), by NMR it appears to be pure material containing residual toluene.

**Methyl 4-(3-butyl-1,2,4-oxadiazol-5-yl)benzoate (1d).** $^1$H NMR (300 MHz, $CDCl_3$): δ 8.13 (m, 4H), 7.13-7.30 (m, 4H), 3.99 (s, 3H), 2.85 (t, 2H), 2.38 (s, 2H), 1.83 (quin, 2H), 1.47 (sx, 2H), 1.00 (t, 3H) ppm.

The compound **Id** (1.273 g, 1 Eq, 4.891 mmol) and NaOH (536 mg, 2.74 Eq, 13.4 mmol) were dissolved in water (50 ml) and 2-propanol (10 ml) and stirred at room temperature overnight. The reaction mixture was washed with DCM (2 x 50 ml), acidified with acetic acid, extracted with DCM (2 x 50 ml), dried over $Na_2SO_4$, filtered and concentrated, obtaining Compound **1e** (4-(3-Butyl-1,2,4-oxadiazol-5-yl)benzoic acid) was synthesized as an off-white solid (793 mg, 65.8% yield).

**4-(3-Butyl-1,2,4-oxadiazol-5-yl)benzoic acid (1e).** $^1$H NMR (300 MHz, $CDCl_3$): δ 8.27 (s, 4H), 7.28 (s, 4H), 2.84 (t, 2H), 1.82 (quin, 2H), 1.47 (sx, 2H), 0.99 (t, 3H) ppm.

The hydrazide **If** was obtained by two step reaction, in a first step (130 mg, 1.0 Eq, 528 μmol) of **1e** (4-(3-Butyl-1,2,4-oxadiazol-5-yl)benzoic acid) was dissolved in DCM (25 ml), cooled using an ice/water-bath and oxalyl chloride (1.4 g, 1.0 ml, 22 Eq, 11 mmol) was added. The reaction mixture was stirred for 4 h and then concentrated. The residue was dissolved in DCM (25 ml) and cooled using an ice/water-bath. Pyridine (0.10 g, 0.10 ml, 2.5 Eq, 1.3 mmol) was added, followed by (96 mg, 1 Eq, 0.51 mmol) of 3-chloro-2-fluorobenzohydrazide **1h.** The reaction mixture was stirred, while the ice-bath reached room temperature overnight. The reaction mixture was washed with water (2 x 25 ml) and concentrated. Compound **If** was obtained as a white solid (204 mg, 96% yield).

**3-Butyl-5-(4-(5-(3-chloro-2-fluorophenyl)-1,3,4-oxadiazol-2-yl)phenyl)-1,2,4-oxadiazole** (If). $^1$H NMR (300 MHz, $CDCl_3$): δ 8.27 (d, 3H), 8.05 (d, 1H), 7.21-7.32 (m, 3H), 2.84 (t, 2H), 1.83 (quin, 2H), 1.47 (sx, 2H), 0.99 (t, 3H) ppm.

Compound 1 was prepared from (75 mg, 1 Eq, 0.18 mmol) of compound **If** in toluene (5 ml) and phosphorus oxychloride (0.8 g, 0.5 ml, 30 Eq, 5 mmol) were refluxed for 3.5 h. The reaction mixture was concentrated and partitioned between KOH 1 M (25 ml) and DCM (25 ml). The layers were separated and the aqueous phase was extracted with DCM (25 ml). The combined organic layers were dried with $Na_2SO_4$, filtered and concentrated. Compound 1 was recovered as a white solid (45 mg, 63% yield).

**3-Butyl-5-(4-(5-(3-chloro-2-fluorophenyl)-1,3,4-oxadiazol-2-yl)phenyl)-1,2,4-oxadiazole** (1). Yield=63%; Purity=95%; $^1$H NMR (300 MHz, $CDCl_3$): δ 8.32 (s, 4H), 8.12 (t, 1H), 7.65 (t, 1H), 7.36-7.24 (m, 1H), 2.84 (t, 2H), 1.83 (quin, 2H), 1.47 (sx, 2H), 0.99 (t, 3H) ppm.

**Compound 2**

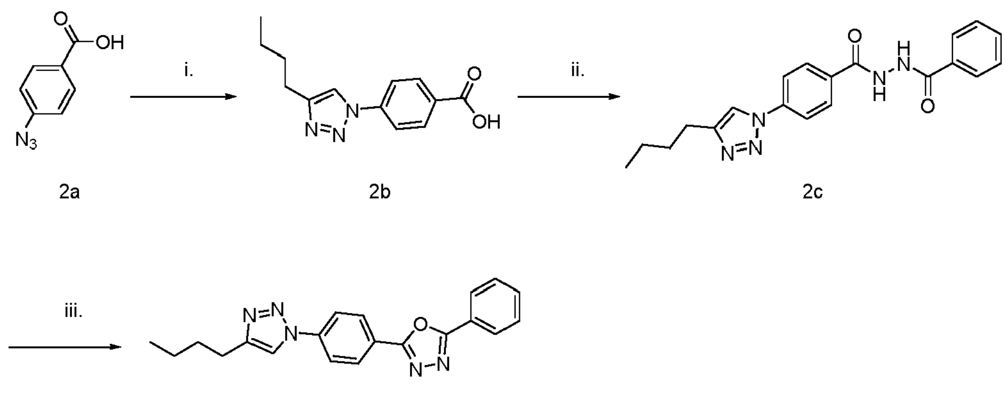


2a 2b 2c

2

**Reagents and conditions:** *i.* $CuSO_4 \cdot 5H_2O$ (0.12 eq.), Sodium Ascorbate (0.5 eq.), 1-hexene (1.5 eq.), $H_2O$:t-BuOH (1:1 v/v), MW 125°C, 15 min; *ii.* oxalyl chloride (2 eq.), DMF (1 drop), DCM, 0°C to RT, 30 min, followed by benzohydrazide (1 eq.), pyridine (2.5 eq.), DCM, RT, overnight; iii. Burgess reagent (3 eq.), acetonitrile, reflux, overnight.

**Procedure for the synthesis of compound 2:**

Compound **2b** was obtained by click reaction in a microwave tube equipped with a small stirring bar, adding (2.015 g, 1 Eq, 12.35 mmol) of compound **2a** (4-azidobenzoic acid, Sigma-Aldrich, catalogue code 778877), water (15 ml), t-BuOH (15 ml), hex-1-yne (1.522 g, 2.14 ml, 1.5 Eq, 18.53 mmol), in the presence of $CuSO_4 \cdot 5H_2O$ (367 mg, 0.119 Eq, 1.47 mmol) and sodium ascorbate (1.214 g, 0.4961 Eq, 6.128 mmol). The tube was sealed and then submitted to irradiation: the suspension became thicker on mixing. NMR analysis after 1h stirring at room temperature showed no conversion. The mixture was divided over 2 microwave vials, closed, and each heated at 125°C for 15 min. The mixtures were partitioned between water (200 ml) and EtOAc (200 ml). A solid remained on the interface of the layers and was filtered off, washed with DCM and air-dried, to afford the product as a greenish solid, 853 mg (batch A). The aqueous layer was extracted with EtOAc (2 x 75 ml). The combined organics were washed with brine, dried ($Na_2SO_4$) and concentrated in vacuum, 1.526 g, white solid. The solid was stirred up in DCM, filtered and washed with DCM and toluene to afford an off-white solid, 1.3 g, (batch B), 71% combined yield (Crude was directly taken to next step without further purification).

**4-(4-Butyl-1H-1,2,3-triazol-1-yl)benzoic acid (2b).** Yield=71%; $^1H$ NMR (300 MHz, $CDCl_3$): δ 13.19 (s, 1H), 8.68 (s, 1H), 8.11 (d, 2H), 8.01 (d, 2H), 2.69 (t, 2H), 1.66 (quin, 2H), 1.37 (sx, 2H), 0.91 (t, 3H) ppm.

In a first step 1 drop of DMF was added to **2b** (100 mg, 1 Eq, 408 μmol) in Dichloromethane (5 ml) at 0°C, followed by oxalyl chloride (103 mg, 71.4 μL, 2 Eq, 815 μmol). The ice-bath was removed and the mixture was stirred at RT for 0.5 h. The solvent was removed in vacuum. The residue was dissolved in DCM (4 ml) and added to a solution of benzohydrazide (Sigma-Aldrich, catalogue code: B13071) (55.5 mg, 1 Eq, 408 μmol) and pyridine (80.6 mg, 82 μL, 2.5 Eq, 1.02 mmol) in dichloromethane (3 ml) at 0°C. The ice-bath was removed and the mixture was allowed to reach room temperature overnight. The mixture was concentrated in vacuum, partitioned between saturated $NaHCO_3$ in water (5-6 ml) and EtOAc (20 ml). The solid was filtered over a glass filter. The filtrate was separated. The aqueous layer extracted with EtOAc (2 x 20 ml). The combined organics were washed with brine, dried ($Na_2SO_4$) and filtered. The filtrate was concentrated in vacuum obtaining 178 mg of crude. The crude was mixed with DCM and stirred for 30 min at room temperature, filtered and the solid was washed with DCM. The solid N'-benzoyl-4-(4-butyl-1H-1,2,3-triazol-1-yl)benzohydrazide **2c** was collected, added to the first solid and both were dried in vacuum obtaining off-white solid (67 mg, 45% yield) (Crude was directly taken to next step without further purification).

**N'-benzoyl-4-(4-butyl-1H-1,2,3-triazol-1-yl)benzohydrazide (2c).** Yield=45%; $^1H$ NMR (300 MHz, $CDCl_3$): δ 8.03 (d, 2H), 7.83 (7, 3H), 7.57-7.37 (m, 4H), 7.26 (s, 1H), 2.74 (t, 2H), 1.66 (quin, 2H), 1.37 (sx, 2H), 0.90 (t, 3H) ppm.

To a suspension of **2c**, (67 mg, 1 Eq, 0.18 mmol) in Acetonitrile (10 ml) was added Burgess reagent (0.13 g, 3 Eq, 0.55 mmol) and the mixture was heated at reflux overnight. The mixture was concentrated in vacuum. The residue was mixed with DCM (2 ml), methanol (0.4 ml) and water (2 ml) and stirred for 10 min at RT. The organic layer was removed by pipette and concentrated in vacuum to afford 62 mg of an off-white crystalline solid. The solid was dissolved in boiling ethanol (2 ml) and the mixture was allowed to cool down to room temperature with stirring. The solid was filtered off, washed with ethanol, collected with ethanol and transferred to a 4 ml vial, concentrated with a stream of nitrogen and dried at high vacuum, obtaining Compound 2 (27 mg, 41% yield).

**2-(4-(4-butyl-1H-1,2,3-triazol-1-yl)phenyl)-5-phenyl-1,3,4-oxadiazole (2).** Yield=41%; Purity=89.1%; $^1H$ NMR (300 MHz, $CDCl_3$): δ 8.30 (d, 2H), 7.95 (d, 2H), 7.57-7.54 (m, 3H), 7.26 (s, 1H), 2.83 (t, 2H), 1.75 (quin, 2H), 1.45 (sx, 2H), 0.98 (t, 3H) ppm. MS (ES+) m/z 346 [M+1], 713 [2M+23].

**Compound 3**

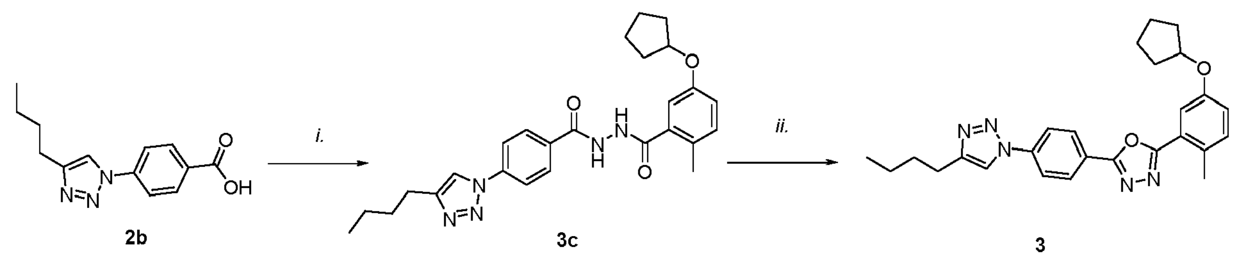


2b 3c 3

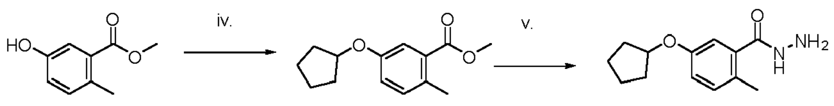


3d 3e 3f

**Reagents and conditions:** *i.* Oxalyl chloride (1.53 eq.), DMF (3 drops), 0°C to RT, 30 min, followed by 5-(cyclopentyloxy)-2-methylbenzohydrazide **3f** (1 eq.), pyridine (2.5 eq.), DCM, RT, overnight; *iii.* Burgess reagent, acetonitrile, reflux overnight; *iv.* Cyclopentyl bromide (1.3 eq.), DMF, 70°C, 24 h; v. hydrazine hydrate (3 eq.), reflux, methanol, 2 days.

**Procedure for the synthesis of compound 3:**

A mixture of compound **3d** (methyl 5-hydroxy-2-methylbenzoate, Combi-Blocks, Inc. catalogue code YC-0867) (1 g, 1 Eq, 6.02 mmol), potassium iodide (20.0 mg, 0.02 Eq, 120 μmol) and potassium carbonate (1.25 g, 1.5 Eq, 9.03 mmol) in anhydrous DMF (7 ml), heated to about 65°C is treated with of cyclopentyl bromide (Sigma-Aldrich, catalogue code C115207) (1.17 g, 839 μL, 1.3 Eq, 7.82 mmol) and heated at 70°C (external) for 24h. After cooling to RT the mixture was diluted with DCM (50 ml), washed with NaOH 2 N (3 x 25 ml), brine (25 ml), dried ($Na_2SO_4$) and concentrated in vacuum to obtain Methyl 5-(cyclopentyloxy)-2-methylbenzoate Compound **3e** as light-brown oil (1.265 g, 90% yield).
**Methyl 5-(cyclopentyloxy)-2-methylbenzoate (3e).** Yield=90%; $^1H$ NMR (300 MHz, $CDCl_3$): δ 7.42 (d, 1H), 7.12 (d, 1H), 6.92 (dd, 1H), 4.77 (quin, 1H), 3.88 (s, 3H), 2.51 (s, 3H), 1.97-1.71 (m, 6H), 1.70-1.55 (m, 2H) ppm.

Compound **3e** (1.265 g, 1 Eq, 5.4 mmol) was reacted with hydrazine hydrate (786 μL, 3 Eq, 16 mmol) in methanol (10 ml), heated at reflux overnight. After 24h only 25% conversion was observed. Then hydrazine hydrate (1 ml, 3.9 Eq, 20 mmol) was added. After 2 days reflux almost full conversion was observed. The mixture was allowed to cool down to room temperature and concentrated in vacuum to a white solid. It was stirred up in methanol, filtered, washed with small amount of methanol, air-dried obtaining hydrazide Compound **3f** as white solid (0.646 g, 51% yield).
**5-(Cyclopentyloxy)-2-methylbenzohydrazide (3f).** Yield=51%; $^1H$ NMR (300 MHz, $CDCl_3$): δ 7.27 (d, 1H), 7.11 (d, 1H), 6.88 (s, 1H), 6.86 (dd, 1H), 4.73 (quin, 1H), 2.92 (s, 2H), 2.36 (s, 3H), 1.97-1.71 (m, 6H), 1.70-1.55 (m, 2H) ppm.

Compound **2b** (4-(4-butyl-1H-1,2,3-triazol-1-yl)benzoic acid) was prepared as described above in the procedure for the synthesis of compound 2.

Compound **3c**, was obtained by a two-step synthesis. In the first step 3 drops of DMF was added to **2b** (4-(4-butyl-1H-1,2,3-triazol-1-yl)benzoic acid) (416 mg, 3 Eq, 1.70 mmol) in DCM (9 ml) cooled in ice, followed by oxalyl chloride (331 mg, 228 μL, 4.61 Eq, 2.61 mmol). The ice-bath was removed and the mixture was stirred at RT for 0.5 h. The solvent was removed in vacuum. The residue was dissolved in DCM (9 ml) and divided over 3 reaction mixtures adding 5-(cyclopentyloxy)-2-methylbenzohydrazide **3f** (132 mg, 1 Eq, 565 μmol) and pyridine (112 mg, 0.11 ml, 2.5 Eq, 1.41 mmol) in dichloromethane (2 ml) at RT. After a night stirring at RT the suspension was filtered over Celite and the pad washed with DCM. The filtrate was washed with saturated $NaHCO_3$, dried with $Na_2SO_4$ and concentrated to a brown solid, 250 mg. It was stirred up in TBME/EtOAc and partially concentrated. The resulting suspension was filtered and the solid washed with EtOAc. The filtrate was collected and concentrated to afford Compound **3c** (190 mg, 69% yield), containing residual ethyl acetate.
**N'-(4-(4-butyl-1H-1,2,3-triazol-1-yl)benzoyl)-5-(cyclopentyloxy)-2-methylbenzohydrazide (3c).** Yield=69%; $^1H$ NMR (300 MHz, $CDCl_3$): δ 10.16 (s, 1H), 8.96 (s, 1H), 8.02 (s, 2H), 7.80 (s, 2H), 7.27 (s, 1H), 7.09 (d, 1H), 6.88 (d, 1H), 6.86 (dd, 1H), 4.72 (quin, 1H), 4.12 (q, 1H), 2.82 (s, 2H), 2.39 (s, 3H), 2.03 (s, 2H), 1.96-1.33 (m, 13H), 1.25 (t, 2H), 0.98 (t, 3H) ppm.

Compound **3** (2-(4-(4-butyl-1H-1,2,3-triazol-1-yl)phenyl)-5-phenyl-1,3,4-oxadiazole) was prepared from compound **3c** (180 mg, 1 Eq, 390 μmol) adding the Burgess reagent (279 mg, 3 Eq, 1.17 mmol) in acetonitrile at reflux, overnight. Then the heating was switched off. On cooling a precipitate formed. The mixture was filtered off and the solid

washed with Acetonitrile and air-dried, Compound **3** was isolated as off-white solid (67 mg, 39% yield).

**2-(4-(4-butyl-1H-1,2,3-triazol-1-yl)phenyl)-5-phenyl-1,3,4-oxadiazole (3).** Yield=39%; Purity=98.1%; $^{1}$H NMR (300 MHz, $CDCl_3$): δ 8.29 (d, 2H), 7.97 (d, 2H), 7.57 (s, 1H), 7.27 - 7.25 (m, 3H), 6.99 (d, 1H), 4.84 (quin, 1H), 3.10-2.75 (m, 1H), 2.84 (br, 1H), 2.69 (s, 3H), 2.02-1.73 (m, 8H), 1.74-1.63 (sx, 2H), 1.57 (s, 8H), 1.52-1.25 (m, 2H), 0.98 (t, 3H) ppm. MS (ES+) m/z 346 [M+1], 713 [2M+23].

**Compound 4**

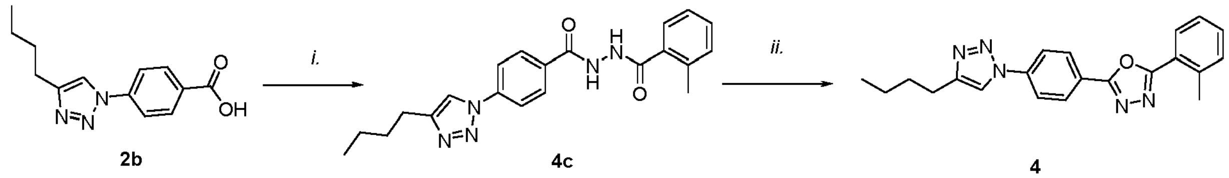


2b 4c 4

**Reagents and conditions:** i. Oxalyl chloride (1.59 eq.), DMF (1 drop), DCM, 0°C to RT, 30 min, followed by 2-methyl-benzohydrazide (1 eq.), pyridine (2.5 eq.), DCM, 0°C to RT, overnight; *ii.* Burgess reagent (3 eq.), acetonitrile, reflux, overnight.

**Procedure for the synthesis of compound 4:**

Compound **2b** (4-(4-butyl-1H-1,2,3-triazol-1-yl)benzoic acid) was prepared as described above in the procedure for the synthesis of compound **2.**

Compound **4c** was obtained by a two-step procedure. In the first step a drop of DMF was added to **2b** (4-(4-butyl-1H-1,2,3-triazol-1-yl)benzoic acid) (125 mg, 1 Eq, 510 μmol) and oxalyl chloride (103 mg, 71.0 μL, 1.59 Eq, 812 μmol) in DCM (5 ml) at 0°C. The ice-bath was removed and the mixture was stirred at RT for 30 min to afford a yellow solution and then concentrated in vacuum, dissolved in DCM (2.5 ml) added to a solution of 2-methylbenzohydrazide (Combi-Blocks, catalogue code OR-0369) (76.5 mg, 1 Eq, 510 μmol) and pyridine (101 mg, 0.10 ml, 2.5 Eq, 1.27 mmol) in DCM (3 ml) at 0°C. The ice-bath was removed and the mixture was allowed to reach RT overnight. The suspension was filtered (plastic filter) and the solid washed with cold DCM (slow filtration). The solid N'-(4-(4-butyl-1H-1,2,3-triazol-1-yl)benzoyl)-2-methylbenzohydrazide Compound **4c** was air-dried (89 mg, 46% yield).

**N'-(4-(4-butyl-1H-1,2,3-triazol-1-yl)benzoyl)-2-methylbenzohydrazide (4c).** Yield=46%; $^{1}$H NMR (300 MHz, $CDCl_3$): δ 8.03 (d, 2H), 7.83 (d, 2H),7.80 (d, 1H), 7.50 (d, 1H), 7.34 (t, 1H), 7.27 (s, 1H), 7.21 (t, 1H), 2.94 (s, 4H), 2.77 (t, 2H), 2.46 (s, 3H), 1.68 (quin, 2H), 1.39 (sx, 2H), 0.92 (t, 3H) ppm. MS (ES+) m/z 378 [M+1].

Compound **4** was prepared from compound **4c,** (89 mg, 1 Eq, 0.24 mmol) in Acetonitrile (10 ml) was added Burgess reagent (0.17 g, 3.0 Eq, 0.71 mmol) and the mixture was heated at reflux, overnight. The organic layer was removed, the aqueous washed with DCM (2 x 20 ml) and the combined organic layers were concentrated in vacuum obtaining a white solid, 87 mg. Ethanol 15 ml was added and the mixture was heated until all dissolved and allowed to cool down to RT with stirring. Filtered, washed with ethanol, air-dried a white solid have been obtained, NMR showed ca. 15% of isomer. The solid was collected with DCM and concentrated. The filtrate from ethanol mixture was concentrated as well, obtaining a white solid, 37 mg. Both aliquots were purified by automated chromatography (0-2% MeOH/DCM gradient) and combined obtaining Compound **4** as white solid (22 mg, 29% yield).

**2-(4-(4-butyl-1H-1,2,3-triazol-1-yl)phenyl)-5-(o-tolyl)-1,3,4-oxadiazole (4).** Yield=29%; Purity=97.3%; $^{1}$H NMR (300 MHz, $CDCl_3$): δ 8.27 (d, 2H), 8.06 (d, 1H), 7.94 (d, 2H), 7.81 (s, 1H), 7.50-7.33 (m, 3H), 7.23 (s, 2H), 2.89-2.72 (m, 5H), 1.71 (quin, 2H), 1.55 (s, 4H), 1.44 (sx, 2H), 0.98 (t, 3H) ppm. MS (ES+) m/z 360 [M+1], 741 [2M+23].

**Compound 5**

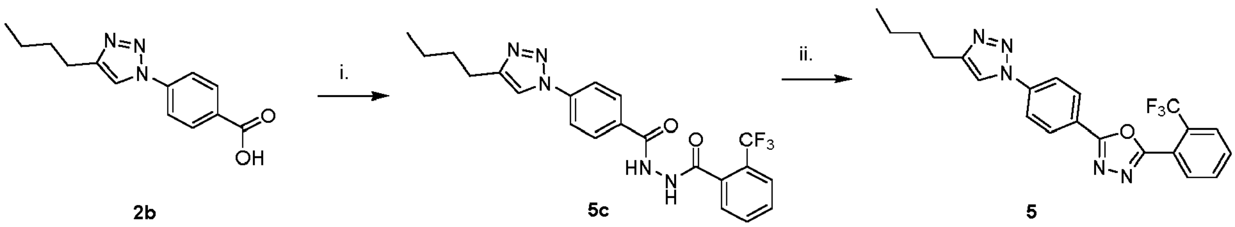


2b 5c 5

**Reagents and conditions:** *i.* Oxalyl chloride (1.59 eq.), DMF (1 drop), DCM, 0°C to RT, 30 min, followed by 2-(Trifluoromethyl)benzoic acid hydrazide (1 eq.), pyridine (2.5 eq.), DCM, 0°C to RT, overnight; *ii.* Burgess reagent (3 eq.), acetonitrile, reflux, overnight.

**Procedure for the synthesis of compound 5:**

Compound **2b** (4-(4-butyl-1H-1,2,3-triazol-1-yl)benzoic acid) was prepared as described above in the procedure for the synthesis of compound **2.**

Compound **5c** was obtained by a two-step procedure. In the first step a drop of DMF was added to **2b** (46 mg, 1 Eq, 0.188 mmol) and oxalyl chloride (32 μL, 2 Eq, 0.376 mmol) in DCM (2 ml) at 0°C and stirred for 30 min at RT during which time it became homogeneous. The volatile compounds were concentrated under reduced pressure to afford a sticky solid. The latter was taken up in DCM (4 ml) and added at RT to a solution of compound 2-(trifluoromethyl)benzoic acid hydrazide (Combi-Blocks, catalogue code QA-6000), (38.4 mg, 1 Eq, 0.188 mmol), pyridine (0.05 ml, 3 Eq, 0.564 mmol) in DCM (4 ml) at 0°C. The reaction mixture was stirred overnight at RT. The resulting suspension was filtered (plastic filter) and the solid washed with cold DCM. The solid was air-dried, transferred to a flask with DCM and concentrated to a white solid Compound **5c** (81 mg, 57% yield). **N'-(4-(4-butyl-1H-1,2,3-triazol-1-yl)benzoyl)-2-(trifluoromethyl)benzohydrazide (5c).** MS (ES+) m/z 432 [M+1].

Compound **5** was prepared from compound **5c** (81 mg, 1 Eq, 0.188 mmol) adding Burgess Reagent (134 mg, 3 Eq, 0.564 mmol) in refluxing acetonitrile, overnight. The reaction mixture was concentrated to dryness, coated on hydromatrix and purified by automated silica gel chromatography (0-2% MeOH/DCM gradient) Compound **5** was isolated, 35mg, 45% yield.

**2-(4-(4-butyl-1H-1,2,3-triazol-1-yl)phenyl)-5-(2-(trifluoromethyl)phenyl-1,3,4-oxadiazole (5).** Yield=45%; Purity=90.3%; $^1H$ NMR (300 MHz, $CDCl_3$): δ 8.23 (t, 2H), 8.21 (d, 1H), 7.99-7.88 (m, 3H), 7.82 (s, 1H), 7.76-7.67 (m, 2H), 2.82 (t, 2H), 1.76 (quin, 2H), 1.26 (sx, 2H), 0.98 (t, 3H) ppm. MS (ES+) m/z 414 [M+1].

**Compound 6**

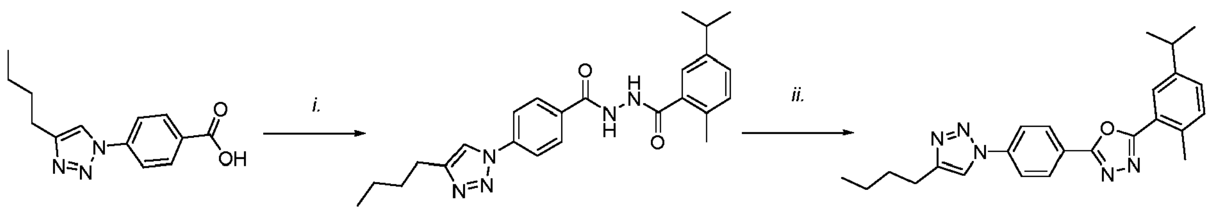


2b 6c 6

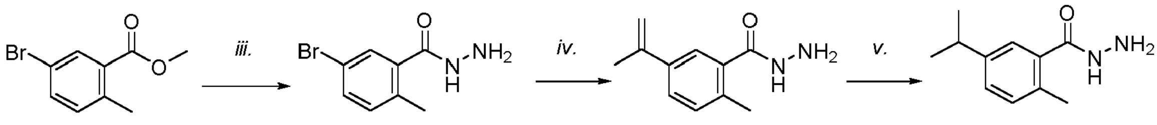


6d 6e 6f 6g

**Reagents and conditions:** *i.* Oxalyl chloride (1.3 eq.), DMF (1 drop), DCM, 0°C to RT, 30 min, followed by 5-isopropyl-2-methyl benzohydrazide **6g** (1 eq.), pyridine (2.5 eq.), DCM, RT, overnight; *ii.* $POCl_3$ (10 eq.), toluene, reflux,

4h; *iii*. Hydrazine hydrate (4.7 eq.), methanol, reflux, over the weekend; i*v*. Suzuki coupling: 4,4,5,5-tetramethyl-2-(prop-1-en-2-yl)-1,3,2-dioxaborolane (1.2 eq.), $K_2CO_3$ (3.0 eq.), $PdCl_2$(dppf)-$CH_2Cl_2$ (0.026 eq.), 1,4-dioxane:water (5:1, v:v), stirred ca. 90°C, overnight; *v*. hydrogen, Pd/C (point spatula), methanol, RT, over the weekend.

**Procedure for the synthesis of compound 6:**

Compound **6e** was obtained by reaction of (2.5 g, 1 Eq, 10.914 mmol) of **6d** (methyl 5-bromo-2-ethylbenzoate, Combi-Blocks catalogue code OS-1093) with hydrazine hydrate (2.6 g, 2.5 ml, 4.7 Eq, 52 mmol) in methanol (25 ml), heated at reflux over the weekend. The white suspension was filtered (P3 filter) and washed with methanol, TBME and air-dried. Obtaining Compound **6e,** as white solid (1.944 g, 78% yields).

**5-Bromo-2-methylbenzohydrazide (6e).** Yield=78%; $^1$H NMR (300 MHz, $CDCl_3$): δ 9.49 (s, 1H), 7.51 (dd, 1H), 7.41 (d, 1H), 7.20 (d, 1H), 4.44 (s, 2H), 3.31 (s, 4H), 2.49 (t, 8H), 2.26 (s, 3H) ppm.

Compound **6f,** was obtained by Suzuki coupling of 500 mg (1 Eq, 2.18 mmol) of compound **6e,** with 4,4,5,5-tetramethyl-2-(prop-1-en-2-yl)-1,3,2-dioxaborolane (Sigma Aldrich catalogue code 663212) (440 mg, 492 μL, 1.2 Eq, 2.62 mmol) and potassium carbonate (905 mg, 3.0 Eq, 6.55 mmol) in 1,4-dioxane (6 ml) and water (1.2 ml), the mixture was degassed with nitrogen. $PdCl_2$(dppf)-$CH_2Cl_2$ adduct (46 mg, 0.026 Eq, 56 μmol) was added and the opening was rinsed with a little dioxane. The mixture was degassed once again (5 min) with nitrogen and placed in a 110°C sand-bath, heated to 100°C and stirred overnight at ca. 90°C internal temperature. The mixture was allowed to cool down to RT and concentrated in vacuum. The residue was partitioned between DCM and water. The organic layer was dried with $Na_2SO_4$ and concentrated in vacuum to a red oil (750 mg), solidifying at RT. The solid was mixed with DCM (ca. 5 ml), filtered off and the white solid washed with a little DCM and discarded. The filtrate was concentrated in vacuum (450 mg) and purified by chromatography ($SiO_2$, 0-5% MeOH/DCM gradient) affording to product, white solid, as a mixture of both starting material and product Compound **6f,** (231 mg, ratio 0.4:1.0, 36 % corrected yield).

**2-Methyl-5-(prop-1-en-2-yl)benzohydrazide (6f).** Yield=36%; $^1$H NMR (300 MHz, $CDCl_3$): δ 7.44-7.35 (m, 2H), 7.13 (d, 1H), 5.30 (s, 1H), 5.03 (s, 1H), 3.13 (s, 4H), 2.36 (s, 3H), 2.31 (s, 1H), 2.08 (s, 3H) ppm.

Compound **6g,** was obtained by hydrogenation of mixture of **6e** and **6f** previously obtained (231 mg, 1 Eq, 0.867 mmol) with Pd/C (point spatula) in methanol (10 ml), in a round-bottom flask with a rubber septum. The reaction mixture was stirred applying vacuum to the mixture and subsequently adding hydrogen, the previous two steps was repeated for three times. The mixture was stirred at RT over the weekend. The crude was filtered on a Celite pad, and washed with methanol. The filtrate was concentrated, 300 mg, yellow oil. Automated chromatography ($SiO_2$, 0-5% MeOH/DCM): afforded a light-orange wax (100 mg, 60% yield), which could be the product based on the NMR spectrum. HPLC did not show any identifiable mass. The subsequent step was successful, confirming the identity of the product Compound **6g.**

**5-Isopropyl-2-methylbenzohydrazide (6g).** $^1$H NMR (300 MHz, $CDCl_3$): δ 7.31-7.11 (m, 4H), 4.89 (s, 2H), 2.86 (sept, 1H), 2.38 (s, 3H), 1.21 (d, 6H) ppm.

Compound **2b** (4-(4-butyl-1H-1,2,3-triazol-1-yl)benzoic acid) was prepared as described above in the procedure for the synthesis of compound **2.**

Compound **6c,** was obtained by a two-step procedure. In the first step a drop of DMF was added to **2b** (128 mg, 1 Eq, 520 μmol) and oxalyl chloride (85.8 mg, 59.2 μL, 1.3 Eq, 676 μmol) in DCM (3 ml) at 0°C. The ice-bath was removed and the mixture was stirred at RT for 0.5 h. The solvent was removed in vacuum. The residue was mixed with DCM (3 ml) and added to **6g** (3.6, 100 mg, 1 Eq, 520 μmol) and pyridine (103 mg, 0.10 ml, 2.5 Eq, 1.30 mmol) in dichloromethane (2 ml) cooled in ice. The ice-bath was removed and the mixture stirred at RT overnight. The mixture was filtered over Celite, washed with DCM (20 ml). The organic filtrate was washed with saturated $NaHCO_3$ (20 ml), brine, dried with $Na_2SO_4$ and concentrated obtaining a brown oil (334 mg). TBME was added, finally to give a suspension with little precipitate. The mixture was concentrated and mixed with TBME (2-3 ml), filtered and the solid washed with TBME and air-dried (25 mg, 12% yield).

**N'-(4-(4-butyl-1H-1,2,3-triazol-1-yl)benzoyl)-5-isopropyl-2-methylbenzo hydrazide (6c).** Yield=12%; Purity=82-84%; $^1$H NMR (300 MHz, $CDCl_3$): δ 8.76 (m, 1H), 8.40-7.87 (m, 4H), 7.43 (s, 1H), 7.35-7.20 (m, 5H), 2.93 (sept, 1H), 2.80 (t, 2H), 2.51 (s, 3H), 1.71 (quin, 2H), 1.64-1.39 (m, 2H), 1.27 (d, 6H) (1.04 (t, 3H) ppm. MS (ES+) m/z 420 [M+1].

Compound 6 was prepared from compound **6c** (26 mg, 1 Eq, 62 μmol) in Toluene (5 ml) was added phosphorus oxychloride (95 mg, 58 μL, 10 Eq, 0.62 mmol) and the mixture was heated at reflux for 4 h. The mixture was concentrated in vacuum. DCM (2 ml) was added, followed by IN NaOH (2 ml). The layers were transferred to a test-tube, with additional DCM (ca. 2 ml) and stirred for 30 min at RT. The organic layer was removed with a pipette. The aqueous layer was extracted with DCM (2 x 20 ml), the organics were dried ($Na_2SO_4$) and concentrated in vacuum, obtaining a light-yellow solid (39 mg). The NMR tube solution was put back in the flask, the whole was concentrated. To the residue ethanol (2 ml) was added and the mixture was heated until almost all solid had dissolved. It was allowed to cool down to RT. The solid was filtered off, air-dried. It was transferred to a vial. Both NMR as HPLC solutions showed very small undissolved particles. The NMR solution was used to dissolve the solid and it was filtered over a microfilter, washing with DCM. The

filtrate was concentrated with a stream of nitrogen, then at high vacuum to afford Compound **6,** a white solid (13.6 mg, 55% yield).

**2-(4-(4-butyl-1H-1,2,3-triazol-1-yl)phenyl)-5-(5-isopropyl-2-methylphenyl)-1,3,4-oxadiazole (6).** Yield=55%; Purity=92%; $^1$H NMR (300 MHz, $CDCl_3$): δ 8.29 (d, 2H), 7.89 (d, 2H), 7.85 (s, 1H), 7.77 (s, 1H), 7.34 (d, 2H), 7.27 (d, 2H), 3.00 (sept, 1H), 2.80 (t, 2H), 2.69 (s, 3H), 1.71 (quin, 2H), 1.53 (s, 3H), 1.44 (sx, 2H), 1.30 (d, 6H), 0.98 (t, 3H) ppm. MS (ES+) m/z 360 [M+1], 741 [2M+23].

**Compound 7**

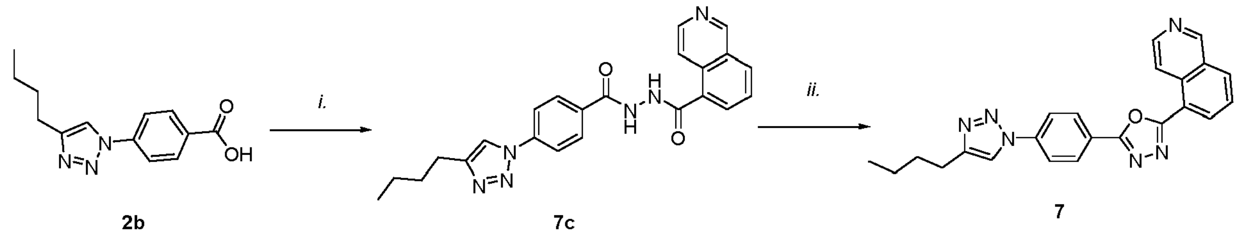


**2b** **7c** **7**

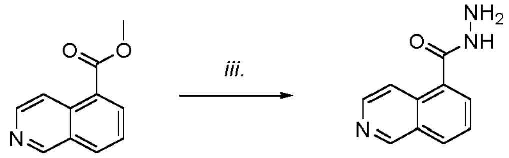


**7d** **7e**

**Reagents and conditions:** *i.* Oxalyl chloride (2 eq.), DMF (3 drops), DCM, 0°C to RT, 0.5h, followed by isoquinoline-5-carbohydrazide, **7e** (1 eq.), pyridine (2.5 eq.), DCM, RT, stirring overnight; *ii.* $POCl_3$ (5.8 Eq.) in toluene, reflux, overnight; *iii.* Hydrazine hydrate (3 eq.), reflux, methanol, overnight.

**Procedure for the synthesis of compound 7:**

The hydrazide **7e** was prepared from ester **7d** (Methyl isoquinoline-5-carboxylate, Combi-Blocks catalogue code QB-2477) (1 g, 1 Eq, 5.342 mmol) adding hydrazine hydrate (802 mg, 778 μL, 3 Eq, 16.0 mmol), in methanol (10 ml), at reflux, 80°C, overnight, with full conversion. The solid was isolated by filtration and washed with water (5 ml) and methanol (10 ml). EtOAc (ca. 10 ml) was added and the suspension was stirred at RT for 10 min, filtered and the white solid was washed with EtOAc and dried at high vacuum, Compound 7e isolated as off-white solid, with trace of EtOAc (866 mg, 88% yield).

**Isoquinoline-5-carbohydrazide** (7e). Yield=88%; $^1$H NMR (300 MHz, $CDCl_3$): δ 9.36 (s, 1H), 8.54 (d, 1H), 8.21 (dd, 1H), 8.10 (dd, 1H), 7.85 (dd, 1H), 7.70 (t, 1H), 4.61 (s, 2H), 3.31 (s, 3H), 2.49 (s, 3H) ppm.

Compound 2b (4-(4-butyl-1H-1,2,3-triazol-1-yl)benzoic acid) was prepared as described above in the procedure for the synthesis of compound 2.

Compound **7c** was obtained by a two-step procedure. In the first step 3 drops of DMF was added to **2b** (416 mg, 3 Eq, 1.70 mmol) and oxalyl chloride (331 mg, 228 μL, 4.61 Eq, 2.61 mmol) in DCM (9 ml) cooled in ice.
The ice-bath was removed and the mixture was stirred at RT for 0.5 h. The solvent was removed in vacuum. The residue was dissolved in DCM (9 ml) and divided in 3 portions and one portion was added to **7e** (106 mg, 1 Eq, 565 μmol) and pyridine (112 mg, 0.11 ml, 2.5 Eq, 1.41 mmol) in DCM (2 ml) at RT. The mixture was stirred at RT overnight. The suspension was filtered off, washed with DCM. The residue was mixed with DCM and transferred to a flask and concentrated to afford Compound **7c** a light-brown solid (192 mg, 82% yield).

**N'-(4-(4-butyl-1H-1,2,3-triazol-1-yl)benzoyl)isoquinoline-5-carbohydrazide (7c).** Yield=88%; $^1$H NMR (300 MHz, $CDCl_3$): δ 10.81 (s, 1H), 9.76 (s, 1H), 8.74 (d, 1H), 8.71 (s, 1H), 8.61 (d, 1H), 8.54 (d, 1H), 8.25 (d, 1H), 8.18 (d, 2H), 8.13 (d, 2H), 8.09 (dd, 1H), 7.99 (t, 2H), 1.66 (quin, 2H), 1.38 (sx, 2H), 0.93 (t, 3H) ppm. MS (ES+) m/z 413 [M-1].

Compound **7** was prepared from compound **7c** (192 mg, 1 Eq, 463 μmol), with $POCl_3$ (0.41 g, 0.25 ml, 5.8 Eq, 2.7 mmol) in toluene (5 ml), at reflux overnight. The mixture was allowed to cool down to RT. It was partitioned between IN NaOH (55 ml) and DCM (50 ml). The aqueous layer was extracted with DCM (25 ml). The combined organics were washed with brine, dried with $Na_2SO_4$ and concentrated, orange-yellow solid (173 mg). Several manipulations with the solid were carried out (stirring up in solvents: methanol, DCM, acetonitrile), but the product remained insufficiently pure.

All fractions were combined with DCM/MeOH and impregnated on hydromatrix. The material was purified over silica (0-2% MeOH/DCM gradient). The most intense pure fractions (#3) were concentrated to obtain Compound **7** as white solid (41 mg, 22% yield).

**2-(4-(4-butyl-1H-1,2,3-triazol-1-yl)phenyl)-5-(isoquinolin-5-yl)-1,3,4-oxadiazole (7).** Yield=22%; Purity=93.1%; $^1$H NMR(300 MHz, $CDCl_3$): δ 9.50 (s, 1H), 9.06 (d, 1H), 8.82-8.70 (m, 3H), 8.49-8.36 (m, 3H), 8.19 (d, 2H), 7.95 (t, 1H), 3.30 (s, 16H), 2.76 (t, 2H), 2.49 (s, 82H), 1.68 (quin, 2H), 1.38 (sx, 2H), (0.93 (t, 3H) ppm. MS (ES+) m/z 397 [M+1], 815.0 [2M+23].

**Compound 8**

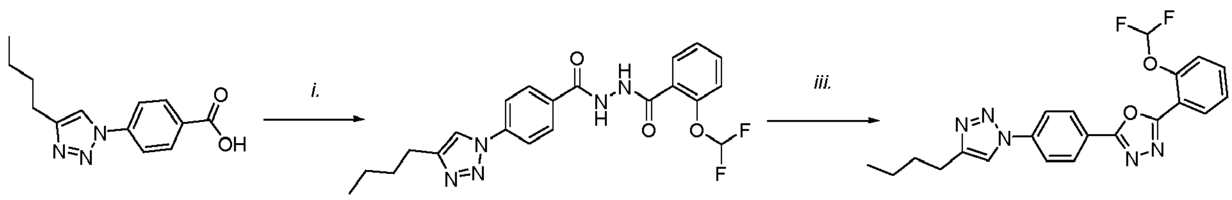


2b 8c 8

**Reagents and conditions:** *i.* Oxalyl chloride (4.61 eq.), DMF (3 drops), DCM, 0°C to RT, 0.5h, followed by 2-(difluoromethoxy)-benzohydrazide (1 eq.), pyridine (2.5 eq.), DCM, RT, overnight; *ii.* Burgess reagent (3 eq.), acetonitrile, reflux, overnight.

**Procedure for the synthesis of compound 8:**

Compound **2b** (4-(4-butyl-1H-1,2,3-triazol-1-yl)benzoic acid) was prepared as described above in the procedure for the synthesis of compound **2.**

Compound **8c** was obtained by a two-step procedure. In the first step 3 drops of DMF was added to **2b** (416 mg, 3 Eq, 1.70 mmol) and oxalyl chloride (331 mg, 228 μL, 4.61 Eq, 2.61 mmol) in DCM (9 ml) cooled in ice bath. The ice bath was removed and the mixture was stirred at RT for 0.5 h. The solvent was removed in vacuum. The residue was dissolved in dichloromethane (9 ml) and divided in 3 portions. To one portion was added to 2-(difluoromethoxy)benzohydrazide (ENAMINE Ltd. Catalogue code EN300-228518), (114 mg, 1 Eq, 565 μmol) and pyridine (112 mg, 0.11 ml, 2.5 Eq, 1.41 mmol) in DCM (2 ml) at RT and stirred, overnight. It was filtered over Celite, washed with DCM. The filtrate was concentrated in vacuum, partitioned between EtOAc (10 ml) and water saturated with $NaHCO_3$ (10 ml). The aqueous layer was washed with EtOAc. The combined organic layers were washed with brine, dried with $Na_2SO_4$ and concentrated in vacuum obtaining a crude yellow solid/wax, containing Compound **8c** (216 mg) as shown by NMR (Crude was directly taken to next step without further purification).

**N'-(4-(4-butyl-1H-1,2,3-triazol-1-yl)benzoyl)-2-(difluoromethoxy)benzohydrazide (8c).** $^1$H NMR (300 MHz, $CDCl_3$): δ 8.34 (m, 4H), 8.12 (dt, 1H), 7.66 (dt, 1H), 7.32 (dt, 1H), 7.27 (s, 4H), 2.56 (s, 3H), 1.83 (quin, 2H), 1.47 (sx, 2H), (0.99 (t, 3H) ppm.

Compound **8** was prepared from compound **8c** (216 mg, 1 Eq, 503 μmol) in Acetonitrile (20 ml) adding Burgess reagent (360 mg, 3 Eq, 1.51 mmol) and the reaction mixture was heated at reflux overnight. Incomplete conversion was observed by HPLC analysis. Additional Burgess reagent (150 mg, 1.25 Eq, 0.63 mmol) was added and the mixture was heated at reflux overnight. Conversion was still incomplete, but sufficient for the target amount. The mixture was concentrated. The residue was taken up in DCM (8 ml), MeOH (4 ml) and water (8 ml). The organic layer was removed and concentrated in vacuum obtaining a light-yellow solid (243 mg). The solid was mixed with ethanol (10 ml) and heated until all dissolved. After cooling to RT the suspension was filtered and the solid washed with ethanol and air-dried. NMR and HPLC samples were prepared, but the solutions remained faintly turbid. The solid was dissolved in DCM/$CDCl_3$ and filtered over a microfilter. The green/brown hue remained in the filter and the filtrate was almost colorless. It was concentrated by nitrogen stream and then at high vacuum affording Compound **8** a white solid (49 mg, 24% yield).

**2-(4-(4-butyl-1H-1,2,3-triazol-1-yl)phenyl)-5-(2-(difluoromethoxy)phenyl)-1,3,4-oxadiazole (8).** Yield=24%; Purity=95.8%; $^1$H NMR (300 MHz, $CDCl_3$): δ 8.29 (d, 2H), 8.21 (d, 1H), 7.89 (d, 2H), 7.77 (s, 1H), 7.57 (t, 1H), 7.45-7.34 (m, 2H), 7.26 (s, 4H), 6.67 (t, 1H, j=6.9 Hz), 2.84 (t, 2H), 1.76 (quin, 2H), 1.55 (s, 9H), 1.43 (sx, 2H), (0.97 (t, 3H) ppm. MS (ES+) m/z 412 [M+1]

**Compound 9**

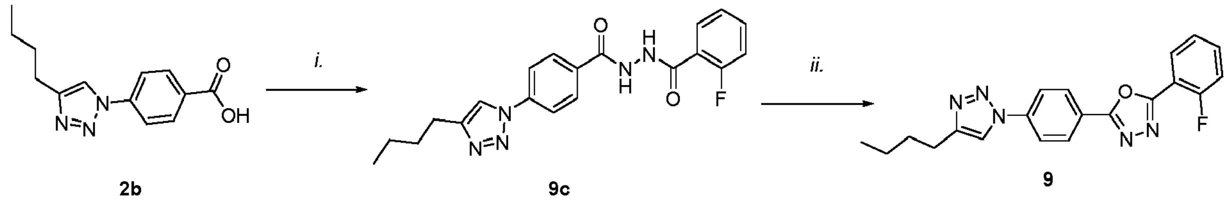


2b 9c 9

**Reagents and conditions:** *i.* Oxalyl chloride (1.59 eq.), DMF (1 drop), DCM, 0°C to RT, 0.5h, followed by o-fluoro-benzohydrazide (1 eq.), pyridine (2.5 eq.), DCM, RT, overnight; *ii.* Burgess reagent, acetonitrile, reflux 90°C, 3 h.

**Procedure for the synthesis of compound 9:**

Compound **2b** (4-(4-butyl-1H-1,2,3-triazol-1-yl)benzoic acid) was prepared as described above in the procedure for the synthesis of compound **2.**

Compound **9c** was obtained by a two-step procedure. In the first step a drop of DMF was added to a suspension of **2b** (125 mg, 1 Eq, 510 μmol) in DCM (5 ml) at 0°C, followed by oxalyl chloride (103 mg, 71.0 μL, 1.59 Eq, 812 μmol). The ice bath was removed and the mixture was stirred at RT for 0.5 h to afford a yellow solution and then concentrated in vacuum, dissolved in DCM (2.5 ml) added to a solution of 2-fluorobenzohydrazide (ENAMINE Ltd. catalogue code EN300-03671) (78.6 mg, 1 Eq, 510 μmol) and pyridine (101 mg, 0.10 ml, 2.5 Eq, 1.27 mmol) in DCM (3 ml) at 0°C. The ice bath was removed and the mixture was allowed to reach RT overnight. The resulting suspension was filtered (plastic filter) and the solid washed with cold DCM. The solid was air dried, transferred to a flask with DCM and concentrated to afford Compound **9c** as white solid (109 mg, 56% yield). NMR identifies the regioisomer as main impurity (Crude was directly taken to next step without further purification).

**N'-(4-(4-butyl-1H-1,2,3-triazol-1-yl)benzoyl)-2-fluorobenzohydrazide (9c).** Yield=56%; Purity=81-82%; $^1$H NMR (300 MHz, $CDCl_3$): δ 8.34 (d, 2H), 8.04 (d, 2H), 7.98 (dt, 2H), 7.88 (s, 1H), 7.82 (d, 2H), 7.49 (dt, 1H), 7.30-7.21 (m, 3H), 7.14 (dt, 1H), 3.44 (s, 12H), 3.29 (quin, 1H), 2.74 (t, 2H), 1.66 (quin, 2H), 1.37 (sx, 2H), (0.89 (t, 3H) ppm. MS (ES+) m/z 382 [M+1].

Compound **9** was prepared from a suspension of **9c** (109 mg, 1 Eq, 286 μmol) in Acetonitrile (10 ml) adding Burgess reagent (204 mg, 3 Eq, 857 μmol) and the mixture was heated at reflux overnight. HPLC analysis showed full conversion. The mixture was concentrated in vacuum. The residue was taken up in DCM (4 ml), MeOH (2 ml) and water (4 ml). The organic layer was removed, the aqueous washed with DCM and the combined organic layers were concentrated in vacuum obtaining a white solid (140 mg). The solid was heated in ethanol (8 ml) to allow almost full dissolution. The mixture was allowed to cool down to RT, filtered and the solid was washed with ethanol, affording an off-white solid (70 mg). The latter almost all dissolved in boiling ethanol (ca. 8 ml). Allowed to cool down to RT over the weekend. The filtrate was concentrated to obtain an off-white solid (17 mg). The previous solid filtered off was washed with ethanol (ca. 50 ml) and the filtrate was concentrated to afford a white solid (41 mg). The two aliquots (17+41 mg) were purified by automated chromatography $SiO_2$ (0-2% MeOH/DCM gradient). Fractions 9-10 + 13-14 were combined to afford Compound 9 (15.1 mg, 16% yield).

**2-(4-(4-butyl-1H-1,2,3-triazol-1-yl)phenyl)-5-(2-fluorophenyl)-1,3,4-oxadiazole (9).** Yield=16%; Purity=92.5%; $^1$H NMR (300 MHz, $CDCl_3$): δ 8.34 (d, 2H), 8.18 (t, 1H), 7.89 (d, 2H), 7.76 (s, 1H), 7.56 (d, 1H), 7.37-7.29 (m, 2H), 7.24 (s, 4H), 2.78 (t, 2H), 1.79 (quin, 2H), 1.54 (s, 7H), 1.42 (sx, 2H), (0.98 (t, 3H) ppm. MS (ES+) m/z 364 [M+1]

**Compounds 10, 11**

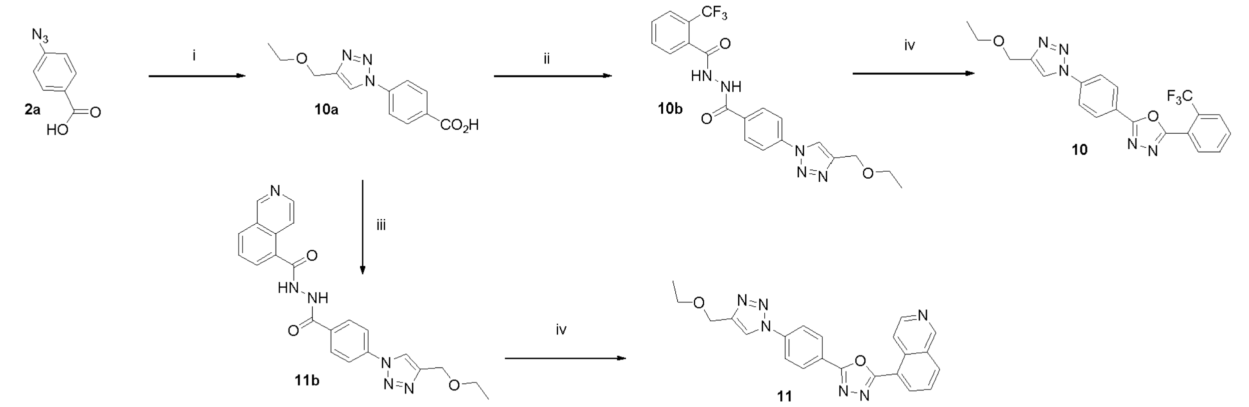


**Reagents and conditions:** *i.* $CuSO_4.5H_2O$ (0.5 eq.), Sodium Ascorbate (0.5 eq.), 3-ethoxyprop-1-yne (1.5 eq.), $H_2O$:t-BuOH (1:1 v/v), MW 120°C, 20 min; *ii.* oxalyl chloride (2 eq.), DMF (1 drop), DCM, 0°C to RT, 50 min, followed by 2-(trifluoromethyl)benzohydrazide (1 eq.), pyridine (3eq.), DMAP (point spatula), DCM, RT, overnight; *iii.* Oxalyl chloride (2 eq.), DCM, 0°C to RT, 50 min, followed by isoquinoline-5-carbohydrazide **(7e)** (1 eq.), pyridine (3 eq.), DMAP (point spatula), DCM, RT, 5h; *iv.* Burgess reagent (3 eq.), acetonitrile, reflux, 90°C, 2.5h.

**Procedure for the synthesis of compound 10a:**

In a microwave tube equipped with a small stirring bar were successively added: **2a** (104.0 mg, 1 Eq, 0.6375 mmol), water (1 ml), t-BuOH (1 ml), 3-ethoxyprop-1-yne (0.1 ml, 1.5 Eq, 0.9563 mmol), copper (II) sulfate pentahydrate (79.6 mg, 0.5 Eq, 0.3187 mmol) and sodium ascorbate (63.15 mg, 0.5 Eq, 0.3188 mmol). The tube was sealed and then submitted to irradiation: 1 min pre-stirring, then 120°C for 20 mins. After cooling to RT, the heterogeneous red mixture was poured in a mixture of EtOAc/water (1:1, v:v). The aqueous layer was further extracted with EtOAc (3 x 20 ml). The organic layers were gathered and washed with brine (1 x). The organic layers were dried over $Na_2SO_4$, filtered and concentrated to afford a "wet" yellow solid. The solids were further washed with heptanes and collected by filtration over a glass filter. After drying pure Compound **10a** was isolated as a mustard yellow solid (144 mg, 91.4% yield).
**4-(4-(ethoxymethyl)-1H-1,2,3-triazol-1-yl)benzoic acid (10a).** Yield=91.4%; $^1H$ NMR (300 MHz, DMSO D-6): $\delta$ 13.16 (s, 1H), 8.88 (s, 1H), 8.10 (d, 2H), 8.03 (d, 2H), 4.57 (s, 2H), 3.53 (q, 2H), 1.15 (t, 3H) ppm.

**Procedure for the synthesis of compound 10b:**

In a first step a drop of DMF was added to a heterogeneous mixture of **10a** (25 mg, 1 Eq, 0.202 mmol) and oxalyl chloride (35.5 $\mu$L, 2 Eq, 0.404 mmol) in DCM (2 ml) at 0°C. The ice bath was removed and the reaction mixture was stirred for 50 min at RT, during which time it became homogeneous. The volatile compounds were concentrated under reduced pressure to afford a yellow sticky solid. The latter was taken up in DCM (4 ml) and added at RT to a solution of 2-(trifluoromethyl)benzohydrazide (Combi-Blocks Inc. catalogue code QA-6000) (41.2 mg, 1 Eq, 0.202 mmol), pyridine (0.05 ml, 3 Eq, 0.606 mmol) and DMAP (point spatula) in DCM (4 ml). The reaction mixture was stirred overnight at RT during which time it turned from homogeneous to heterogeneous (within 2h). The latter was diluted with EtOAc and washed with saturated aqueous sodium bicarbonate. The aqueous layer was further extracted with EtOAc (2 x). The organic layers were washed with brine, dried over $Na_2SO_4$, filtered and concentrated to a brown solid. The latter was triturated with heptanes; the solids were collected on a glass filter, washed with heptanes and dried under reduced pressure to afford Compound **10b** as a tan solid (63.6 mg, 72.6% yield).
**N'-(4-(4-(Ethoxymethyl)-1H-1,2,3-triazol-1-yl)benzoyl)-2-(trifluoromethyl) benzohydrazide (10b)**. Yield=72.6; Purity=62%; $^1H$ NMR (300 MHz, $CDCl_3$): $\delta$ 8.07 (s, 1H), 7.99 (d, 2H), 7.81 (d, 2H), 7.78-7.68 (m, 3H), 7.68-7.57 (m, 1H), 4.73(s, 2H), 3.66 (q, 2H), 1.28 (t, 3H) ppm. MS (ES+) m/z 434 [M+1]

**Procedure for the synthesis of compound 10:**

Burgess reagent (105 mg, 3 Eq, 0.44 mmol) was added to a solution of **10b** (63.6 mg, 1 Eq, 147 $\mu$mol) in ACN (5 ml) and the reaction mixture was refluxed (90°C ext.) for 2.5 h. The reaction mixture was concentrated to dryness, coated on hydromatrix and purified by automated silica gel chromatography using a gradient from 2.5 to 7.5% MeOH in DCM. Fractions 2 and 3 contained the product but fraction 3 was also contaminated with a lower response factor impurity. Finally, fraction 2 was concentrated to a white solid (22 mg, batch A) was isolated (pure product). Fraction 3 was concentrated to afford a second batch (32 mg, batch B) which was re-purified by automated silica gel chromatography

using a gradient from 2.5 to 7.5% MeOH in DCM. Compound **10** was obtained as white solid (54 mg, 88.6% yield).
**2-(4-(4-(Ethoxymethyl)-1H-1,2,3-triazol-1-yl)phenyl)-5-(2-(trifluoromethyl)phenyl)-1,3,4-oxadiazole (10).** Yield=88.6%; Purity=96.3%; $^1$H NMR (300 MHz, $CDCl_3$): δ 8.31 (d, 2H), 8.23 (d, 1H), 8.09 (s, 1H), 8.02-7.89 (m, 3H), 7.81-7.70 (m, 2H), 4.75 (s, 2H), 4.68 (q, 2H), 1.28 (t, 3H) ppm. MS (ES+) m/z 416 [M+1]

**Procedure for the synthesis of compound 11b:**

First a drop of DMF was added to a heterogeneous mixture of **10a** (50.6 mg, 1 Eq, 0.204 mmol) and then oxalyl chloride (36 μL, 2 Eq, 0.408 mmol) in DCM (2 ml) at 0°C. The ice bath was removed and the reaction mixture was stirred for 50 minutes at RT during which time it became homogeneous. The volatile compounds were concentrated under reduced pressure to afford a yellow sticky solid. The latter was taken up in DCM (2.5 ml) and added at RT to a mixture of **7e** (38.4 mg, 1 Eq, 0.204 mmol), pyridine (0.05 ml, 3 Eq, 0.614 mmol) and DMAP (point spatula) in DCM (2.5 ml). The reaction mixture was stirred for 5 h at RT during which time it turned from homogeneous to heterogeneous. The mixture was diluted with EtOAc (not very soluble product) and washed with saturated aqueous sodium bicarbonate. THF (20% related to EtOAc) was added in combination with EtOAc in order to increase the solubility of the product in the organic layer. Sodium chloride was added to the aqueous layer in order to limit the emulsion. The aqueous layer was further extracted with EtOAc/THF (8:2, v:v) (3 x 20 ml). The organic layers were washed with brine, dried over sodium sulfate, filtered and concentrated to a brown solid. The latter was triturated with EtOAc/heptanes (1:4, v:v); the solids were collected on a glass filter, washed with heptanes and dried under reduced pressure to afford Compound **11b** as a tan solid (62.4 mg, 73.2% yield).
**N'-(4-(4-(Ethoxymethyl)-1H-1,2,3-triazol-1-yl)benzoyl)isoquinoline-5-carbohydrazide (11b).** Yield=73.2%; Purity=59.1%; $^1$H NMR (300 MHz, DMSO D-6): δ 10.75 (s, 2H), 9.42 (s, 1H), 8.94 (s, 1H), 8.62 (d, 1H), 8.31 (d, 2H), 8.24-8.07 (m, 4H), 8.02 (d, 1H), 7.80 (t, 1H), 4.60 (s, 2H), 3.55 (q, 2H), 1.15 (t, 3H) ppm. MS (ES+) m/z 417 [M+1]

**Procedure for the synthesis of compound 11:**

Burgess reagent (107.2 mg, 3 Eq, 0.45 mmol) was added to a suspension of **11b** (62.4 mg, 1 Eq, 150 μmol) in ACN (5 ml) and the reaction mixture was refluxed (90°C external) for 2.5 h. The reaction mixture became shortly homogeneous then heterogeneous again. The reaction mixture was cooled to RT and the solids were collected on a glass filter, washed with ACN, heptanes and dried under reduced pressure. The resulting tan solids (~60 mg) were taken up in a mixture of MeOH/DCM and coated on silica gel. The resulting cake was purified by silica gel chromatography using a gradient from 2.5 to 7.5% MeOH in DCM. A first spot (blue by UV detection) came out of the column followed by the desired product (very intense blue by UV detection). Mono spot fractions were collected and concentrated to afford Compound 11 as an off white solid (42 mg, 70.4% yield).
**2-(4-(4-(Ethoxymethyl)-1H-1,2,3-triazol-1-yl)phenyl)-5-(isoquinolin-5-yl)-1,3,4-oxadiazole (11).** Yield=70.4%; Purity=98.5%; $^1$H NMR (300 MHz, DMSO D-6): δ 9.51 (s, 1H), 9.04 (d, 1H), 8.99 (s, 1H), 8.78-8.71 (m, 2H), 8.49-8.39 (m, 5H), 8.24 (d, 3H), 7.95 (t, 1H), 4.61 (s, 2H), 3.56 (q, 2H), 1.16 (t, 3H) ppm. MS (ES+) m/z 399 [M+1]

**Compound 12**

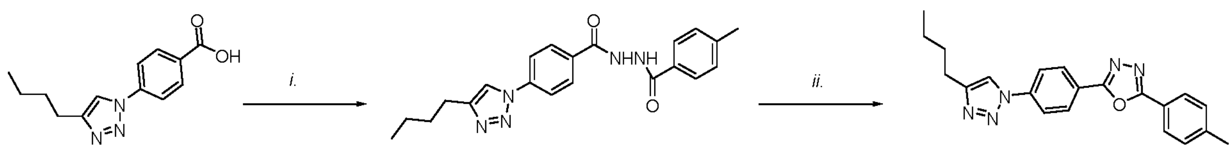


2b 12b 12

**Reagents and conditions:** *i.* 4-methylbenzohydrazide (1.1 eq.), EDC.HCl (1.5 eq.), DMAP (1.5 eq.), DMF, 0°C to RT, 5 h; *ii.* Burgess reagent (7.0 eq.), ACN, 90°C to RT, 4 h.

**Procedure for the synthesis of compound 12b:**

Compound **2b** (4-(4-butyl-1H-1,2,3-triazol-1-yl)benzoic acid) was prepared as described above in the procedure for the synthesis of compound **2.**
To a stirred solution of **2b** (300 mg, 1 Eq, 1.22 mmol) in DMF (6 ml), was added 4-methyl benzohydrazide (ENAMINE Ltd. catalogue code EN300-18501) (202 mg, 1.1 Eq, 1.346 mmol), reaction mixture was cooled to 0°C, was

added DMAP (224 mg, 1.5 Eq, 1.836 mmol) and EDC.HCl (352 mg, 1.5 Eq, 1.836 mmol) at 0°C. Reaction mixture was stirred for 5 h at RT. Reaction progress was monitored by TLC (EtOAc, Rf: 0.5). After completion of reaction, reaction mixture was poured in to ice cold water (20 ml). The precipitate was formed; Solid was filtered, washed with water (2 x 20 ml) and dried under vacuum to afford Compound **12b** as light brown solid (250 mg, 54% yield).

**4-(4-butyl-1H-1,2,3-triazol-1-yl)-N'-(4-methylbenzoyl)benzohydrazide (12b).** Yield=54%; Purity=98.9%; $^1$H NMR (400 MHz, DMSO D-6): δ 10.65 (s, 1H), 10.49 (s, 1H), 8.71 (s, 1H), 8.13 (d, 2H), 8.08 (d, 2H), 7.84 (d, 2H), 7.34 (d, 2H), 2.74 (t, 2H), 2.39 (s, 3H), 1.68 (quin, 2H), 1.39 (sx, 2H), 0.94 (t, 3H) ppm. MS (ES+) m/z 378.26[M+1]

**Procedure for the synthesis of compound 12:**

To a stirred solution of **12b** (250 mg, 1 Eq, 0.663 mmol) in ACN (5 ml), was added Burgess reagent (1.1 g, 7 Eq, 4.64 mmol), the reaction mixture was heated to 90°C for 4 h. reaction progress was monitored by TLC (50% EtOAc/Petroleum ether v:v, Rf: 0.5). After completion of reaction, the reaction mixture was cooled to RT and poured in to an ice cooled water (50ml). The solid precipitate formed was filtered, and dried, crude compound was purified by column chromatography (Silica gel 100-200 mesh). Eluent gradient 30% EtOAc/Petroleum ether (v:v). Combined column fractions distilled and dried to afford Compound 12 as pale yellow solid (40 mg, 16% yield).

**2-(4-(4-butyl-1H-1,2,3-triazol-1-yl)phenyl)-5-(p-tolyl)-1,3,4-oxadiazole (12).** Yield=16%; Purity=99.3%; $^1$H NMR (400 MHz, DMSO D-6): δ 8.76 (s, 1H), 8.33 (d, 2H), 8.17 (d, 2H), 8.06 (d, 2H), 7.46 (d, 2H), 2.74 (t, 2H), 2.43 (s, 3H), 1.68 (quin, 2H), 1.39 (sx, 2H), 0.94 (t, 3H) ppm. MS (ES+) m/z 360.33[M+1]

**Compound 13**

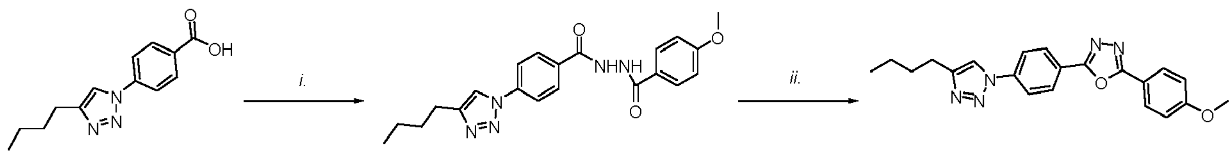


2b 13b 13

**Reagents and conditions:** *i.* 4-methoxybenzohydrazide (1.1 eq.), EDC.HCl (1.5 eq.), DMAP (1.5 eq.), DMF, 0°C to RT, 5h; *ii.* Burgess reagent (7.0 eq.), ACN, 90°C, 4h.

**Procedure for the synthesis of compound 13b:**

Compound **2b** (4-(4-butyl-1H-1,2,3-triazol-1-yl)benzoic acid) was prepared as described above in the procedure for the synthesis of compound **2.**

To a stirred solution of **2b** (150 mg, 1 Eq, 0.612 mmol) in DMF (3 ml), was added 4-methoxybenzohydrazide (ENAMINE Ltd. catalogue code EN300-18129) (646 mg, 1 Eq, 0.612 mmol), and DMAP (112 mg, 1.5 Eq, 0.9183 mmol) at RT. The reaction mixture was cooled to 0°C then added EDC.HCl (176 mg, 1.5 Eq, 0.9183 mmol) at 0°C. The reaction mixture temperature raised to RT, and was stirred for 5 h at RT. Reaction progress was monitored by TLC (EtOAc, Rf : 0.4). After completion of reaction, the reaction mixture was poured in to cold water (20 ml). The solid precipitate formed, was filtered off, washed with water (2 x 20 ml) and dried under vacuum to afford Compound **13b** as off white solid (90 mg, 37% yield).

**4-(4-butyl-1H-1,2,3-triazol-1-yl)-N'-(4-methoxybenzoyl)benzohydrazide (13b).** Yield=37%; Purity=87.5%; $^1$H NMR (400 MHz, DMSO D-6): δ 10.65 (s, 1H), 10.43 (s, 1H), 8.72 (s, 1H), 8.13 (d, 2H), 8.07 (d, 2H), 7.94 (d, 2H), 7.07 (d, 2H), 3.85 (s, 3H), 2.74 (t, 2H), 1.67 (quin, 2H), 1.39 (sx, 2H), 1.24 (s, 2H), 0.94 (t, 3H) ppm. MS (ES+) m/z 394.28 [M+1]

To a stirred solution of **13b** (90 mg, 1 Eq, 0.229 mmol) in ACN (5 ml), was added Burgess reagent (327mg, 6 Eq, 1.374 mmol), the reaction mixture was stirred at 90°C for 4h. The reaction progress was monitored by TLC (EtOAc, Rf: 0.7). After completion of reaction, the reaction mixture was diluted with cold water precipitate was formed, solid was filtered off, washed with water (2 x 10 ml) and dried under vacuum. Crude compound was washed with Diethyl ether (15 ml) to afford Compound 13 as off white solid (32 mg, 37% yield).

**2-(4-(4-butyl-1H-1,2,3-triazol-1-yl)phenyl)-5-(4-methoxyphenyl)-1,3,4-oxadiazole (13).** Yield=37%; Purity=91.6%; $^1$H NMR (400 MHz, DMSO D-6): δ 8.75 (s, 1H), 8.32 (d, 2H), 8.16 (d, 2H), 8.11 (d, 2H), 7.19 (d, 2H), 3.88 (s, 3H), 2.74 (t, 2H), 1.68 (quin, 2H), 1.39 (sx, 2H), 0.94 (t, 3H) ppm. MS (ES+) m/z 376.29 [M+1]

**Compound 14**

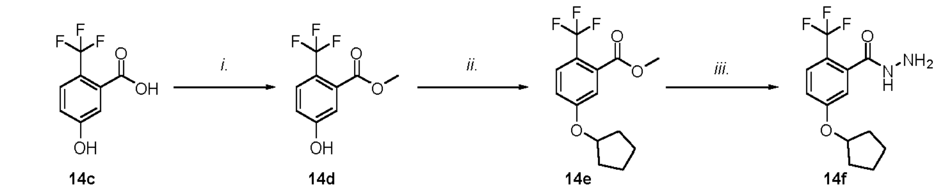


14c 14d 14e 14f

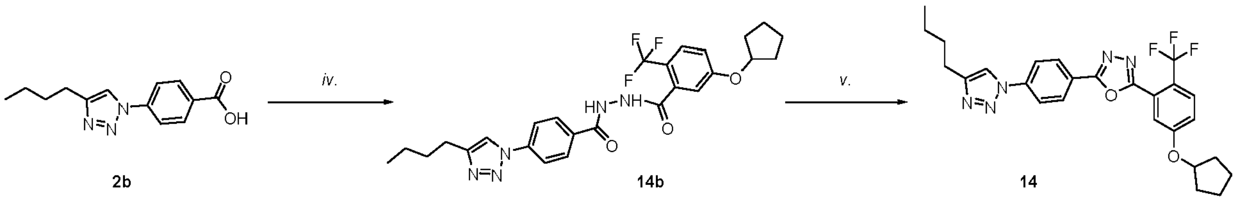


2b 14b 14

**Reagents and conditions:** *i.* $H_2SO_4$ (1.2 eq.), MeOH, 55°C, 16h; *ii.* Cyclopentanol (1.2 eq.), TPP (2.0 eq.), DEADCAT (2.0 eq.), THF, 0 °C to RT, 2h; *iii.* $NH_2$-$NH_2$.$H_2O$ (5.0 eq.), MeOH, 60°C, 18h; *iv.* 5-(cyclopentyloxy)-2-(trifluoromethyl)benzohydrazide **14f** (1.1 eq.), EDC.HCl (1.5 eq.), DMAP (1.5 eq.), DMF, 0°C to RT, 5h; *v.* Burgess reagent (7.0 eq.), ACN, 90°C to RT, 4h.

**Procedure for the synthesis of compound 14:**

Compound **2b** (4-(4-butyl-1H-1,2,3-triazol-1-yl)benzoic acid) was prepared as described above in the procedure for the synthesis of compound **2.**

To a stirred solution of **14c** (5-hydroxy-2-(trifluoromethyl)benzoic acid, ENAMINE Ltd. catalogue code EN300-171219) (0.870 g, 1 Eq, 4.223 mmol) and $H_2SO_4$ (0.496 g, 1.2 Eq, 5.0679 mmol) in MeOH (10 ml), reaction mixture was heated to 60°C for 4 h. Reaction progress was monitored by TLC (30% EtOAc in Petroleum ether, v:v, Rf : 0.8). After completion of reaction, reaction mixture was concentrated under vacuum and crude was diluted with cold water (50 ml), extracted with DCM (2 x 50 ml), combined organic layer was dried over anhydrous $Na_2SO_4$, filtrated and concentrated under reduced pressure to afford Compound **14d** as white solid (0.87 g, 93% yield).

**Methyl 5-hydroxy-2-(trifluoromethyl)benzoate (14d).** Yield=93%; Purity=97.27%; $^1$H NMR (500 MHz, $CDCl_3$): $\delta$ 7.67 (s, 2H), 7.61 (d, 1H), 6.02 (br s, 1H), 3.96 (s, 3H) ppm. MS (ES+) m/z 211.18[M+1]

To a stirred solution of **14d** (0.870 g, 1 Eq, 3.9545 mmol), TPP (2.07 g, 2 Eq, 7.9090 mmol) and Cyclopentanol (0.408 g, 1.2 Eq, 4.7454 mmol) in THF (17.4 ml), cooled to 0°C. To this DEADCAT was added dropwise (1.37 g, 2 Eq, 7.9090 mmol), the reaction mixture stirred at RT for 2 h. Reaction progress was monitored by TLC (30% EtOAc in Petroleum ether, v:v, Rf: 0.8). After completion of reaction, reaction mixture was concentrated under vacuum and directly purified under column chromatography (Silica gel 100-200 mesh). Eluent gradient 5% EtOAc/Petroleum ether (v:v). Combined pure column fractions distilled and dried under reduced pressure to afford Compound **14e** as white solid (0.87 g, 76.9% yield).

**Methyl 5-(cyclopentyloxy)-2-(trifluoromethyl)benzoate (14e).** Yield=76.9%; Purity=93%; $^1$H NMR (400 MHz, $CDCL_3$): $\delta$ 7.68-7.58 (d, 3H), 4.97 (quin, 1H), 3.95 (s, 3H), 2.00-1.88 (m, 2H), 1.82-1.56 (m, 6H) ppm. MS (ES+) m/z 289.26[M+1]

To a stirred solution of **14e** (0.87g, 1 Eq, 3.0416 mmol) in MeOH (10 ml), was added hydrazine hydrate (0.755 g, 5 Eq, 15.104 mmol), stirred for 10 min and heated at 60°C for 18 h. Reaction progress was monitored by TLC (50% EtOAc in Petroleum ether, v:v, Rf: 0.1). After completion of reaction, the reaction mixture was completely distilled off. Crude solid was washed with diethyl ether (15 ml) and dried under vacuum to afford Compound **14f** as white solid (500 mg, 57% yield).

**N'-(4-(4-butyl-1H-1,2,3-triazol-1-yl)benzoyl)-5-(cyclopentyloxy)-2-(trifluoromethyl) benzohydrazide (14f)** Yield=57%; Purity=90.35%; $^1$H NMR (500 MHz, DMSO D-6): $\delta$ 10 (s br, 1H), 7.68 (d, 1H), 7.61 (s, 1H), 7.49 (d, 1H), 5.09 (quin, 1H), 4.58 (s br, 2H), 2.00-1.76 (m, 6H), 1.71-1.60 (m, 2H) ppm. MS (ES+) m/z 289.26[M+1]

To a stirred solution of **2b** (300 mg, 1 Eq, 1.224 mmol) in DMF (9 ml, 30V), was added **14f** (423 mg, 1.2 Eq, 1.469 mmol), DMAP (224 mg, 1.5 Eq, 1.836 mmol) at RT. The reaction mixture was cooled to 0°C then added EDC.HCl (352 mg, 1.5 Eq, 1.836 mmol) at 0°C. The reaction mixture was stirred at RT for 5 h. Reaction progress was monitored by TLC (EtOAc, Rf: 0.5). After completion of reaction, the reaction mixture was poured in to cold water (50 ml), precipitated

solid was filtered off, washed with water (2 x 20 ml) and dried under vacuum to afford Compound **14b** as pale yellow solid (230 mg, crude).

**N'-(4-(4-buty|-1H-1,2,3-triazol-1-yl)benzoyl)-5-(cyclopentyloxy)-2-(trifluoromethyl) benzohydrazide (14b).** Purity=54%; MS (ES+) m/z 516.32[M+1]

To a stirred solution of **14b** (230 mg, 1 Eq, 0.449 mmol) in ACN (10 ml), was added Burgess reagent (534 mg, 5 Eq, 2.246 mmol), the reaction mixture was stirred at 90°C for 4 h. Reaction progress was monitored by TLC (50% EtOAc/Petroleum ether, v:v, Rf: 0.7). After completion of reaction, reaction mixture was diluted with cold water. The precipitated solid was filtered off, washed with water (2 x 20 ml) and dried under vacuum. Crude compound was washed with ether to afford Compound **14** as white solid (55 mg, 24% yield).

**2-(4-(4-butyl-1H-1,2,3-triazol-1-yl)phenyl)-5-(5-(cyclopentyloxy)-2-(trifluoromethyl) phenyl)-1,3,4-oxadiazole (14).** Yield=24%; Purity=95.6%; $^1$H NMR (500 MHz, DMSO D-6): δ 8.76 (s, 1H), 8.38 (d, 2H), 8.17 (d, 2H), 7.91 (s, 1H), 7.86 (dd, 2H), 5.26 (quin, 1H), 2.74 (t, 2H), 2.06-1.96 (m, 2H), 1.87-1.64 (m, 8H), 1.41 (sx, 2H), 0.95 (t, 3H) ppm. MS (ES+) m/z 498.26[M+1]

**Compound 15**

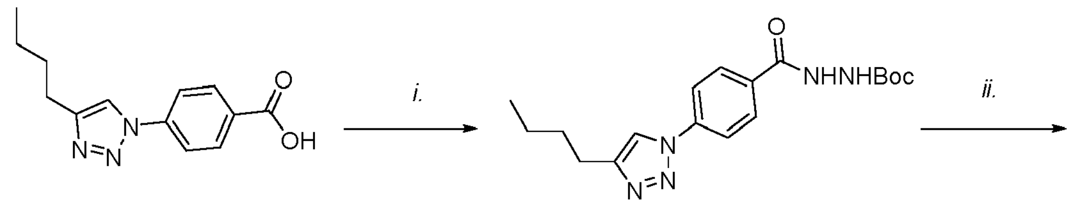


**2b** **15b**

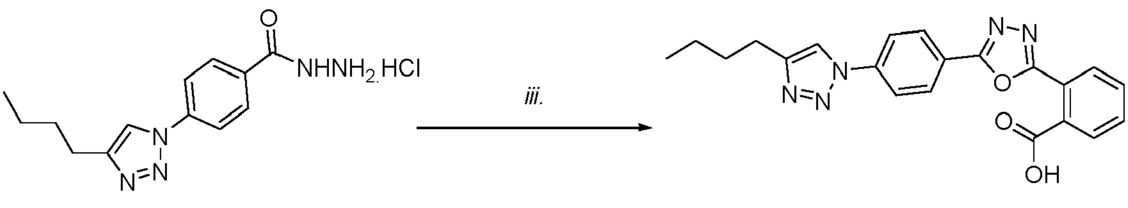


**15c** **15**

**Reagents and conditions:** *i.* Boc-hydrazide (1.1 eq.), EDC.HCl (1.5 eq.), DMAP (1.5 eq.), DMF, 0°C to RT, 5h; *ii.* DCM, 1,4-Dioxane HCl (4 N), 0°C to RT, 3h; *iii.* 2-(methoxycarbonyl)benzoic acid (1.0 eq.), TEA (3 eq.), DMAP (0.1 eq.), DMF, EDC.HCl (2 eq.), 0°C to RT, 18h.

**Procedure for the synthesis of compound 15:**

Compound **2b** (4-(4-butyl-1H-1,2,3-triazol-1-yl)benzoic acid) was prepared as described above in the procedure for the synthesis of compound **2.**

To a stirred solution of **2b** (500 mg, 1 Eq, 2.0408 mmol) in DMF (10 ml), was added Boc-hydrazide (323 mg, 1.2 Eq, 2.4489 mmol), and DMAP (374 mg, 1.5 Eq, 3.0612 mmol) at RT. The reaction mixture was cooled to 0°C then added EDC.HCl (586 mg, 1.5 Eq, 3.0612mmol) at 0°C. The reaction mixture temperature raised to RT, and stirred for 5 h at RT. Reaction progress was monitored by TLC (EtOAc, Rf: 0.4). After completion of reaction, the reaction mixture was poured in to cold water (20 ml). The precipitate was formed; Solid was filtered off, washed with water (2 x 20 ml) and dried under vacuum to afford Compound **15b** as yellow solid (450 mg, 61% yield).

**Tert-butyl 2-(4-(4-butyl-1H-1,2,3-triazo|-1-yl)benzoyl)hydrazine-1-carboxylate (15b).** Yield=61%; Purity=91.9%; $^1$H NMR (400 MHz, DMSO D-6): δ 10.35 (s, 1H), 8.99 (s, 1H), 8.70 (s, 1H), 8.10-7.97 (m, 4H), 2.72 (t, 2H), 1.66 (quin, 2H), 1.45 (s, 9H), 1.39 (sx, 2H), 0.94 (t, 3H) ppm. MS (ES+) m/z 360.37[M+1]

To a stirred solution of **15b** (450 mg, 1 Eq, 1.253 mmol) in DCM (10ml), was added 4 N 1,4dioxane HCl (2.25 ml), the reaction mixture was stirred at RT for 3h. The reaction progress was monitored by TLC (10% MeOH in DCM, Rf: 0.1). After completion of reaction, the reaction mixture was evaporated under vacuum. Crude was washed with

Diethyl ether to afford Compound **15c** as yellow solid (360 mg, 97% yield).

**4-(4-butyl-1H-1,2,3-triazol-yl)benzohydrazide (15c).** Yield=97%; Purity=91.8%; $^1$H NMR (400 MHz, DMSO D-6): δ 11.99 (s br, 1H), 8.75 (s, 1H), 8.18 (d, 2H), 8.11 (d, 2H), 4.67 (s br, 2H), 2.72 (t, 2H), 1.67 (quin, 2H), 1.39 (sx, 2H), 0.94 (t, 3H) ppm. MS (ES+) m/z 260.28[M+1]

To a stirred solution of **15c** (250 mg, 1 Eq, 0.8474 mmol) in DMF (5 ml), was added 2-(methoxycarbonyl)benzoic acid (Sigma Aldrich catalogue code 317640) (152 mg, 1 Eq, 0.8474 mmol) and TEA (256mg, 3 Eq, 2.542 mmol) DMAP (10 mg, 0.1 Eq, 0.0847 mmol) at RT. The reaction mixture was cooled to 0°C, and then added EDC.HCl (242 mg, 1.2711 mmol) at 0°C. The reaction mixture temperature raised to RT and stirred for 18 h. Reaction progress was monitored by TLC (EtOAc, Rf: 0.7). After completion of reaction, the reaction mixture was poured in to cold water (10 ml), and Extracted with ethyl acetate (2 x 20 ml), combined organic layers, dried over anhydrous $Na_2SO_4$, filtered and concentrated under vacuum. Crude was purified under silica gel (100-200 mesh) column, eluted with 50% ethyl acetate in Petroleum ether (v:v), pure fractions were concentrated under vacuum to afford Compound **15** as white solid (21mg, 6.3% yield).

**2-(5-(4-(4-butyl-1H-1,2,3-triazol-1-yl)phenyl)-1,3,4-oxadiazol-2-yl)benzoic acid (15).** Yield=6.3%; Purity=94.8%; $^1$H NMR (500 MHz, DMSO D-6): δ 11.48 (s, 1H), 8.74 (s, 1H), 8.18 (d, 2H), 8.13 (d, 2H), 8.05-8.01 (m, 2H), 8.01-7.97 (m, 2H), 2.74 (t, 2H), 1.68 (quin, 2H), 1.39 (sx, 2H), 0.94 (t, 3H) ppm. MS (ES+) m/z 390.23[M+1]

**Compound 16**

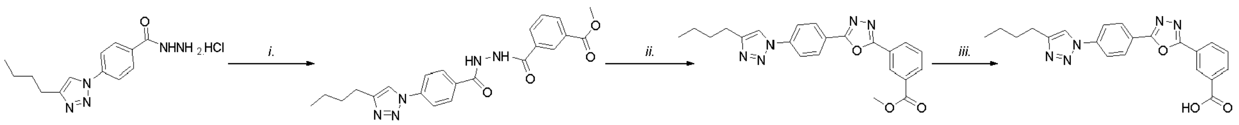


15c 16b 16c 16

**Reagents and conditions:** *i.* 3-(methoxycarbonyl)benzoic acid (1.0 eq.), TEA (3 eq.), DMAP (0.1 eq.), DMF, EDC.HCl (1.5 eq.), 0°C to RT, 4h; *ii.* Burgess reagent (7.0 eq.), ACN, 90°C to RT, 4h; LiOH (1.4 eq.), THF, Water, RT, 2h.

**Procedure for the synthesis of compound 16:**

Compound **15c** 4-(4-butyl-1H-1,2,3-triazol-1-yl)benzohydrazide was prepared as described above in the procedure for the synthesis of compound **15.**

To a stirred solution of **15c** (250 mg, 1 Eq, 0.8474 mmol) in DMF (5 ml), was added 3-(methoxycarbonyl)benzoic acid (Sigma Aldrich catalogue code 555401) (173 mg, 1 Eq, 0.8474 mmol), TEA (256 mg, 3 Eq, 2.5423 mmol) and DMAP (10 mg, 0.1 Eq, 0.0847 mmol) at RT. The reaction mixture was cooled to 0°C. To this EDC.HCl (243mg, 1.5 Eq, 1.2711 mmol) was added at 0°C, reaction mixture temperature raised to RT, stirred for 4 h. Reaction progress was monitored by TLC (100% EtOAc (Rf : 0.4). After completion of reaction, the reaction mixture was poured in to cold water (20 ml). The solid precipitate was formed, filtered off, washed with water (2 x 20 ml) and dried under vacuum to afford Compound 16b as pale yellow solid (238 mg, crude).

**Methyl 3-(2-(4-(4-butyl-1H-1,2,3-triazo|-1-yl)benzoyl)hydrazine-1-carbonyl)benzoate (16b).** Purity=47.1%; MS (ES+) m/z 422.28[M+1]

To a stirred solution of **16b** (238 mg, 1 Eq, 0.5653 mmol) in ACN (5 ml), was added Burgess reagent (807 mg, 6 Eq, 3.3919 mmol), the reaction mixture was stirred at 90°C for 4 h. The reaction progress was monitored by TLC (EtOAc, Rf: 0.7). After completion of reaction, the reaction mixture was diluted with cold water precipitate was formed, solid was filtered off, washed with water (2 x 10 ml) and dried under vacuum. Crude compound was purified with grace column using silica gel 100-200 mesh, eluted with 50% ethyl acetate in Petroleum ether (v:v) and pure fractions concentrated under vacuum to afford Compound **16c** as off white solid (40 mg, 17% yield).

**Methyl 3-(5-(4-(4-butyl-1H-1,2,3-triazol-1-yl)phenyl)-1,3,4-oxadiazol-2-yl)benzoate (16c).** Yield=17%; Purity=97.8%; $^1$H NMR (400 MHz, DMSO D-6): δ 8.77 (s, 1H), 8.66 (s, 1H), 8.45 (d, 1H), 8.37 (d, 2H), 8.26-8.13 (m, 3H), 7.83 (t, 1H), 3.95 (s, 3H), 2.75 (t, 2H), 1.68 (quin, 2H), 1.39 (sx, 2H), 0.95 (t, 3H) ppm. MS (ES+) m/z 404.30[M+1]

To a stirred solution of **16c** (40 mg, 1 Eq, 0.0992 mmol) in THF (3 ml) and LiOH (3 mg, 1.4 Eq, 0.14 mmol) was dissolved in water (1 ml) was added at 0°C and stirred t RT for 4 h. The reaction progress was monitored by TLC (EtOAc, Rf: 0.2). After completion of reaction, the reaction mixture was evaporated under vacuum, crude was adjusted pH to 3 with HCl 1 N, precipitate was formed, solid was filtered and washed with water (2 x 10 ml) and dried under vacuum. Crude compound was purified with grace column using silica gel 100-200 mesh; compound was eluted with ethyl acetate and evaporated pure fractions under vacuum to afford Compound 16 as white solid (13.4 mg, 34% yield).

**3-(5-(4-(4-butyl-1H-1,2,3-triazol-1-yl)phenyl)-1,3,4-oxadiazol-2-yl)benzoic acid (16).** Yield=34%; Purity=94.3%; $^1$H

NMR (500 MHz, DMSO D-6): δ 8.76 (s, 1H), 8.66 (s, 1H), 8.36 (d, 3H), 8.19 (d, 1H), 8.18 (d, 2H), 7.76 (t, 1H), 2.74 (t, 2H), 1.68 (quin, 2H), 1.39 (sx, 2H), 0.94 (t, 3H) ppm. MS (ES+) m/z 390.27[M+1]

**Compound 17**

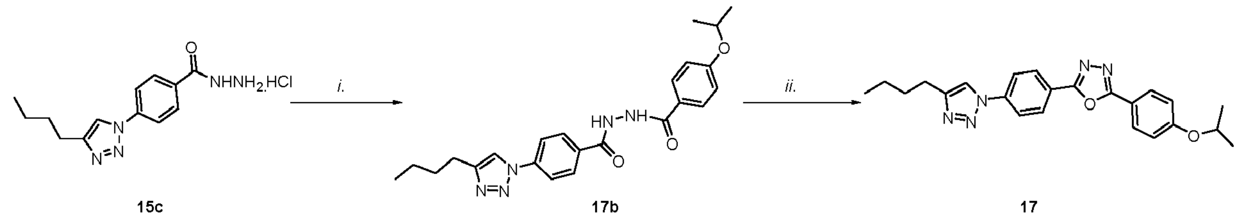


15c 17b 17

**Reagents and conditions:** *i.* 4-isopropoxybenzoic acid (1.0 eq.), TEA (3 eq.), DMAP (1.5 eq.), DMF, EDC.HCl (1.5 eq.), 0°C to RT, 4 h; ii. Burgess reagent (6.0 eq.), ACN, 90°C, 4 h.

**Procedure for the synthesis of compound 17:**

Compound **15c** 4-(4-butyl-1H-1,2,3-triazol-1-yl)benzohydrazide was prepared as described above in the procedure for the synthesis of compound **15.**

To a stirred solution of **15c** (200 mg, 1 Eq, 0.677 mmol) in DMF (4 ml), was added 4-isopropoxybenzoic acid (ENAMINE Ltd. catalogue code EN300-17039) (122 mg, 1 Eq, 0.677 mmol), and TEA (205 mg, 3 Eq, 2.033 mmol), DMAP (124 mg, 1.5 Eq, 1.0169 mmol) at RT. The reaction mixture was cooled to 0°C, added EDC.HCl (194 mg, 1.5 Eq, 1.0169 mmol) at 0°C. The reaction mixture was stirred for 4 h at RT. Reaction progress was monitored by TLC (EtOAc, Rf: 0.5). After completion of reaction, the reaction mixture was poured in to cold water (20 ml). The solid precipitate was formed, filtered off, washed with water (2 x 20 ml) and dried under vacuum to afford Compound **17b** as pale yellow solid (160 mg, crude).

**4-(4-butyl-1H-1,2,3-triazol-1-yl)-N'-(4-isopropoxybenzoyl)benzohydrazide (17b).** Purity=23%; MS (ES+) m/z 422.36[M+1]

To a stirred solution of **17b** (160 mg, 1 Eq, 0.3800 mmol) in ACN (5 ml), was added Burgess reagent (542mg, 6 Eq, 2.2802 mmol) and heated to 90°C for 4h. The reaction progress was monitored by TLC (EtOAc, Rf: 0.7). After completion of reaction, the reaction mixture was diluted with cold water precipitate solid was filtered, washed with water (2 x 10 ml) and dried under vacuum. Crude compound was purified under grace column using silica gel 100-200 mesh; compound was eluted with 50% ethyl acetate in petroleum ether (v:v), pure fraction concentrated under vacuum to afford Compound **17** as white solid (29.9 mg, 19.5% yield).

**2-(4-(4-buty|-1H-1,2,3-triazol-1-yl)phenyl)-5-(4-isopropoxyphenyl)-1,3,4-oxadiazole (17).** Yield=19.5%; Purity=94.2%; $^1$H NMR (400 MHz, DMSO D-6): δ 8.75 (s, 1H), 8.32 (d, 2H), 8.16 (d, 2H), 8.08 (d, 2H), 7.16 (d, 2H), 4.78 (sept, 1H), 2.74 (t, 2H), 1.68 (quin, 2H), 1.37 (sx, 2H), 1.32 (d, 6H), 0.94 (t, 3H) ppm. MS (ES+) m/z 404.39[M+1]

**Compound 18**

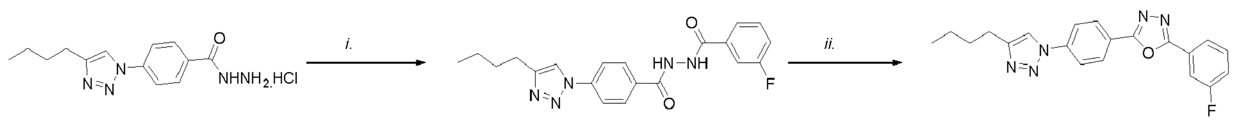


15c 18b 18

**Reagents and conditions:** *i.* 3-fluorobenzoic acid (1.0 eq.), TEA (3 eq.), DMAP (1.5 eq.), DMF, EDC.HCl (2 eq.), 0°C to RT, 4h; *ii.* Burgess reagent (6.0 eq.), ACN, 90°C, 4h.

**Procedure for the synthesis of compound 18:**

Compound **15c** 4-(4-butyl-1H-1,2,3-triazol-1-yl)benzohydrazide was prepared as described above in the procedure for the synthesis of compound **15.**

To a stirred solution of **15c** (300 mg, 1 Eq, 1.0169 mmol) in DMF (6 ml), was added 3-fluorobenzoic acid (Sigma Aldrich catalogue code F6605) (142 mg, 1 Eq, 1.0169 mmol), TEA (308 mg, 3 Eq, 3.0508 mmol) and DMAP (186 mg, 1.5 Eq, 1.5254mmol) at RT. The reaction mixture was cooled to 0°C, added EDC.HCl (291mg, 1.5 Eq, 1.5254 mmol) at 0°C. The reaction mixture was stirred for 4 h at RT. Reaction progress was monitored by TLC (EtOAc, Rf: 0.4). After completion of reaction, the reaction mixture was poured in to cold water (20 ml). The solid precipitate was formed, filtered off, washed with water (2 x 20 ml) and dried under vacuum to afford Compound **18b** as pale yellow solid (250 mg, 64.5% yield).

**N'-(4-(4-butyl-1H-1,2,3-triazol-1-yl)benzoyl)-3-fluorobenzohydrazide (18b).** Yield=64.5%; Purity=94.6%; $^1$H NMR (400 MHz, DMSO D-6): δ 9.72 (s, 2H), 8.72 (s, 1H), 8.14 (d, 2H), 8.08 (d, 2H), 7.81 (d, 1H), 7.73 (d, 1H), 7.62 (dd, 1H), 7.49 (dt, 1H), 2.73 (t, 2H), 1.68 (quin, 2H), 1.39 (sx, 2H), 0.94 (t, 3H) ppm. MS (ES-) m/z 380.45[M-1]; MS (ES+) m/z 382.29[M+1].

To a stirred solution of **18b** (250 mg, 1 Eq, 0.6561 mmol) in ACN (5 ml), was added Burgess reagent (937mg, 6 Eq, 3.9370 mmol) heated at 90°C for 4h. The reaction progress was monitored by TLC (EtOAc, Rf: 0.7). After completion of reaction, the reaction mixture was diluted with cold water; precipitated solid was filtered, washed with water (2 x 10 ml) and dried under vacuum. Crude compound was purified under grace column using silica gel 100-200 mesh; compound was eluted with 50% ethyl acetate in petroleum ether (v:v). Pure fractions were concentrated under vacuum to afford Compound **18** as white solid (14.6 mg, 6% yield).

**2-(4-(4-butyl-1H-1,2,3-triazol-1-yl)phenyl)-5-(3-fluorophenyl)-1,3,4-oxadiazole (18).** Yield=6%; Purity=95.9%; $^1$H NMR (400 MHz, DMSO D-6): δ 8.77 (s, 1H), 8.37 (d, 2H), 8.18 (d, 2H), 8.03 (t, 2H), 7.71 (dd, 1H), 7.54 (dt, 1H), 2.74 (t, 2H), 1.68 (quin, 2H), 1.39 (sx, 2H), 0.94 (t, 3H) ppm. MS (ES+) m/z 364.27[M+1]

**Compound 19**

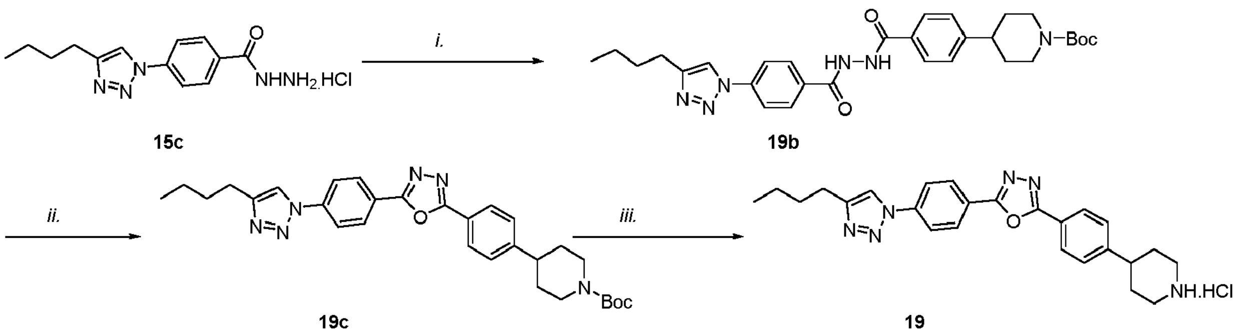


15c 19b 19c 19

**Reagents and conditions:** *i.* 4-(1-(tert-butoxycarbonyl)piperidin-4-yl)benzoic acid (1.0 eq.), TEA (3 eq.), DMAP (1.5 eq.), DMF, EDC.HCl (2 eq.), 0°C to RT, 5 h; *ii.* Burgess reagent (6.0 eq.), ACN, 90°C, 8h; *iii.* 1,4-dioxane, 1,4-dioxane HCl (4 N), 0°C to RT, 6h.

**Procedure for the synthesis of compound 19:**

Compound **15c** 4-(4-butyl-1H-1,2,3-triazol-1-yl)benzohydrazide was prepared as described above in the procedure for the synthesis of compound **15.**

To a stirred solution of **15c** (500 mg, 1 Eq, 1.69 mmol) in DMF (10 ml), cooled to 0°C, was added TEA (513 mg, 3 Eq, 5.084 mmol) and stirred for 5 min at 0°C. To this 4-(1-(tert-butoxycarbonyl)piperidin-4-yl)benzoic acid (Sigma Aldrich catalogue code 916870) (516 mg, 1 Eq, 1.649 mmol), DMAP (310 mg, 1.5 Eq, 2.542 mmol) and EDC.HCl (487 mg, 1.5 Eq, 2.542 mmol) was added at 0°C. The reaction mixture was stirred at RT for 5 h. Reaction progress was monitored by TLC (EtOAc (Rf: 0.5). After completion of reaction, the reaction mixture was poured in to ice cold water (50 ml). The precipitated solid was filtered, washed with water (2 x 50 ml) and dried to afford Compound **19b** as off white solid (300 mg, crude).

**Tert-butyl 4-(4-(2-(4-(4-butyl-1H-1,2,3-triazol-1-yl)benzoyl)hydrazine-1-carbonyl)phenyl)piperidine-1-carboxylate (19b).** Purity=34%; MS (ES+) m/z 547.36 [M+1]

To a stirred solution of **19b** (300 mg, 1 Eq, 0.549 mmol) in ACN (9 ml), was added Burgess reagent (784 mg, 6 Eq, 3.29 mmol) and heated to 90°C for 4 h. Reaction progress was monitored by TLC (50% EtOAc, Rf : 0.5). After completion of reaction, the reaction mixture was cooled to RT and poured in to ice cold water (50 ml), precipitated solid was filtered, washed with water (50 ml) and dried, crude compound was purified by column chromatography (Silica gel

100-200 mesh). Eluent gradient 30% EtOAc/Petroleum ether (v:v). Combined column fractions distilled and dried to afford Compound **19c** as off white solid (100 mg, 34% yield).

**Tert-butyl 4-(4-(5-(4-(4-butyl-1H-1,2,3-triazol-1-yl)phenyl)-1,3,4-oxadiazol-2-yl)phenyl)piperidine-1-carboxylate (19c).** Yield=34%; Purity=88.7%; MS (ES+) m/z 529.43 [M+1]

To a stirred solution of **19c** (100 mg, 1 Eq, 0.189 mmol) in 1,4 Dioxane (3 ml) was cooled to 0°C. To this 4 N 1,4 Dioxane HCl (0.2 ml) was added, stirred at RT for 6 h. Reaction progress was monitored by TLC (100% EtOAc, Rf: 0.1). After completion of reaction, the reaction mixture was concentrated under reduced pressure, compound was triturated with diethyl ether (20 ml), filtered and dried to afford Compound **19** as off white solid (70 mg, 79% yield).

**2-(4-(4-butyl-1H-1,2,3-triazol-1-yl)phenyl)-5-(4-(piperidin-4-yl)phenyl)-1,3,4-oxadiazole hydrochloride (19).** Yield=79%; Purity=91%; $^1$H NMR (400 MHz, DMSO D-6): δ 8.90-8.82 (b, 1H), 8.76 (s, 1H), 8.73-8.65 (b, 1H), 8.33 (d, 2H), 8.18 (d, 2H), 8.15 (d, 2H), 7.51 (d, 2H), 3.44-3.37 (m, 2H), 3.10-2.96 (m, 3H), 2.74 (t, 2H), 2.04-1.97 (m, 2H), 1.96-1.83 (m, 2H), 1.68 (quin, 2H), 1.39 (sx, 2H), 0.94 (t, 3H) ppm. MS (ES+) m/z 429.38 [M+1]

**Compound 20**

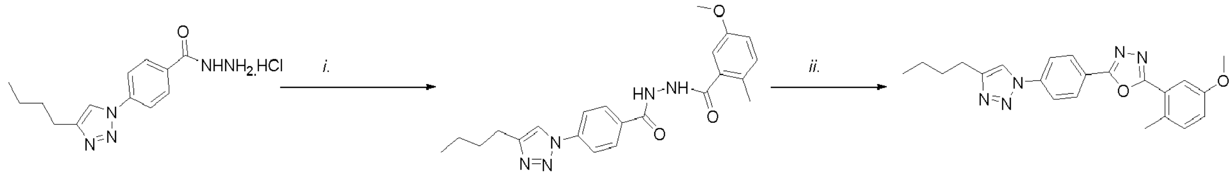


15c 20b 20

**Reagents and conditions:** *i.* 5-methoxy-2-methylbenzoic acid (1.0 eq.), TEA (3 eq.), DMAP (0.2 eq.), DMF, EDC.HCl (1.5 eq.), 0°C to RT, 4h; *ii.* Burgess reagent (6.0 eq.), ACN, 90°C to RT, 4h.

**Procedure for the synthesis of compound 20:**

Compound **15c** 4-(4-butyl-1H-1,2,3-triazol-1-yl)benzohydrazide was prepared as described above in the procedure for the synthesis of compound **15.**

To a stirred solution of **15c** (248mg, 1 Eq, 0.8433 mmol) in DMF (3.5 ml), was added 5-methoxy-2-methylbenzoic acid (Combi-Blocks Inc. catalogue code QA-2928) (140 mg, 1 Eq, 0.8433 mmol), TEA (255 mg, 3 Eq, 2.5301 mmol) and DMAP (20 mg, 0.2 Eq, 0.1686 mmol) at RT, cooled to 0°C. To this EDC.HCl (242 mg, 1.5 Eq, 1.265 mmol) was added at 0°C. The reaction mixture was stirred at RT for 4h. Reaction progress was monitored by TLC (EtOAc, Rf: 0.5). After completion of reaction, reaction mixture was poured in to cold water (10 ml) and extracted with EtOAc (2 x 10 ml), combined organic layers, dried over anhydrous $Na_2SO_4$, filtered and concentrated under vacuum to afford Compound **20b** as pale yellow solid (395mg, crude).

**N'-(4-(4-butyl-1H-1,2,3-triazol-1-yl)benzoyl)-5-methoxy-2-methylbenzohydrazide (20b).** Purity=44.1%; MS (ES+) m/z 408.17 [M+1]

To a stirred solution of **20b** (395 mg, 1 Eq, 0.9705 mmol) in ACN (5 ml), was added Burgess reagent (1.38g, 6 Eq, 5.823 mmol), stirred at 90°C for 4h. The reaction progress was monitored by TLC (EtOAc) (Rf: 0.7). After completion of reaction, the reaction mixture was diluted with cold water; precipitate solid was filtered, washed with water (2 x 10 ml) and dried under vacuum. Crude compound was purified under grace column using silica gel 100-200 mesh; compound was eluted with 50% ethyl acetate in petroleum ether (v:v) to afford Compound **20** as off white solid (55 mg, 14.5% yield).

**2-(4-(4-butyl-1H-1,2,3-triazol-1-yl)phenyl)-5-(5-methoxy-2-methylphenyl)-1,3,4-oxadiazole (20).** Yield=14.5%; Purity=95.6%; $^1$H NMR (400 MHz, DMSO D-6): δ 8.75 (s, 1H), 8.34 (d, 2H), 8.16 (d, 2H), 7.63 (d, 1H), 7.40 (d, 1H), 7.14 (dd, 1H), 3.86(s, 3H), 2.74 (t, 2H), 2.63 (s, 3H), 1.68 (quin, 2H), 1.39 (sx, 2H), 0.94 (t, 3H) ppm. MS (ES+) m/z 390.32[M+1]

**Compound 21**

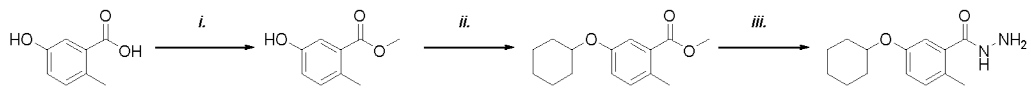


21c 21d 21e 21f

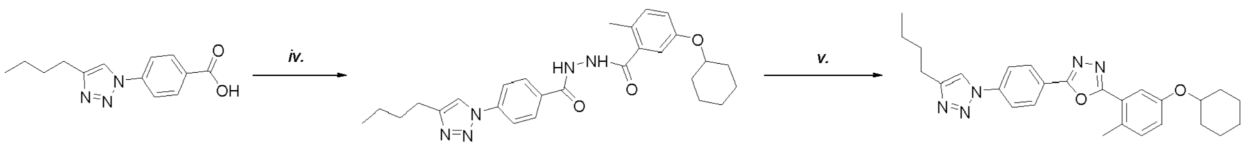


15b 21b 21

**Reagents and conditions:** *i.* $H_2SO_4$ (3.0 eq.), MeOH, RT, 2h; *ii.* Cyclohexanol (2.0 eq.), TPP (2.0 eq.), DEADCAT (2.0 eq.), THF, 0°C to RT, 16h; *iii.* Hydrazine hydrate (5.0 eq.), MeOH, 70°C, 16h; *iv.* 5-(cyclohexyloxy)-2-methyl-benzohydrazide **21f** (1.1 eq.), DMAP (1.5 eq.), EDC.HCl (1.5 eq.), DMF, 0°C to RT, 5h; *v.* Burgess reagent (6.0 eq.), ACN, 90°C to RT, 4h.

**Procedure for the synthesis of compound 21:**

Compound **2b** (4-(4-butyl-1H-1,2,3-triazol-1-yl)benzoic acid) was prepared as described above in the procedure for the synthesis of compound **2.**

To a stirred solution of **21c** (5-Hydroxy-2-methylbenzoic acid) (Sigma Aldrich catalogue code 696366) (1 g, 1 Eq, 6.57 mmol) in MeOH (20 ml), was added $H_2SO_4$ (1.93 g, 3 Eq, 19.73 mmol), stirred at RT for 2 h. Reaction progress was monitored by TLC (100% EtOAc in Petroleum ether, v:v, Rf: 0.5). After completion of reaction, the reaction mixture was concentrated under reduced pressure, basified with saturated $NaHCO_3$ solution up to PH~8 compound was extracted with EtOAc (2 x 50 ml), combined organic layer was dried over anhydrous $Na_2SO_4$, filtrated and concentrated to afford Compound **21d** as off white solid (1 g, crude).

**Methyl 5-hydroxy-2-methylbenzoate (21d).** Purity=91.5%; MS (ES+) m/z 167.16 [M+1]

To a stirred solution of **21d** (1 g, 6.024 mmol) and cyclohexanol (Sigma Aldrich catalogue code 822328) (1.2 g, 2 Eq, 12.048 mmol) in THF (20 ml) was added TPP (3.1 g, 2 Eq, 12.048 mmol) was cooled to 0°C. To this DEADCAT (2 g, 2 Eq, 12.048 mmol) was added and stirred at RT for 16 h. Reaction progress was monitored by TLC (30% EtOAc/Petroleum ether, v:v, Rf : 0.6). After completion of reaction, the reaction mixture was poured in to ice cooled water, extracted with EtOAc (2 x 200 ml), combined organic layer was washed with Brine solution (200 ml) and dried over anhydrous $Na_2SO_4$, filtrated and concentrated. Crude compound was purified by column chromatography (Silica gel 100-200 mesh). Eluted with gradient of 15% EtOAc/Petroleum ether (v:v) to afford Compound **21e** as off white solid (900 mg, 60% yield).

**Methyl 5-(cyclohexyloxy)-2-methylbenzoate (21e).** Yield=60%; Purity=79.1%; MS (ES+) m/z 249.82 [M+1]

To a stirred solution of **21e** (900 mg, 1 Eq, 3.62 mmol) in MeOH (18 ml), was added hydrazine hydrate (907 mg, 5 Eq, 18.145 mmol), the reaction mixture was heated to 70°C for 16 h. Reaction progress was monitored by TLC (100% EtOAc, Rf: 0.2). After completion of reaction, the reaction mixture was cooled to RT, concentrated under reduced pressure, crude compound triturated with diethyl ether (20 ml) filtered and dried under vacuum to afford Compound **21f** as off white solid (800 g, crude).

**5-(cyclohexyloxy)-2-methylbenzohydrazide (21f).** Purity=90.9%; MS (ES+) m/z 249.36 [M+1]

To a stirred solution of **2b** (200 mg, 1 Eq, 0.816 mmol) in DMF (4 ml), was added 21f (222 mg, 1.1 Eq, 0.897 mmol), the reaction mixture was cooled to 0°C, was added DMAP (149 mg, 1.5 Eq, 1.221 mmol), EDC.HCl (234 mg, 1.2 Eq, 0.958 mmol) at 0°C. The reaction mixture was stirred for 5 h, at RT. Reaction progress was monitored by TLC (EtOAc, Rf: 0.3). After completion of reaction, reaction mixture was poured in to ice cooled water (100 ml). The precipitate was formed, solid was filtered; the crude was purified by column chromatography (Silica gel 100-200 mesh). Eluent gradient 30% EtOAc/Petroleum ether (v:v). Combined column fractions distilled dried to afford Compound **21b** as off white solid (140 mg, 36% yield).

**N'-(4-(4-buty|-1H-1,2,3-triazol-1-yl) benzoyl)-5-(cyclohexyloxy)-2-methylbenzohydrazide (21b).** Yield=36%; Purity=76%; $^1$H NMR (400 MHz, DMSO D-6): δ 10.65 (s, 1H), 10.23 (s, 1H), 8.71 (s, 1H), 8.14 (d, 2H), 8.06 (d, 2H), 7.18 (d, 1H), 7.00-6.91 (m, 2H), 4.33 (quin, 1H), 2.73 (t, 2H), 2.35 (s, 3H), 1.93 (b, 2H), 1.80-1.64 (m, 4H), 1.60-1.21 (m, 8H), 0.94 (t, 3H) ppm. MS (ES+) m/z 476.38 [M+1].

To a stirred solution of **21b** (140 mg, 1 Eq, 0.294 mmol) in ACN (4.2 ml), was added Burgess reagent (420 mg, 6 Eq, 1.76 mmol), the reaction mixture was heated to 90°C for 4 h. Reaction progress was monitored by TLC (50% EtOAc, Rf: 0.5) After completion of reaction, reaction mixture was cooled to RT and poured in to an ice cold water (50

ml) precipitated is formed and filtered solid was washed with water (50 ml) and dried to afford Compound 21 as off white solid (65 mg 48% yield).

**2-(4-(4-butyl-1H-1,2,3-triazol-1-yl)phenyl)-5-(5-(cyclohexyloxy)-2-methylphenyl)-1,3,4-oxadiazole (21).** Yield=48%; Purity=94.8%; $^1$H NMR (400 MHz, DMSO D-6): δ 8.75 (s, 1H), 8.34 (d, 2H), 8.17 (d, 2H), 7.60 (d, 1H), 7.37 (d, 1H), 7.15 (dd, 1H), 4.50-4.42 (b, 1H), 2.74 (t, 2H), 2.62 (s, 3H), 2.00-1.92 (b, 2H), 1.78-1.64 (m, 4H), 1.60-1.24 (m, 8H), 0.94 (t, 3H) ppm. MS (ES+) m/z 458.33[M+1]

**Compound 22**

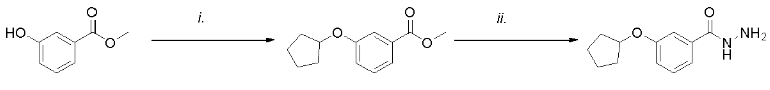


22c 22d 22e

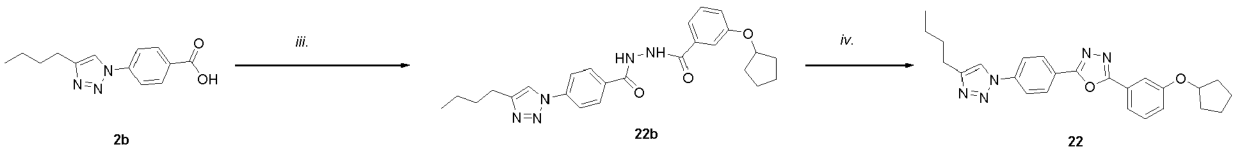


2b 22b 22

**Reagents and conditions:** *i.* Cyclopentanol (1.2 eq.), TPP (2.0 eq.), DEADCAT (2.0 eq.), THF, 0°C to RT, 2h; *ii*. Hydrazine hydrate (3.0 eq.), MeOH, 60°C, 18h; *iii*. 3-(cyclopentyloxy)benzohydrazide **22e** (1.1 eq.), DMAP (1.5 eq.), DMF, EDC·HCl (1.5 eq.), 0°C to RT, 5h; *iv*. Burgess reagent (6.0 eq.), ACN, 90°C, 4h.

**Procedure for the synthesis of compound 22:**

Compound **2b** (4-(4-butyl-1H-1,2,3-triazol-1-yl)benzoic acid) was prepared as described above in the procedure for the synthesis of compound **2.**

To a stirred solution of methyl 3-hydroxybenzoate (Sigma Aldrich catalogue code 252794) (1.0 g, 1 Eq, 6.5746 mmol) and Cyclopentanol (0.68 g, 1.2 Eq, 7.8895 mmol) in THF (20 ml), added TPP (3.44 g, 2 Eq, 13.1492 mmol) and cooled to 0°C. To this DEADCAT (2.0 ml, 2 Eq, 13.1492 mmol) was added, stirred at RT for 2 h. Reaction progress was monitored by TLC (30% EtOAc in Petroleum ether, v:v, Rf: 0.8). After completion of reaction, reaction mixture was concentrated and directly purified through column chromatography (Silica gel 100-200 mesh). Eluent gradient 5% EtOAc/Petroleum ether (v:v). Combined pure fractions distilled and dried under reduced pressure to afford Compound **22d** as light yellow liquid (0.9 g, 64% yield).

**Methyl 3-(cyclopentyloxy) benzoate (22d).** Yield=64%; Purity=72.7%; $^1$H NMR (500 MHz, $CDCl_3$): δ 7.59 (d, 1H), 7.54 (s, 1H), 7.31 (t, 1H), 7.05 (d, 1H), 4.82 (quin, 1H), 3.91 (s, 3H), 1.90-1.99 (m, 2H), 1.89-1.65 (m, 4H), 1.69-1.60 (m, 2H) ppm. MS (ES+) m/z 221.12[M+1]

To a stirred solution of **22d** (900 mg, 1 Eq, 4.08 mmol) in MeOH (18 ml), was added hydrazine hydrate (0.66 ml, 3 Eq, 12.2576 mmol), stirred for 10 min and heated at 60°C for 18 h. Reaction progress was monitored by TLC (30% EtOAc in Petroleum ether, v:v, Rf: 0.1). After completion of reaction, reaction mixture was completely distilled off. Crude solid was triturated with diethyl ether (15 ml), decant and dried under vacuum to afford Compound **22e** as off white solid (600 mg, 66% yield).

**3-(cyclopentyloxy)benzohydrazide (22e).** Yield=66%; Purity=97.5%; $^1$H NMR (400 MHz, $CDCl_3$): δ 7.36-7.25 (m, 2H), 7.22 (d, 1H), 7.01 (d, 1H), 4.80 (quin, 1H), 4.10 (s br, 2H), 2.03-1.59 (m, 8H) ppm. MS (ES+) m/z 221.22[M+1].

To a stirred solution of **2b** (300 mg, 1 Eq, 1.22 mmol) in DMF (9 ml), was added **22e** (296 mg, 1.1 Eq, 1.34 mmol), DMAP (224 mg, 1.5 Eq, 1.83 mmol) at RT, cooled to 0°C. To this added EDC·HCl (352 mg, 1.5 Eq, 1.83 mmol) at 0°C. The reaction mixture was stirred at RT for 5 h. Reaction progress was monitored by TLC (70% EtOAc in Petroleum ether, v:v, Rf: 0.5). After completion of reaction, reaction mixture was poured in to cold water (50 ml). The precipitated solid was filtered off, washed with water (2 x 50 ml) and dried under vacuum to afford Compound **22b** as off light green solid (200 mg, Crude). (Crude was directly taken to next step without further purification).

**N'-(4-(4-butyl-1H-1,2,3-triazol-1-yl)benzoyl)-3-(cyclopentyloxy)benzohydrazide (22b).** Yield=47%; Purity=99.1%; $^1$H NMR (400 MHz, $CDCl_3$): δ 9.27 (br, 2H), 8.05 (d, 2H), 7.87 (d, 2H), 7.80 (s, 1H), 7.63 (s, 1H), 7.39 (s, 2H), 7.08 (d, 1H), 4.83 (quin, 1H), 2.83 (t, 2H), 2.00-1.36 (m, 12H), 0.98 (t, 3H) ppm. MS (ES+) m/z 448.32[M+1]

To a stirred solution of **22b** (200 mg, 1 Eq, 0.4474 mmol) in ACN (4 ml) was added Burgess reagent (638 mg,

6 Eq, 2.6844 mmol), was heated at 90°C for 4 h. Reaction progress was monitored by TLC (20% EtOAc in Petroleum ether, v:v, Rf: 0.5). After completion of reaction, reaction mixture was diluted with cold water (30 ml). The precipitated solid was filtered off, washed with water (2 x 20 ml) and dried under vacuum. Crude compound was purified through column chromatography (Silica gel 100-200 mesh). Eluent gradient 20% EtOAc/Petroleum ether (v:v). Combined column fractions were concentrated to afford Compound **22** as off white solid (30 mg, 12% yield).

**2-(4-(4-butyl-1H-1,2,3-triazol-1-yl)phenyl)-5-(3-(cyclopentyloxy)phenyl)-1,3,4-oxadiazole (22).** Yield=12%; Purity=99.1%; $^1H$ NMR (400 MHz, DMSO D-6): δ 8.76 (s, 1H), 8.35 (d, 2H), 8.17 (d, 2H), 7.71 (d, 1H), 7.63 (s, 1H), 7.54 (t, 1H), 7.21 (dd, 1H), 4.98 (quin, 1H), 2.74 (t, 2H), 2.04-1.95 (m, 2H), 1.73-1.60 (m, 8H), 1.39 (sx, 2H), 0.94 (t, 3H) ppm. MS (ES+) m/z 430.34[M+1]

**Compound 23**

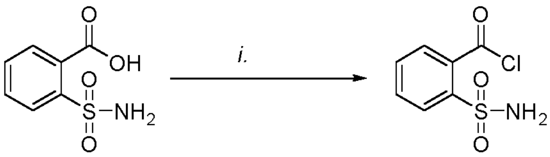


**23b** **23c**

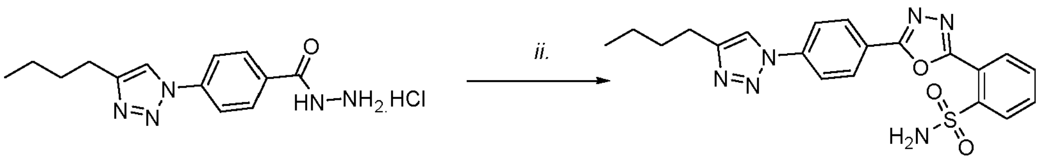


**15b** **23**

**Reagents and conditions:** *i.* $SOCl_2$ (3eq.), DCM, DMF, 0°C, reflux, 2h; *ii.* TEA (5.0 eq.), THF, RT, 18h.

**Procedure for the synthesis of compound 23:**

Compound **15c** 4-(4-butyl-1H-1,2,3-triazol-1-yl)benzohydrazide was prepared as described above in the procedure for the synthesis of compound **15.**

To a stirred solution of **23b** (2-sulfamoylbenzoic acid, ENAMINE Ltd. Catalogue code EN300-24313) (140 mg, 1 Eq, 0.6965 mmol) in DCM (3 ml), was added $SOCl_2$ (246 mg, 3 Eq, 2.0895 mmol), and DMF (point spatula) was added at 0°C. The reaction mixture was refluxed, for 2 h. Reaction progress was monitored by TLC (10% MeOH in DCM, Rf: 0.5). After completion of reaction, the reaction mixture was evaporated precipitate was formed to afford Compound **23c** (176 mg, crude). (Crude was directly taken to next step without further purification).

**2-sulfamoylbenzoyl chloride (23c).**

To a stirred solution of **15c** (230 mg, 1 Eq, 0.7796 mmol) in THF (4.6 ml), and TEA (393mg, 5 Eq, 3.898 mmol) stirred for 20 mins at RT, then added **23c** (174 mg, 0.95 Eq, 0.7406 mmol) in THF (5 ml) was added at 0°C. The reaction mixture temperature raised to RT, stirred for 18 h at RT. Reaction progress was monitored by TLC (10% MeOH in DCM, Rf : 0.7). After completion of reaction, the reaction mixture was poured in to cold water (10 ml), and extracted with 10% MeOH in DCM (2 x 20 ml), combined organic layer and evaporated. Crude was purified by preparative HPLC; Column: X-Bridge C18 5um (19 * 250 mm) Mobile Phase: 10 mM ABC in $H_2O$/CAN; Solubility: ACN + water + THF Flow: 15.0 ml/min. Pure fractions was lyophilized to afford Compound **23** as white solid (40 mg 12.1% yield).

**2-(5-(4-(4-butyl-1H-1,2,3-triazol-1-yl)phenyl)-1,3,4-oxadiazol-2-yl)benzenesulfonamide (23).** Yield=12.1%; Purity=97.2%; $^1H$ NMR (400 MHz, DMSO D-6): δ 10.09 (s br, 1H), 8.69 (s, 1H), 8.03 (s, 4H), 7.85 (d, 1H), 7.73 (d, 1H), 7.61 (t, 2H), 7.10 (s br, 1H), 2.72 (t, 2H), 1.67 (quin, 2H), 1.39 (sx, 2H), 0.94 (t, 3H) ppm. MS (ES+) m/z 425.22[M+1]

**Compound 24**

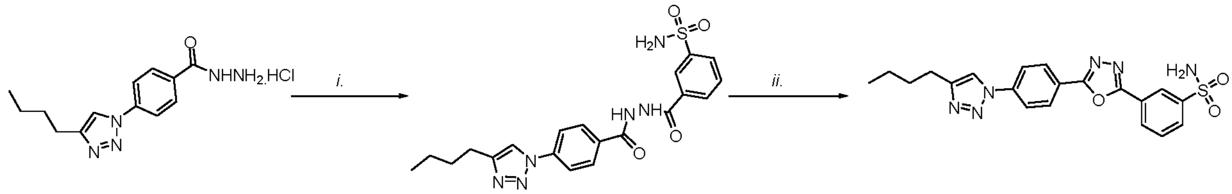


15c 24b 24

**Reagents and conditions:** *i.* 3-sulfamoylbenzoic acid (1.1 eq.), TEA(3.3 eq.), DMAP (0.55 eq.), DMF, EDC·HCl (1.66 eq.), 0°C to RT, 5h.; *ii.* Burgess reagent (6.0 eq.), ACN, 90°C to RT, 6h.

**Procedure for the synthesis of compound 24:**

Compound **15c** 4-(4-butyl-1H-1,2,3-triazol-1-yl)benzohydrazide was prepared as described above in the procedure for the synthesis of compound **15.**

To a stirred solution of **15c** (350 mg, 1 Eq, 1.22 mmol) in DMF (7 ml), was added 3-sulfamoylbenzoic acid (Sigma Aldrich catalogue code CDS011670) (271 mg, 1.1 Eq, 1.35 mmol), TEA (0.56 ml, 3.3 Eq, 4.05 mmol) and DMAP (82 mg, 0.55 Eq, 0.67 mmol) at RT. The reaction mixture was cooled to 0°C then added EDC·HCl (388 mg, 1.66 Eq, 2.02 mmol) at 0°C. The reaction mixture was stirred at for 5 h. Reaction progress was monitored by TLC (70% EtOAc in Petroleum ether, v:v, Rf: 0.5). After completion of reaction, reaction mixture was poured in to cold water (50 ml). The precipitated solid was filtered off, washed with water (2 x 50 ml) and dried under vacuum to afford Compound **24b** as brown solid (120 mg, crude). (Crude was directly taken to next step without further purification).

**3-(2-(4-(4-butyl-1H-1,2,3-triazol-1-yl)benzoyl)hydrazine-1-carbonyl)benzenesulfonamide (24b).** Purity=62.4%; MS (ES+) m/z 443.24[M+1]

To a stirred solution of **24b** (120 mg, 1 Eq, 0.2684 mmol) in ACN (4.8 ml), was added Burgess reagent (383 mg, 6 Eq, 1.6104 mmol), was stirred at 90°C for 6 h. Reaction progress was monitored by TLC (70% EtOAc in Petroleum ether, v:v, Rf: 0.7). After completion of reaction, the reaction mixture was diluted with cold water. The precipitated solid was filtered off, washed with water (2 x 20 ml) and dried under vacuum. Crude compound was purified using preparative HPLC; Column: X-Bridge C18 5um (19 * 250 mm); Mobile Phase: 10 mM ABC in $H_2O$, CAN; Solubility: ACN + water +THF Flow: 15.0 ml/min. Fractions were concentrated to afford Compound **24** as off white solid (20 mg, 17% yield).

**3-(5-(4-(4-butyl-1H-1,2,3-triazol-1-yl)phenyl)-1,3,4-oxadiazol-2-yl)benzenesulfonamide (24).** Yield=17%; Purity=97.6%; $^1$H NMR (400 MHz, DMSO D-6): δ 8.76 (s, 1H), 8.58 (s, 1H), 8.39 (d, 1H), 8.36 (d, 2H), 8.19 (d, 2H), 8.08 (d, 1H), 7.87 (t, 1H), 7.63 (s, 2H), 2.74 (t, 2H), 1.68 (quin, 2H), 1.39 (sx, 2H), 0.94 (t, 3H) ppm. MS (ES+) m/z 425.26[M+1]

**Compound 25**

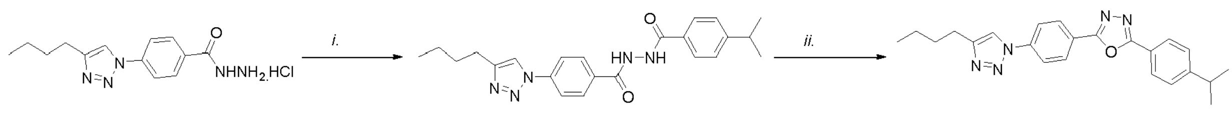


15c 25b 25

**Reagents and conditions:** *i.* 4-isopropylbenzoic acid (0.8 eq.), TEA (2.4 eq.), DMAP (0.08 eq.), DMF, EDC·HCl (1.2 eq.), 0°C to RT, 5h; *ii.* Burgess reagent (6.0 eq.), ACN, 90°C to RT, 6h.

**Procedure for the synthesis of compound 23:**

Compound **15c** 4-(4-butyl-1H-1,2,3-triazol-1-yl)benzohydrazide was prepared as described above in the procedure for the synthesis of compound **15.**

To a stirred solution of **15c** (250 mg, 1 Eq, 1.22 mmol) in DMF (5.0 ml) was added 4-isopropylbenzoic acid (Sigma Aldrich catalogue code 268402) (158 mg, 0.79 Eq, 0.965 mmol), TEA (0.4 ml, 2.4 Eq, 2.89 mmol) and DMAP

(11 mg, 0.08 Eq, 0.096 mmol) at RT, was cooled to 0°C then added EDC·HCl (277 mg, 1.2 Eq, 1.447 mmol) at 0°C, stirred for 5 h at RT. Reaction progress was monitored by TLC (50% EtOAc in Petroleum ether, v:v, Rf: 0.2). After completion of reaction, reaction mixture was poured in cold water (50 ml). The precipitated solid was filtered off, washed with water (2 x 50 ml) and dried under vacuum to afford Compound **25b** as brown solid (150 mg, crude).
**4-(4-butyl-1H-1,2,3-triazol-1-yl)-N'-(4-isopropylbenzoyl)benzohydrazide (25b).** Purity=14%; $^1$H NMR (400 MHz, $CDCl_3$): δ 7.88-7.68 (m, 5H), 7.36 (d, 2H), 7.23 (d, 2H), 3.30 (sept, 1H), 2.82 (t, 2H), 1.83-1.68 (m, 2H), 1.50-1.40 (m, 2H), 1.36-1.19 (m, 6H), 0.97 (t, 3H) ppm. MS (ES+) m/z 406.20[M+1]

To a stirred solution of **25b** (150 mg, 1 Eq, 0.3699 mmol) in ACN (6.0 ml), was added Burgess reagent (528 mg, 6 Eq, 2.219 mmol), the reaction mixture was stirred at 90°C for 6 h. Reaction progress was monitored by TLC (50% EtOAc in Petroleum ether, v:v, Rf: 0.5). After completion of reaction, reaction mixture was diluted with cold water. The precipitated solid was filtered off, washed with water (2 x 20 ml) and dried under vacuum. Crude compound was purified using normal phase column chromatography using silica gel (100-200 mesh) and elution gradient 25% ethyl acetate in petroleum ether (v:v) to afford Compound **25** as off white solid (20 mg, 13% yield).
**2-(4-(4-butyl-1H-1,2,3-triazol-1-yl)phenyl)-5-(4-isopropylphenyl)-1,3,4-oxadiazo|e (25).** Yield=13%; Purity=92.8%; $^1$H NMR (400 MHz, DMSO D-6): δ 8.75 (s, 1H), 8.33 (d, 2H), 8.17 (d, 2H), 8.09 (d, 2H), 7.52 (d, 2H), 3.00 (sept, 1H), 2.74 (t, 2H), 1.68 (quin, 2H), 1.39 (sx, 2H), 1.26 (d, 6H), 0.94 (t, 3H) ppm. MS (ES+) m/z 388.33[M+1]

**Compound 26**

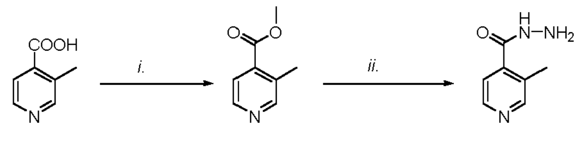


26d 26e 26f

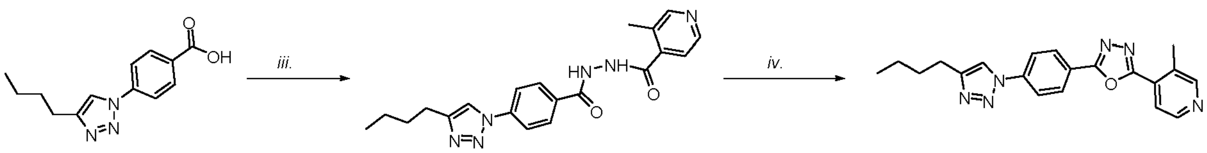


2b 26c 26

**Reagents and conditions:** *i.* $H_2SO_4$ (2.0 eq.), MeOH, 70°C, 16h.; *ii.* Hydrazine hydrate (5.0 eq.), MeOH, 70°C, 16h; *iii.* 3-methylisonicotinohydrazide 26f (1.0 eq.), DMAP (1.5 eq.), EDC.HCl (1.5 eq.), DMF, 0°C to RT, 3h; *iv.* Burgess reagent (6.0 eq.), ACN, 90°C, 4h.

**Procedure for the synthesis of compound 26:**

Compound **2b** (4-(4-butyl-1H-1,2,3-triazol-1-yl)benzoic acid) was prepared as described above in the procedure for the synthesis of compound **2.**

To a stirred solution of **26d** (3-methylisonicotinic acid, Combi-Blocks Inc. catalogue code OR-2584) (500 mg, 1 Eq, 3.64 mmol) in MeOH (5 ml), was added $H_2SO_4$ (0.4 ml, 2 Eq, 7.29 mmol) at 0°C, was heated to 70°C for 16 h. Reaction progress was monitored by TLC (EtOAc, Rf : 0.5). After completion of reaction, the reaction mixture was concentrated under reduced pressure. Crude compound was basified with saturated $NaHCO_3$ solution up to PH~8 and extracted with EtOAC (2 x 50 ml), combined organic layer was dried over anhydrous $Na_2SO_4$, filtrated and concentrated to afford Compound **26e** as off white solid (200 mg, crude).
**Methyl 3-methylisonicotinate (26e).** Purity=91.6%; MS (ES+) m/z 152.08[M+1]

To a stirred solution of **26e** (200 mg, 1 Eq, 1.32 mmol) in MeOH (5 ml), was added hydrazine hydrate (0.3 ml, 5 Eq, 6.62 mmol) at RT, and the mixture heated to 70°C for 16 h. Reaction progress was monitored by TLC (EtOAc, Rf: 0.3). After completion of reaction, the reaction mixture was concentrated under reduced pressure, compound was triturated with diethyl ether (20 ml), filtered and dried to afford Compound **26f** as off white solid (160 mg, crude).
**3-methylisonicotinohydrazide (26f).** Purity=95.1%; MS (ES+) m/z 152.17[M+1]

To a stirred solution of **2b** (160 mg, 1 Eq, 1.059 mmol) in DMF (6 ml), was added **26f** (260 mg, 1 Eq, 1.059 mmol) was cooled to 0°C. To this DMAP (194 mg, 1.5 Eq, 1.58 mmol), EDC·HCl (304 mg, 1.5 Eq, 1.58 mmol) was added

at 0°C, was stirred for 3 h at RT. Reaction progress was monitored by TLC (50% EtOAc in Petroleum ether, v:v, Rf: 0.5). After completion of reaction, the reaction mixture was poured in ice cooled water (30 ml), extracted in EtOAc (2 x 100 ml), organic layer was washed with water (100 ml) and dried over anhydrous $Na_2SO_4$, filtrated and concentrated under vacuum to afford Compound **26c** as off light brown solid (200 mg, crude).

**N'-(4-(4-butyl-1H-1,2,3-triazol-1-yl)benzoyl)-3-methylisonicotinohydrazide (26c).** Purity=57.3%; $^1$H NMR (400 MHz, DMSO D-6): δ 10.76 (s, 1H), 10.52 (s, 1H), 8.71 (s, 1H), 8.57 (d, 2H), 8.13 (d, 2H), 8.07 (d, 2H), 7.39 (d, 1H), 2.73 (t, 2H), 2.43 (s, 3H), 1.67 (quin, 2H), 1.39 (sx, 2H), 0.94 (t, 3H) ppm. MS (ES+) m/z 379.27[M+1]

To a stirred solution of **26c** (200 mg, 1 Eq, 0.529 mmol) in ACN (6 ml), was added Burgess reagent (756 mg, 6 Eq, 3.174 mmol), was heated to 90°C for 4 h. Reaction progress was monitored by TLC (50% EtOAc/Petroleum ether, v:v, Rf: 0.5). After completion of reaction, the reaction mixture was poured in to ice cooled water (50 ml), crude compound was extracted in EtOAc (2 x 100 ml), organic layer was washed with water (100 ml) organic layer was dried over anhydrous $Na_2SO_4$, filtrated and concentrated. Crude compound was purified by column chromatography (Silica gel 100-200 mesh). Eluent gradient 30% EtOAc/Petroleum ether (v:v). Combined column fractions distilled dried to afford Compound **26** as white solid (40 mg, 20% yield).

**2-(4-(4-butyl-1H-1,2,3-triazol-1-yl)phenyl)-5-(3-methylpyridin-4-yl)-1,3,4-oxadiazole (26).** Yield=20%; Purity=99.4%; $^1$H NMR (400 MHz, DMSO D-6): δ 8.75 (d, 2H), 8.69 (d, 1H), 8.36 (d, 2H), 8.19 (d, 2H), 8.07 (d, 1H), 2.77-2.71 (m, 5H), 1.68 (quin, 2H), 1.39 (sx, 2H), 0.94 (t, 3H) ppm. MS (ES+) m/z 361.33[M+1]

**Compound 27**

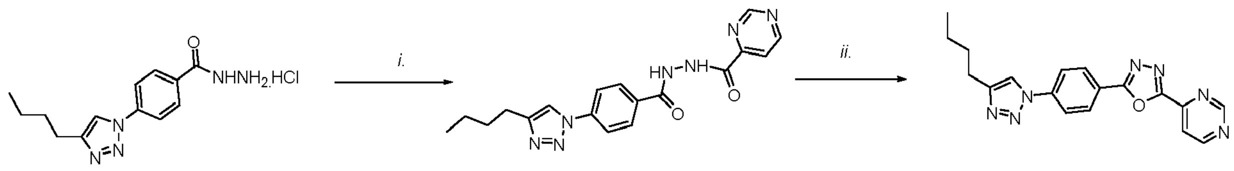


15c 27b 27

**Reagents and conditions:** *i.* Pyrimidine-4-carboxylic acid (1.0 eq.), TEA (3 eq.), DMAP (1.5 eq.), EDC·HCl (1.5 eq.), DMF, 0°C to RT, 3h; *ii.* Burgess reagent (6.0 eq.), ACN, 90°C, 4h.

**Procedure for the synthesis of compound 27:**

Compound **15c** 4-(4-butyl-1H-1,2,3-triazol-1-yl)benzohydrazide was prepared as described above in the procedure for the synthesis of compound **15.**

To a stirred solution of **15c** (300 mg, 1 Eq, 1.15 mmol) in DMF (6 ml), the reaction mixture was cooled to 0°C, was added TEA (0.4 ml, 3 Eq, 3.47 mmol) and reaction mixture was stirred for 5 min, then was added pyrimidine-4-carboxylic acid (Sigma Aldrich catalogue code 730831) (143 mg, 1 Eq, 1.15 mmol), DMAP (212 mg, 1.5 Eq, 1.73 mmol), EDC·HCl (333 mg, 1.5 Eq, 1.73 mmol) at 0°C. The reaction mixture was stirred at RT for 3 h. Reaction progress was monitored by TLC (50% EtOAc in Petroleum ether, v:v, Rf: 0.5). After completion of reaction, the reaction mixture was poured in to ice cooled water (100 ml), extracted with EtOAc (2 x 100 ml), organic layer was washed with water (100 ml) and dried over anhydrous $Na_2SO_4$, filtrated and concentrated under vacuum to afford Compound **27b** as light green solid (166 mg, crude).

**N'-(4-(4-butyl-1H-1,2,3-triazol-1-yl)benzoyl)pyrimidine-4-carbohydrazide (27b).** Purity=66.5%; MS (ES+) m/z 366.10[M+1]

To a stirred solution of **27b** (166 mg, 1 Eq, 0.454 mmol) in ACN (5 ml), was added Burgess reagent (650 mg, 6 Eq, 2.72 mmol), the reaction mixture was heated to 90°C for 4 h. Reaction progress was monitored by TLC (50% EtOAc in Petroleum ether, v:v, Rf: 0.5). After completion of reaction, the reaction mixture was poured in to ice cooled water (50 ml), crude compound was extracted in EtOAc (2 x 100 ml), and organic layer was washed with water (100 ml), dried over anhydrous $Na_2SO_4$, filtrated and concentrated. Crude compound was purified by column chromatography (Silica gel 100-200 mesh), eluted with the gradient 30% EtOAc/Petroleum ether (v:v). Combined column fractions distilled dried to afford Compound **27** as off white solid (38 mg, 24% yield).

**2-(4-(4-butyl-1H-1,2,3-triazol-1-yl)phenyl)-5-(pyrimidin-4-yl)-1,3,4-oxadiazole (27).** Yield=24%; Purity=92.3%; $^1$H NMR (400 MHz, DMSO D-6): δ 9.48 (s, 1H), 9.14 (d, 1H), 8.77 (s, 1H), 8.38-8.33 (m, 3H), 8.20 (d, 2H), 2.74 (t, 2H), 1.68 (quin, 2H), 1.39 (sx, 2H), 0.94 (t, 3H) ppm. MS (ES+) m/z 348.31[M+1]

**Compound 28**

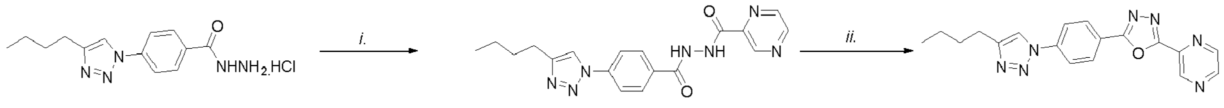


15c 28c 28

**Reagents and conditions:** *i.* Pyrazine-2-carboxylic acid (1.0 eq.), TEA (3 eq.), DMAP (1.5 eq.), EDC.HCl (1.5 eq.), DMF, 0°C to RT, 3h; *ii.* Burgess reagent (5.1 eq.), ACN, 90°C, 4h.

**Procedure for the synthesis of compound 28:**

Compound **15c** 4-(4-butyl-1H-1,2,3-triazol-1-yl)benzohydrazide was prepared as described above in the procedure for the synthesis of compound **15.**

To a stirred solution of **15c** (300 mg, 1 Eq, 1.15 mmol) in DMF (6 ml), the reaction mixture was cooled to 0°C, was added TEA (0.4 ml, 3 Eq, 3.47 mmol) and stirred for 5 min, then was added pyrazine-2-carboxylic acid (Sigma Aldrich catalogue code P56100) (143 mg, 1 Eq, 1.15 mmol), DMAP (212 mg, 1.5 Eq, 1.73 mmol), EDC·HCl (333 mg, 1.5 Eq, 1.73 mmol) at 0°C. The reaction mixture was stirred for 3 h at RT. Reaction progress was monitored by TLC (50% EtOAc in Petroleum ether, v:v, Rf: 0.5). After completion of reaction, the reaction mixture was poured in to ice cooled water (100 ml) crude compound was extracted in EtOAc (2 x 100 ml), organic layer was washed with water (100 ml), organic layer was dried over anhydrous $Na_2SO_4$, filtrated and concentrated to afford Compound **28c** as off light green solid (195 mg, crude).

**N'-(4-(4-butyl-1H-1,2,3-triazol-1-yl)benzoyl)pyrazine-2-carbohydrazide (28c).** Purity=67.4%; MS (ES+) m/z 366.10[M+1]

To a stirred solution of **28c** (195 mg, 1 Eq, 0.534 mmol) in ACN (6 ml), was added Burgess reagent (650 mg, 5.1 Eq, 2.72 mmol), the reaction mixture was heated to 90°C for 4 h. Reaction progress was monitored by TLC (50% EtOAc in Petroleum ether, v:v, Rf: 0.5). After completion of reaction, the reaction mixture was poured in to ice cooled water (50ml), crude compound was extracted in EtOAc (2x100 ml), and organic layer was washed with water (100 ml) and dried over anhydrous $Na_2SO_4$, filtrated and concentrated. Crude compound was purified by column chromatography (Silica gel 100-200 mesh). Eluent gradient 30% EtOAc/Petroleum ether (v:v). Combined column fractions distilled dried to afford Compound **28** as off white solid (40 mg, 20% yield).

**2-(4-(4-butyl-1H-1,2,3-triazol-1-yl)phenyl)-5-(pyrazin-2-yl)-1,3,4-oxadiazole (28).** Yield=20%; Purity=93.8%; $^1H$ NMR (400 MHz, DMSO D-6): δ 9.51 (s, 1H), 9.93 (s, 2H), 8.77 (s, 1H), 8.35 (d, 2H), 8.20 (d, 2H), 2.74 (t, 2H), 1.68 (quin, 2H), 1.39 (sx, 2H), 0.94 (t, 3H) ppm. MS (ES+) m/z 348.31[M+1]

**Compound 29**

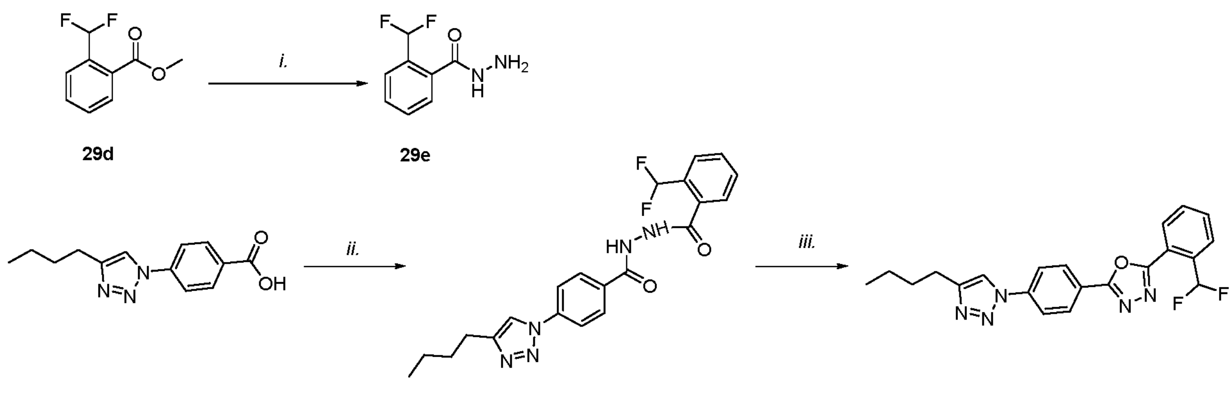


29d 29e

2b 29c 29

**Reagents and conditions:** *i.* Hydrazine hydrate (5.0 eq.), MeOH, 60°C, 18h; *ii.* 2-(difluoromethyl)benzohydrazide **29e** (1.2 eq.), DMAP (1.5 eq.), DCM, EDC·HCl (1.5 eq.), 0°C to RT, 5h; *iii.* Burgess reagent (5.0 eq.), ACN, 90°C, 4h.

**Procedure for the synthesis of compound 29:**

Compound **2b** (4-(4-butyl-1H-1,2,3-triazol-1-yl)benzoic acid) was prepared as described above in the procedure for the synthesis of compound **2.**

To a stirred solution of **29d** (methyl 2-(difluoromethyl)benzoate, Sigma Aldrich catalogue code 765805) (500mg, 1 Eq, 2.6852 mmol) in MeOH (10ml), was added Hydrazine hydrate (671mg, 5 Eq, 13.426 mmol), and heated to 60°C. The reaction mixture was stirred for 18 h at 60°C. Reaction progress was monitored by TLC (EtOAc, Rf: 0.1). After completion of reaction, the reaction mixture was evaporated and added ether; precipitate was formed and filtered to afford Compound **29e** as white solid (450 mg, 90% yield).

**2-(difluoromethyl) benzohydrazide (29e).** Yield=90%; Purity=91.6%; MS (ES+) m/z 187.05[M+1]

To a stirred solution of **2b** (300 mg, 1 Eq, 1.224 mmol) in DCM (6 ml, 20V), was added **29e** (273 mg, 1.2 Eq, 1.46 mmol), and DMAP (224mg, 1.5 Eq, 1.837 mmol) at RT. The reaction mixture was cooled to 0°C then added EDC·HCl (352 mg, 1.5 Eq, 1.8367 mmol) at 0°C. The reaction mixture temperature raised to RT, and was stirred for 5 h at RT. Reaction progress was monitored by TLC (EtOAc, Rf : 0.4). After completion of reaction, the reaction mixture was poured in to cold water (20 ml). Extracted with ethyl acetate (2 x 20 ml). Combined ethyl acetate fractions were evaporated under vacuum to afford Compound **29c** as brown solid (400 mg, 79.2% yield).

**N'-(4-(4-butyl-1H-1,2,3-triazol-1-yl)benzoyl)-2-(difluoromethyl)benzohydrazide (29c).** Yield=79.2%; Purity=61.9%; MS (ES+) m/z 414.30[M+1]

To a stirred solution of **29c** (400 mg, 1 Eq, 0.96 mmol) in ACN (5 ml), was added Burgess reagent (1.15g, 5 Eq, 4.8426 mmol), the reaction mixture was stirred at 90°C for 4h. The reaction progress was monitored by TLC (EtOAc, Rf: 0.7). After completion of reaction, the reaction mixture was diluted with cold water precipitate was formed, solid was filtered off, washed with water (2 x 10 ml) and dried under vacuum. Crude was washed with Diethyl ether to afford Compound **29** as off white solid (80 mg, 20% yield).

**2-(4-(4-butyl-1H-1,2,3-triazol-1-yl)phenyl)-5-(2-(difluoromethyl)phenyl)-1,3,4-oxadiazole (29).** Yield=20%; Purity=96.1%; $^1$H NMR (500 MHz, DMSO D-6): δ 8.76 (s, 1H), 8.34 (d, 2H), 8.28 (d, 1H), 8.19 (d, 2H), 7.95 (d, 1H), 7.84 (t, 2H), 7.76 (t, J=109 Hz, 1H), 2.74 (t, 2H), 1.68 (quin, 2H), 1.39 (sx, 2H), 0.94 (t, 3H) ppm. $^{19}$F NMR (DMSO D-6, 470 MHz): δ -112.13 (d, J = 116 Hz, 2F) ppm. MS (ES+) m/z 396.28[M+1]

**Compound 30**

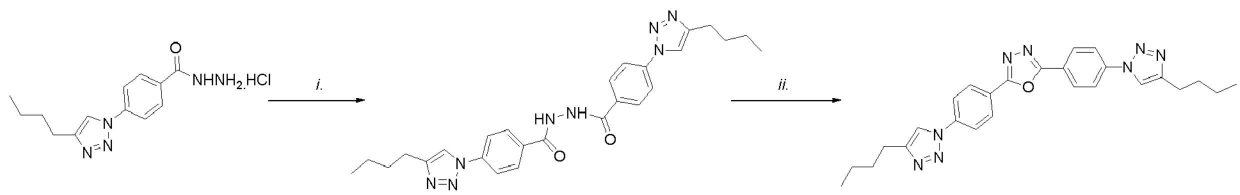


15c 30c 30

**Reagents and conditions:** *i.* 4-(4-butyl-1H-1,2,3-triazol-1-yl)benzoic acid **2b** (1.0 eq.), DIPEA (5.0 eq.), HATU (1.5 eq.), DMF, 0°C to RT, 16h; *ii.* Burgess reagent (10.0 eq.), ACN, 90°C, 18h.

**Procedure for the synthesis of compound 30:**

Compound **2b** (4-(4-butyl-1H-1,2,3-triazol-1-yl)benzoic acid) was prepared as described above in the procedure for the synthesis of compound **2.**

Compound **15c** 4-(4-butyl-1H-1,2,3-triazol-1-yl)benzohydrazide was prepared as described above in the procedure for the synthesis of compound **15.**

To a stirred solution of **15c** (300 mg, 1 Eq, 1.0204 mmol) in DMF (6 ml) was added **2b** (250 mg, 1 Eq, 1.0204 mmol), and DIPEA (655 mg, 5 Eq, 5.0847 mmol) at RT. The reaction mixture was cooled to 0°C, and then added HATU (579mg, 1.5 Eq, 1.52 mmol) at 0°C. The reaction mixture was raised to RT and stirred at RT for 16 h. Reaction progress was monitored by TLC (EtOAc, Rf: 0.3). After completion of reaction, the reaction mixture was poured in to cold water (20 ml). The precipitate was formed, Solid was filtered off, washed with water (2 x 20 ml) and dried under vacuum to afford Compound **30c** as off white solid (310 mg, crude).

**4-(4-butyl-1H-1,2,3-triazol-1-yl)-N'-(4-(4-butyl-1H-1,2,3-triazol-1-yl)benzoyl)benzohydrazide (30c).** MS (ES+) m/z 487.25[M+1]

To a stirred solution of **30c** (310 mg, 1 Eq, 0.6365 mmol) in ACN (5 ml), was added Burgess reagent (1.51g, 10 Eq, 6.365 mmol), the reaction mixture was stirred at 90°C for 18 h. The reaction progress was monitored by TLC (EtOAc, Rf: 0.7). After completion of reaction, reaction mixture was diluted with cold water precipitate was formed, solid was filtered off, washed with water (2 x 10 ml) and dried under vacuum. Purification: Crude compound was purified under silica gel (100-200 mesh) column; compound was eluted with 2% MeOH in DCM. Pure fractions were concentrated under vacuum to afford Compound **30** as white solid (30 mg, 10% yield).

**2,5-bis(4-(4-butyl-1H-1,2,3-triazol-1-yl)phenyl)-1,3,4-oxadiazole (30).** Yield=10%; Purity=96.8%; $^1$H NMR (400 MHz, $CDCl_3$): δ 8.33 (d, 4H), 7.97 (d, 4H), 8.83 (s, 2H), 2.84 (t, 4H), 1.75 (quin, 4H), 1.47 (sx, 4H), 0.98 (t, 6H) ppm. MS (ES+) m/z 469.28[M+1]

**Compound 31**

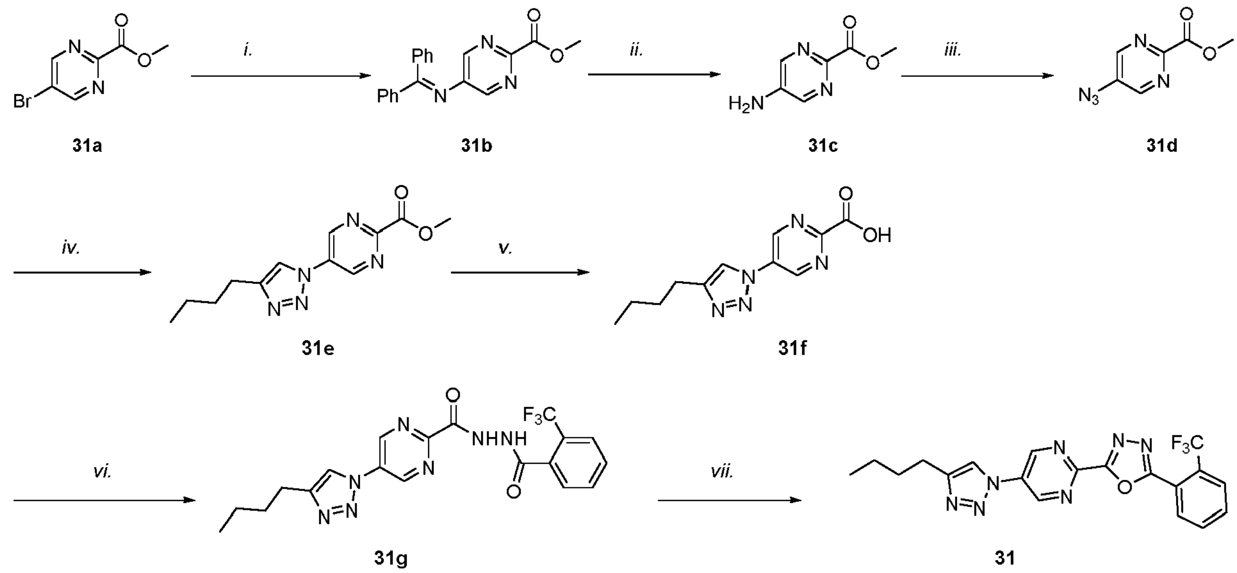


**Reagents and conditions:** *i.* Benzophenone imine (1.5 eq.), $Cs_2CO_3$ (1.5 eq.), Toluene, $Pd(OAc)_2$ (0.05 eq.), BINAP (0.1 eq.), 110°C, 4h; *ii.* Aq. HCl, THF, RT, overnight; *iii.* Isoamyl nitrite (1.5 eq.), TMS-azide (1.2 eq.), ACN, 0°C to RT, 3h; iv. Hex-1-yne (1 eq.), TEA (3 eq.), DMAP (1.5 eq.), EDC.HCl (1.5 eq.), 0°C to RT, 3h; v. NaOH (3.0 eq.), Methanol:water (1:1, v/v), 0°C to RT, 6h; vi. 2-(trifluoromethyl)benzohydrazide (1.2 eq.), DMAP (1.5 eq.), DMF, EDC.HCl (1.5 eq.), 0°C to RT, 16h; *vii.* Burgess reagent (10.0 eq.), ACN, 80 °C, 16h.

**Procedure for the synthesis of compound 31:**

To a stirred solution of **31a** (methyl 5-bromopyrimidine-2-carboxylate, ENAMINE Ltd catalogue code EN300-111521) (500 mg, 1 Eq, 2.304 mmol) in toluene (25 ml) was added benzophenone imine (Sigma Aldrich catalogue code 293733) (626 mg, 1.5 Eq, 3.456 mmol) and cesium carbonate (1.12 g, 1.5 Eq, 3.456 mmol) added and argon purged for 10 min then $Pd(OAc)_2$ (77.6 mg, 0.05 Eq, 0.1152 mmol), BINAP (143 mg, 0.1 Eq, 0.2304 mmol) was added and again argon purged for another 10 min, then the reaction mixture was heated to 110°C for 4 h. Reaction progress was monitored by TLC (50% EtOAc in Petroleum ether, v:v, Rf: 0.4). After completion of reaction, reaction mixture was cooled to RT and filtered through the Celite bed, the bed washed with ethyl acetate (20 ml), filtrate concentrated. Crude compound was purified through column chromatography (Silica gel 100-200 mesh). Eluent gradient 30% EtOAc/Petroleum ether (v:v). Combined column fractions were distilled under vacuum to afford Compound **31b** as pale yellow solid (800 mg, 59% yield).

**Methyl 5-((diphenylmethylene)amino)pyrimidine-2-carboxylate (31b).** Yield=59%; Purity=92.2%; $^1$H NMR (400 MHz, $CDCl_3$): δ 8.32 (s, 2H), 7.80 (d, 2H), 7.56 (t, 1H), 7.46 (t, 2H), 7.40-7.30 (m, 3H), 7.11 (d, 2H), 4.02 (s, 3H) ppm. MS (ES+) m/z 318.10[M+1]

To a stirred solution of **31b** (800 mg, 1 Eq, 2.523 mmol) in THF (12 ml) was added aqueous HCl (4 ml) and

stirred overnight at RT. Reaction progress was monitored by TLC (EtOAc, Rf: 0.2). After completion of reaction, the precipitated solid was filtered off, washed with THF (5 ml) and dried under vacuum, then solid was dissolved in methanol (25 ml) basified with Amberlite resin (basic) filtered and concentrated and dried under reduced pressure to afford Compound **31c** as off white solid (400 mg, crude).

**Methyl 5-aminopyrimidine-2-carboxylate (31c).** Purity=98.8%; $^1$H NMR (400 MHz, DMSO D-6): δ 8.15 (s, 2H), 6.35 (d, 2H), 3.79 (s, 3H) ppm. MS (ES+) m/z 154.18[M+1]

To a stirred solution of **31c** (2 g, 1 Eq, 13.071 mmol) in ACN (60 ml) was added Isoamyl nitrite (Sigma Aldrich catalogue code 150495) (2.29 g, 1.5 Eq, 19.607 mmol) and cooled to 0°C, then TMS-azide (Sigma Aldrich catalogue code 155017) (1.80 g, 1.2 Eq, 15.685 mmol) added dropwise at 0°C. The reaction mixture slowly allowed to reach RT for 3 h (Raised to RT and sudden increased to reflux temperature observed). Reaction progress was monitored by TLC (10% MeOH in DCM, Rf: 0.5). After completion of reaction, the reaction mixture was concentrated under vacuum and dried under reduced pressure to afford Compound **31d** as brown gummy (2 g, crude).

**Methyl 5-azidopyrimidine-2-carboxylate (31d).** Purity=73.7%; MS (ES+) m/z 180.18[M+1]

To a stirred solution of **31d** (2 g, 33.4 Eq, 38.43 mmol) in DMF (6 ml) was added hex-1-yne (Sigma Aldrich catalogue code 244422) (143 mg, 1 Eq, 1.15 mmol), TEA (0.4 ml, 3 Eq, 3.47 mmol) and DMAP (212 mg, 1.73 mmol) at RT. The reaction mixture was cooled to 0°C then added EDC·HCl (333 mg, 1.5 Eq, 1.73 mmol) at 0°C. The reaction mixture was stirred for 3 h at RT. Reaction progress was monitored by TLC (50% EtOAc in Petroleum ether, v:v, Rf: 0.5). After completion of reaction, the reaction mixture was poured in to water (100 ml), precipitated solid was filtered off, washed with water (2 x 100 ml) and dried under vacuum to afford Compound **31e** as lite light green solid (166 mg, crude).

**Methyl 5-(4-butyl-1H-1,2,3-triazol-1-yl)pyrimidine-2-carboxylate (31e).** Purity=94.4%; $^1$H NMR (400 MHz, DMSO D-6): δ 9.54 (s, 2H), 8.85 (s, 1H), 3.95 (s, 3H), 2.76 (t, 2H), 1.67 (quin, 2H), 1.39 (sx, 2H), 0.95 (t, 3H) ppm. MS (ES+) m/z 262.25[M+1]

To a stirred solution of **31e** (1.0 g, 1 Eq, 3.8314 mmol) in methanol (15 ml) was added NaOH solution (459 mg, 3 Eq, 11.494 mmol in 15 ml water) at 0°C, slowly raised to RT for 6 h. Reaction progress was monitored by TLC (10% MeOH in DCM, Rf : 0.1), after completion of reaction, the reaction mixture was concentrated under vacuum, residue diluted with water (20 ml) and cooled to 0°C, pH adjusted to 6 using 2 N HCl solution. The precipitated solid was filtered off, washed with water (2 x 10 ml) and dried under vacuum to afford Compound **31f** as off white solid (600 mg, 63% yield).

**5-(4-butyl-1H-1,2,3-triazol-1-yl)pyrimidine-2-carboxylic acid (31f).** Yield=63%; Purity=95.5%; $^1$H NMR (500 MHz, DMSO D-6): δ 9.67-9.54 (s br, 2H), 8.79 (s, 1H), 2.76 (t, 2H), 1.67 (quin, 2H), 1.39 (sx, 2H), 0.95 (t, 3H) ppm. MS (ES+) m/z 248.22[M+1]

To a stirred solution of **31f** (250 mg, 1 Eq, 1.01 mmol) in DMF (12.5 ml), was added 2-(trifluoromethyl)benzohydrazide (Combi-Blocks Inc. catalogue code QA-6000) (247 mg, 1.2 Eq, 1.21 mmol) and DMAP (185.2 mg, 1.5 Eq, 1.52 mmol) at RT. The reaction mixture was cooled to 0°C then added EDC·HCl (290.7 mg, 1.5 Eq, 1.52 mmol), stirred for 16 h at RT. Reaction progress was monitored by TLC (10% MeOH in DCM, Rf : 0.3). After completion of reaction, the reaction mixture was poured in to water (100 ml), precipitated solid was filtered off, washed with water (2 x 100 ml) and dried under vacuum, solid was triturated with diethyl ether (10 ml), filtered and dried to afford Compound **31g** as light brown solid (150 mg, 34% yield).

**5-(4-butyl-1H-1,2,3-triazol-1-yl)-N'-(2-(trifluoromethyl)benzoyl)pyrimidine-2-carbohydrazide (31g).** Yield=34%; Purity=92.3%; $^1$H NMR (500 MHz, DMSO D-6): δ 11.43 (s br, 1H), 9.45 (s, 2H), 8.80 (s, 1H), 7.89-7.52 (m, 4H), 2.75 (t, 2H), 1.68 (quin, 2H), 1.40 (sx, 2H), 0.94 (t, 3H) ppm. MS (ES+) m/z 434.21 [M+1]

To a stirred solution of **31g** (150 mg, 1 Eq, 0.346 mmol) in ACN (7.5 ml) was added Burgess reagent (823 mg, 10 Eq, 3.460 mmol), the reaction mixture was stirred at 80°C for 16 h. Reaction progress was monitored by TLC (EtOAc, Rf: 0.6). After completion of reaction, the reaction mixture was poured in to water (10 ml) and stirred for 20 min. The precipitated solid was filtered off, washed with water (2 x 10 ml) and dried under vacuum, solid was triturated with diethyl ether (10 ml) for 30 min, filtered and dried to afford Compound **31** as pale brown solid (70 mg, 48% yield).

**2-(5-(4-butyl-1H-1,2,3-triazol-1-yl)pyrimidin-2-yl)-5-(2-(trifluoromethyl)phenyl)-1,3,4-oxadiazole (31).** Yield=48%; Purity=98.4%; $^1$H NMR (400 MHz, DMSO D-6): δ 9.65 (s, 2H), 8.86 (s, 1H), 8.18 (d, 1H), 8.09 (d, 1H), 7.99 (t, 1H), 7.97 (t, 1H), 2.78 (t, 2H), 1.69 (quin, 2H), 1.40 (sx, 2H), 0.94 (t, 3H) ppm. MS (ES+) m/z 416.27[M+1]

**Compound 32**

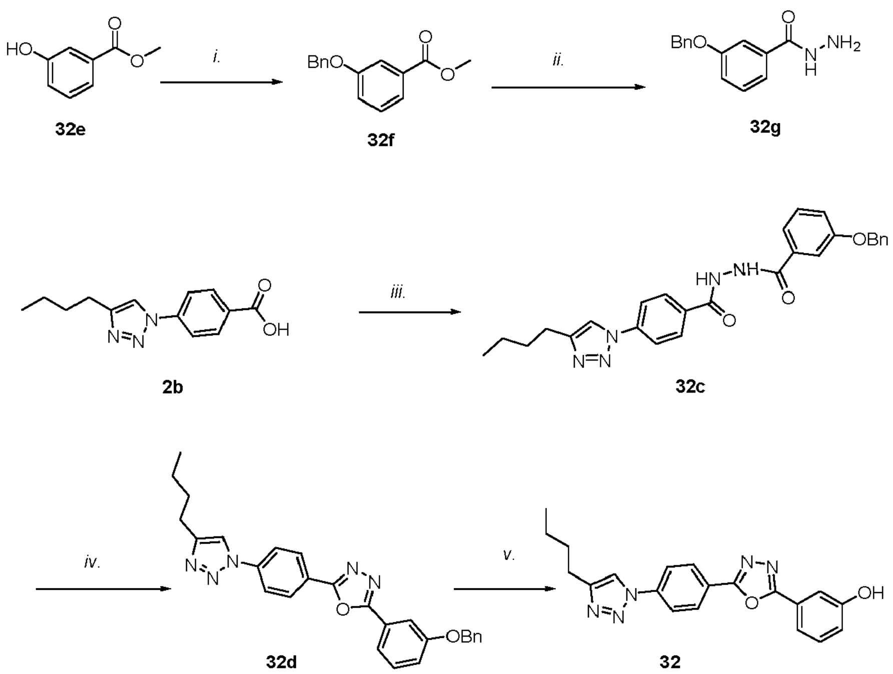


32e 32f 32g

2b 32c

32d 32

**Reagents and conditions:** *i.* NaH (1.2 eq.), DMF, 0°C, 30min then Benzyl Bromide (1.1 eq.), RT, 2h; *ii.* Hydrazine hydrate (10 eq.), MeOH, 60°C, 6h; *iii.* 3-(benzyloxy)benzohydrazide 32g (1.1 eq.), DMAP (1.5 eq.), DMF, EDC·HCl (1.5 eq.), 0°C to RT, 5h; *iv.* Burgess reagent (6.0 eq.), ACN, 90 °C, 4h; *v.* THF:DCM:MeOH (3:3:1 v/v) Pd/C (17% w/w), $H_2$, RT, 7h.

**Procedure for the synthesis of compound 32:**

Compound **2b** (4-(4-butyl-1H-1,2,3-triazol-1-yl)benzoic acid) was prepared as described above in the procedure for the synthesis of compound **2.**

To a stirred solution of **32e** (methyl 3-hydroxybenzoate, Sigma Aldrich catalogue code 252794) (2 g, 1 Eq, 13.157 mmol) in DMF (20 ml) cooled to 0°C, 50% NaH (0.75mg, 1.2 Eq, 15.788 mmol) was portion wise added to reaction mixture, and stirred at 0°C for 30 min. To this Benzyl Bromide (Sigma Aldrich catalogue code B17905) (2.47 g, 1.1 Eq, 14.47 mmol) was added, and stirred at RT for 2 h. Reaction progress was monitored by TLC (30% EtOAc in Petroleum ether, v:v, Rf: 0.7). After completion of reaction, the reaction mixture was poured in to ice cold water (100 ml). The precipitated solid was filtered, washed with water (2 x 50 ml) and dried under vacuum to afford Compound **32f** as off white solid (1.9 g, 61% yield).

**Methyl 3-(benzyloxy)benzoate (32f).** Yield=61%; Purity=98.8%; $^1$H NMR (400 MHz, $CDCl_3$): δ 7.66 (d, 2H), 7.44 (t, 2H), 7.40 (t, 1H), 7.38 (s, 1H), 7.34 (d, 2H), 7.16 dt, 1H), 5.11 (s, 2H), 3.91 (t, 3H) ppm. MS (ES+) m/z 243.18[M+1]

To a stirred solution **of 32f** (1.2 g, 1 Eq, 4.95 mmol) in MeOH (12 ml), was added hydrazine hydrate (2.69 ml, 10 Eq, 49.53 mmol) stirred for 10 min, then heated at 60°C for 6 h. Reaction progress was monitored by TLC (50% EtOAc in Petroleum ether, v:v, Rf : 0.1). After completion of reaction, the reaction mixture was completely distilled off. Crude solid was triturated with diethyl ether (2 x 20 ml), decant and dried under vacuum to afford Compound **32g** as off white solid (1.0 g, 66% yield).

**3-(benzyloxy)benzohydrazide (32g).** Yield=66%; Purity=98.0%; $^1$H NMR (400 MHz, $CDCl_3$): δ 7.49 (s, 1H), 7.47-7.34 (m, 7H), 7.13 dt, 1H), 5.13 (s, 2H), 4.11 (s br, 2H) ppm. MS (ES+) m/z 243.18[M+1]

To a stirred solution of **2b** (500 mg, 1 Eq, 2.040 mmol) in DMF (10 ml), was added **32g** (542 mg, 1.1 Eq, 2.24 mmol), DMAP (373 mg, 1.5 Eq, 3.06 mmol) at RT, cooled to 0°C. To this EDC·HCl (586 mg, 1.5 Eq, 3.06 mmol) was added at 0°C, and stirred at RT for 5 h. Reaction progress was monitored by TLC (70% EtOAc in Petroleum ether, v:v, Rf: 0.5). After completion of reaction, the reaction mixture was poured in to cold water (50 ml). The precipitated solid was filtered off, washed with water (2 x 50 ml) and dried under vacuum to afford Compound **32c** as light brown solid (900 mg, crude). (Crude was directly taken to next step without further purification).

**3-(benzyloxy)-N'-(4-(4-butyl-1H-1,2,3-triazol-1-yl)benzoyl)benzohydrazide (32c).** Purity=96.1%; MS (ES+) m/z 470.10[M+1]

To a stirred solution of **32c** (0.9g, 1 Eq, 1.9189 mmol) in ACN (36 ml), was added Burgess reagent (2.7g, 6 Eq, 11.5138 mmol), heated to 90°C for 4 h. Reaction progress was monitored by TLC (50% EtOAc in Petroleum ether,

v:v, Rf: 0.5). After completion of reaction, the reaction mixture was diluted with cold water. The precipitated solid was filtered and dried under vacuum to afford Compound **32d** as brown solid (0.7 g, Crude).

**2-(3-(benzyloxy)phenyl)-5-(4-(4-butyl-1H-1,2,3-triazol-1-yl)phenyl)-1,3,4-oxadiazole (32d).** Purity=99.0%; $^1H$ NMR (400 MHz, DMSO D-6): δ 8.76 (s,1H), 8.36 (d, 2H), 8.18 (d, 2H), 7.78 (s, 1H), 7.77 (d, 1H), 7.57 (t, 1H), 7.54 (d, 2H), 7.43 (t, 2H), 7.37 (d, 1H), 7.34 (d, 1H), 5.26 (s, 2H), 2.74 (t, 2H), 1.68 (quin, 2H), 1.40 (sx, 2H), 0.94 (t, 3H) ppm. MS (ES+) m/z 452.23[M+1]

To a stirred solution of **32d** (0.3 g, 1 Eq, 6.6518 mmol) in THF (9 ml), DCM (9 ml) and MeOH (3 ml) (poor soluble) was added 10% Pd/C (50 mg, 17% w/w), and applied $H_2$ (balloon) pressure was stirred at RT for 7 h. Reaction progress was monitored by TLC (50% EtOAc in Petroleum ether, v:v, Rf: 0.5). After completion of reaction, the reaction mixture was filtered through Celite bed washed with MeOH:DCM (1:1) (15 ml) and concerted under vacuum. Crude comp was washed with diethyl ether (10 ml), filtered and dried to afford Compound **32** as off white solid (0.17 g, 70% yield).

**3-(5-(4-(4-butyl-1H-1,2,3-triazol-1-yl)phenyl)-1,3,4-oxadiazol-2-yl)phenol (32).** Yield=70%; Purity=96.6%; $^1H$ NMR (500 MHz, DMSO D-6): δ 10.03 (s, 1H), 8.76 (s,1H), 8.32 (d, 2H), 8.18 (d, 2H), 7.60 (d, 1H), 7.54 (t, 1H), 7.45 (t, 1H), 7.05 (dd, 1H), 2.74 (t, 2H), 1.67 (quin, 2H), 1.39 (sx, 2H), 0.94 (t, 3H) ppm. MS (ES+) m/z 362.08[M+1]

## EXAMPLE 3: BIOLOGICAL DATA

## MATERIALS AND METHODS

### Enzymatic binding

The binding of compounds to DDX3X have been assessed by nanoDSF analysis (NanoTemper Technologies GmbH, Flößergasse 4, 81369 München, Germany). NanoDSF technology is a Differential Scanning Fluorimetry method which measures the protein stability with high resolution without the need of a dye. In particular it measures the changes of the intrinsic tryptophan fluorescence at 330 and 350 nm and it is used to determine the melting temperature of a protein. In the folded state the residues of tryptophan are buried in the hydrophobic core. During denaturation the residues are more exposed and the fluorescence changes in both intensity and wavelength. In a typical experiment, the protein is mixed with the ligand in the reaction buffer and transferred into a capillary. The fluorescence is monitored over temperature gradient between 20 and 95°C with an increment of 2.5°C per minute. The interaction of the ligand to the folded state of the protein shifts the protein melting temperature (Tm) at higher or lower temperature.

Experimental conditions: the experiments were conducted with the device Prometheus NT.48 (NanoTemper Technologies GmbH), the enzyme used was the human DDX3X (residues 132-607, UniProtID: 000571, produced by Proteros GmbH). The assessed compounds were dissolved in pure DMSO adequate to obtain a concentration of compound of 100μM and DMSO 1% (v:v) in final solution. Reaction buffer for DDX3X was 20 mM HEPES, pH 7.5, 0.5 mM TCEP, 500 mM NaCl, 10% (v:v) Glycerin, with a final reaction volume of 10 μL and a final DMSO concentration of 1% (v:v). The reaction was performed in "High sensitivity capillaries" in the range of 20-95°C with a heating rate of 2.5°C/min, with an incubation time of 60 minutes.

Pipetting sequence:

1) Add appropriate volume of target enzyme in reaction buffer
2) Add compound(s) in 100% DMSO (1% final DMSO conc.)
3) Fill the capillary
4) Measure fluorescence

The assay development was performed with DDX3X protein alone showing a Tm at 54.6 °C.

### Helicase Assay

The DDX3X protein was obtained by immunoprecipitation from 293T cells ($3x10^5$) were seeded in 6-well plates and, after 24h, transfected with 1 μg of HA-DDX3 (Addgene plasmid # 44975) by using the Lipofectamine reagent (Thermo Fischer Scientific). After 36 h, cells were lysed in NP-40 Lysis Buffer (20 mM HEPES pH=7.5, 10 mM EGTA pH=8, 40 mM β-glycerolphosphate, 1% NP40, 2.5 mM MgCl2, 2 mM Orthovanadate, 2 mM NaF, 1 mM DTT, and 1X protease inhibitors mixture) and 2 mg of proteins were immunoprecipitated with anti-HA antibodies (Covance) in NP40 lysis buffer. Immunoprecipitated samples were washed twice with lysis buffer and one time in helicase reaction buffer (50 mM Tris HCl pH=8, 1 mM DTT, 5% glycerol, 10 mM $MgCl_2$). Beads were then resuspended in 100 μl in helicase

reaction buffer.

The helicase activity of DDX3 was tested by measuring the unwinding of an 18/36mer double stranded (ds) RNA oligonucleotide (Garbelli et al., Targeting the Human DEAD-Box Polypeptide 3 (DDX3) RNA Helicase as a Novel Strategy to Inhibit Viral Replication. Curr Med Chem 2011, 18, 3015):

| | |
|---|---|
| **r36mer** | 5'-ACCAGCUUUGUUCCUUGGGUUCUUGGGAGCAGCAGG-3' |
| **r18mer_FAM** | [6FAM]-5'-CCCAAGAACCCAAGGAAC-3' |

The 36mer oligonucleotides sequence is complementary to the 18mer. A RNA oligonucleotide homologous to the 18mer (without 6-FAM modification) was used as competitor to avoid reannealing of the probed RNA after unwinding. Annealing reactions were performed in 50 mM Tris HCl pH=8, 10mM $MgCl_2$, 0.2 μM of r18mer and 0.2 μM of r36mer. A sample with the final concentration of 20 nM of dsRNA plus 100 nM of the competitor were heated at 90°C for 1 min to test efficiency of competition.

Ultimately, reactions were performed in a final volume 100 μl of helicase reaction buffer plus ATP 4 mM, 30 μl of beads used to immunoprecipitate HA-DDX3X, 20 nM of 18/36mer dsRNA and 100 nM of competitor at 37°C for 1 hour and stopped by adding 10 μl of a stopping solution (EDTA 60 mM pH=8, 40% glycerol, 0.6% SDS, 1% Orange G). Samples were next loaded on a 15% polyacrylamide TBE gel containing 1% SDS in 1x TBE buffer with 0.1% SDS and run at 4°C for 1.5 hours. The product of the unwinding reaction, r18mer_FAM and the dsRNA were visualized by an Image-Quant Las4000 (GE Healthcare) with Cy3 detection filter and Green Epi light. Quantification of the ssRNA was performed by the ImageJ software (version 1.53, NIH).

**Cell lines**

Cell lines were purchased from ATCC, except HuH7 and Peripheral Blood Mononuclear Cells (PBMC) (Table 4). Peripheral Blood Mononuclear Cells (PBMC) were isolated from healthy donors blood using Ficoll gradient centrifugation according to manufacturer (Pharmacia). Huh-7 cells were obtained from Japanese Cancer Research Resources Bank (JCRB0403).

Table 4. Source and description of the cell lines employed to characterize the compounds described in the current invention; if applicable for each cell line the ATCC ID (https://www.lgcstandards-atcc.org/) is reported.

| Name | Catalogue ID | Source | Known mutated genes and reported resistance | Cell origin | Medium |
|---|---|---|---|---|---|
| U87 | ATCC: HTB-14 | Glioblastoma | CDKN2A; PTEN; CDKN2C | Primary | EMEM |
| Huh-7 | JCRB: JCRB0403 | Hepatocellular carcinoma | TP53 | Primary | DMEM |
| A549 | ATCC: CCL-185 | Lung adenocarcinoma | K-RAS; HER2neu; LKB1; CDKN2A; gefitinib/erlotinib resistance reported | Primary | RPMI-1640 |
| A549 Red-FLuc | Perkin Elmer catalogue number: bw119266 | Lung adenocarcinoma | K-RAS; HER2neu; LKB1; CDKN2A; gefitinib-erlotinib resistance reported | Primary | RPMI-1640 |
| A375 | ATCC: CRL-1619 | malignant melanoma | BRAF; CDKN2A | Primary | DMEM |
| PANC-1 | ATCC: CRL-1469 | epithelioid pancreatic carcinoma | TP53 | Primary | DMEM |
| K562 | ATCC: CCL-243 | chronic myelogenous leukemia | CDKN2A; TP53 | Primary | DMEM |
| CALU1 | ATCC: HTB-54 | Lung adenocarcinoma | K-RAS; HER2neu; TERT; p53; gefitinib-erlotinib resistance reported | Pleural metastasis | DMEM |

(continued)

| Name | Catalogue ID | Source | Known mutated genes and reported resistance | Cell origin | Medium |
|---|---|---|---|---|---|
| SKLU1 | ATCC: HTB-57 | Lung adenocarcinoma | K-RAS; CDKN2A; p53; ARID1A; HER2neu; gefitinib-erlotinib resistance reported | Primary | DMEM |
| MCF10a | ATCC: CRL-10317 | Normal breast tissue | INK4 p16/p15 locus | n.a. | DMEM/F12 |
| DU145 | ATCC: HTB-81 | Prostate cancer | RB1; CDKN2A | Brain metastasis | EMEM |
| H9 | ATCC: HTB-176 | cutaneous T lymphocyte lymphoma | CDKN2A; N-RAS; RB1; | Primary | RPMI-1640 |
| MDAMB 231 | ATCC: HTB-26 | Triple Negative breast cancer | BRCA1; BRCA2; B-RAF; K-RAS; CDKN2A; TERT; p53; Wnt7b; low sensitivity to Olaparib reported | Pleural effusion | RPMI-1640 |
| PBMC | Cell Applications, Inc., catalogue number: 690PBK-100a | Peripheral Blood from healthy donors | n.a. | n.a. | RPMI-1640 |

**Cytotoxicity assays**

The anti-tumor activity of the compounds of the present invention was evaluated as the effect on the proliferation and survival of tumor cells by counting the number of living cells in presence or absence of the compounds ("cell count method"), and as the effect on the viability and metabolic activity of the cells, by using particular substrates that are converted by cellular enzymes into coloured or luminescent products, proportionally with metabolic activity and state of the cell, such as the MTS tetrazolium compound ("MTS method") or luciferin ("ATP-based method") respectively.

The non-specific toxicity of the compounds is evaluated in non-tumor immortalized cells with cell count and / or MTS methods, and in primary Peripheral Blood Mononuclear Cells (PBMC) with the ATP-based method.

Tumor and non-tumor immortalized cells were cultured in the suggested medium supplemented with 10% FBS, 2 mM L-glutamine and 1000 units/ml Penicillin/Streptomycin at 37°C in 5% $CO_2$ atmosphere according to manufacturer instructions (for the medium used with each cell line see reference in Table 4). Cells were seeded at 3 to 5 x $10^4$ cells/well density depending on cell lines and maintained at 37°C in 5% $CO_2$. After 24 hours cells were treated with ascending concentrations of the investigational compounds and the reference (5-fluoro-Uracyl) in a range from 0 to 100 $\mu$M. Compounds were dissolved in DMSO which final concentration in each well was 0.5% v:v. After 72 h of incubation with compounds, the cells were detached with trypsin and re-suspended in 1 ml of complete medium; the cell number was evaluated using a Z2 Coulter Counter ("cell count method") (Attallah and Johnson, A simple highly sensitive methods for the determination of cell viability using an electronic particle analyzer, Coulter counter J. Immunol. Methods. 1981, 41, 155; Leonardi et al., Activated kinase screening identifies the IKBKE oncogene as a positive regulator of autophagy. Autophagy, 2019, 15, 312). Alternatively, after the 72 hours of incubation with compounds, the MTS solution (CellTiter 96® AQueous One Solution Cell Proliferation Assay) is added to each well and absorbance at 490 nm wavelength is read after 2 hours of incubation ("MTS method"). Results in Tables 8-13, 17, 18 were obtained with the cell count method. Results in Tables 14-15 were obtained with the MTS method. Results in Table 16 were obtained with ATP-based method.

Primary human Peripheral Blood Mononuclear Cells (PBMC) are incubated at 37°C and 5% CO2 for the viability assay with CellTiter Glo 2.0 kit, an ATP-based method (Promega, Catalog number: G9241). 48 h prior to the assay, 2 $\mu$g/ml of phytohemagglutinin-A (PHA) are added to PBMC culture and cells are incubated for 48 h at 37°C and 5% $CO_2$. PBMC are centrifuged at 300 G for 10 minutes, then re-suspended in growth medium and plated at density of 6 x $10^4$ cells/well. Compounds are diluted in DMSO and Lymphocyte growth medium and serial twofold dilutions are prepared. Cells are incubated at 37°C and 5% $CO_2$ for three days with the different dilutions; Lymphocyte growth medium is prepared with RPMI 1640 (Eurobio) supplemented with 10% of heat-inactivated fetal bovine serum (FBS, Euroclone), 100 U/ml penicillin (Euroclone), 100 $\mu$g/ml streptomycin (Euroclone), interleukin-2 (Roche) 20 U/ml, HEPES 12.5 mM.

PBMC incubated with the same amount of DMSO present in the compound dilutions are used as control. After

the incubation, 50 µl of cell cultures from each well are transferred in a luminescence plate after vigorous pipetting and 50 µl of CellTiter Glo 2.0 are added in each well, then the plate is incubated in the Glomax Discovery Reader (Promega, Catalog number: GM3000) following the instructions. Relative luminescence units (RLU) calculated in each well is elaborated with GraphPad software: 100% viability is determined as the mean RLU value measured in the wells containing untreated PBMCs. $IC_{50}$ was calculated by GraphPad Prism 8.0 software.

**Kinetic Solubility**

Kinetic solubility was assessed in phosphate buffered saline at pH 7.4 according to the turbidimetry method. Turbidity (precipitation) was measured by absorbance at 620 nm following incubation at 37°C for 2 h. Stock solution of compounds in DMSO were prepared to produce the desired final range of concentrations, then these were added to the buffer to obtain the final test compound concentrations (1 µM, 3 µM, 10 µM, 30 µM, 100 µM), with a final DMSO concentration 1%). Solubility was estimated from the concentration of tested compound that produced a significant increase in absorbance (≥ 0.003 OD at 620 nm) above vehicle control (1% DMSO in water). Miconazole, Reserpine and Propranolol were used as controls.

In general, compounds with solubility values > 100 µM (such as Propanolol) were regarded as soluble, whereas compounds with values between 30-100 µM (such as Reserpine) were considered to be partially soluble, compounds with values between 10-30 µM (such as Miconazole) were considered scarcely soluble, and compound with solubility below 10 µM are considered insoluble.

**Plasma Stability**

Plasma previously separated from whole blood in EDTA-treated tubes and frozen; blood samples obtained from healthy volunteers, Sprague Dawley rats and CD1 mice, was thawed at room temperature and centrifuged at 1400x RCF 4°C, for 15 minutes. Approximately 90% of the clear supernatant fraction was transferred to a separate tube and was used for the assay.

Stocks (1 mM and 25 µM) of test compound were prepared in acetonitrile:water (50:50 v/v). Plasma samples were heat inactivated at 54°C. 3 µl of 25 µM test compound were added to 72 µl of heat inactivated plasma. A 50 µL aliquot of the mixture was taken and crashed with 200 µl of acetonitrile containing Telmisartan 100 ng/ml as internal standard and was further processed along with other time points: 10, 30 60 and 120 minutes.

Final working stock of 1 µM was prepared by diluting 12 µl of 25 µM stock in 288 µl of plasma. The obtained solution (300 µl) of plasma containing the test compound was incubated for 240 min at 37°C in shaker water bath with gentle shaking. 50 µl aliquot of sample at 10, 30, 60 and 120 min was precipitated immediately with 200 µl of acetonitrile containing internal standard and centrifuged at 4000x RCF, 4°C for 20 minutes. 150 µl of supernatant was diluted with 150 µl of water and analyzed by LC-MS/MS.

The % remaining of the test substance was calculated as: (Peak Area ratio at time (min))/(Peak Area Ratio at 0 min) * 100

The API400 MS and Agilent 1260 Infinity LC were used. Mobile Phase included 10mM Ammonium acetate with 0.1% formic acid in MilliQ water, and Methanol. The column was Phenomenex, KINETIC EVO C18 100A 5u 50*4.6 mm, 10 µl injection volume.

**Plasma Protein Binding**

In vitro evaluation of test compounds for protein binding in plasma was performed using the rapid equilibrium dialysis method. Ready to use PBS, 9.07-10.00 g, (Sigma Aldrich, catalogue code P3813) was dissolved in 1l of MilliQ water and pH was adjusted to 7.4. 10 mM stock of the test compound was prepared in DMSO. 1 mM solution was prepared by diluting 10 mM stock in methanol. 10 µM working stock was prepared in plasma by spiking 3 µl of 1 mM solution into 297 µl of plasma. An aliquot of 200 µl of plasma containing test compound was spiked into donor well (red chamber) of the insert. 350 µl of PBS was spiked into receiver well (white chamber) of the insert. The plate was incubated at 37 °C in thermo-mixer at 400 rpm for 5 hours. The samples were matrix equilibrated with opposite matrix: 25 µl of plasma sample was matched with 25 µl of blank buffer (PBS) and 25 µl of buffer sample was matched with 25 µl of plasma.

Matrix matched samples were precipitated with 200 µl of acetonitrile containing Telmisartan 100 ng/ml as internal standard. Buffer samples were of phosphate buffer saline pH 7.4 incubated for 5.5 h along with plasma samples. Blank samples were the ones matched with Phosphate buffer Saline pH 7.4 maintained at room temperature.

Samples were vortexed at 1000 rpm for 5 min and centrifuged at 4000 rpm for 10 min. Supernatant was separated, diluted 2 fold with water and analyzed in LC-MS/MS.

To control samples were processed immediately after the preparation of plasma working stock solutions. These samples serve as a measure for calculating the percentage recovery from plasma and buffer of test compounds. The

percentage of plasma unbound (% Unbound) and bound (% Bound) fractions were calculated by the following equations:

$$\%\text{Unbound} = \left(1 - \frac{(T_c - F_c)}{T_c}\right) * 100$$

$$\%\text{Bound} = 100 - \%\text{Unbound}$$

$$\%\text{Recovery} = 100 * \frac{F_c + T_c}{T_0}$$

$T_C$ = Compound concentration as determined by the calculated concentration on the plasma side of the membrane (donor well); $F_C$ = Free compound concentration as determined by the calculated concentration on the buffer side of the membrane (receiver well); $T_0$ = Total compound concentration as determined before dialysis.

The API400 MS and Agilent 1260 Multi sampler LC were used, with 10 uL injection volumes. Typically, plasma protein binding is determined over ≥ 4 hours at 37°C. Under these conditions chemical and/or enzymatic degradation of compounds can occur. The determination of compound recovery is, therefore, of pivotal importance. A low % recovery can flag a variety of potential problems:

- The compound is hydrolytically or metabolically unstable
- The compound is highly bound to the rapid equilibrium dialysis device
- The compound is poorly soluble

**hERG inhibition**

HEK293 cells (Eurofins Discover X, Catalogue number CYL3039) stably expressing hERG channels (human ether-a-go-go-related gene potassium channels were plated onto 12 mm coverslips, 12 to 18 h before performing the assay. Cells with coverslips were placed in recording chamber of molecular devices profiled with extracellular solution. Glass capillary with a tip resistance from 2 to 3 mΩ was back filled with intracellular solution. Giga seal was attained using a sequence of positive and negative pressure.

Whole cell configuration was attained using a gentle suction. Cell capacitance and series resistance compensation was done using software controls provided in multi clamp 700B commander. The cells are maintained at a holding potential of -80mV; hERG currents are activated by cell depolarization to +20mV for 2 seconds followed by a 3 second repolarization to -60mV (frequency: 0.05Hz).

Electrical signals were filtered at 2 KHz and digitized at 5 KHz. Series resistance compensation was typically 60% to 70% such that voltage errors were less than 5 mV. No leak subtraction was applied, cells exhibiting leak conductance >10% maximal conductance were excluded from the study. Data were acquired using Clampex acquisition software. Five increasing consecutive concentration of test article are supervised to establish a concentration-response relationship in 3 cells.

Amplitude of the tail current upon repolarization to -60mV (pA) and inhibition of the hERG tail current with respect to untreated cells (%) are measured. If no effect is observed (inhibition <50-60%) at the highest test concentration the reference compound is added to test responsiveness of the cell. Data were analyzed in Microsoft excel and GraphPad Prism to calculate the Inhibitory Concentration 50% ($IC_{50}$) and the Hill Slope.

Test compound concentrations: 300nM, 1 μM, 3 μM, 10μM and 30μM
Reference compound (Quinidine, a known hERG inhibitor) concentrations: 1nM, 3nM, 10nM, 30nM and 100nM
Vehicle: DMSO
Compound application: 15-30 mins at room temperature (25 °C)
Intracellular solution: 130 nmol/l KCl, 1 nmol/l $MgCl_2$, 1 nmol/l $CaCl_2$, 5 nmol/l MgATP, 10 nmol/l EGTA, 10 nmol/l HEPES (pH 7.2 with KOH)
Extracellular solution: 140 nmol/l NaCl, 4 nmol/l KCl, 1.8 nmol/l $CaCl_2$, 1 nmol/l $MgCl_2$, 10 nmol/l Glucose 10 nmol/l HEPES (pH 7.4 with NaOH)
Stimulation Protocol: The cells are maintained at a holding potential of -80mV. hERG currents are activated by cell depolarization to +20mV for 2 seconds followed by a 3 second repolarization to -60mV (frequency: 0.05Hz).
Compound dilutions: 30mM stock of the compound was prepared in 100% DMSO. Test compound and reference

compound were diluted in extracellular solution to achieve the desired test concentration.

## Cytochrome P450 inhibition

Ready to use PBS, 9.07-10.00 g, (Sigma Aldrich, catalogue code P3813) was dissolved in 11 of MilliQ water and pH was adjusted to 7.4.

Substrate pool was done pooling 14 μl of Phenacitin 10 mM (CYP1A2 substrate, Sigma Aldrich, catalogue number 7740), 14 μl Diclofenac 10 mM (CYP2C9 substrate, Sigma Aldrich, catalogue number D6899), 14 μl Dextromethorphan 10 mM (CYP2D6 substrate, Sigma Aldrich, catalogue number D9684), 20.28 μl Midazolam 2.76 mM (CYP3A4 substrate, Sigma Aldrich, catalogue number UC430) and 14 μl Bupropion HCl 10 mM (CYP2B6 substrate, Sigma Aldrich, catalogue number B102), and diluted to 2800 μl with PBS buffer pH 7.4.

Inhibitors of the cytochromes tested were the following: Alpha-naphthoflavone (CYP1A2, Sigma Aldrich, catalogue number N5757); Sulfaphenazole (CYP2C9, Sigma Aldrich, catalogue number S0758); Ticlopidine (CYP2B6, Sigma Aldrich, catalogue number T6654); Quinidine (CYP2D6, Sigma Aldrich, catalogue number Q3625); Ketoconazole (CYP3A4, Sigma Aldrich, catalogue number K1003).

158 μl of Human Liver Microsome working stock solution in PBS buffer pH7.4 was added to reaction plate (0.253 mg/ml, 158 μl/well).

Test compounds and inhibitors were prepared in DMSO and diluted with buffer to prepare working solution with the final concentration of 3000, 999, 333, 111, 37 and 12 μM. 2 μl of working solution of the test compounds or inhibitors listed above were added to reaction plate (2 μl/ well). For control, 2 μl of Acetonitrile containing DMSO (without inhibitor) was added to reaction plate. 20 μl of substrate pool was added to all the wells of the reaction plate. The samples were mixed using the vortex and pre-incubated at 37°C for 5 min.

The NADPH solution 10 mM in PBS buffer pH 7.4 was pre-warmed at 37°C for 5 min. 20 μl of pre-warmed NADPH solution was added to the reaction plate and then incubated at 37°C for 10 min.

The reaction was terminated by adding cold ACN (200 μl/well) to the reaction mixture and centrifuged at 4,000 rpm for 20 minutes.

After centrifugation, 200 μl of supernatant was submitted for LC-MS/MS analysis. Mobile phases include 0.1% formic acid in water and methanol:acetonitrile, 50% (v/v).

Column is Phenomenex kinetic C18, 50 x 4.6 mm, 5μM. $IC_{50}$ values were calculated using Graphpad Prism (version 4.03).

## In-Vivo Experiments

### Animals

Animals were housed in cages with clean bedding, maintained and monitored for good health at the discretion of the laboratory animal veterinarian.

Certified rodent diet was provided. Water was available ad libitum. A periodic analysis of the water was performed and the results were archived at the Test Facility. Environmental controls for the animal room were set to maintain a temperature of 22 to 25°C, humidity of 40-70% relative humidity and a 12-hour light/12-hour dark cycle.

The pharmacokinetic study was performed in male Swiss albino mice from Hylasco Biotechnology Pvt. Ltd. (A Charles River technology Licensee). The tissue distribution study was performed in male Swiss albino mice and in male FOXN1$^{nu/nu}$ nude mice (Envigo spa). The efficacy study was performed in male FOXN1$^{nu/nu}$ nude mice.

## Pharmacokinetic studies

Single intravenous administration studies were done as follows:

1. Compound 5

- dose: 1 mg/kg
- dose concentration: 0.2 mg/ml in 10% DMSO and 10% Solutol in WFI (90%).
- volume: 5 ml/kg
- blood withdrawal time points: 0.083, 0.25, 0.5, 1, 2, 4, 8, 24 hours post administration

2. Compound 3

- dose: 5 mg/kg

- dose concentration: 0.2 mg/ml in 10% DMSO and 10% Solutol in WFI (90%).
- volume: 5 ml/kg
- blood withdrawal time points: 0.083, 0.25, 0.5, 1, 2, 4, 8, 24 hours post administration

Single intraperitoneal administration studies were done as follows:

1. Compound 5

- dose 1: 45 mg/kg
- dose concentration: 12 mg/ml in 10% DMSO, 5% TWEEN 80 and WFI (85%)
- volume: 5 ml/kg
- blood withdrawal time points: 0.25, 0.5, 1, 2, 4, 6, 8, 24 hours post administration
- brain, liver and lungs collected at 4, 8 and 24 hours

2. Compound 5

- dose 2: 10 mg/kg
- dose concentration: 2 mg/ml in 10% DMSO, 10% Solutol and WFI (90%).
- volume: 5 ml/kg
- blood withdrawal time points: 0.25, 0.5, 1, 2, 4, 6, 8, 24 hours post administration

Single oral administration studies were done as follows:

1. Compound 5

- dose: 45 mg/kg
- dose concentration: 4.5 mg/ml in Labrasol (30%), Capmul PG 8 (30%), Cremophor EL (10%) and PEG (30%)
- volume given by oral gavage: 10 ml/Kg
- blood withdrawal time points: 0.08, 0.25, 0.5, 1, 2, 4, 6, 8, 24 hours post administration

Single intra tracheal administration studies were done as follows:

1. Compound 5

- dose: 0.08 mg/kg
- dose concentration: 0.04 mg/ml in DMSO (1%) and WFI (99%)
- volume given by intra tracheal route: 2 ml/Kg
- blood withdrawal time points: 0.03, 0.08, 0.17, 0.25, 0.75, 1.5, 2, 4 hours post administration

Animals were observed up to 24 hours after dose administration for behavioral or physical alterations. For blood withdrawal, mice were anesthetized using gaseous anesthesia and 0.2 to 0.3 ml of blood samples were collected in Li-Heparin containing tubes at the time points above indicated, stored on ice, centrifuged within 15 minutes to separate plasma at 1540 rcf (5000 rpm) at 4°C for 10 minutes; plasma was separated and transferred to new tubes.

Selected organs were collected at 4, 8 and 24 hours for drug distribution studies: mice were anesthetized and intra-cardiac whole body perfusion was performed using chilled saline. Separated tissues were immediately washed, dried, weighed and frozen at -80±10°C until homogenization. The levels of compound in the tissues and in plasma were quantified by LC-MS/MS, using as instrument Q-TRAP 500, with a column Phenomenex, KINETEX 5μ EVO C18 50 mm X 4.5 mm (Phenomenex). The mobile phase employed was composed as follows: aqueous reservoir, 10 mM Ammonium Acetate with 0.1% Formic acid in MilliQ water; organic reservoir, Acetonitrile:Methanol (50:50, v/v); flow rate 1 ml/min; injection volume 10μl; rinsing solution, Acetonitrile:Methanol:Water (20:60:20, v/v).

Calibration Curve & Quality Control samples preparation: 0.5 μl of calibration curve standards were diluted 25X with blank matrix (i.e. Acetonitrile:Methanol (1:1, v/v)) and precipitated with 200 μl of acetonitrile containing internal standard at 200 ng/ml concentration, then vortexed for 5 min at 850 rpm, centrifuged at 4000 rpm for 5 min at 4°C, from this 110 μl of supernatant was separated and diluted with 130 μl of methanol:water, (1:1 v/v) .

Telmisartan (200 ng/ml) was used as internal standard. Telmisartan was spiked in 100% acetonitrile to a final concentration of 200 ng/ml. Sample Preparation: 12.5 to 50 μl of test sample was taken and precipitated with 200 μl of acetonitrile containing internal standard at 200 ng/ml, then vortexed for 5 min at 850 rpm, centrifuged at 4000 rpm for 5 min at 4°C, from this 110 μl of supernatant was separated and diluted with 130 μl of (methanol:water, 1:1 v/v).

Vd (Volume of distribution apparent) is based on the terminal phase and calculated for non-steady-state data by formula:

$$V_d = \frac{Dose}{K_{el} * AUC_{\text{inf}}}$$

AUC is measured by integration of the plot. $AUC_{0\text{-last}}$ is Area under the curve from the time of dosing to the time of the last measurable (positive) concentration ($T_{last}$) calculated by Trapezoidal rule.

$AUC_{0\text{-inf}}$ is AUC from time of dosing extrapolated to infinity, based on the last observed concentration. It is calculated by formula:

$$AUC_{0-last} = AUC_{last} * (\frac{C_{last}}{K_{el}})$$

$AUC\%_{extra}$ is percentage of $AUC_{inf}$ due to extrapolation from $T_{last}$ to infinity. It is calculated:

$$AUC\%_{extra} = \left(\frac{AUC\,\text{inf} \quad - AUClast}{AUCinf}\right) * 100$$

MRT is Mean residence time from the time of dosing to the time of the last measurable concentration with the formula:

$$MRT = \frac{AUMClast}{AUClast}$$

Elimination half-life ($T_{1/2}$) is calculated by formula:

$$\mathrm{T}_{1/2} = \frac{0.693}{\mathrm{K}_{el}}$$

where $K_{e1}$ is elimination rate constant.

Bioanalysis of LC-MS/MS data was performed to obtain the concentration data shown in the Figure 17, and calculate the pharmacokinetic parameters. A fit-for-purpose bioanalytical method was developed for analyzing the plasma samples. One set of nine standards were analyzed before the sample batch and were used to plot a calibration curve. Quality control samples were prepared at a minimum of 3 concentrations; Lower quality control, not more than 5 times to that of lowest standard concentration; High quality control, not less than 75% of the highest standard concentration; Medium quality control, between the low and high concentration. A minimum of 6 quality control samples (three concentrations in duplicate). One set of quality controls samples as described above were analyzed before and after a sample batch. The analytical run was considered acceptable if at least two third of the calibration standards and quality controls meet the stated acceptance criteria. Calibration standards are considered acceptable if the back-calculated concentrations do not deviate by more than 20%. In case the lowest standard is excluded the reported measured concentration of the study samples must be above the nest lowest acceptable standard. If the highest standard is excluded, the reported highest calibration standard is the second highest standard. If the study sample concentration above the upper limit of quantification, the study sample will be diluted with blank matrix, processed and analyzed. The pharmacokinetic parameters were calculated for mean concentrations by non-compartmental model with Phoenix software (version 8.1).

Distribution to tissues is expressed as ratio between the compound level in tissues (ng compounds/ mg tissue) divided by the compound levels in blood (ng/ml), considering 1 ml of blood equal to 1 mg.

**Subcutaneous xenograft mice model**

Human cancer cell lines were inoculated subcutaneously in nude FOXN1$^{nu/nu}$ mice (Envigo spa) in both flanks.

The number of cells inoculated varied between different cell lines:

- A549 and A549 Red-FLuc: 5 x $10^6$ in medium:matrigel (1:1 v/v)
- MDAMB231: 2.8 x $10^6$ in medium:matrigel (1:2 v/v)

When tumors reach the appropriate volume for each cell line, the compounds were administered I.P. every 48 hours, 3 times a week every Monday, Wednesday and Friday for 5 weeks, at a dose selected from the PK study, as follows:

- vehicle: 10% DMSO, 5% TWEEN 80 and WFI (85%)

  - treatment group dose: 45 mg/kg
  - control group: vehicle only

- dose concentration: 12 mg/ml in 10% DMSO, 5% TWEEN 80 and WFI (85%)
- volume: 5 ml/kg

Tumor volume was evaluated twice a week with caliper and calculated with the standard formula (Length * Height * Height)/2. Mice were weighed twice a week. Tumor growth is calculated with respect to the first day of treatment (day 0) as ratio between the volume at each day of measurement and day 0.

At study closure, animals were sacrificed and tumor and tissues removed for further studies of distribution and morphology. Mice from the efficacy study were anesthetized using gaseous anesthesia and blood samples collected in Li-Heparin containing tubes by intracardiac puncture, stored on ice, centrifuged within 15 minutes to separate plasma at 1540 ref (5000 rpm), at 4°C for 10 minutes; plasma was separated and transferred to new tubes. After blood collection, tissues for bio-analyses were separated, washed in PBS, dried and frozen at -80±10°C until homogenization.

Tissues for histological analyses were washed in PBS and paraffin embedded soon after separation. Dehydration and Haematoxylin and Eosin (H&E) staining was performed on 5 μm thick sections followed by microscopic examination with Nikon Ni-e microscope (Nikon Instruments, Calenzano, Italy).

The levels of compound in the tissues were quantified by LC-MS/MS as follows.

UHPLC-MS/MS analysis was performed on a Q-Exactive Plus (ThermoFisher Scientific) mass spectrometer coupled with an UHPLC Ultimate 3000SD (ThermoFisher Scientific).

Chromatographic separation of the analytes was performed on an Acquity UPLC BEH Shield RP18 column (1.7 μM, 2.1 mm x 50 mm, ThermoFisher) with a column temperature at 40°C.

The flow rate was 250 μl/min. The mobile phase was a gradient of a mixture of 10 mM ammonium acetate with 0.1% formic acid (phase A) and Acetonitrile:Methanol (50:50 v/v) as phase B. The gradient profile used began with an elution of (A:B) 70:30 v/v for 1 min, followed by a gradual decrease of A to (A:B) 5:95 v/v until 5 min, a constant gradient for 1 min of 95% of B, then a gradual decrease of B to the initial condition until 7 min and then a final conditioning of the column for about 4 min in order to equilibrate for the next injection.

The electrospray ionization source (ESI) was operated in positive mode. Parallel reaction monitoring (PRM) methods was applied to quantify compound 5. The selected transitions m/z 414.20-243.10 for compound 5 and m/z 360.18- 243.12 for the internal standard (compound 4) were considered. Each sample was assayed in duplicate.

Preparation of plasma samples: Mouse plasma (10 μl) was placed into a tube containing 188 μl of a chilled Acetonitrile:Methanol solution (50:50 v/v) in order to precipitate proteins. Each sample was spiked with a fixed concentration (200 nM) of compound 4 used as internal standard. After 1 min of vortex, the mixture was centrifuged at 10,000 x g for 15 minutes at 4 °C to separate the protein material from the supernatant. The last one of each sample was transferred to a new sample tube and the solvent was evaporated under nitrogen stream. The dried material was re-suspended in 100 μl of mobile phase. The resulting solution was then analyzed by UHPLC-MS/MS without further modifications.

Preparation of tissue samples was as follows: the tissues of interest were washed briefly with chilled water to remove traces of blood, if necessary, and cut into small pieces, keeping them on ice during all the operations. Then the samples were transferred to a tube and homogenized in purified water using the homogenizer TissueRuptor of Qiagen. In general, 300 μl of purified water was added to 50 mg of tissue. The homogenized samples were centrifuged at 10,000 x g for 15 minutes at 4°C to pellet cell debris, and then the supernatant were transferred to a clean tube in order to perform protein precipitation using a chilled solution of Acetonitrile:Methanol (50:50 v/v) and vortexed for 1 min. Before protein precipitation a fixed concentration (200 nM) of internal standard (Compound 4) was added in every sample. After centrifugation, the supernatant was separated from protein pellet and dried under nitrogen stream. 50 μl of mobile phase was used to re-suspend each dried sample.

For the calibration curve a stock solution of 20 mM Compound 5 in DMSO was prepared and diluted stepwise with methanol-acetonitrile (50:50, v/v) to prepare standard solutions at a final concentration from 8 nM to 5 μM (eight points calibration curve for the analyte). Each standard solution for the calibration curve was spiked with internal standard (compound 4) at the fixed concentration of 200 nM.

Distribution to tissues is expressed as ratio between the compound levels in tissue (ng compound/mg tissue) divide by the compound levels in blood (ng/ml), considering 1 ml of blood equal to 1 mg.

**RESULTS**

**Enzymatic Binding**

Binding of compound 5 was assessed with the NanoDSF technique, evaluating the association of the aforementioned compound with APO DDX3X and in presence of a competitor for the ATP binding site, Adenylyl imidodiphosphate (AMPPNP) to assess competition of compound 5 for the DDX3X ATP binding site.

Table 6. Final protein and compound concentrations in the various assay setup:

| Experimental conditions | Protein conc. [μM] | AMPPNP conc. [μM] | Compound conc. [μM] |
|---|---|---|---|
| DDX3 (1% DMSO) | 4 | - | - |
| AMPPNP (1% DMSO) | 4 | 100 | - |
| Compound (1% DMSO) | 4 | - | 100 |
| AMPPNP + Compound (1% DMSO) | 4 | 100 | 100 |

All the measures were performed in triplicate and the average (Mean Tm) and standard deviation (SD) were calculated with the following equations:

$$Mean\ Tm = \frac{1}{n}\sum_{i=1}^{n} Tm_i \quad n = 3$$

and

$$SD = \sqrt{\frac{\sum(Tm - Mean\ Tm)^2}{n-1}}$$

A change of 5xSD from the 1% DMSO control mean is considered to be a significant Tm change. Test of DDX3X with 1% DMSO was performed to obtain the reference melting temperature of the protein alone. Compounds are considered as binders if the Tm shifts by >= 5 times the standard deviation of the control (protein in the absence of compound).

Table 7. DDX3X melting temperatures (Tm) at various experimental conditions

| **Compound** | **Compound conc. [μM]** | **AMPPNP conc. [μM]** | **DDX3 conc. [μM]** | **Mean Tm [°C]** | **SD** | **Comment** |
|---|---|---|---|---|---|---|
| DMSO (1%) | - | - | 4 | 54.6 | 0.05 | |
| Compound 5 | 100 | - | 4 | 56.9 | 0.13 | significant Tm shift to higher Tm, compound interference |
| DMSO (1%) | - | 100 | 4 | 54.7 | 0.07 | |
| Compound 5 | 100 | 100 | 4 | 56.8 | 0.26 | significant Tm shift to higher Tm, compound interference |

The Tm of 54.6°C was measured for DDX3X alone and a Tm of 54.7°C with AMPPNP. AMPPNP is an ATP mimetic compound and is reported to bind ATP site of DDX3X (Högbom et al., Crystal structure of conserved domains 1 and 2 of the human DEAD-box helicase DDX3X in complex with the mononucleotide AMP, J Mol Biol, 2007, 372, 150;

Floor et al., Autoinhibitory Interdomain Interactions and Subfamily-specific Extensions Redefine the Catalytic Core of the Human DEAD-box Protein DDX3, J Biol Chem, 2016, 291, 2412; Epling et al., Cancer-associated mutants of RNA helicase DDX3X are defective in RNA-stimulated ATP hydrolysis, J Mol Biol, 2015, 427, 1779). Although AMPPNP is a binder of DDX3X there is no significant effect on Tm upon binding. Compound 5 tested with DDX3X alone and in presence of APMPNP 100 μM; in both the conditions it showed a significant shift to higher Tm with minor interference in the measurement.

**Helicase assay**

Additional confirmation of the ability of compounds to inhibit the DDX3X helicase come from the in vitro assay performed using DDX3X human protein immunoprecipitated from 293T cells transfected with HA-tagged DDX3X. As reference treatment of DDX3X with and without ATP was used to define maximum and minimum activity of DDX3X. Then DDX3X was treated in turn with compounds 5 and 16, in presence of ATP. Both compounds show inhibitory effect on DDX3X helicase activity, correlating with the concentration of the compounds tested (Figure 13 and 14).

**Cytotoxicity**

Cytotoxicity was measured in the cell lines described in Table 4. Compounds show anti-proliferative and cytotoxic activity in several cancer cell lines (Tables 8-15 and 18), whereas in non-cancer cell lines no activity was observed (Tables 16-17). Compound 5 was the most active compound tested, with comparable activity in almost all the cancer cell lines and toxicity above 100 μM in primary human cells (Table 16) or non tumor cell lines (Table 17). Compounds 2, 3 and 5 have shown an $IC_{50}$ in the low micromolar or sub-micromolar range showing a strong antitumor activity.

Table 8. Anti-proliferative activity against triple negative breast cancer (MDAMB231 cell line). Anti-proliferative activity is expressed as the concentration inhibiting cell proliferation and survival to 50% with respect to untreated cells ($IC_{50}$) during 72 hours of incubation measured with the cell count method.

| Compound | MDAMB231 |
|---|---|
| | $IC_{50}$ (μM) |
| 5 | 0.58 |
| 2 | 8.113 |
| 3 | 6.072 |
| 4 | 38.85 |
| 6 | 34.63 |
| 9 | 43.13 |
| 7 | 215.8 |
| 8 | 1000 |
| 1 | 1000 |

Table 9. Anti-proliferative activity against human lung adenocarcinoma (A549, SK-LU1 and CALU1 cell lines). Anti-proliferative activity is expressed as the concentration inhibiting cell proliferation and survival to 50% with respect to untreated cells ($IC_{50}$) during 72 hours of incubation measured with the cell count method.

| Compound | A549 | A549 Red-FLuc | SK-LU1 | CALU1 |
|---|---|---|---|---|
| | $IC_{50}$ (μM) | $IC_{50}$ (μM) | $IC_{50}$ (μM) | $IC_{50}$ (μM) |
| 5 | 0.35 | 6.04 | 0.42 | 0.37 |
| 16 | | 18.07 | | |
| 19 | | 1.7 | | |
| 22 | | 3.42 | | |

Table 10. Anti-proliferative activity against human hepatocellular carcinoma (HUH7 cell line). Anti-proliferative activity is expressed as the concentration inhibiting cell proliferation and survival to 50% with respect to untreated cells ($IC_{50}$) during 72 hours of incubation measured with the cell count method.

| Compound | HUH7 |
|---|---|
| | $IC_{50}$ (μM) |
| 5 | 3.28 |

Table 11. Anti-proliferative activity against human prostate cancer (DU145 cell line). Anti-proliferative activity is expressed as the concentration inhibiting cell proliferation and survival to 50% with respect to untreated cells ($IC_{50}$) during 72 hours of incubation measured with the cell count method.

| Compound | DU145 |
|---|---|
| | $IC_{50}$ (μM) |
| 5 | 3.7 |

Table 12. Anti-proliferative activity against human malignant melanoma (A375 cell line). Anti-proliferative activity is expressed as the concentration inhibiting cell proliferation and survival to 50% with respect to untreated cells ($IC_{50}$) during 72 hours of incubation measured with the cell count method.

| Compound | A375 |
|---|---|
| | $IC_{50}$ (μM) |
| 5 | 0.94 |

Table 13. Anti-proliferative activity against human pancreas epithelioid carcinoma (PANC-1 cell line). Anti-proliferative activity is expressed as the concentration inhibiting cell proliferation and survival to 50% with respect to untreated cells (IC50) during 72 hours of incubation measured with the cell count method.

| Compound | PANC-1 |
|---|---|
| | $IC_{50}$ (μM) |
| 5 | 0.72 |

Table 14. Anti-proliferative activity against chronic myelogenous leukemia (CML, K562 cell line). Anti-proliferative activity is expressed as the concentration inhibiting cell viability to 50% with respect to untreated cells ($IC_{50}$) during 72 hours of incubation measured with the MTS method.

| Compound | K562 |
|---|---|
| | $IC_{50}$ (μM) |
| 5 | 20.33 |

Table 15. Anti-proliferative activity against human cutaneous T-lymphocyte lymphoma. Anti-proliferative activity is expressed as the concentration inhibiting cell viability to 50% with respect to untreated cells ($IC_{50}$) during 72 hours of incubation measured with the ATP-based method.

| Compound | H9 |
|---|---|
| | $IC_{50}$ (μM) |
| 5 | 50 |

Table 16. Anti-proliferative activity against human peripheral blood cell from healthy donor. Anti-proliferative activity is expressed as the concentration inhibiting cell viability to 50% with respect to untreated cells ($IC_{50}$) during 72 hours of incubation measured with the ATP-based method.

| Compound | PBMC |
|---|---|
| | $IC_{50}$ (μM) |
| 5 | 200 |

Table 17. Anti-proliferative activity against normal human breast cells. Anti-proliferative activity is expressed as the concentration inhibiting cell proliferation and survival to 50% with respect to untreated cells ($IC_{50}$) during 72 hours of incubation measured with the cell count method.

| Compound | MCF10a |
|---|---|
| | $IC_{50}$ (μM) |
| 5 | 440 |

Table 18. Anti-proliferative activity against glioblastoma cells. Anti-proliferative activity is expressed as the concentration inhibiting cell proliferation and survival to 50% with respect to untreated cells ($IC_{50}$) during 72 hours of incubation measured with the cell count method.

| Compound | U87 |
|---|---|
| | $IC_{50}$ (μM) |
| 5 | 3.9 |

**Kinetic Solubility**

Table 19. Absorbance of solutions of control compounds (Miconazole, Reserpine, Propanolol) and Compound 5.

| **conc. (μM)** | **Compound 5** | **Miconazole** | **Reserpine** | **Propranolol** |
|---|---|---|---|---|
| 100 | 0.038 | 0.053 | 0.058 | 0.037 |
| 30 | 0.037 | 0.044 | 0.040 | 0.038 |
| 10 | 0.037 | 0.037 | 0.037 | 0.039 |
| 3 | 0.037 | 0.037 | 0.037 | 0.037 |
| 1 | 0.037 | 0.037 | 0.037 | 0.038 |
| 0 | 0.037 | 0.036 | 0.036 | 0.036 |

Compound 5 shows solubility > 100 μM and is defined as soluble. Compound 5 did not show significant increase of absorbance within the range of concentration tested as the reference compound for high solubility (Propanolol > 100 μM), while the reference compounds for scarce solubility (Miconazole, 10-30 μM) and medium solubility (Reserpine, 30-100 μM), show increase of absorbance in the range expected (Figure 15).

***In vitro* ADME**

*In vitro* characterization of some ADME-properties of compound 5 has shown that the compound has a high plasma stability in human, rat and mice (Table 20), and that a high fraction is bound to plasma proteins (Table 21).

Table 20. Plasma stability expressed as percentage of compound 5 found after incubation with plasma at 0, 10, 30, 60 and 120 minutes. Values above 100% can be due to the automated peak area measurement by LC-MS/MS instrument, and should be intended as 100% stability.

| Compound 5 | % Remaining ± SD | | |
|---|---|---|---|
| Time (minutes) | Human plasma | Rat plasma | Mouse plasma |
| 0 | 100.0 ± 3.9 | 100.0 ± 1.0 | 100.0 ± 1.8 |
| 10 | 107.1 ± 7.7 | 107.1 ± 4.1 | 117.2 ± 8.6 |
| 30 | 87.2 ± 10.3 | 103.3 ± 2.3 | 111.1 ± 0.3 |
| 60 | 88.3 ± 7.0 | 99.0 ± 6.8 | 108.2 ± 4.8 |
| 120 | 88.2 ± 8.2 | 104.8 ± 5.6 | 123.2 ± 26.5 |

Table 21. Plasma protein binding, fraction of compound bound to plasma proteins and percentage of recovered compound measured in three species, human, rat and mouse.

| Compound ID | Species / Matrix | % Bound in Plasma ± SD | % Recovery ± SD |
|---|---|---|---|
| 5 | Human plasma | 99.78 ± 0.28 | 78.98 ± 0.99 |
| 5 | Rat plasma | 99.95 ± 0.01 | 63.37 ± 2.74 |
| 5 | Mouse plasma | 99.97 ± 0.005 | 83.74 ± 13.37 |

No drug-drug interaction can be anticipated based on the CYP-inhibition assay results, as compound 5 is not inhibiting the activity of the 5 enzymes more frequently involved in drug metabolism (Table 24). These data support the possibility to administrate compound 5 in combination with other drugs without toxicity due to drug-drug interaction.

Table 24. Cytochrome P450 inhibition, measured in subtypes 1A2, 2C9, 2B6, 2D6 and 3A4

| Compound | $IC_{50}$ (μM) | | | | |
|---|---|---|---|---|---|
| | 1A2 | 2C9 | 2B6 | 2D6 | 3A4 |
| 5 | >30 | >30 | >30 | >30 | >30 |

**hERG channel inhibition**

No effect on the conductance of the hERG channel has been observed in presence of compound 5, even in high concentration (Figure 16), ruling out the possibility of hERG mediated cardiac toxicity in vivo.

**Pharmacokinetics**

Pharmacokinetic parameters for compound 5 are reported in Table 25. Noteworthy the compound has a high volume of distribution (Vd) reflecting a high distribution to tissues. The intraperitoneal route, the route used for efficacy studies, ensures a high exposure of compound 5 in mice, as shown by the absolute bioavailability (F) above 100%. Intraperitoneal administration shows slow elimination ratio maintaining the plasmatic levels after 24 hours.

Dose: Administered dose reported as mg of drug over body weight in Kg
Co: Concentration extrapolated at time 0 for I.V. administration
$C_{max}$: Maximum concentration measured for all the administration routes excluding IV
$T_{max}$: Time when Cmax is reached, that represents the equilibrium between absorption and excretion
$T_{1/2}$: Half-life of administered drug
$AUC_{0-last}$: Area under the curve from time zero to the last time-point measured. It represents the quantity of compound absorbed
$AUC_{0-inf}$: Area under the curve from time 0 to infinite, it represents the theoretical quantity of compound absorbed
$AUC_{Extra}$: Percentage of the area under the curve extrapolated

CL: Clearance in vivo by the entire route of elimination and excretion
Vd: Volume of distribution, can be calculated only for I.V. administration route
$MRT_{0\text{-}last}$: average time the drug molecules spend in the system before being eliminated
F: Bioavailability applicable for all the route of administration excluding IV
Rsq: R square value indicate the fit of the pharmacokinetic model applied over the experimental data

Table 25. Pharmacokinetic parameters for different routes of administration intravenous (I.V.), intraperitoneal (I.P.; *1: TWEEN-based vehicle; **2: Solutol-based vehicle), oral (P.O.), intra tracheal (I.T.).

| Single Administration | I.V. | I.P. 1* | I.P. 2* | P.O. | I.T. |
|---|---|---|---|---|---|
| Dose (mg/kg) | 1 | 45 | 10 | 45 | 0.08 |
| $C_0$ (ng/ml) | 879.02 | n.a. | n.a. | n.a. | n.a. |
| $C_{max}$ (ng/ml) | n.a. | 3695.66 | 691.81 | 1347.01 | 1950.67 |
| $T_{max}$ (h) | n.a. | 2 | 2 | 4 | 0.03 |
| $T_{1/2}$ (h) | 2.37 | n.a. | n.a. | n.a. | n.a. |
| $AUC_{0\text{-}last}$ (ng·h/ml) | 399.94 | 21772.49 | 5349.5 | 14769,87 | 791.38 |
| $AUC_{0\text{-}inf}$ (ng·h/ml) | 416.53 | 25573.44 | 5632.07 | 17775,48 | 815.21 |
| $AUC_{Extra}$ (%) | 3.98 | 14.86 | 5.02 | 16.91 | 2.92 |
| CL (ml/min/Kg) | 40.01 | n.a. | n.a. | n.a. | n.a. |
| Vd (l/Kg) | 8.2 | n.a. | n.a. | n.a. | n.a. |
| $MRT_{0\text{-}last}$ (h) | 1.09 | 7.46 | 6.49 | 9.05 | 0.72 |
| F (%) | n.a. | 136 | 135 | 95 | n.a. |
| Rsq | 0.9187 | 0.9834 | 0.9988 | 0,9650 | 0.99 |
| n.a.: not applicable | | | | | |

The pharmacokinetic profile for I.P. (I.P. 1 in the table 25), P.O. and I.V. (P.O. and IV in the table 25) with administration of compound 5 is reported in Figure 17. Compound 5 blood concentrations is reported in ng/ml (reported in Log scale) and measured for I.V. at 5 min, 15 min, 1h, 2 h, 4 h, 8 h and 24 h, while for P.O. administration the measurements were at 5 min, 15 min, 30 min, 1 h, 2 h, 4 h, 8 h and 24 h and for I.P. at 15 min, 30 min, 1 h, 2 h, 4 h, 6 h, 8 h and 24 h. The high Vd indicates distribution in tissues, and high binding to peripheral tissues, as confirmed below by the tissue distribution experiments. The CL measured in vivo can be influenced by the high plasma protein binding and high tissue distribution, which protect the compound 5 from hepatic metabolism.

**Tissue distribution**

Analyses of compound 5 levels in tissue have shown high distribution in the tested tissues in different conditions of treatment (Table 26a, 26b) confirming that the compound is able to distribute to relevant organs.

Table 26a. Plasma levels and Liver, Brain and Kidney to plasma ration after single administration of 45 mg/kg of compound 5 via intraperitoneal route to male Swiss Albino Mice.

| Hours after single administration I.P. 45 mg/Kg | Plasma (ng/ml) | Liver to plasma | Brain to plasma | Kidney to plasma |
|---|---|---|---|---|
| 4 | 2171 | 11.2 | 2.4 | 11.5 |
| 8 | 775 | 9.2 | 2.1 | 9.2 |
| 24 | 273 | 9.2 | 1.75 | 9.3 |

Table 26b. Plasma levels and tissue to plasma ratios after administration of 45 mg/kg of compound 5 via intraperitoneal route to FOXN1$^{nu/nu}$ nude mice (Envigo spa) in different conditions and schedules: #1: single I.P. administration, 45 mg/Kg, withdrawal 4 hours after administration; #1A: two I.P. administration, 45 mg/Kg, at distance of 48 hours, withdrawal 4 hours after last administration; #1B: 6 weeks of repeated I.P. administration, 45 mg/kg, every 48 hours, withdrawal 4 hours after last administration

| Conditions | Plasma (ng/ml) | Liver to plasma | Brain to plasma | Kidney to plasma | Lung to plasma | Breast to plasma | Spleen to plasma | Intestine to plasma | Tumor to plasma | Lymph node to plasma | Adipose to plasma |
|---|---|---|---|---|---|---|---|---|---|---|---|
| #1 | 2099.8 | 7.3 | 0.5 | 23.1 | 12.6 | 42.9 | 26.1 | 5.8 | 11.3 | 17.9 | 14.3 |
| #1A | 2188.6 | 12.9 | 1.1 | 21.6 | 10.3 | 10.9 | 23.3 | 4.2 | 39.9 | 137.7 | 31.8 |
| #1B | 4658.9 | 12.4 | 0.7 | 18.3 | 9.6 | 18.5 | 21.4 | 10.9 | 14.9 | 27.5 | 233.3 |

Considering the variability of the animal model the values of ratio within ± 10% of variability can be considered equivalent.

**Systemic and organ toxicity**

No treatment related morphological changes were recorded in brain, liver, lung (not shown), spleen and kidney of mice after 6 weeks of repeated treatment (I.P. 45mg/Kg every 48 hours) (Figure 18).

No systemic toxicities or altered behavior have been observed in mice after single as well as repeated administration of different doses of compound 5:

- I.V. 1 mg/kg single administration: no toxicities (mice observed for 24 hours)
- I.P. 10 mg/kg single administration: no toxicities (mice observed for 24 hours)
- I.P. 45 mg/kg single administration: no toxicities (mice observed for 24 hours)
- I.P. 45 mg/kg double administration: no toxicities (mice observed for 48 hours)
- I.P. 45 mg/kg every 48 hours, 6 weeks: no toxicities observed for the whole study, including 1 week follow up
- P.O. 45 mg/kg single administration: no toxicities (mice observed for 24 hours)
- I.T. 0.08 mg/kg single administration: no toxicities (mice observed for 24 hours)

***In vivo* efficacy**

Repeated administrations of compound 5 in a subcutaneous xenograft mice model of Triple Negative Breast (Figure 19a) and in two Lung cancer ones (Figure 19b-c) counteract tumor growth in different conditions. In fact in among the two studies in the lung cancer models there were small differences: the cell lines was the same (A549, Figure 19b) but in one case the cell line was engineered with a luciferase reporter (A549 Red-FLuc, Figure 19c); the administration of the compound started in two different stage of the tumor growth, being more advanced in the study of Figure 19b; the rate of growth of the tumor in untreated condition was higher in the study of Figure 19c. Compound 5 was able to contrast tumor growth both when the tumor was already well established and grown, as well as when the tumor was smaller but with higher growth rate.

## BIBLIOGRAPHY

Waterhouse, A., et al., SWISS-MODEL: homology modeling of protein structures and complexes. Nucleic Acids Res. 46(W1), W296-W303 (2018).

Guex, N., et al., Automated comparative protein structure modeling with SWISS-MODEL and Swiss-PdbViewer: A historical perspective. Electrophoresis 30, S162-S173 (2009).

Bienert, S., et al., The SWISS-MODEL Repository - new features and functionality. Nucleic Acids Res. 45, D313-D319 (2017).

Marco Biasini, et al. (2014). SWISS-MODEL: modeling protein tertiary and quaternary structure using evolutionary information. Nucleic Acids Research (1 July 2014) 42 (W1): W252-W258

Arnold, K., et al. (2006) The SWISS-MODEL workspace: a web-based environment for protein structure homology modeling. Bioinformatics, 22, 195-201.

Benkert, P., et al. (2011) Toward the estimation of the absolute quality of individual protein structure models. Bioinformatics, 27, 343-350

Altschul, S.F., et al. (1997) Gapped BLAST and PSI-BLAST: a new generation of protein database search programs. Nucleic Acids Res, 25, 3389-3402.

Remmert, M., et al. HHblits: lightning-fast iterative protein sequence searching by HMM-HMM alignment. Nat Methods, 9, 173-175 (2012).

Guex, N. and Peitsch, M.C. (1997) SWISS-MODEL and the Swiss-PdbViewer: an environment for comparative protein modeling. Electrophoresis, 18, 2714-2723.

Sali, A. and Blundell, T.L. (1993) Comparative protein modeling by satisfaction of spatial restraints. J Mol Biol, 234, 779-815.

Benkert, P., et al. (2011) Toward the estimation of the absolute quality of individual protein structure models. Bioinformatics, 27, 343-350.

Mariani, V., et al. (2011) Assessment of template based protein structure predictions in CASP9. Proteins, 79 Supp 10, 37-58.

## Claims

1. A compound of formula (I):

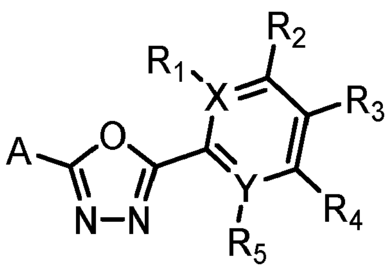

(I)

or a salt, solvate, stereoisomer or tautomer thereof, wherein:

A is substituted or unsubstituted aryl or substituted or unsubstituted heteroaryl;
wherein the substituted aryl and the substituted heteroaryl are independently substituted with one or more substituents each independently selected from the group consisting of: unsubstituted or substituted $C_1$-$C_6$ alkyl, unsubstituted or substituted $C_2$-$C_6$ alkenyl, unsubstituted or substituted $C_2$-$C_6$ alkynyl, unsubstituted or substi-

tuted cyclic $C_3$-$C_8$ alkyl, unsubstituted or substituted cyclic $C_3$-$C_8$ alkenyl, a cyclic amine selected from the group of pyrrolidine, piperidine, morpholine, piperazine, halogen, $-OR_A$, $-SR_A$, $-NR_AR_B$, $-S(O)R_A$, $-S(O)_2R_A$, $-S(O)_2OR_A$, $-S(O)_2NR_AR_B$, - $NR_AS(O)_2R_B$, $-COOR_A$, $-C(O)NR_AR_B$, $-C(O)R_A$, $-OC(O)R_A$, $-NR_BC(O)R_A$, $-NR_CC(O)NR_AR_B$, - $OP(O)(OR_A)_2$, $-OP(O)(NR_AR_B)_2$, $-NO_2$, -NO, -C≡N, $-N^+≡C^-$, -SCN, -OCN and 5-membered heteroaromatic ring optionally substituted with $C_1$-$C_6$ alkyl;

**X** and **Y** are each independently selected from the group consisting of: C and N.

$\mathbf{R_1}$, $\mathbf{R_2}$, $\mathbf{R_4}$ and $\mathbf{R_5}$ are each independently selected from the group consisting of: H, unsubstituted or substituted $C_1$-$C_6$ alkyl, unsubstituted or substituted $C_2$-$C_6$ alkenyl, unsubstituted or substituted $C_2$-$C_6$ alkynyl, unsubstituted or substituted cyclic $C_3$-$C_8$ alkyl, unsubstituted or substituted cyclic $C_3$-$C_8$ alkenyl, halogen, $-OR_A$, $-SR_A$, $-NR_AR_B$, $-S(O)R_A$, $-S(O)_2R_A$, $-S(O)_2OR_A$, $-S(O)_2NR_AR_B$, - $NR_AS(O)_2R_B$, $-COOR_A$, $-C(O)NR_AR_B$, $-C(O)R_A$, $-OC(O)R_A$, $-NR_AC(O)R_B$, $-NR_CC(O)NR_AR_B$, - $OP(O)(OR_A)_2$, $-OP(O)(NR_AR_B)_2$, $-NO_2$, -NO, -C≡N, $-N^+≡C^-$, -SCN and -OCN;

$\mathbf{R_3}$ is a heteroaryl group selected from the group consisting of:

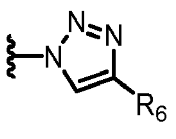 , 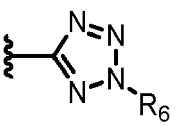 , 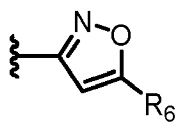 , 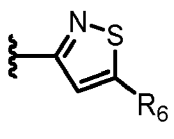 , 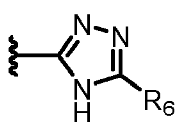 , 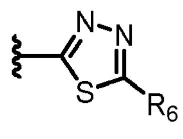 ,

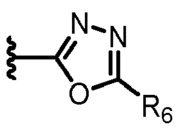 , 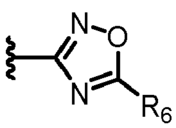 , 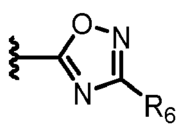 , 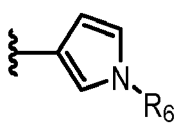 , 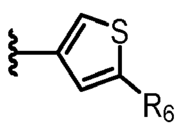 , 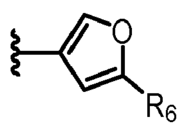 ,

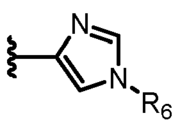 , 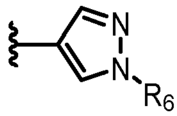 , , 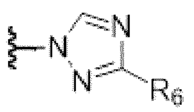 , 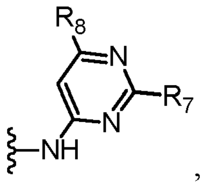 , 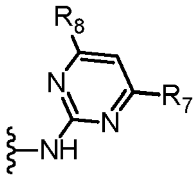

and

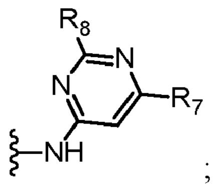 ;

$\mathbf{R_6}$ is unsubstituted or substituted $C_1$-$C_8$ alkyl;

wherein the substituted $C_1$-$C_8$ alkyl is substituted with one or more substituents each independently selected from the group consisting of: unsubstituted or substituted $C_1$-$C_6$ alkyl, unsubstituted or substituted $C_2$-$C_6$ alkenyl, unsubstituted or substituted $C_2$-$C_6$ alkynyl, unsubstituted or substituted cyclic $C_3$-$C_8$ alkyl, unsubstituted or substituted cyclic $C_3$-$C_8$ alkenyl, halogen, $-OR_A$, $-SR_A$, $-NR_AR_B$, $-S(O)R_A$, $-S(O)_2R_A$, $-S(O)_2OR_A$, $-S(O)_2NR_AR_B$, $-NR_AS(O)_2R_B$, - $COOR_A$, $-C(O)NR_AR_B$, $-C(O)R_A$, $-OC(O)R_A$, $-NR_BC(O)R_A$, $-NR_CC(O)NR_AR_B$, $-OP(O)(OR_A)_2$, $-OP(O)(NR_AR_B)_2$, $-NO_2$, -NO, -C≡N, $-N^+≡C^-$, -SCN and -OCN;

wherein any one or more of said substituted $C_1$-$C_6$ alkyl, substituted $C_2$-$C_6$ alkenyl, substituted $C_2$-$C_6$ alkynyl, substituted cyclic $C_3$-$C_8$ alkyl and substituted cyclic $C_3$-$C_8$ alkenyl are each independently substituted with one or more substituents each independently selected from the group consisting of: halogen, $-OR_A$, $-SR_A$, $-NR_AR_B$, $-S(O)R_A$, $-S(O)_2R_A$, $-S(O)_2OR_A$, - $S(O)_2NR_AR_B$, $-NR_AS(O)_2R_B$, $-COOR_A$, $-C(O)NR_AR_B$, $-C(O)R_A$, $-OC(O)R_A$, $-NR_BC(O)R_A$,$-NR_CC(O)NR_AR_B$, $-OP(O)(OR_A)_2$, $-OP(O)(NR_AR_B)_2$, $-NO_2$, -NO, -C≡N, $-N^+≡C^-$, -SCN and-OCN;

$\mathbf{R_7}$ is N-piperidinyl, N-pyrrolidinyl or N-morpholinyl;

$\mathbf{R_8}$ is H or unsubstituted or substituted $C_1$-$C_8$ alkyl;

wherein the substituted $C_1$-$C_8$ alkyl is substituted with one or more substituents each independently selected from the group consisting of: unsubstituted or substituted $C_1$-$C_6$ alkyl, unsubstituted or substituted $C_2$-$C_6$ alkenyl, unsubstituted or substituted $C_2$-$C_6$ alkynyl, unsubstituted or substituted cyclic $C_3$-$C_8$ alkyl, unsubstituted or

substituted cyclic $C_3$-$C_8$ alkenyl, halogen, -$OR_A$, -$SR_A$, -$NR_AR_B$, -S(O)$R_A$, -S(O)$_2R_A$, -S(O)$_2OR_A$, -S(O)$_2NR_AR_B$, -$NR_AS(O)_2R_B$, - $COOR_A$, -C(O)$NR_AR_B$, -C(O)$R_A$, -OC(O)$R_A$, -$NR_BC(O)R_A$, -$NR_CC(O)NR_AR_B$, -OP(O)($OR_A$)$_2$, -OP(O)($NR_AR_B$)$_2$, -$NO_2$, -NO, -C≡N, -$N^+$≡$C^-$, -SCN and -OCN;

wherein any one or more of said substituted $C_1$-$C_6$ alkyl, substituted $C_2$-$C_6$ alkenyl, substituted $C_2$-$C_6$ alkynyl, substituted cyclic $C_3$-$C_8$ alkyl and substituted cyclic $C_3$-$C_8$ alkenyl are each independently substituted with one or more substituents each independently selected from the group consisting of: halogen, -$OR_A$, -$SR_A$, -$NR_AR_B$, -S(O)$R_A$, -S(O)$_2R_A$, -S(O)$_2OR_A$, - S(O)$_2NR_AR_B$, -$NR_AS(O)_2R_B$, -$COOR_A$, -C(O)$NR_AR_B$, -C(O)$R_A$, -OC(O)$R_A$, -$NR_BC(O)R_A$,-$NR_CC(O)NR_AR_B$, -OP(O)($OR_A$)$_2$, -OP(O)($NR_AR_B$)$_2$, -$NO_2$, -NO, -C≡N, -$N^+$≡$C^-$, -SCN and-OCN;

**$R_A$, $R_B$** and **$R_C$** are each independently selected from the group consisting of: H, unsubstituted or substituted $C_1$-$C_6$ alkyl, unsubstituted or substituted $C_2$-$C_6$ alkenyl, unsubstituted or substituted $C_2$-$C_6$ alkynyl, unsubstituted or substituted cyclic $C_3$-$C_8$ alkyl, unsubstituted or substituted cyclic $C_3$-$C_8$ alkenyl,

or **$R_A$** and **$R_B$** together with the nitrogen to which they are attached, form a 4-7 membered saturated or partially unsaturated ring optionally containing one or more additional heteroatoms independently selected from N, S and O, wherein the substituted $C_1$-$C_6$ alkyl, the substituted $C_2$-$C_6$ alkenyl, the substituted $C_2$-$C_6$ alkynyl, the substituted cyclic $C_3$-$C_8$ alkyl and the substituted cyclic $C_3$-$C_8$ alkenyl in position **$R_A$,** $R_B$ and/or **$R_C$** and the 4-7 membered saturated or partially unsaturated ring are each independently substituted with one or more substituents each independently selected from the group consisting of: $C_1$-$C_6$ alkyl, $C_2$-$C_6$ alkenyl, $C_2$-$C_6$ alkynyl, cyclic $C_3$-$C_8$ alkyl, cyclic $C_3$-$C_8$ alkenyl, $C_1$-$C_6$ alkoxy, halogen, $C_1$-$C_6$ haloalkyl, OH, -$NO_2$, -NO, -C≡N, -$N^+$≡$C^-$, -SCN and -OCN;

with the proviso that if $R_3$ is

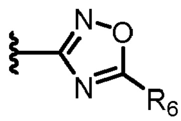

then A is not

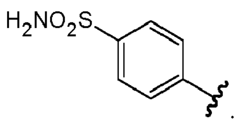.

2. The compound, salt, solvate, stereoisomer or tautomer according to claim 1, wherein the aryl is phenyl or the heteroaryl is selected from the group consisting of: isoquinoline, quinoline, pyridine, pyrimidine, pyrazine and pyridazine.

3. The compound, salt, solvate, stereoisomer or tautomer according to claim 1 or 2, wherein the substituted aryl and the substituted heteroaryl are independently substituted with one or more substituents each independently selected from the group consisting of: unsubstituted or substituted $C_1$-$C_6$ alkyl, halogen, -$OR_A$, -$NR_AS(O)_2R_B$, -$NR_BC(O)R_A$ and -C(O)N($R_A$)$R_B$;
wherein **$R_A$** is selected from the group consisting of: H, unsubstituted or substituted $C_1$-$C_6$ alkyl and unsubstituted or substituted cyclic $C_3$-$C_8$ alkyl;
wherein **$R_B$** is unsubstituted or substituted $C_1$-$C_6$ alkyl or unsubstituted or substituted cyclic $C_3$-$C_8$ alkyl;
wherein any one of said substituted $C_1$-$C_6$ alkyl and cyclic $C_3$-$C_8$ alkyl is independently substituted with one or more halogens.

4. The compound, salt, solvate, stereoisomer or tautomer according to any one of the previous claims, wherein **$R_1$, $R_2$, $R_4$** and **$R_5$** are each independently selected from the group consisting of: H, OMe, OEt, F, Cl and NHMe.

5. The compound, salt, solvate, stereoisomer or tautomer according to any one of the previous claims, wherein **$R_3$** is selected from the group consisting of:

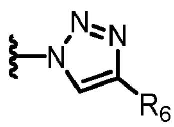, 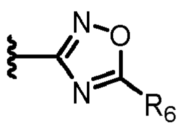,

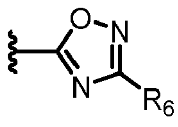 and 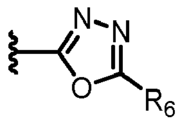 .

6. The compound, salt, solvate, stereoisomer or tautomer according to any one of the previous claims, wherein **$R_6$** is unsubstituted or substituted $C_1$-$C_5$ alkyl, preferably **$R_6$** is selected from the group consisting of: substituted or unsubstituted n-butyl, substituted or unsubstituted n-pentyl, substituted or unsubstituted n-propyl, substituted or unsubstituted ethyl, substituted or unsubstituted methyl, substituted or unsubstituted tert-butyl and substituted or unsubstituted isopropyl, wherein any one of said substituted $C_1$-$C_5$ alkyl is independently substituted with one or more halogens.

7. The compound, salt, solvate, stereoisomer or tautomer according to any one of the previous claims being selected from the group consisting of:

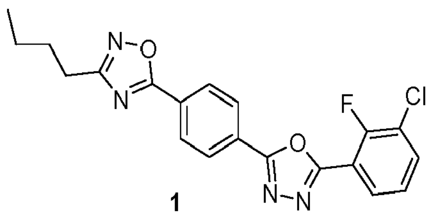

**1** ,

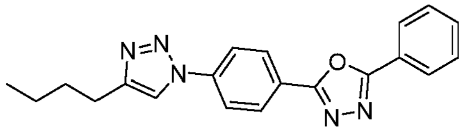

**2** ,

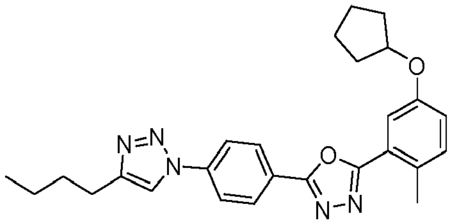

**3** ,

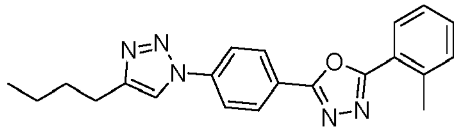

**4** ,

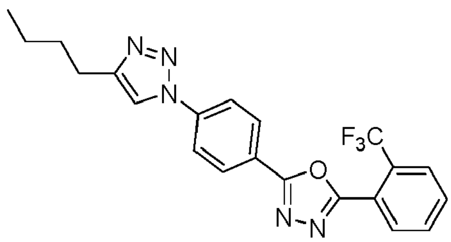

**5** ,

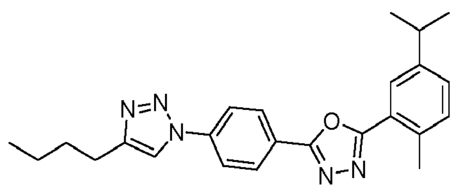

**6** ,

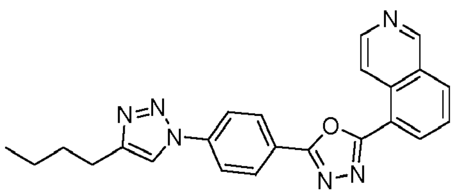

**7** ,

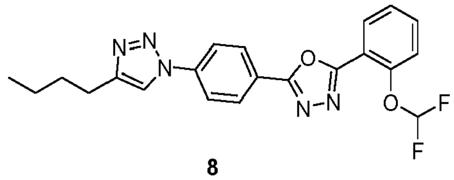

**8** ,

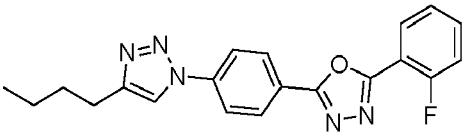

9 ,

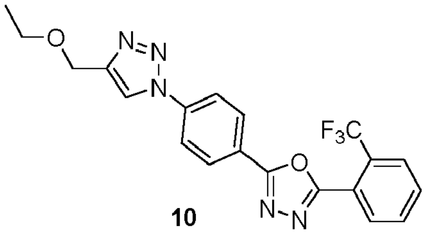

10 ,

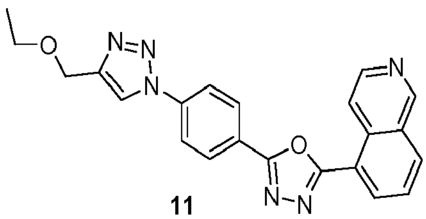

11 ,

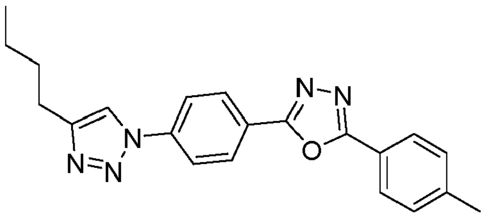

12 ,

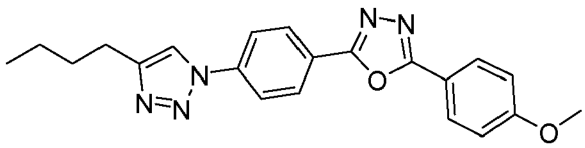

13 ,

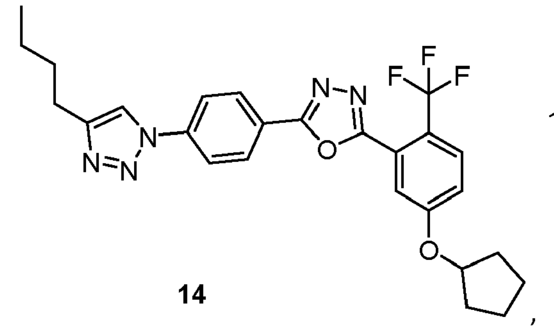

14 ,

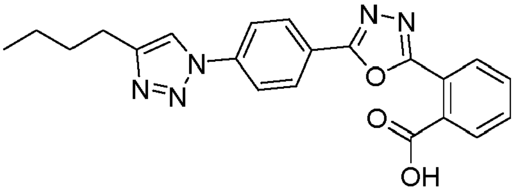

15 ,

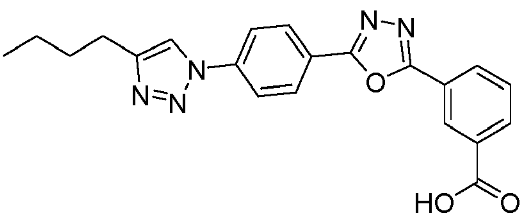

16 ,

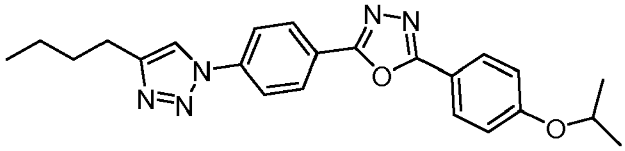

17 ,

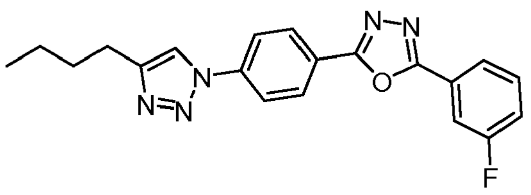

**18** ,

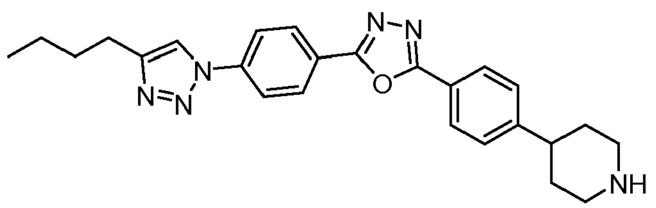

**19** ,

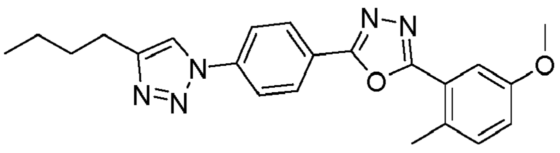

**20** ,

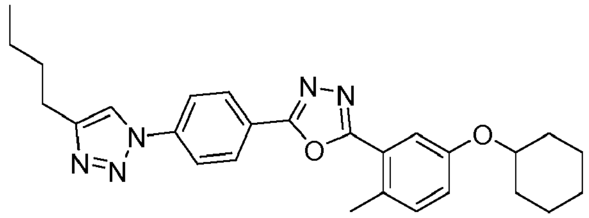

**21** ,

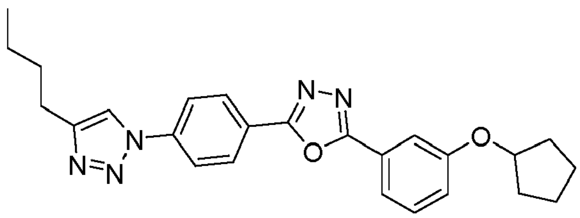

**22** ,

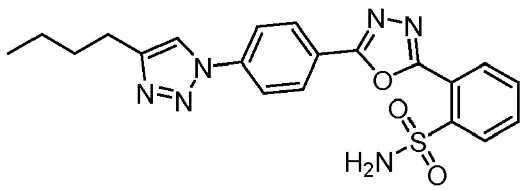

**23** ,

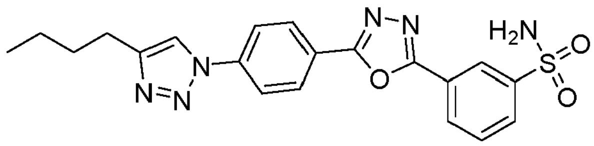

**24** ,

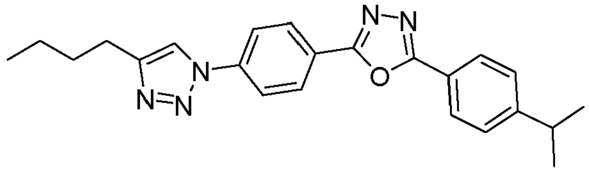

**25** ,

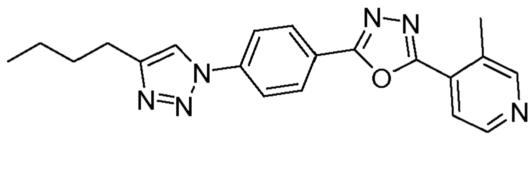

**26** ,

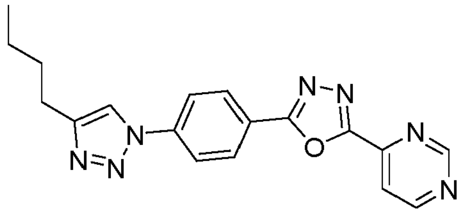

**27** ,

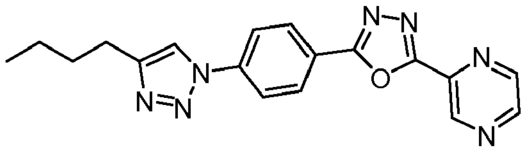

**28**

,

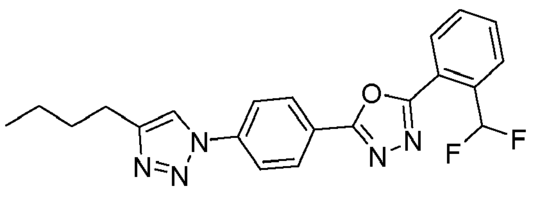

**29**

,

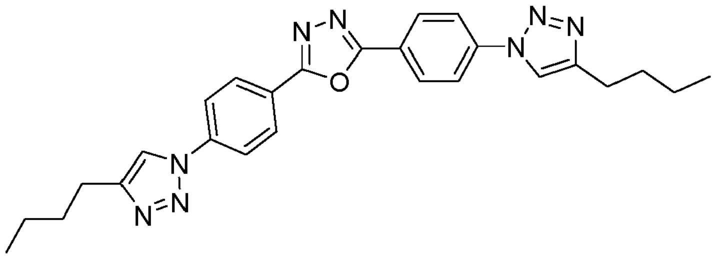

**30**

,

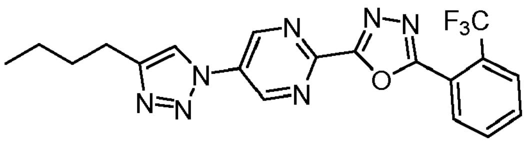

**31**

and

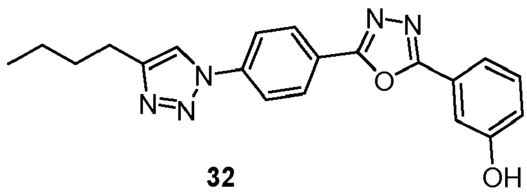

**32**

or salt, solvate, stereoisomer or tautomer thereof.

8. The compound, salt, solvate, stereoisomer or tautomer according to any one of the previous claims being an inhibitor of at least one DDX helicase, preferably being an inhibitor of at least two DDX helicases, preferably said DDX helicase is selected from the group consisting of DDX3X, DDX4, DDX5, DDX17, DDX21, DDX23, DDX18, DDX24, DDX48, DDX1, DDX2A and DDX2B.

9. The compound, salt, solvate, stereoisomer or tautomer as defined in any one of the previous claims for medical use.

10. The compound, salt, solvate, stereoisomer or tautomer as defined in any one of the previous claims for use in the prevention and/or treatment of a hyperproliferative disorder.

11. The compound, salt, solvate, stereoisomer or tautomer for use according to claim 10, wherein said hyperproliferative disorder is dependent on a DDX helicase, preferably said hyperproliferative disorder is dependent on at least two DDX helicases, preferably said DDX helicase is selected from the group consisting of: DDX3X, DDX4, DDX5, DDX17, DDX21, DDX23, DDX18, DDX24, DDX48, DDX1, DDX2A and DDX2B.

12. The compound, salt, solvate, stereoisomer or tautomer for use according to claim 10 or 11, wherein said hyperproliferative disorder is selected from the group consisting of: cancer, neurofibromatosis, hemangiomas, hyperproliferative arterial stenosis, a myelodysplastic disorder, arthritis, inflammatory arthritis, familial intestinal polyposes, histiocytosis, hyperkeratosis, including keloids, hypertrophic scars, oral leukoplakias and actinic keratosis, and papulosquamous eruptions including, pityriasis rosea, lichen planus and psoriasis; preferably said cancer is primary or metastatic; preferably said cancer is selected from the group consisting of: hepatocellular carcinoma, lung cancer, melanoma, pancreatic cancer, prostate cancer, cutaneous T lymphocyte lymphoma, breast cancer, colon cancer, colorectal cancer, lymphoma, liver cancer, ovarian cancer, thyroid cancer, esophagus cancer, stomach cancer, gastrointestinal cancer, brain or central and peripheral nervous system cancer, testis cancer, bone cancer, retinoblastoma, cervical carcinoma, kidney cancer, myeloma, leukemia, endocrine cancer, sarcoma, skin cancer, sinus cancer, urethral cancer, bladder cancer, genitourinary cancer, oral cancer, head and neck squamous carcinoma, pontine tumors, hepatoblastoma, gallbladder cancer; preferably said familial intestinal polyposes is Gardner syndrome; preferably said lung cancer is lung adenocarcinoma; preferably said pancreatic cancer is epithelioid pancreas carcinoma; preferably said breast cancer is triple negative breast cancer; preferably said brain or central and peripheral nervous system cancer is selected from the group consisting of: glioblastoma, glioma, medulloblastoma and neuroblastoma; preferably said cervical carcinoma is cervical carcinoma-in-situ; preferably said leukemia is acute lymphocytic leukemia, acute myelogenous leukemia, chronic lymphocytic leukemia and chronic myelogenous leukemia; preferably said sarcoma is selected from the group consisting of: angiosarcomas, osteosarcomas, fibrosarcomas, muscle rhabdomyosarcoma and ewing sarcoma; preferably said cancer is resistant to one or more of: gefitinib, erlotinib, olaparib, an anthracycline, temozolomide, Paclitaxel, Trastuzumab, Epirubicin, Doxorubicin, Adriamycin, Vincristine, cisplatin, 5-fluorouracil, oxaliplatin, mitomycin C, Bortezomib, Thalidomide, Dexamethasone, Tamoxifen, Etoposide, Lapatinib, Melphalan, Adriamycin, an EGFR inhibitor, a pro-apoptotic agent, a monoclonal antibody, an interleukin or interferon; preferably said cancer has a mutation in one or more of the following genes: CDKN2A, PTEN, CDKN2C, N-RAS, TERT, TP53, K-RAS, HER2neu, LKB1, BRAF, p53, ARID1A, RB1, BRCA1, BRCA2, Wnt7b, MDR1, ABCG2, PDCD4, P-gp, MRP1, ABCB1, BAFF, CCND1, GRB2, ERK2, RSK1, RSK2, , AKT, MET, ACVR2A, EP300, PI3KCA, TGFBR2, KDR, APC, ALK, EGFR, ROS1.

**13.** The compound, salt, solvate, stereoisomer or tautomer for use according to any one of claims 9-12, wherein said compound, salt, solvate, stereoisomer or tautomer is combined with a further therapeutic intervention, preferably said further therapeutic intervention is: surgery, radiotherapy or chemotherapy.

**14.** A pharmaceutical composition comprising:

- the compound, salt, solvate, stereoisomer or tautomer as defined in any one of claims 1 to 8,
- a pharmaceutically acceptable vehicle, and
- optionally a further therapeutic agent,

preferably said further therapeutic agent is an anti-proliferative agent, anti-pain agent, anti-emetic agent (such as Aprepitant, Fosaprepitant, Dolasetron, Granisetron, Ondansetron, Palonosetron, Tropisetron, Ramosetron or Dexamethasone), preferably said further therapeutic agent is selected from the group consisting of: Gefitinib, Erlotinib, Olaparib, an Anthracycline, Temozolomide, Paclitaxel, Trastuzumab, Epirubicin, Doxorubicin, Adriamycin, Vincristine, Cisplatin, 5-fluorouracil, Oxaliplatin, Mitomycin C, Bortezomib, Thalidomide, Dexamethasone, Tamoxifen, Etoposide, Lapatinib, Melphalan, Adriamycin and EGFR inhibitors.

**15.** The pharmaceutical composition according to claim 14 for the use as defined in any one of claims 10-13.

**16.** A process of making the compound, salt, solvate, stereoisomer or tautomer as defined in any one of claims 1-8, comprising the step of reacting a compound of formula (II):

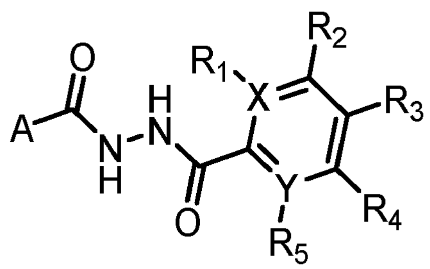

(II)

with Burgess Reagent or with $POCl_3$ thus obtaining cyclisation of the -C(O)-NH-NH-C(O)-moiety.

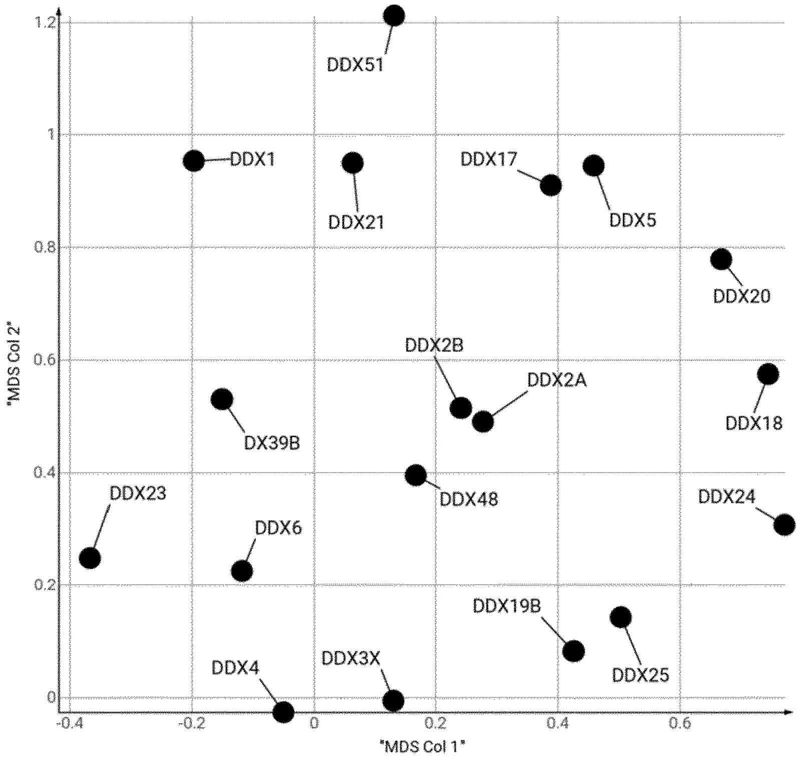
DDX51
DDX1
DDX21
DDX17
DDX5
DDX20
DDX2B
DDX2A
DX39B
DDX18
DDX48
DDX23
DDX24
DDX6
DDX19B
DDX25
DDX4
DDX3X
"MDS Col 2"
"MDS Col 1"
1.2
1
0.8
0.6
0.4
0.2
0
-0.4
-0.2
0
0.2
0.4
0.6

Fig. 1

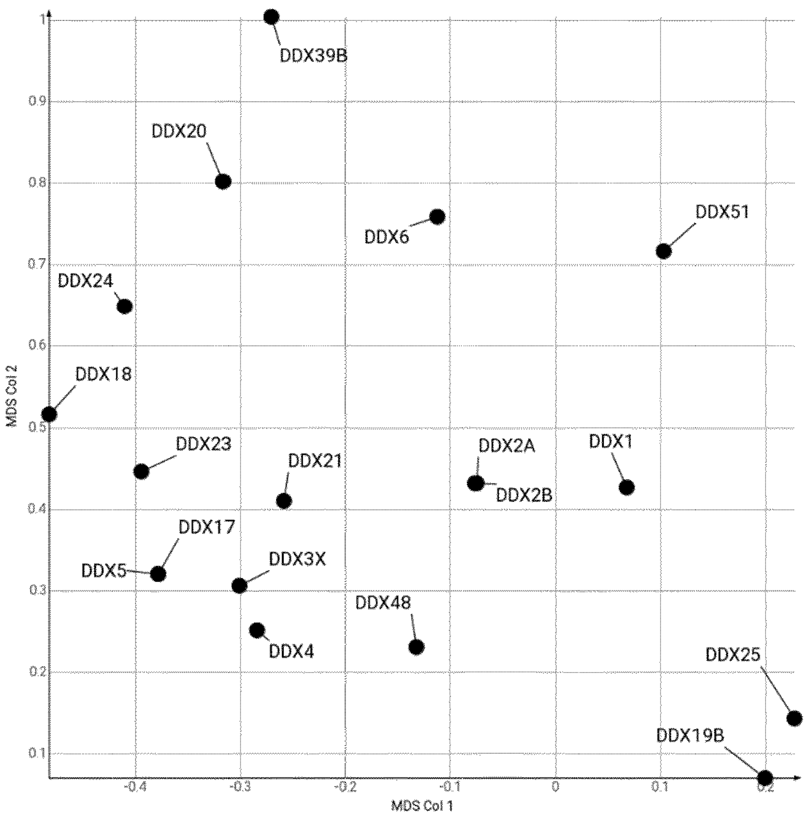
DDX39B
DDX20
DDX6
DDX51
DDX24
DDX18
DDX23
DDX21
DDX2A
DDX1
DDX2B
DDX17
DDX5
DDX3X
DDX48
DDX4
DDX25
DDX19B
MDS Col 2
MDS Col 1
1
0.9
0.8
0.7
0.6
0.5
0.4
0.3
0.2
0.1
-0.4
-0.3
-0.2
-0.1
0
0.1
0.2

Fig. 2

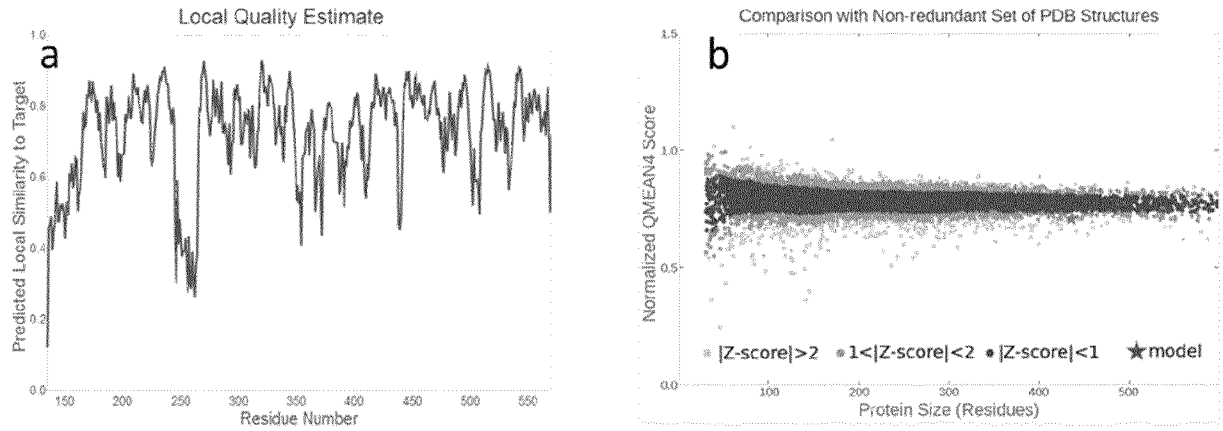


Fig. 3

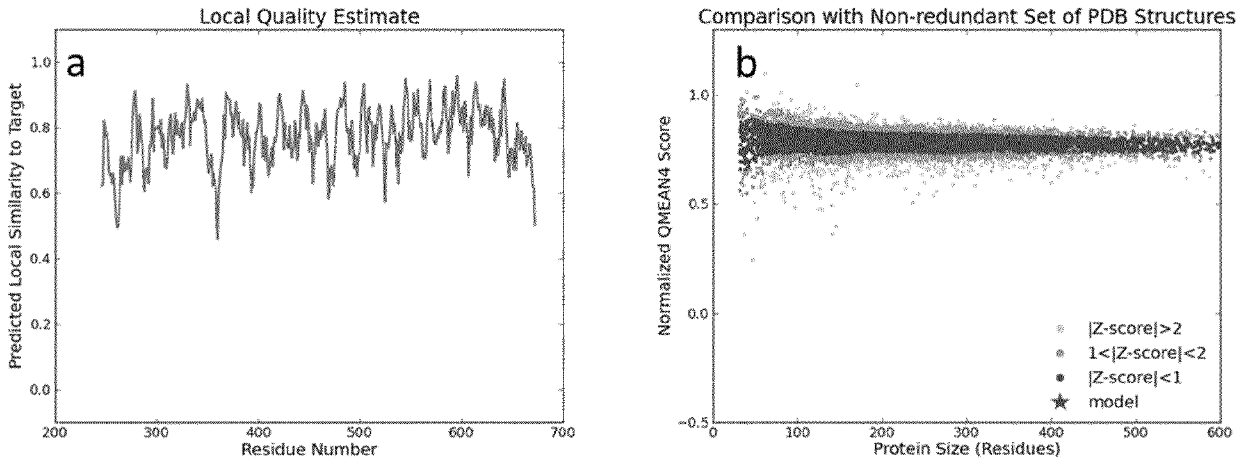


Fig. 4

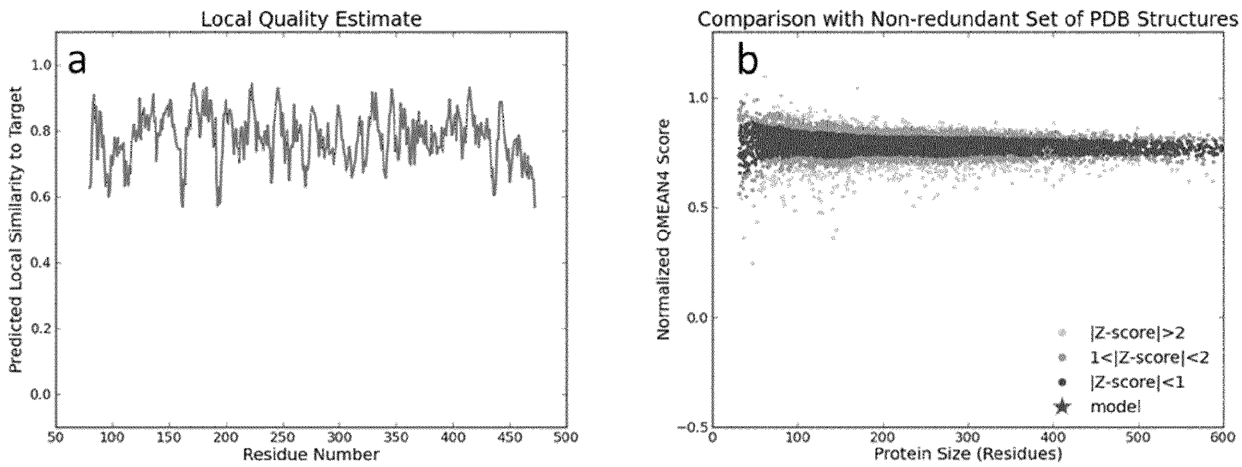


Fig. 5

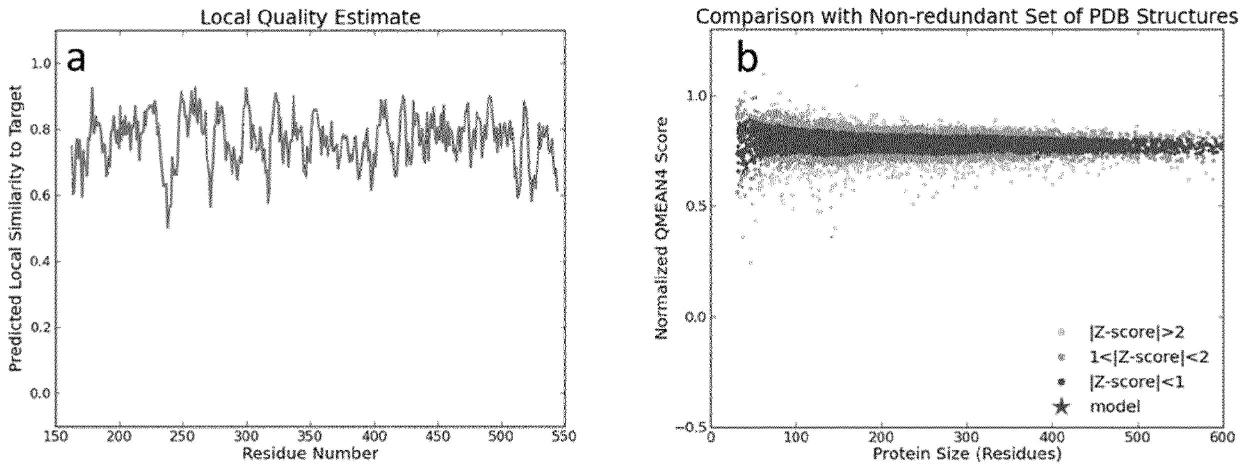


Fig. 6

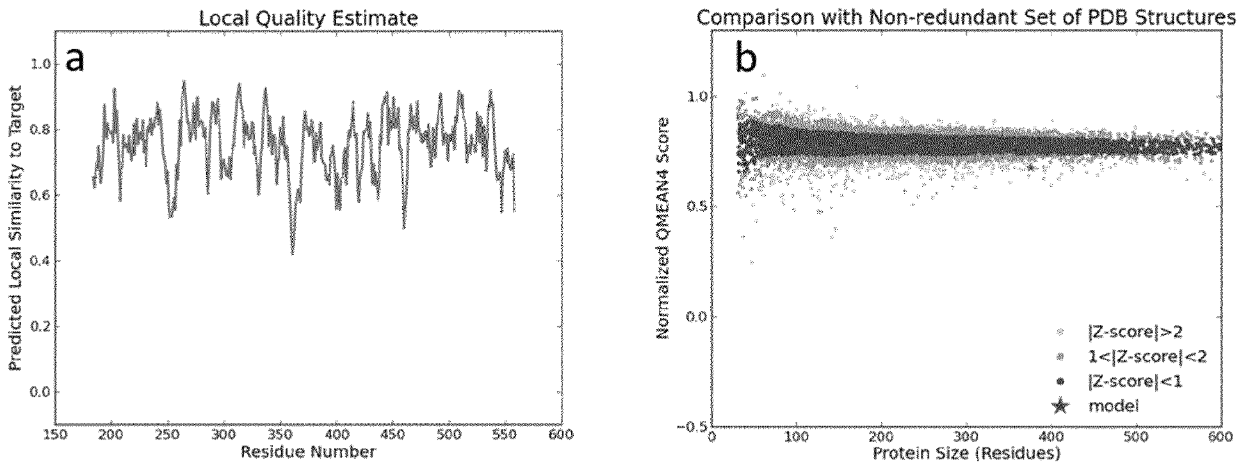


Fig. 7

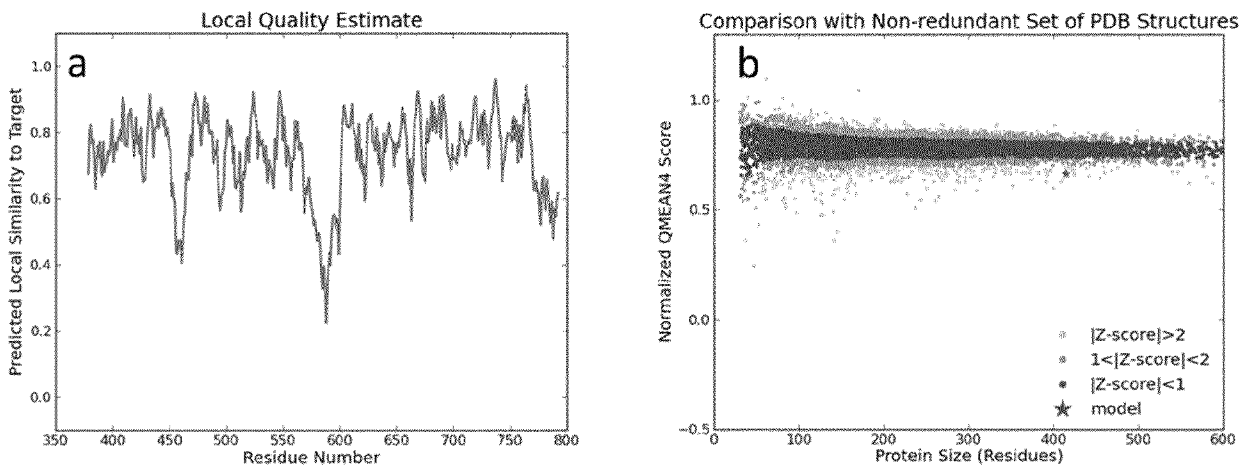


Fig. 8

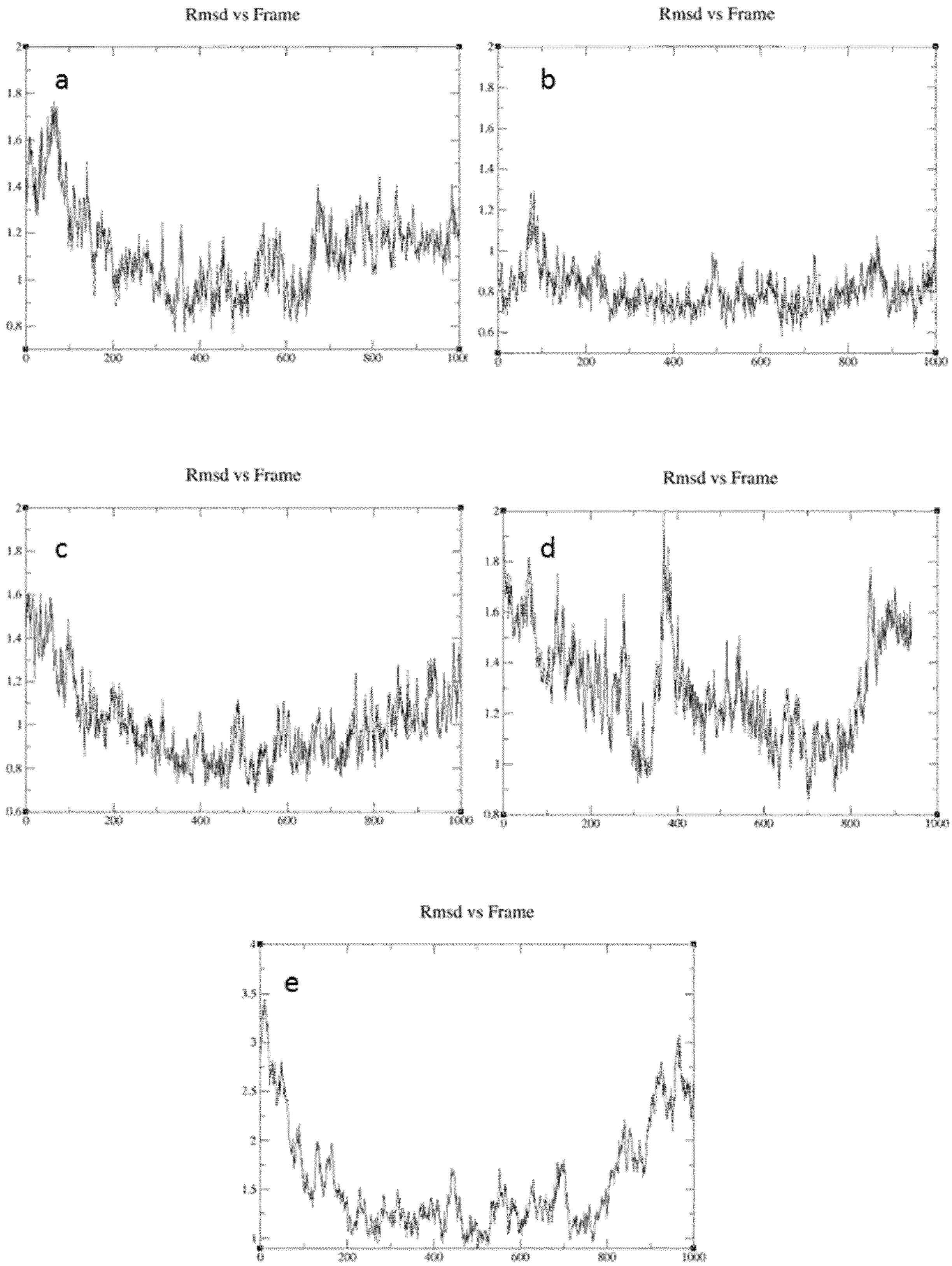
Rmsd vs Frame
a
2
1.8
1.6
1.4
1.2
1
0.8
0
200
400
600
800
1000
Rmsd vs Frame
b
2
1.8
1.6
1.4
1.2
1
0.8
0.6
0
200
400
600
800
1000
Rmsd vs Frame
c
2
1.8
1.6
1.4
1.2
1
0.8
0.6
0
200
400
600
800
1000
Rmsd vs Frame
d
2
1.8
1.6
1.4
1.2
1
0.8
0
200
400
600
800
1000
Rmsd vs Frame
e
4
3.5
3
2.5
2
1.5
1
0
200
400
600
800
1000

Fig. 9

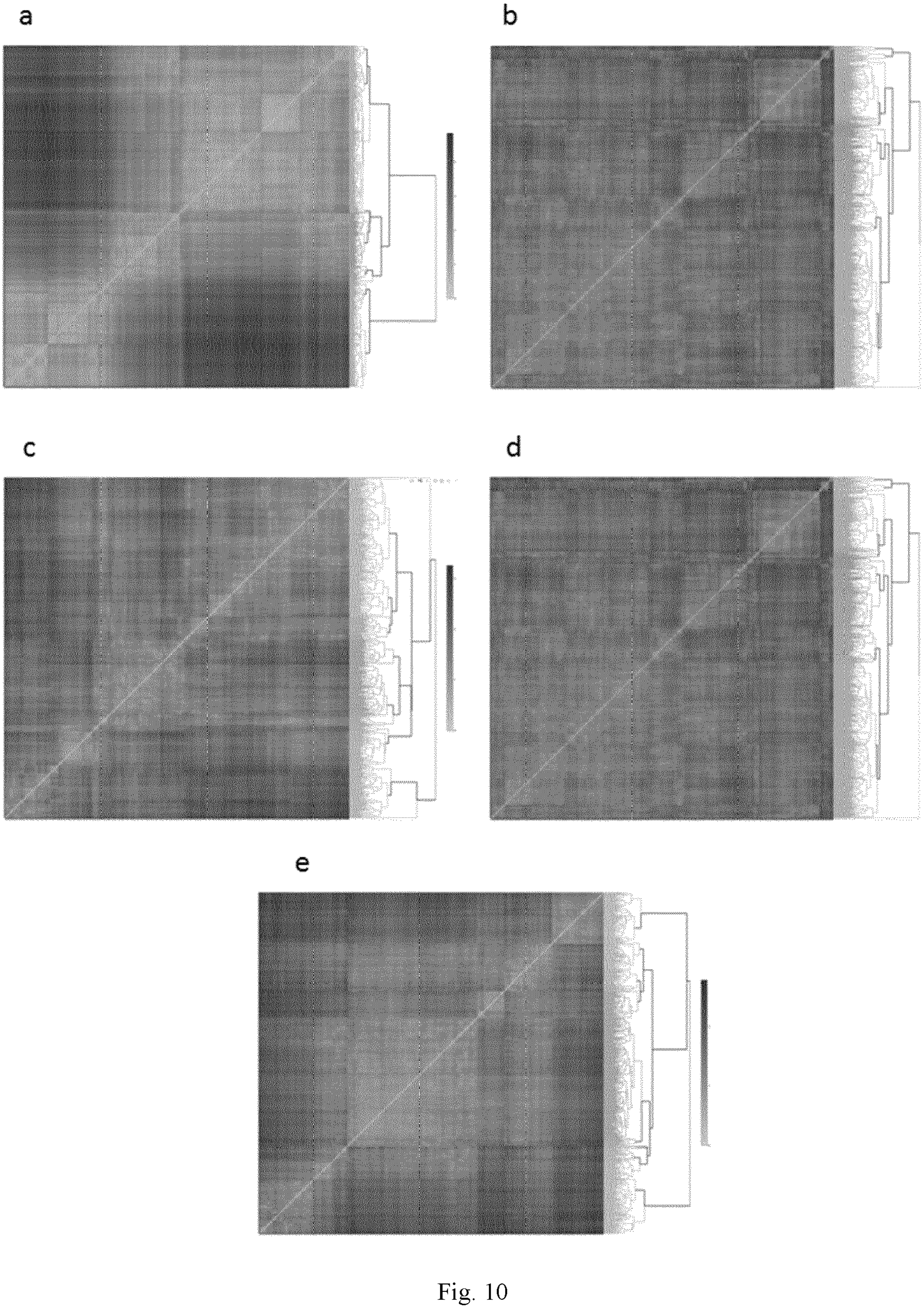


Fig. 10

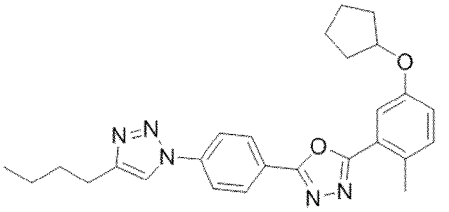

**3**

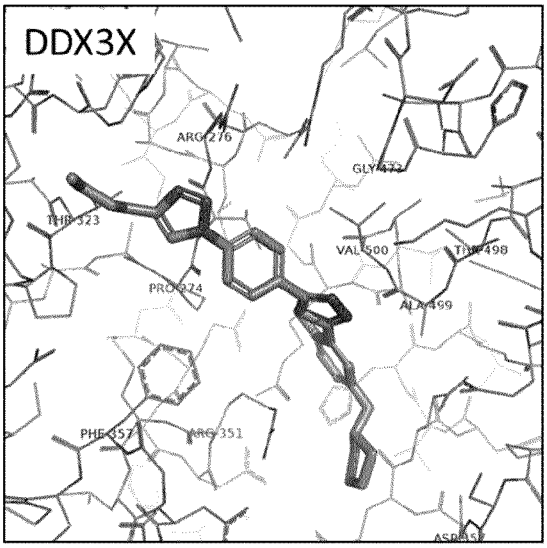


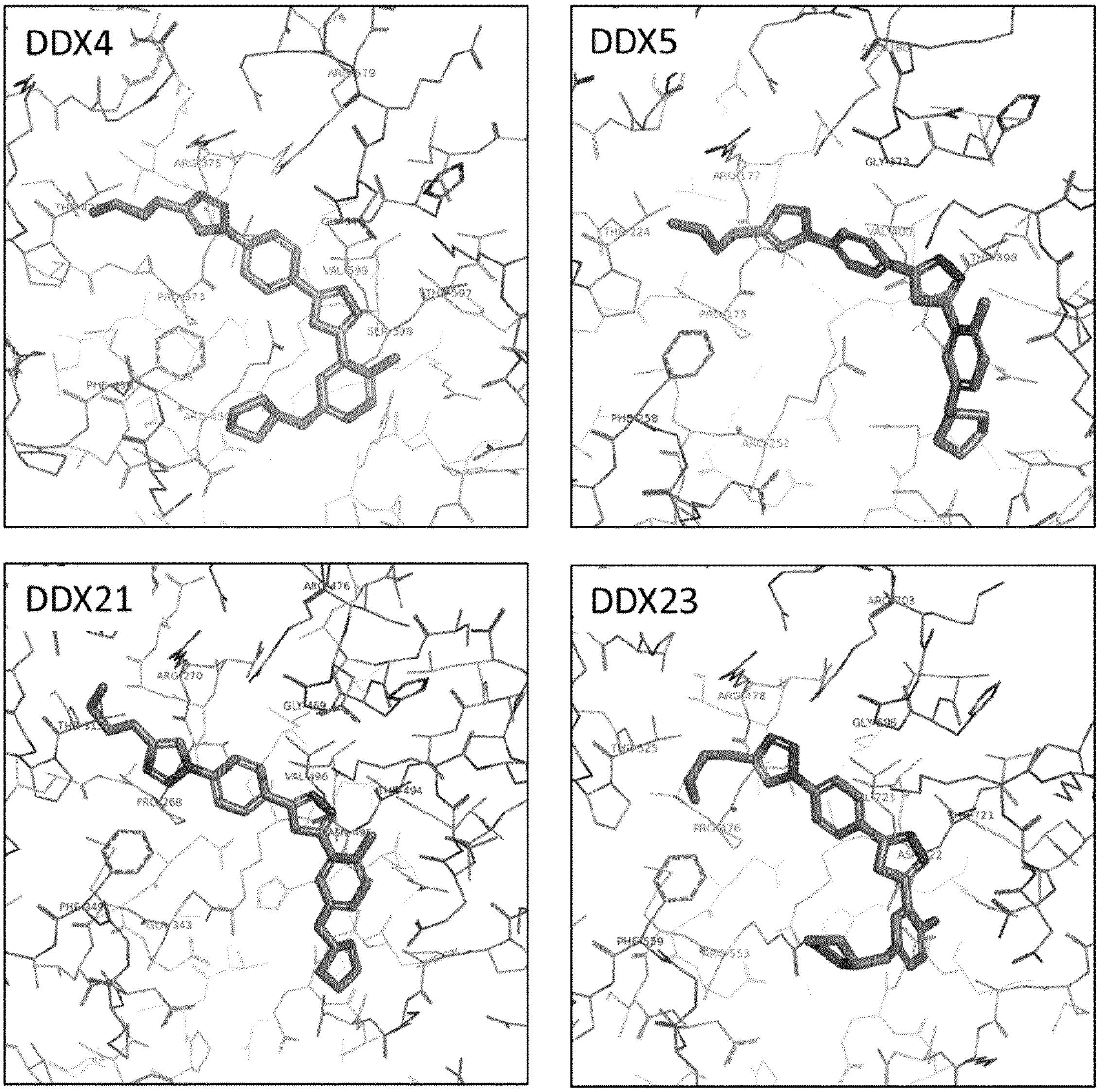


Fig. 11

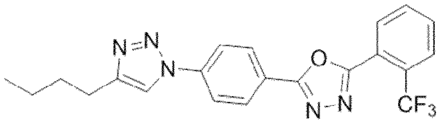

5

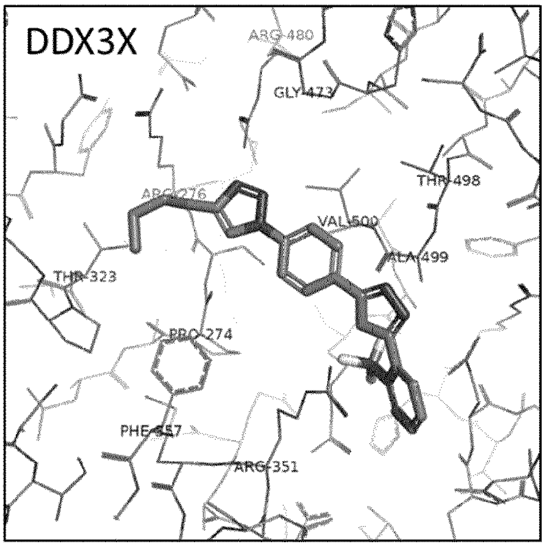


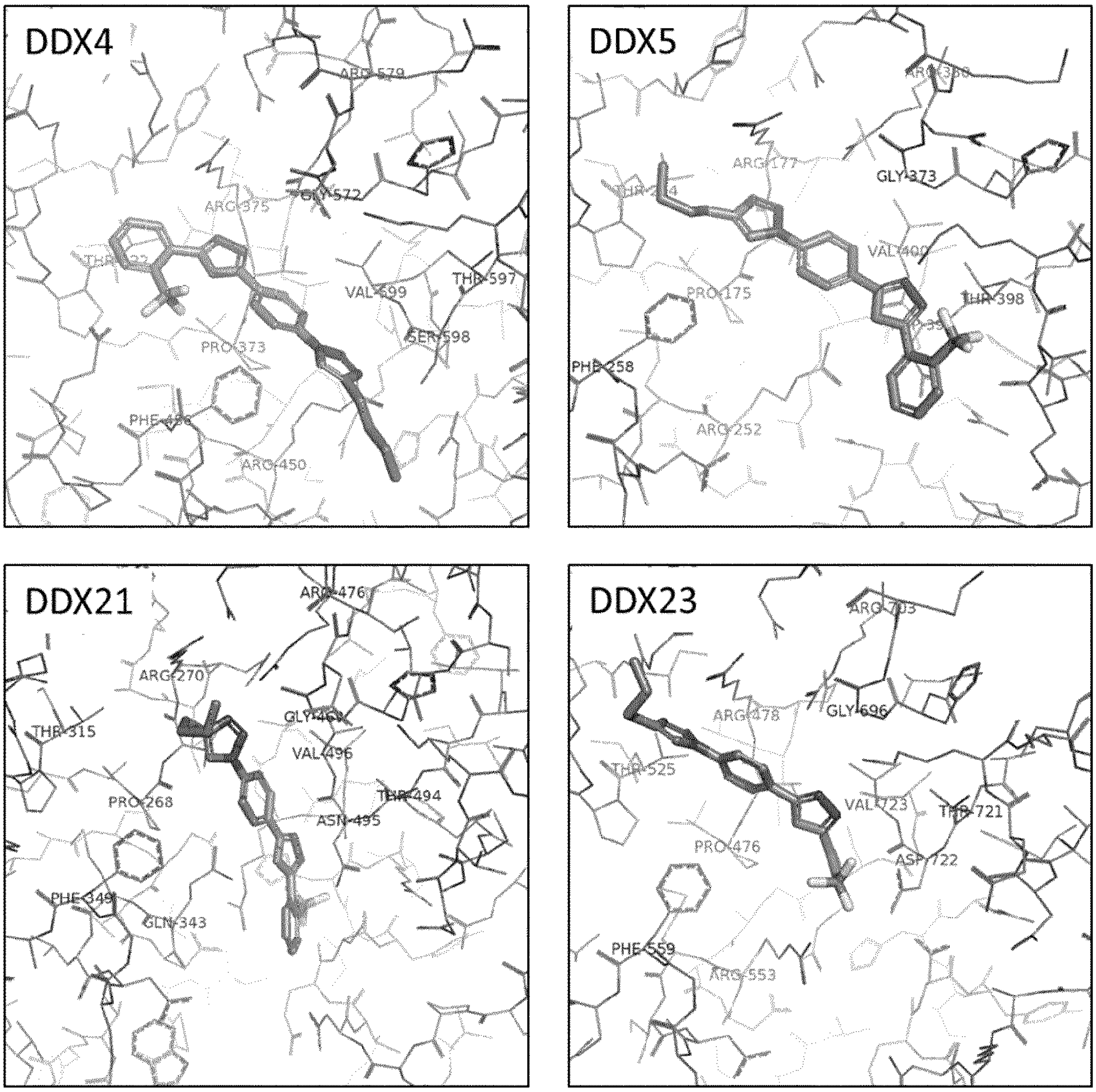


Fig. 12

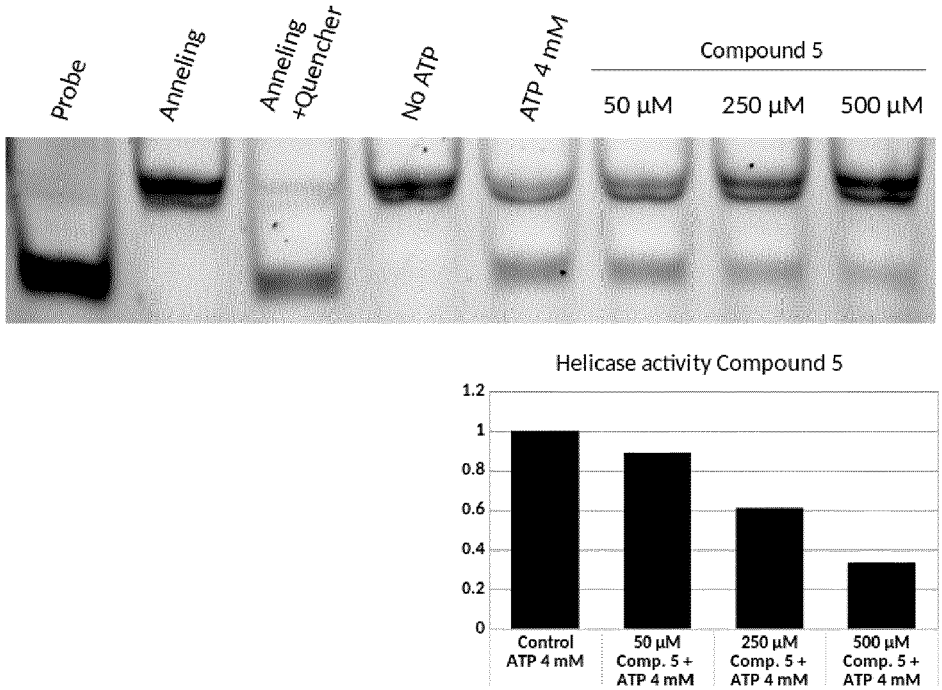
Probe
Anneling
Anneling +Quencher
No ATP
ATP 4 mM
Compound 5
50 µM
250 µM
500 µM
Helicase activity Compound 5
1.2
1
0.8
0.6
0.4
0.2
0
Control ATP 4 mM
50 µM Comp. 5 + ATP 4 mM
250 µM Comp. 5 + ATP 4 mM
500 µM Comp. 5 + ATP 4 mM

Fig. 13

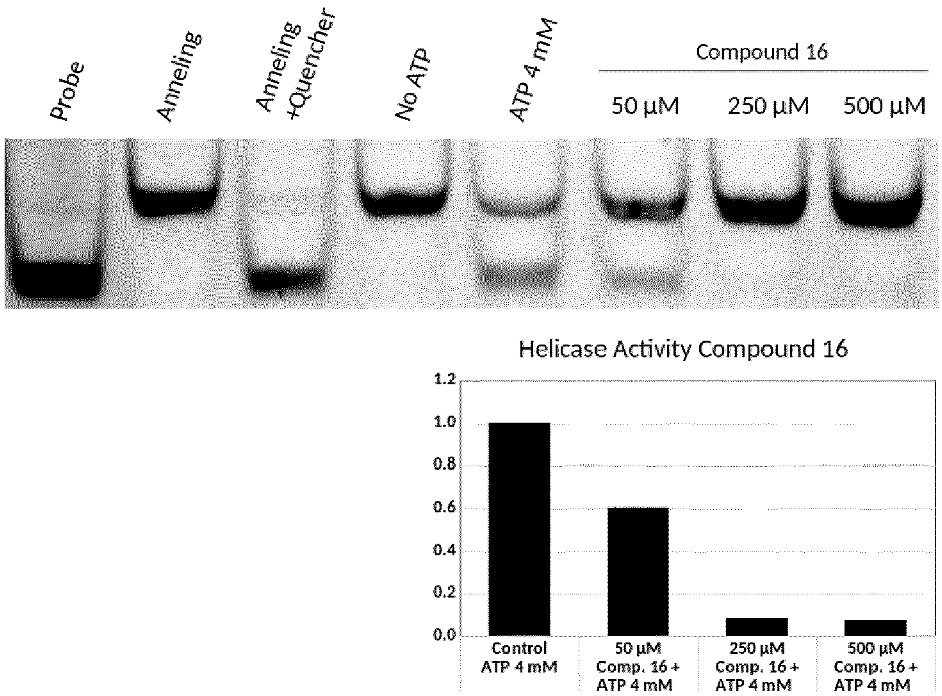
Probe
Anneling
Anneling +Quencher
No ATP
ATP 4 mM
Compound 16
50 µM
250 µM
500 µM
Helicase Activity Compound 16
1.2
1.0
0.8
0.6
0.4
0.2
0.0
Control ATP 4 mM
50 µM Comp. 16 + ATP 4 mM
250 µM Comp. 16 + ATP 4 mM
500 µM Comp. 16 + ATP 4 mM

Fig. 14

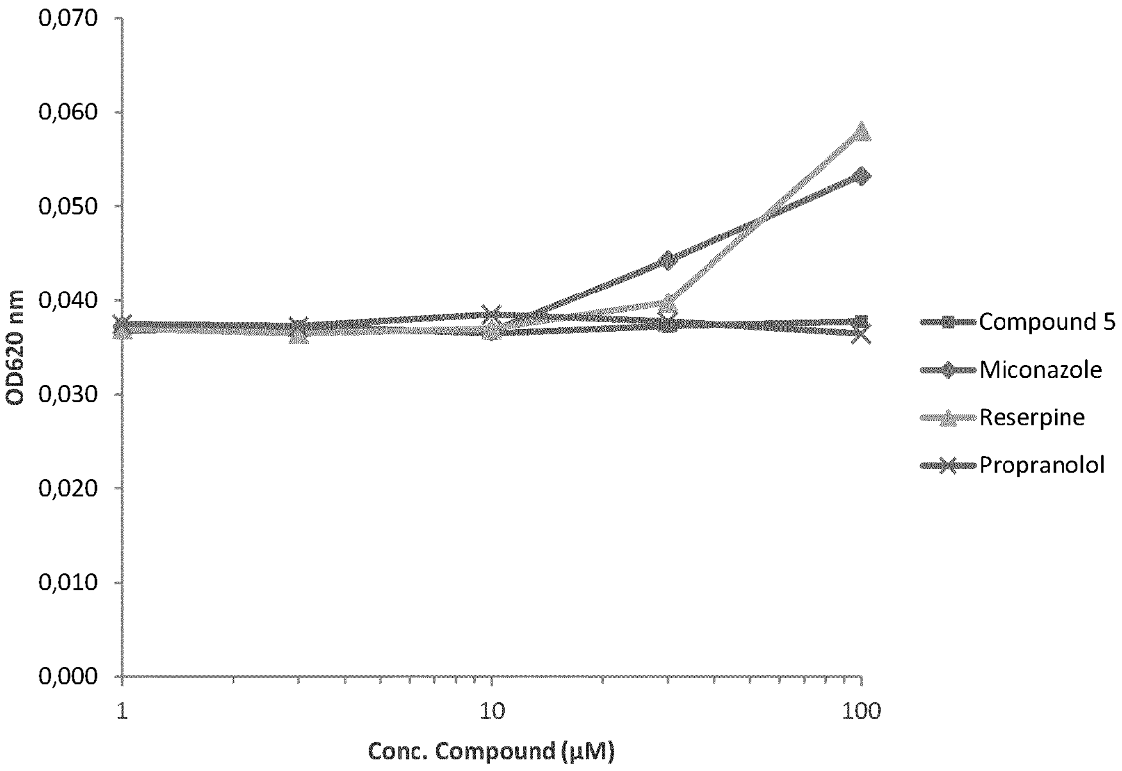


Fig. 15

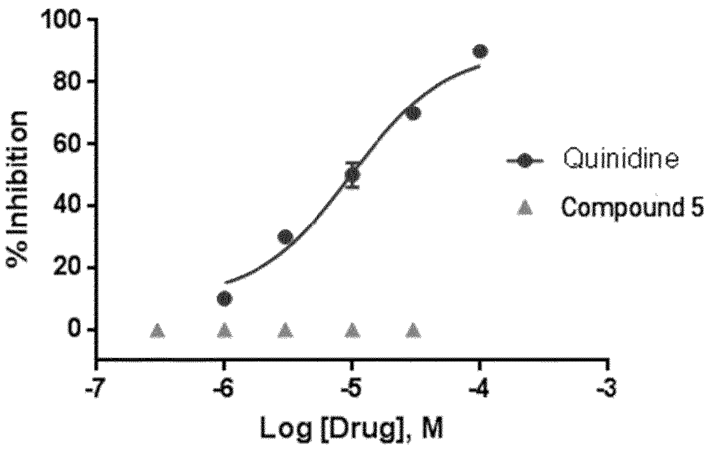


| Compound ID | $IC_{50}$ (uM) | Hill Slope |
|---|---|---|
| **5** | >30 | NA |
| **Quinidine (reference)** | 9.807 | 1.176 |

Fig. 16

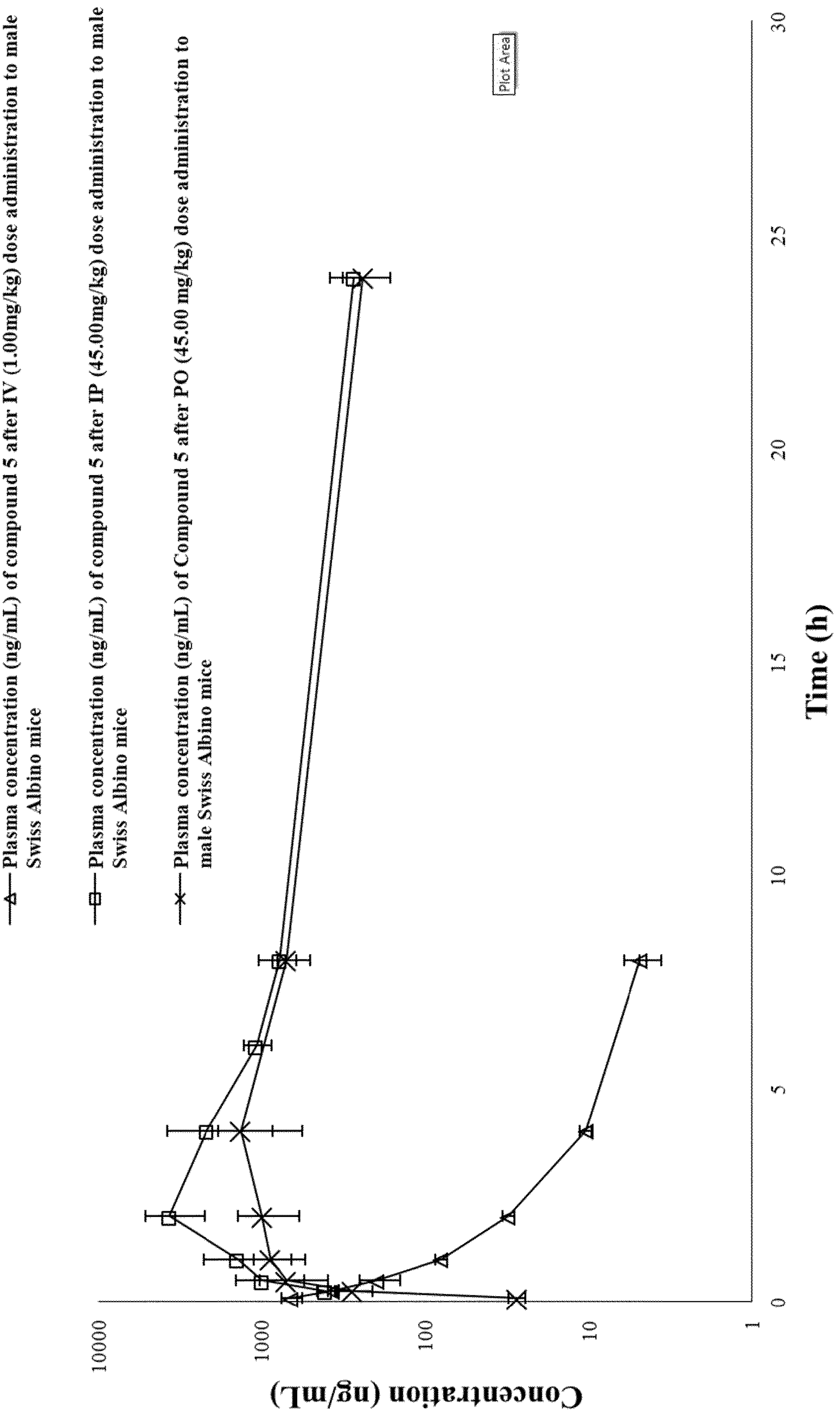
Plasma concentration (ng/mL) of compound 5 after IV (1.00mg/kg) dose administration to male Swiss Albino mice
Plasma concentration (ng/mL) of compound 5 after IP (45.00mg/kg) dose administration to male Swiss Albino mice
Plasma concentration (ng/mL) of Compound 5 after PO (45.00 mg/kg) dose administration to male Swiss Albino mice
Plot Area
Concentration (ng/mL)
10000
1000
100
10
1
Time (h)
0
5
10
15
20
25
30

Fig. 17

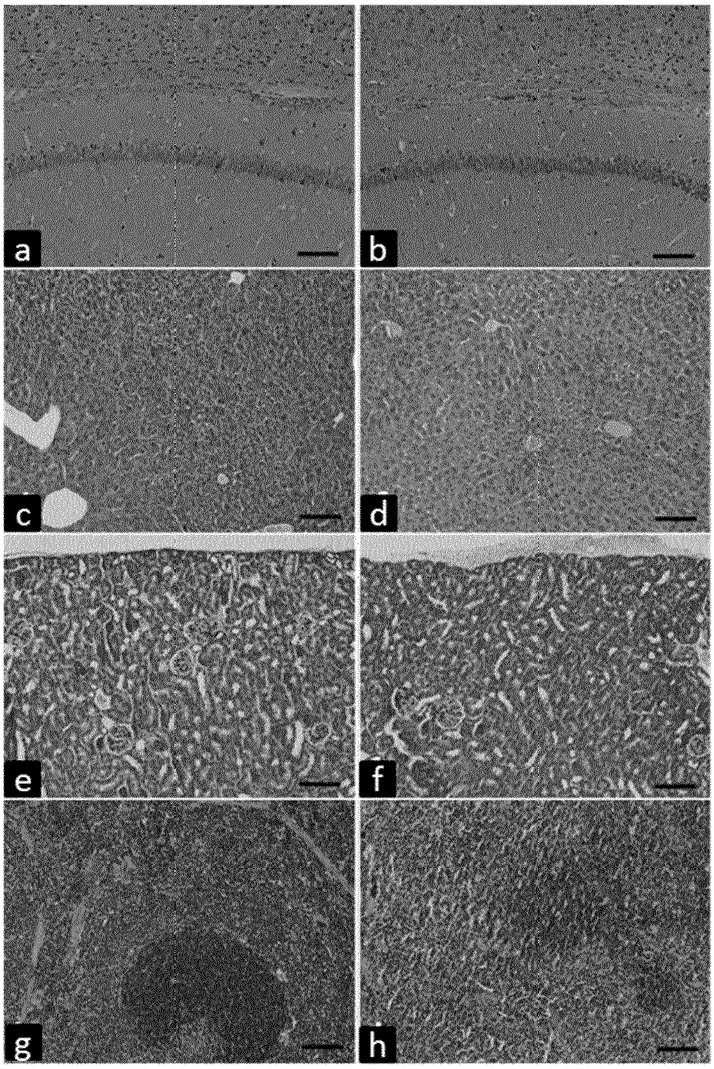


Fig. 18

a.

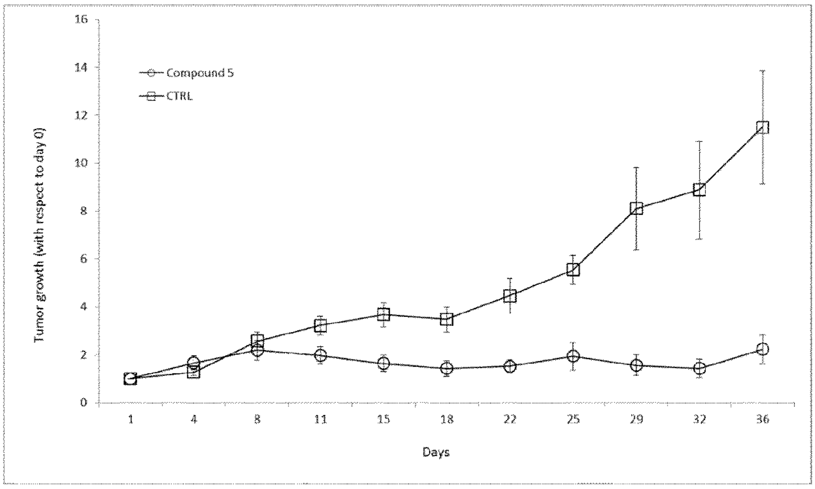
Compound 5
CTRL
Tumor growth (with respect to day 0)
Days
0
2
4
6
8
10
12
14
16
1
4
8
11
15
18
22
25
29
32
36

b.

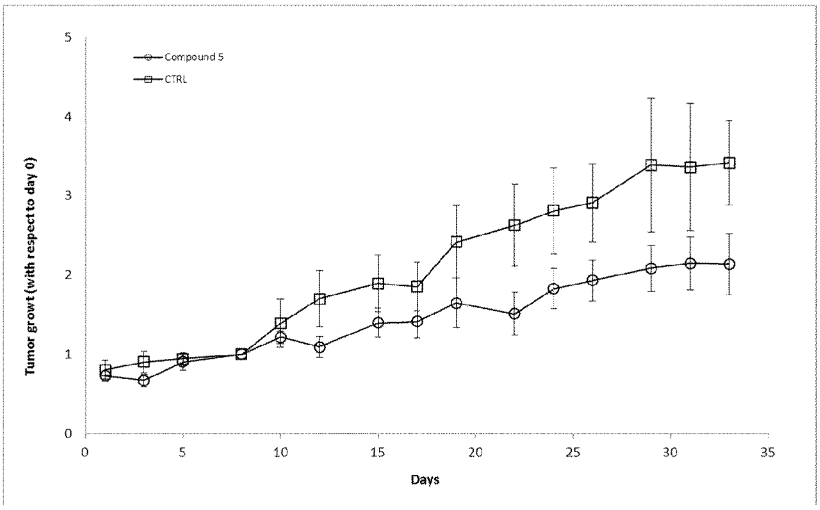
Compound 5
CTRL
Tumor growt (with respect to day 0)
Days
0
1
2
3
4
5
0
5
10
15
20
25
30
35

c.

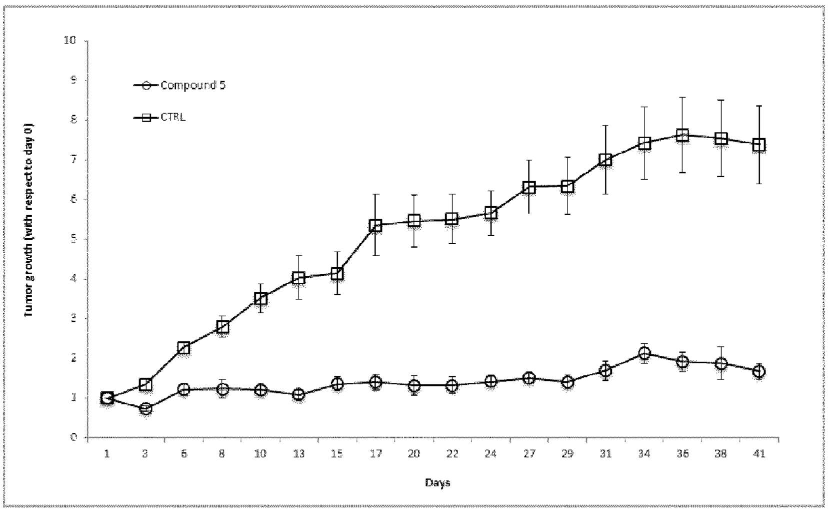
Compound 5
CTRL
Tumor growth (with respect to day 0)
Days
0
1
2
3
4
5
6
7
8
9
10
1
3
6
8
10
13
15
17
20
22
24
27
29
31
34
36
38
41

Fig. 19

Europäisches Patentamt
European Patent Office
Office européen des brevets

# EUROPEAN SEARCH REPORT

Application Number
EP 21 15 9563

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | SIWACH ANKIT ET AL: "Therapeutic potential of oxadiazole or furadiazole containing compounds", BMC CHEMISTRY, vol. 14, no. 70, 7 December 2020 (2020-12-07), pages 1-40, XP055826594, DOI: 10.1186/s13065-020-00721-2 Retrieved from the Internet: URL:https://bmcchem.biomedcentral.com/track/pdf/10.1186/s13065-020-00721-2.pdf> * scheme 12; page 13 - page 21; table 12 * | 1-16 | INV. C07D271/107 C07D413/10 C07D413/14 A61P35/00 A61P1/08 A61K31/4245 |
| A | CHAKRAPANI B ET AL: "Synthesis and Anticancer Evaluation of 1,2,4-Oxadiazole Linked Imidazothiadiazole Derivatives", RUSSIAN JOURNAL OF GENERAL CHEMISTRY, M A I K NAUKA - INTERPERIODICA, RU, vol. 88, no. 5, 26 June 2018 (2018-06-26), pages 1020-1024, XP036535023, ISSN: 1070-3632, DOI: 10.1134/S1070363218050304 [retrieved on 2018-06-26] * scheme 1 * | 1-16 | TECHNICAL FIELDS SEARCHED (IPC) C07D A61K A61P |
| A | WO 2006/031348 A2 (MERCK & CO INC [US]; COX CHRISTOPHER D [US] ET AL.) 23 March 2006 (2006-03-23) * claims 4, 6, 7 * | 1-16 | |
| A | WO 2009/043890 A1 (MERCK SERONO SA [CH]; MONTAGNE CYRIL [FR] ET AL.) 9 April 2009 (2009-04-09) * see e.g. compounds I57 on page 156; claims 1, 12, 15 * | 1-16 | |

1

The present search report has been drawn up for all claims

| Place of search | Date of completion of the search | Examiner |
|---|---|---|
| Munich | 23 July 2021 | Grégoire, Ariane |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT ON EUROPEAN PATENT APPLICATION NO. EP 21 15 9563

This annex lists the patent family members relating to the patent documents cited in the above-mentioned European search report. The members are as contained in the European Patent Office EDP file on
The European Patent Office is in no way liable for these particulars which are merely given for the purpose of information.

23-07-2021

| Patent document cited in search report | Publication date | Patent family member(s) | Publication date |
|---|---|---|---|
| WO 2006031348 A2 | 23-03-2006 | AU 2005285422 A1 | 23-03-2006 |
| | | CA 2576057 A1 | 23-03-2006 |
| | | CN 101005837 A | 25-07-2007 |
| | | EP 1781286 A2 | 09-05-2007 |
| | | JP 2008510013 A | 03-04-2008 |
| | | US 2008153887 A1 | 26-06-2008 |
| | | WO 2006031348 A2 | 23-03-2006 |
| WO 2009043890 A1 | 09-04-2009 | AR 068730 A1 | 02-12-2009 |
| | | AU 2008306886 A1 | 09-04-2009 |
| | | BR PI0817597 A2 | 07-04-2015 |
| | | CA 2696829 A1 | 09-04-2009 |
| | | CN 101815707 A | 25-08-2010 |
| | | CN 104478821 A | 01-04-2015 |
| | | EA 201070423 A1 | 29-10-2010 |
| | | EP 2193126 A1 | 09-06-2010 |
| | | ES 2547877 T3 | 09-10-2015 |
| | | IL 204520 A | 21-04-2016 |
| | | JP 5727223 B2 | 03-06-2015 |
| | | JP 6224771 B2 | 01-11-2017 |
| | | JP 2010540593 A | 24-12-2010 |
| | | JP 2015025001 A | 05-02-2015 |
| | | JP 2016169223 A | 23-09-2016 |
| | | KR 20100083814 A | 22-07-2010 |
| | | US 2010305104 A1 | 02-12-2010 |
| | | US 2013109676 A1 | 02-05-2013 |
| | | WO 2009043890 A1 | 09-04-2009 |
| | | ZA 201001217 B | 28-04-2011 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 5077211 A, Yarosh
- US 4621 A
- US 023 A, Redziniak
- US 4508 A
- US 703 A, Redziniak


### Non-patent literature cited in the description


- **WANPEI CAI et al.** Wanted DEAD/H or Alive: Helicases Winding Up in Cancer. *Journal of the National Cancer Institute,* 2017, vol. 109 (6
- **XUE, Y. et al.** DDX5 promotes hepatocellular carcinoma tumorigenesis via Akt signaling pathway. *Biochemical and biophysical research communications,* 2018, vol. 503, 2885
- **MA, Z. et al.** Knockdown of DDX5 Inhibits the Proliferation and Tumorigenesis in Esophageal Cancer. *Oncology research,* 2017, vol. 25, 887
- **GUTURI, K. K. N. et al.** DEAD-box protein p68 is regulated by β-catenin/transcription factor 4 to maintain a positive feedback loop in control of breast cancer progression. *Breast cancer research,* 2014, vol. 16, 496
- **CHOI, Y.-J. ; LEE, S.-G.** The DEAD-box RNA helicase DDX3 interacts with DDX5, co-localizes with it in the cytoplasm during the G2/M phase of the cycle, and affects its shuttling during mRNP export. *J. Cell. Biochem.,* 2012, vol. 113, 985
- **MAZUREK, A. et al.** DDX5 regulates DNA replication and is required for cell proliferation in a subset of breast cancer cells. *Cancer discovery,* 2012, vol. 2, 812
- **WANG, D. et al.** RNA helicase DDX5 regulates microRNA expression and contributes to cytoskeletal reorganization in basal breast cancer cells. *Molecular & cellular proteomics,* 2012, vol. 11
- **DUTERTRE, M. et al.** Estrogen regulation and physiopathologic significance of alternative promoters in breast cancer. *Cancer research,* 2010, vol. 70, 3760
- **VELLKY, J. E. et al.** Expression and Localization of DDX3 in Prostate Cancer Progression and Metastasis. *The American journal of pathology,* 2019, vol. 189, 1256
- **WANG, Z. et al.** Rottlerin upregulates DDX3 expression in hepatocellular carcinoma. *Biochemical and biophysical research communications,* 2018, vol. 495, 1503
- **CHEN, H.-H. et al.** DDX3 activates CBC-eIF3-mediated translation of uORF-containing oncogenic mRNAs to promote metastasis in HNSCC. *Cancer research,* 2018, vol. 78, 4512
- **TANTRAVEDI, S. et al.** Targeting DDX3 in Medulloblastoma Using the Small Molecule Inhibitor RK-33. *Translational oncology,* 2018, vol. 12, 96
- **CANNIZZARO, E. et al.** DDX3X RNA helicase affects breast cancer cell cycle progression by regulating expression of KLF4. *FEBS letters,* 2018, vol. 592, 2308
- **HEERMA VAN VOSS, M. R. et al.** Combination treatment using DDX3 and PARP inhibitors induces synthetic lethality in BRCA1-proficient breast cancer. *Medical oncology,* 2017, vol. 34, 33
- **HEERMA VAN VOSS, M. R. et al.** The prognostic effect of DDX3 upregulation in distant breast cancer metastases. *Clinical & experimental metastasis,* 2017, vol. 34, 85
- Targeting mitochondrial translation by inhibiting DDX3: a novel radiosensitization strategy for cancer treatment. **VAN VOSS, M. R. H. et al.** Oncogene. Springer Nature, 2017, vol. 37, 63
- **HEERMA VAN VOSS, M. R. et al.** Nuclear DDX3 expression predicts poor outcome in colorectal and breast cancer. *OncoTargets and therapy,* 2017, vol. 10, 3501
- **XIE, M. et al.** RK-33 radiosensitizes prostate cancer cells by blocking the RNA helicase DDX3. *Cancer Res.,* 2016
- **HE, T.-Y. et al.** DDX3 promotes tumor invasion in colorectal cancer via the CK1ε/Dv12 axis. *Sci. Rep.,* 2016, vol. 6, 21483
- **WILKY, B. et al.** RNA helicase DDX3: a novel therapeutic target in Ewing sarcoma. *Oncogene,* 2016, vol. 35, 2574
- **XIE, M. et al.** RK-33 Radiosensitizes Prostate Cancer Cells by Blocking the RNA Helicase DDX3. *Cancer research,* 2016, vol. 76, 6340
- **BOL, G. M. et al.** DDX3, a potential target for cancer treatment. *Mol. Cancer,* 2015, vol. 14, 188

- **CAO, J. et al.** DDX21 promotes gastric cancer proliferation by regulating cell cycle. *Biochemical and biophysical research communications,* 2018, vol. 505, 1189
- **ZHANG, Y. et al.** Elevated DDX21 regulates c-Jun activity and rRNA processing in human breast cancers. *Breast cancer research,* 2014, vol. 16, 449
- **MAO, G. et al.** DDX23-Linc00630-HDAC1 axis activates the Notch pathway to promote metastasis. *Oncotarget,* 2017, vol. 8, 38937-38949
- **YIN, J. et al.** DEAD-box RNA helicase DDX23 modulates glioma malignancy via elevating miR-21 biogenesis. *Brain: a journal of neurology,* 2015, vol. 138, 2553
- **XANDE, J.G. et al.** Bicistronic transfer of CDKN2A and p53 culminates in collaborative killing of human lung cancer cells in vitro and in vivo. *Gene Ther.,* 2020, vol. 27, 51
- **CICENAS J et al.** KRAS, TP53, CDKN2A, SMAD4, BRCA1, and BRCA2 Mutations in Pancreatic Cancer. *Cancers,* 2017, vol. 9 (5), 42
- **HODIS E. et al.** A Landscape of Driver Mutations in Melanoma. *Cell,* 2012, vol. 150 (2), 251
- **GREULICH H.** The Genomics of Lung Adenocarcinoma. Opportunities for Targeted Therapies. *Genes Cancer,* December 2010, vol. 1 (12), 1200
- **HARBECK, N. et al.** Breast cancer. *Nat. Rev. Dis. Primers,* 2019, vol. 5, 66
- **PESHKIN BN et al.** BRCA1/2 mutations and triple negative breast cancers. *Breast Dis.,* 2010, vol. 32 (1-2), 25
- **JANKOWSKY.** Duplex unwinding with DDX proteins. *Methods in molecular biology,* 2010, vol. 587, 245
- **BIZEBARD.** A FRET-based, continuous assay for the helicase activity of DDX proteins. *Methods in molecular biology,* 2015, vol. 1259, 199
- **BERG et al.** Pharmaceutical Salts. *J. Pharm. Sci.,* 1977, vol. 66, 1
- *Drugs of Today,* 1983, vol. 19 (9), 499
- Design of Prodrugs. **H. BUNDGAARD.** Topics in Chemistry. Elsevier, 1985, 306
- **REMINGTON.** The Science and Practice of Pharmacy. Academic Press
- USFDA. Guidance for Industry: Estimating the Maximum Safe Starting Dose in Adult Healthy Volunteer. *Rockville, MD: US Food and Drug Administration,* 2005
- **SIEVERS F. et al.** Fast, scalable generation of high-quality protein multiple sequence alignments using Clustal Omega. *Mol. Syst. Biol.,* 11 October 2011, vol. 7, 539
- KNIME: The {K}onstanz {I}nformation {M}iner, Studies in Classification. **MICHAEL R. BERTHOLD et al.** Data Analysis, and Knowledge Organization (GfKL 2007). Springer, 2007
- **SAMMON JW.** A nonlinear mapping for data structure analysis. *IEEE Transactions on Computers,* 1969, vol. 18, 403
- **BIASINI M et al.** OpenStructure: an integrated software framework for computational structural biology. *Acta Cryst,* 2013
- **TAMINAU, J. et al.** Pharao: pharmacophore alignment and optimization. *J Mol Graph Model,* 2008, vol. 27, 16
- **BRUNS, R. F. ; WATSON, I. A.** Rules for identifying potentially reactive or promiscuous compounds. *J Med Chem,* 2012, vol. 55, 9763
- **BAELL, J. B.** Screening-based translation of public research encounters painful problems. *ACS Med Chem Lett,* 2015, vol. 6, 229
- **DAHLIN, J. L. et al.** PAINS in the assay: chemical mechanisms of assay interference and promiscuous enzymatic inhibition observed during a sulfhydryl-scavenging HTS. *J Med Chem,* 2015, vol. 58, 2091
- **MIKKO J. VAINIO ; MARK S. JOHNSON.** Generating Conformer Ensembles Using a Multiobjective Genetic Algorithm. *Journal of Chemical Information and Modeling,* 2007, vol. 47, 2462
- **J. SANTERI PURANEN et al.** Accurate conformation-dependent molecular electrostatic potentials for high-throughput in-silico drug discovery. *Journal of Computational Chemistry,* 2010, vol. 31, 1722
- **TAMINAU, J. ; THIJS, G. ; DE WINTER, H.** Pharao: pharmacophore alignment and optimization. *J Mol Graph Model,* 2008, vol. 27, 161
- **DOLINSKY TJ et al.** PDB2PQR: Expanding and upgrading automated preparation of biomolecular structures for molecular simulations. *Nucleic Acids Res,* 2007, vol. 35, W522
- **DOLINSKY TJ et al.** PDB2PQR: an automated pipeline for the setup, execution, and analysis of Poisson-Boltzmann electrostatics calculations. *Nucleic Acids Res,* 2004, vol. 32, W665
- **MEAGHER KL ; REDMAN LT.** Carlson HA Development of polyphosphate parameters for use with the AMBER force field. *J Comp Chem,* 2003, vol. 24, 1016
- **PHILLIPS et al.** *J Comp Chem,* 2005, vol. 26, 1781
- **HUMPHREY, W. et al.** VMD - Visual Molecular Dynamics. *J Molec Graphics,* 1996, vol. 14, 33
- **O. TROTT ; A. J. OLSON.** AutoDock Vina: improving the speed and accuracy of docking with a new scoring function, efficient optimization and multithreading. *J Comp Chem,* 2010, vol. 31, 455
- **DAVID RYAN KOES et al.** Lessons Learned in Empirical Scoring with Smina from the CSAR 2011 Benchmarking Exercise. *J Chem Inf Model,* 2013, vol. 538, 1893

- **RUIZ-CARMONA S et al.** rDock: A Fast, Versatile and Open Source Program for Docking Ligands to Proteins and Nucleic Acids. *PLoS Comput Biol,* 2014, vol. 10, e1003571
- **MORLEY, S.D. ; AFSHAR, M.** alidation of an empirical RNA-ligand scoring function for fast flexible docking using Ribodock. *J Comput Aided Mol Des,* 2004, vol. 18, 189
- **WÓJCIKOWSKI, M. et al.** Performance of machine-learning scoring functions in structure-based virtual screening. *Scientific reports,* 2017, vol. 7, 46710
- **CANNIZZARO, E et al.** DDX3X RNA helicase affects breast cancer cell cycle progression by regulating expression of KLF4. *FEBS letters,* 2018, vol. 592, 2308
- **HASHEMI, V. et al.** The role of DEAD-box RNA helicase p68 (DDX5) in the development and treatment of breast cancer. *Journal of cellular physiology,* 2019, vol. 234, 5478
- **ALQAHTANI, H. et al.** DDX17 (P72), a Sox2 binding partner, promotes stem-like features conferred by Sox2 in a small cell population in estrogen receptor-positive breast cancer. *Cellular signalling,* 2016, vol. 28, 42
- **WANG ; YAO.** *Scientific Reports,* 2019, vol. 9, 20149
- **GARBELLI et al.** Targeting the Human DEAD-Box Polypeptide 3 (DDX3) RNA Helicase as a Novel Strategy to Inhibit Viral Replication. *Curr Med Chem,* 2011, vol. 18, 3015
- **COULTER COUNTER.** *J. Immunol. Methods,* 1981, vol. 41, 155
- **LEONARDI et al.** Activated kinase screening identifies the IKBKE oncogene as a positive regulator of autophagy. *Autophagy,* 2019, vol. 15, 312
- **HÖGBOM et al.** Crystal structure of conserved domains 1 and 2 of the human DEAD-box helicase DDX3X in complex with the mononucleotide AMP. *J Mol Biol,* 2007, vol. 372, 150
- **FLOOR et al.** Autoinhibitory Interdomain Interactions and Subfamily-specific Extensions Redefine the Catalytic Core of the Human DEAD-box Protein DDX3. *J Biol Chem,* 2016, vol. 291, 2412
- **EPLING et al.** Cancer-associated mutants of RNA helicase DDX3X are defective in RNA-stimulated ATP hydrolysis. *J Mol Biol,* 2015, vol. 427, 1779
- **WATERHOUSE, A. et al.** SWISS-MODEL: homology modeling of protein structures and complexes. *Nucleic Acids Res.,* 2018, vol. 46 (W1), W296-W303
- **GUEX, N. et al.** Automated comparative protein structure modeling with SWISS-MODEL and Swiss-PdbViewer: A historical perspective. *Electrophoresis,* 2009, vol. 30, S162-S173
- **BIENERT, S. et al.** The SWISS-MODEL Repository - new features and functionality. *Nucleic Acids Res.,* 2017, vol. 45, D313-D319
- **MARCO BIASINI et al.** SWISS-MODEL: modeling protein tertiary and quaternary structure using evolutionary information. *Nucleic Acids Research,* 01 July 2014, vol. 42 (W1), W252-W258
- **ARNOLD, K. et al.** The SWISS-MODEL workspace: a web-based environment for protein structure homology modeling. *Bioinformatics,* 2006, vol. 22, 195-201
- **BENKERT, P. et al.** Toward the estimation of the absolute quality of individual protein structure models. *Bioinformatics,* 2011, vol. 27, 343-350
- **ALTSCHUL, S.F. et al.** Gapped BLAST and PSI-BLAST: a new generation of protein database search programs. *Nucleic Acids Res,* 1997, vol. 25, 3389-3402
- **REMMERT, M. et al.** HHblits: lightning-fast iterative protein sequence searching by HMM-HMM alignment. *Nat Methods,* 2012, vol. 9, 173-175
- **GUEX, N. ; PEITSCH, M.C.** SWISS-MODEL and the Swiss-PdbViewer: an environment for comparative protein modeling. *Electrophoresis,* 1997, vol. 18, 2714-2723
- **SALI, A. ; BLUNDELL, T.L.** Comparative protein modeling by satisfaction of spatial restraints. *J Mol Biol,* 1993, vol. 234, 779-815
- **MARIANI, V. et al.** Assessment of template based protein structure predictions in CASP9. *Proteins,* 2011, vol. 79 (10), 37-58

SEQUENCE LISTING

<110> First Health Biopharmaceutical B.V.

<120> ANTI-PROLIFERATIVE AGENTS

<130> BE 141776

<160> 12

<170> PatentIn version 3.5

<210> 1
<211> 662
<212> PRT
<213> Homo sapiens

<400> 1

Met Ser His Val Ala Val Glu Asn Ala Leu Gly Leu Asp Gln Gln Phe
1               5                   10                  15

Ala Gly Leu Asp Leu Asn Ser Ser Asp Asn Gln Ser Gly Gly Ser Thr
            20                  25                  30

Ala Ser Lys Gly Arg Tyr Ile Pro Pro His Leu Arg Asn Arg Glu Ala
        35                  40                  45

Thr Lys Gly Phe Tyr Asp Lys Asp Ser Ser Gly Trp Ser Ser Ser Lys
    50                  55                  60

Asp Lys Asp Ala Tyr Ser Ser Phe Gly Ser Arg Ser Asp Ser Arg Gly
65                  70                  75                  80

Lys Ser Ser Phe Phe Ser Asp Arg Gly Ser Gly Ser Arg Gly Arg Phe
                85                  90                  95

Asp Asp Arg Gly Arg Ser Asp Tyr Asp Gly Ile Gly Ser Arg Gly Asp
            100                 105                 110

Arg Ser Gly Phe Gly Lys Phe Glu Arg Gly Gly Asn Ser Arg Trp Cys
        115                 120                 125

Asp Lys Ser Asp Glu Asp Asp Trp Ser Lys Pro Leu Pro Pro Ser Glu
    130                 135                 140

Arg Leu Glu Gln Glu Leu Phe Ser Gly Gly Asn Thr Gly Ile Asn Phe
145                 150                 155                 160

Glu Lys Tyr Asp Asp Ile Pro Val Glu Ala Thr Gly Asn Asn Cys Pro
                165                 170                 175

```
Pro His Ile Glu Ser Phe Ser Asp Val Glu Met Gly Glu Ile Ile Met
            180                 185                 190

Gly Asn Ile Glu Leu Thr Arg Tyr Thr Arg Pro Thr Pro Val Gln Lys
        195                 200                 205

His Ala Ile Pro Ile Ile Lys Glu Lys Arg Asp Leu Met Ala Cys Ala
    210                 215                 220

Gln Thr Gly Ser Gly Lys Thr Ala Ala Phe Leu Leu Pro Ile Leu Ser
225                 230                 235                 240

Gln Ile Tyr Ser Asp Gly Pro Gly Glu Ala Leu Arg Ala Met Lys Glu
                245                 250                 255

Asn Gly Arg Tyr Gly Arg Arg Lys Gln Tyr Pro Ile Ser Leu Val Leu
            260                 265                 270

Ala Pro Thr Arg Glu Leu Ala Val Gln Ile Tyr Glu Glu Ala Arg Lys
        275                 280                 285

Phe Ser Tyr Arg Ser Arg Val Arg Pro Cys Val Val Tyr Gly Gly Ala
    290                 295                 300

Asp Ile Gly Gln Gln Ile Arg Asp Leu Glu Arg Gly Cys His Leu Leu
305                 310                 315                 320

Val Ala Thr Pro Gly Arg Leu Val Asp Met Met Glu Arg Gly Lys Ile
                325                 330                 335

Gly Leu Asp Phe Cys Lys Tyr Leu Val Leu Asp Glu Ala Asp Arg Met
            340                 345                 350

Leu Asp Met Gly Phe Glu Pro Gln Ile Arg Arg Ile Val Glu Gln Asp
        355                 360                 365

Thr Met Pro Pro Lys Gly Val Arg His Thr Met Met Phe Ser Ala Thr
    370                 375                 380

Phe Pro Lys Glu Ile Gln Met Leu Ala Arg Asp Phe Leu Asp Glu Tyr
385                 390                 395                 400

Ile Phe Leu Ala Val Gly Arg Val Gly Ser Thr Ser Glu Asn Ile Thr
                405                 410                 415

Gln Lys Val Val Trp Val Glu Glu Ser Asp Lys Arg Ser Phe Leu Leu
            420                 425                 430
```

Asp Leu Leu Asn Ala Thr Gly Lys Asp Ser Leu Thr Leu Val Phe Val
435 440 445

Glu Thr Lys Lys Gly Ala Asp Ser Leu Glu Asp Phe Leu Tyr His Glu
450 455 460

Gly Tyr Ala Cys Thr Ser Ile His Gly Asp Arg Ser Gln Arg Asp Arg
465 470 475 480

Glu Glu Ala Leu His Gln Phe Arg Ser Gly Lys Ser Pro Ile Leu Val
485 490 495

Ala Thr Ala Val Ala Ala Arg Gly Leu Asp Ile Ser Asn Val Lys His
500 505 510

Val Ile Asn Phe Asp Leu Pro Ser Asp Ile Glu Glu Tyr Val His Arg
515 520 525

Ile Gly Arg Thr Gly Arg Val Gly Asn Leu Gly Leu Ala Thr Ser Phe
530 535 540

Phe Asn Glu Arg Asn Ile Asn Ile Thr Lys Asp Leu Leu Asp Leu Leu
545 550 555 560

Val Glu Ala Lys Gln Glu Val Pro Ser Trp Leu Glu Asn Met Ala Tyr
565 570 575

Glu His His Tyr Lys Gly Ser Ser Arg Gly Arg Ser Lys Ser Ser Arg
580 585 590

Phe Ser Gly Gly Phe Gly Ala Arg Asp Tyr Arg Gln Ser Ser Gly Ala
595 600 605

Ser Ser Ser Ser Phe Ser Ser Ser Arg Ala Ser Ser Ser Arg Ser Gly
610 615 620

Gly Gly Gly His Gly Ser Ser Arg Gly Phe Gly Gly Gly Gly Tyr Gly
625 630 635 640

Gly Phe Tyr Asn Ser Asp Gly Tyr Gly Gly Asn Tyr Asn Ser Gln Gly
645 650 655

Val Asp Trp Trp Gly Asn
660

<210> 2
<211> 419

<212> PRT
<213> Drosophila melanogaster

<400> 2

Tyr Ile Pro Pro Glu Pro Ser Asn Asp Ala Ile Glu Ile Phe Ser Ser
1 5 10 15

Gly Ile Ala Ser Gly Ile His Phe Ser Lys Tyr Asn Asn Ile Pro Val
20 25 30

Lys Val Thr Gly Ser Asp Val Pro Gln Pro Ile Gln His Phe Thr Ser
35 40 45

Ala Asp Leu Arg Asp Ile Ile Ile Asp Asn Val Asn Lys Ser Gly Tyr
50 55 60

Lys Ile Pro Thr Pro Ile Gln Lys Cys Ser Ile Pro Val Ile Ser Ser
65 70 75 80

Gly Arg Asp Leu Met Ala Cys Ala Gln Thr Gly Ser Gly Lys Thr Ala
85 90 95

Ala Phe Leu Leu Pro Ile Leu Ser Lys Leu Leu Glu Asp Pro His Glu
100 105 110

Leu Glu Leu Gly Arg Pro Gln Val Val Ile Val Ser Pro Thr Arg Glu
115 120 125

Leu Ala Ile Gln Ile Phe Asn Glu Ala Arg Lys Phe Ala Phe Glu Ser
130 135 140

Tyr Leu Lys Ile Gly Ile Val Tyr Gly Gly Thr Ser Phe Arg His Gln
145 150 155 160

Asn Glu Cys Ile Thr Arg Gly Cys His Val Val Ile Ala Thr Pro Gly
165 170 175

Arg Leu Leu Asp Phe Val Asp Arg Thr Phe Ile Thr Phe Glu Asp Thr
180 185 190

Arg Phe Val Val Leu Asp Glu Ala Asp Arg Met Leu Asp Met Gly Phe
195 200 205

Ser Glu Asp Met Arg Arg Ile Met Thr His Val Thr Met Arg Pro Glu
210 215 220

His Gln Thr Leu Met Phe Ser Ala Thr Phe Pro Glu Glu Ile Gln Arg
225 230 235 240

Met Ala Gly Glu Phe Leu Lys Asn Tyr Val Phe Val Ala Ile Gly Ile
245 250 255

Val Gly Gly Ala Cys Ser Asp Val Lys Gln Thr Ile Tyr Glu Val Asn
260 265 270

Lys Tyr Ala Lys Arg Ser Lys Leu Ile Glu Ile Leu Ser Glu Gln Ala
275 280 285

Asp Gly Thr Ile Val Phe Val Glu Thr Lys Arg Gly Ala Asp Phe Leu
290 295 300

Ala Ser Phe Leu Ser Glu Lys Glu Phe Pro Thr Thr Ser Ile His Gly
305 310 315 320

Asp Arg Leu Gln Ser Gln Arg Glu Gln Ala Leu Arg Asp Phe Lys Asn
325 330 335

Gly Ser Met Lys Val Leu Ile Ala Thr Ser Val Ala Ser Arg Gly Leu
340 345 350

Asp Ile Lys Asn Ile Lys His Val Ile Asn Tyr Asp Met Pro Ser Lys
355 360 365

Ile Asp Asp Tyr Val His Arg Ile Gly Arg Thr Gly Arg Val Gly Asn
370 375 380

Asn Gly Arg Ala Thr Ser Phe Phe Asp Pro Glu Lys Asp Arg Ala Ile
385 390 395 400

Ala Ala Asp Leu Val Lys Ile Leu Glu Gly Ser Gly Gln Thr Val Pro
405 410 415

Asp Phe Leu

<210> 3
<211> 724
<212> PRT
<213> Homo sapiens

<400> 3

Met Gly Asp Glu Asp Trp Glu Ala Glu Ile Asn Pro His Met Ser Ser
1 5 10 15

Tyr Val Pro Ile Phe Glu Lys Asp Arg Tyr Ser Gly Glu Asn Gly Asp
20 25 30

```
Asn Phe Asn Arg Thr Pro Ala Ser Ser Ser Glu Met Asp Asp Gly Pro
        35                  40                  45

Ser Arg Arg Asp His Phe Met Lys Ser Gly Phe Ala Ser Gly Arg Asn
    50                  55                  60

Phe Gly Asn Arg Asp Ala Gly Glu Cys Asn Lys Arg Asp Asn Thr Ser
65                  70                  75                  80

Thr Met Gly Gly Phe Gly Val Gly Lys Ser Phe Gly Asn Arg Gly Phe
                85                  90                  95

Ser Asn Ser Arg Phe Glu Asp Gly Asp Ser Ser Gly Phe Trp Arg Glu
            100                 105                 110

Ser Ser Asn Asp Cys Glu Asp Asn Pro Thr Arg Asn Arg Gly Phe Ser
        115                 120                 125

Lys Arg Gly Gly Tyr Arg Asp Gly Asn Asn Ser Glu Ala Ser Gly Pro
    130                 135                 140

Tyr Arg Arg Gly Gly Arg Gly Ser Phe Arg Gly Cys Arg Gly Gly Phe
145                 150                 155                 160

Gly Leu Gly Ser Pro Asn Asn Asp Leu Asp Pro Asp Glu Cys Met Gln
                165                 170                 175

Arg Thr Gly Gly Leu Phe Gly Ser Arg Arg Pro Val Leu Ser Gly Thr
            180                 185                 190

Gly Asn Gly Asp Thr Ser Gln Ser Arg Ser Gly Ser Gly Ser Glu Arg
        195                 200                 205

Gly Gly Tyr Lys Gly Leu Asn Glu Glu Val Ile Thr Gly Ser Gly Lys
    210                 215                 220

Asn Ser Trp Lys Ser Glu Ala Glu Gly Gly Glu Ser Ser Asp Thr Gln
225                 230                 235                 240

Gly Pro Lys Val Thr Tyr Ile Pro Pro Pro Pro Pro Glu Asp Glu Asp
                245                 250                 255

Ser Ile Phe Ala His Tyr Gln Thr Gly Ile Asn Phe Asp Lys Tyr Asp
            260                 265                 270

Thr Ile Leu Val Glu Val Ser Gly His Asp Ala Pro Pro Ala Ile Leu
```

275 280 285

Thr Phe Glu Glu Ala Asn Leu Cys Gln Thr Leu Asn Asn Asn Ile Ala
290 295 300

Lys Ala Gly Tyr Thr Lys Leu Thr Pro Val Gln Lys Tyr Ser Ile Pro
305 310 315 320

Ile Ile Leu Ala Gly Arg Asp Leu Met Ala Cys Ala Gln Thr Gly Ser
325 330 335

Gly Lys Thr Ala Ala Phe Leu Leu Pro Ile Leu Ala His Met Met His
340 345 350

Asp Gly Ile Thr Ala Ser Arg Phe Lys Glu Leu Gln Glu Pro Glu Cys
355 360 365

Ile Ile Val Ala Pro Thr Arg Glu Leu Val Asn Gln Ile Tyr Leu Glu
370 375 380

Ala Arg Lys Phe Ser Phe Gly Thr Cys Val Arg Ala Val Val Ile Tyr
385 390 395 400

Gly Gly Thr Gln Leu Gly His Ser Ile Arg Gln Ile Val Gln Gly Cys
405 410 415

Asn Ile Leu Cys Ala Thr Pro Gly Arg Leu Met Asp Ile Ile Gly Lys
420 425 430

Glu Lys Ile Gly Leu Lys Gln Ile Lys Tyr Leu Val Leu Asp Glu Ala
435 440 445

Asp Arg Met Leu Asp Met Gly Phe Gly Pro Glu Met Lys Lys Leu Ile
450 455 460

Ser Cys Pro Gly Met Pro Ser Lys Glu Gln Arg Gln Thr Leu Met Phe
465 470 475 480

Ser Ala Thr Phe Pro Glu Glu Ile Gln Arg Leu Ala Ala Glu Phe Leu
485 490 495

Lys Ser Asn Tyr Leu Phe Val Ala Val Gly Gln Val Gly Gly Ala Cys
500 505 510

Arg Asp Val Gln Gln Thr Val Leu Gln Val Gly Gln Phe Ser Lys Arg
515 520 525

```
Glu Lys Leu Val Glu Ile Leu Arg Asn Ile Gly Asp Glu Arg Thr Met
    530                 535                 540

Val Phe Val Glu Thr Lys Lys Lys Ala Asp Phe Ile Ala Thr Phe Leu
545                 550                 555                 560

Cys Gln Glu Lys Ile Ser Thr Thr Ser Ile His Gly Asp Arg Glu Gln
                565                 570                 575

Arg Glu Arg Glu Gln Ala Leu Gly Asp Phe Arg Phe Gly Lys Cys Pro
            580                 585                 590

Val Leu Val Ala Thr Ser Val Ala Ala Arg Gly Leu Asp Ile Glu Asn
        595                 600                 605

Val Gln His Val Ile Asn Phe Asp Leu Pro Ser Thr Ile Asp Glu Tyr
    610                 615                 620

Val His Arg Ile Gly Arg Thr Gly Arg Cys Gly Asn Thr Gly Arg Ala
625                 630                 635                 640

Ile Ser Phe Phe Asp Leu Glu Ser Asp Asn His Leu Ala Gln Pro Leu
                645                 650                 655

Val Lys Val Leu Thr Asp Ala Gln Gln Asp Val Pro Ala Trp Leu Glu
            660                 665                 670

Glu Ile Ala Phe Ser Thr Tyr Ile Pro Gly Phe Ser Gly Ser Thr Arg
        675                 680                 685

Gly Asn Val Phe Ala Ser Val Asp Thr Arg Lys Gly Lys Ser Thr Leu
    690                 695                 700

Asn Thr Ala Gly Phe Ser Ser Ser Gln Ala Pro Asn Pro Val Asp Asp
705                 710                 715                 720

Glu Ser Trp Asp


<210>  4
<211>  424
<212>  PRT
<213>  Drosophila melanogaster

<400>  4

Glu Phe Tyr Ile Pro Pro Glu Pro Ser Asn Asp Ala Ile Glu Ile Phe
1               5                   10                  15
```

```
Ser Ser Gly Ile Ala Ser Gly Ile His Phe Ser Lys Tyr Asn Asn Ile
            20                  25                  30

Pro Val Lys Val Thr Gly Ser Asp Val Pro Gln Pro Ile Gln His Phe
        35                  40                  45

Thr Ser Ala Asp Leu Arg Asp Ile Ile Ile Asp Asn Val Asn Lys Ser
    50                  55                  60

Gly Tyr Lys Ile Pro Thr Pro Ile Gln Lys Cys Ser Ile Pro Val Ile
65                  70                  75                  80

Ser Ser Gly Arg Asp Leu Met Ala Cys Ala Gln Thr Gly Ser Gly Lys
                85                  90                  95

Thr Ala Ala Phe Leu Leu Pro Ile Leu Ser Lys Leu Leu Glu Asp Pro
            100                 105                 110

His Glu Leu Glu Leu Gly Arg Pro Gln Val Val Ile Val Ser Pro Thr
        115                 120                 125

Arg Glu Leu Ala Ile Gln Ile Phe Asn Glu Ala Arg Lys Phe Ala Phe
    130                 135                 140

Glu Ser Tyr Leu Lys Ile Gly Ile Val Tyr Gly Gly Thr Ser Phe Arg
145                 150                 155                 160

His Gln Asn Glu Cys Ile Thr Arg Gly Cys His Val Val Ile Ala Thr
                165                 170                 175

Pro Gly Arg Leu Leu Asp Phe Val Asp Arg Thr Phe Ile Thr Phe Glu
            180                 185                 190

Asp Thr Arg Phe Val Val Leu Asp Glu Ala Asp Arg Met Leu Asp Met
        195                 200                 205

Gly Phe Ser Glu Asp Met Arg Arg Ile Met Thr His Val Thr Met Arg
    210                 215                 220

Pro Glu His Gln Thr Leu Met Phe Ser Ala Thr Phe Pro Glu Glu Ile
225                 230                 235                 240

Gln Arg Met Ala Gly Glu Phe Leu Lys Asn Tyr Val Phe Val Ala Ile
                245                 250                 255

Gly Ile Val Gly Gly Ala Cys Ser Asp Val Lys Gln Thr Ile Tyr Glu
            260                 265                 270
```

Val Asn Lys Tyr Ala Lys Arg Ser Lys Leu Ile Glu Ile Leu Ser Glu
275 280 285

Gln Ala Asp Gly Thr Ile Val Phe Val Glu Thr Lys Arg Gly Ala Asp
290 295 300

Phe Leu Ala Ser Phe Leu Ser Glu Lys Glu Phe Pro Thr Thr Ser Ile
305 310 315 320

His Gly Asp Arg Leu Gln Ser Gln Arg Glu Gln Ala Leu Arg Asp Phe
325 330 335

Lys Asn Gly Ser Met Lys Val Leu Ile Ala Thr Ser Val Ala Ser Arg
340 345 350

Gly Leu Asp Ile Lys Asn Ile Lys His Val Ile Asn Tyr Asp Met Pro
355 360 365

Ser Lys Ile Asp Asp Tyr Val His Arg Ile Gly Arg Thr Gly Arg Val
370 375 380

Gly Asn Asn Gly Arg Ala Thr Ser Phe Phe Asp Pro Glu Lys Asp Arg
385 390 395 400

Ala Ile Ala Ala Asp Leu Val Lys Ile Leu Glu Gly Ser Gly Gln Thr
405 410 415

Val Pro Asp Phe Leu Arg Thr Cys
420

<210> 5
<211> 614
<212> PRT
<213> Homo sapiens

<400> 5

Met Ser Gly Tyr Ser Ser Asp Arg Asp Arg Gly Arg Asp Arg Gly Phe
1 5 10 15

Gly Ala Pro Arg Phe Gly Gly Ser Arg Ala Gly Pro Leu Ser Gly Lys
20 25 30

Lys Phe Gly Asn Pro Gly Glu Lys Leu Val Lys Lys Lys Trp Asn Leu
35 40 45

Asp Glu Leu Pro Lys Phe Glu Lys Asn Phe Tyr Gln Glu His Pro Asp
50 55 60

```
Leu Ala Arg Arg Thr Ala Gln Glu Val Glu Thr Tyr Arg Arg Ser Lys
65                  70                  75                  80

Glu Ile Thr Val Arg Gly His Asn Cys Pro Lys Pro Val Leu Asn Phe
                85                  90                  95

Tyr Glu Ala Asn Phe Pro Ala Asn Val Met Asp Val Ile Ala Arg Gln
            100                 105                 110

Asn Phe Thr Glu Pro Thr Ala Ile Gln Ala Gln Gly Trp Pro Val Ala
        115                 120                 125

Leu Ser Gly Leu Asp Met Val Gly Val Ala Gln Thr Gly Ser Gly Lys
    130                 135                 140

Thr Leu Ser Tyr Leu Leu Pro Ala Ile Val His Ile Asn His Gln Pro
145                 150                 155                 160

Phe Leu Glu Arg Gly Asp Gly Pro Ile Cys Leu Val Leu Ala Pro Thr
                165                 170                 175

Arg Glu Leu Ala Gln Gln Val Gln Gln Val Ala Ala Glu Tyr Cys Arg
            180                 185                 190

Ala Cys Arg Leu Lys Ser Thr Cys Ile Tyr Gly Gly Ala Pro Lys Gly
        195                 200                 205

Pro Gln Ile Arg Asp Leu Glu Arg Gly Val Glu Ile Cys Ile Ala Thr
    210                 215                 220

Pro Gly Arg Leu Ile Asp Phe Leu Glu Cys Gly Lys Thr Asn Leu Arg
225                 230                 235                 240

Arg Thr Thr Tyr Leu Val Leu Asp Glu Ala Asp Arg Met Leu Asp Met
                245                 250                 255

Gly Phe Glu Pro Gln Ile Arg Lys Ile Val Asp Gln Ile Arg Pro Asp
            260                 265                 270

Arg Gln Thr Leu Met Trp Ser Ala Thr Trp Pro Lys Glu Val Arg Gln
        275                 280                 285

Leu Ala Glu Asp Phe Leu Lys Asp Tyr Ile His Ile Asn Ile Gly Ala
    290                 295                 300

Leu Glu Leu Ser Ala Asn His Asn Ile Leu Gln Ile Val Asp Val Cys
305                 310                 315                 320
```

His Asp Val Glu Lys Asp Glu Lys Leu Ile Arg Leu Met Glu Glu Ile
325 330 335

Met Ser Glu Lys Glu Asn Lys Thr Ile Val Phe Val Glu Thr Lys Arg
340 345 350

Arg Cys Asp Glu Leu Thr Arg Lys Met Arg Arg Asp Gly Trp Pro Ala
355 360 365

Met Gly Ile His Gly Asp Lys Ser Gln Gln Glu Arg Asp Trp Val Leu
370 375 380

Asn Glu Phe Lys His Gly Lys Ala Pro Ile Leu Ile Ala Thr Asp Val
385 390 395 400

Ala Ser Arg Gly Leu Asp Val Glu Asp Val Lys Phe Val Ile Asn Tyr
405 410 415

Asp Tyr Pro Asn Ser Ser Glu Asp Tyr Ile His Arg Ile Gly Arg Thr
420 425 430

Ala Arg Ser Thr Lys Thr Gly Thr Ala Tyr Thr Phe Phe Thr Pro Asn
435 440 445

Asn Ile Lys Gln Val Ser Asp Leu Ile Ser Val Leu Arg Glu Ala Asn
450 455 460

Gln Ala Ile Asn Pro Lys Leu Leu Gln Leu Val Glu Asp Arg Gly Ser
465 470 475 480

Gly Arg Ser Arg Gly Arg Gly Gly Met Lys Asp Asp Arg Arg Asp Arg
485 490 495

Tyr Ser Ala Gly Lys Arg Gly Gly Phe Asn Thr Phe Arg Asp Arg Glu
500 505 510

Asn Tyr Asp Arg Gly Tyr Ser Ser Leu Leu Lys Arg Asp Phe Gly Ala
515 520 525

Lys Thr Gln Asn Gly Val Tyr Ser Ala Ala Asn Tyr Thr Asn Gly Ser
530 535 540

Phe Gly Ser Asn Phe Val Ser Ala Gly Ile Gln Thr Ser Phe Arg Thr
545 550 555 560

Gly Asn Pro Thr Gly Thr Tyr Gln Asn Gly Tyr Asp Ser Thr Gln Gln

565 570 575

Tyr Gly Ser Asn Val Pro Asn Met His Asn Gly Met Asn Gln Gln Ala
580 585 590

Tyr Ala Tyr Pro Ala Thr Ala Ala Ala Pro Met Ile Gly Tyr Pro Met
595 600 605

Pro Thr Gly Tyr Ser Gln
610

<210> 6
<211> 398
<212> PRT
<213> Bombyx mori

<400> 6

Ile Ala Val Lys Val Ser Gly Glu Asn Pro Pro Arg Pro Ile Glu Ser
1 5 10 15

Phe Glu Thr Ala Asn Leu Arg Lys Tyr Val Leu Asp Asn Val Leu Lys
20 25 30

Ala Gly Tyr Arg Lys Pro Thr Pro Ile Gln Lys Asn Ala Ile Pro Ile
35 40 45

Ile Met Ser Gly Arg Asp Leu Met Gly Cys Ala Gln Thr Gly Ser Gly
50 55 60

Lys Thr Ala Ala Phe Leu Val Pro Ile Ile Asn Met Leu Leu Gln Asp
65 70 75 80

Pro Lys Asp Leu Ile Ser Glu Asn Gly Cys Ala Gln Pro Gln Val Ile
85 90 95

Ile Val Ser Pro Thr Arg Glu Leu Thr Leu Gln Ile Phe Asn Glu Ala
100 105 110

Arg Lys Phe Ser Tyr Gly Ser Val Leu Lys Val Ala Val Ala Tyr Gly
115 120 125

Gly Thr Ala Val Arg His Gln Gly Asp Asn Ile Ala Arg Gly Cys His
130 135 140

Ile Leu Val Ala Thr Pro Gly Arg Leu His Asp Phe Val Glu Arg Asn
145 150 155 160

Arg Val Ser Phe Gly Ser Val Arg Phe Val Val Leu Asp Gln Ala Asp

```
                165                 170                 175

Cys Met Leu Asp Met Gly Phe Met Pro Ser Ile Glu Lys Met Met Leu
            180                 185                 190

His Pro Thr Met Val Glu Thr Thr Lys Arg Gln Thr Leu Met Phe Ser
        195                 200                 205

Ala Thr Phe Pro Glu Asp Ile Gln His Leu Ala Gly Arg Phe Leu Asn
    210                 215                 220

Asn Tyr Leu Phe Val Ala Val Gly Ile Val Gly Gly Ala Ser Thr Asp
225                 230                 235                 240

Val Glu Gln Ile Phe Ile Glu Val Thr Lys Tyr Glu Lys Arg Asn Ser
                245                 250                 255

Leu Lys Gln Leu Ile Glu Glu Asn Asp Gly Lys Arg Ile Leu Val Phe
            260                 265                 270

Val Glu Thr Lys Arg Asn Ala Asp Phe Ile Ala Ala Met Leu Ser Glu
        275                 280                 285

Gln Gln Leu Leu Thr Ser Ser Ile His Gly Asp Arg Met Gln Arg Glu
    290                 295                 300

Arg Glu Glu Ala Leu Gln Asn Phe Lys Ser Gly Lys His Cys Ile Leu
305                 310                 315                 320

Val Ala Thr Ala Val Ala Ala Arg Gly Leu Asp Ile Lys Asn Val Asp
                325                 330                 335

Ile Val Val Asn Tyr Asp Leu Pro Lys Ser Ile Asp Glu Tyr Val His
            340                 345                 350

Arg Ile Gly Arg Thr Gly Arg Val Gly Asn Arg Gly Lys Ala Val Ser
        355                 360                 365

Phe Tyr Asp Ser Asp Gln Asp Leu Ala Leu Val Ala Asp Leu Ser Lys
    370                 375                 380

Ile Leu Arg Gln Ala Asp Gln Ser Val Pro Asp Phe Leu Lys
385                 390                 395
```

<210> 7
<211> 729
<212> PRT
<213> Homo sapiens

<400> 7

Met Pro Thr Gly Phe Val Ala Pro Ile Leu Cys Val Leu Leu Pro Ser
1               5                   10                  15

Pro Thr Arg Glu Ala Ala Thr Val Ala Ser Ala Thr Gly Asp Ser Ala
            20                  25                  30

Ser Glu Arg Glu Ser Ala Ala Pro Ala Ala Ala Pro Thr Ala Glu Ala
        35                  40                  45

Pro Pro Pro Ser Val Val Thr Arg Pro Glu Pro Gln Ala Leu Pro Ser
    50                  55                  60

Pro Ala Ile Arg Ala Pro Leu Pro Asp Leu Tyr Pro Phe Gly Thr Met
65                  70                  75                  80

Arg Gly Gly Gly Phe Gly Asp Arg Asp Arg Asp Arg Asp Arg Gly Gly
                85                  90                  95

Phe Gly Ala Arg Gly Gly Gly Gly Leu Pro Pro Lys Lys Phe Gly Asn
            100                 105                 110

Pro Gly Glu Arg Leu Arg Lys Lys Lys Trp Asp Leu Ser Glu Leu Pro
        115                 120                 125

Lys Phe Glu Lys Asn Phe Tyr Val Glu His Pro Glu Val Ala Arg Leu
    130                 135                 140

Thr Pro Tyr Glu Val Asp Glu Leu Arg Arg Lys Lys Glu Ile Thr Val
145                 150                 155                 160

Arg Gly Gly Asp Val Cys Pro Lys Pro Val Phe Ala Phe His His Ala
                165                 170                 175

Asn Phe Pro Gln Tyr Val Met Asp Val Leu Met Asp Gln His Phe Thr
            180                 185                 190

Glu Pro Thr Pro Ile Gln Cys Gln Gly Phe Pro Leu Ala Leu Ser Gly
        195                 200                 205

Arg Asp Met Val Gly Ile Ala Gln Thr Gly Ser Gly Lys Thr Leu Ala
    210                 215                 220

Tyr Leu Leu Pro Ala Ile Val His Ile Asn His Gln Pro Tyr Leu Glu
225                 230                 235                 240

```
Arg Gly Asp Gly Pro Ile Cys Leu Val Leu Ala Pro Thr Arg Glu Leu
                245                 250                 255

Ala Gln Gln Val Gln Gln Val Ala Asp Asp Tyr Gly Lys Cys Ser Arg
            260                 265                 270

Leu Lys Ser Thr Cys Ile Tyr Gly Gly Ala Pro Lys Gly Pro Gln Ile
        275                 280                 285

Arg Asp Leu Glu Arg Gly Val Glu Ile Cys Ile Ala Thr Pro Gly Arg
    290                 295                 300

Leu Ile Asp Phe Leu Glu Ser Gly Lys Thr Asn Leu Arg Arg Cys Thr
305                 310                 315                 320

Tyr Leu Val Leu Asp Glu Ala Asp Arg Met Leu Asp Met Gly Phe Glu
                325                 330                 335

Pro Gln Ile Arg Lys Ile Val Asp Gln Ile Arg Pro Asp Arg Gln Thr
            340                 345                 350

Leu Met Trp Ser Ala Thr Trp Pro Lys Glu Val Arg Gln Leu Ala Glu
        355                 360                 365

Asp Phe Leu Arg Asp Tyr Thr Gln Ile Asn Val Gly Asn Leu Glu Leu
    370                 375                 380

Ser Ala Asn His Asn Ile Leu Gln Ile Val Asp Val Cys Met Glu Ser
385                 390                 395                 400

Glu Lys Asp His Lys Leu Ile Gln Leu Met Glu Glu Ile Met Ala Glu
                405                 410                 415

Lys Glu Asn Lys Thr Ile Ile Phe Val Glu Thr Lys Arg Arg Cys Asp
            420                 425                 430

Asp Leu Thr Arg Arg Met Arg Arg Asp Gly Trp Pro Ala Met Cys Ile
        435                 440                 445

His Gly Asp Lys Ser Gln Pro Glu Arg Asp Trp Val Leu Asn Glu Phe
    450                 455                 460

Arg Ser Gly Lys Ala Pro Ile Leu Ile Ala Thr Asp Val Ala Ser Arg
465                 470                 475                 480

Gly Leu Asp Val Glu Asp Val Lys Phe Val Ile Asn Tyr Asp Tyr Pro
                485                 490                 495
```

Asn Ser Ser Glu Asp Tyr Val His Arg Ile Gly Arg Thr Ala Arg Ser
            500                 505                 510

Thr Asn Lys Gly Thr Ala Tyr Thr Phe Phe Thr Pro Gly Asn Leu Lys
        515                 520                 525

Gln Ala Arg Glu Leu Ile Lys Val Leu Glu Glu Ala Asn Gln Ala Ile
    530                 535                 540

Asn Pro Lys Leu Met Gln Leu Val Asp His Arg Gly Gly Gly Gly Gly
545                 550                 555                 560

Gly Gly Gly Arg Ser Arg Tyr Arg Thr Thr Ser Ser Ala Asn Asn Pro
                565                 570                 575

Asn Leu Met Tyr Gln Asp Glu Cys Asp Arg Arg Leu Arg Gly Val Lys
            580                 585                 590

Asp Gly Gly Arg Arg Asp Ser Ala Ser Tyr Arg Asp Arg Ser Glu Thr
        595                 600                 605

Asp Arg Ala Gly Tyr Ala Asn Gly Ser Gly Tyr Gly Ser Pro Asn Ser
    610                 615                 620

Ala Phe Gly Ala Gln Ala Gly Gln Tyr Thr Tyr Gly Gln Gly Thr Tyr
625                 630                 635                 640

Gly Ala Ala Ala Tyr Gly Thr Ser Ser Tyr Thr Ala Gln Glu Tyr Gly
                645                 650                 655

Ala Gly Thr Tyr Gly Ala Ser Ser Thr Thr Ser Thr Gly Arg Ser Ser
            660                 665                 670

Gln Ser Ser Ser Gln Gln Phe Ser Gly Ile Gly Arg Ser Gly Gln Gln
        675                 680                 685

Pro Gln Pro Leu Met Ser Gln Gln Phe Ala Gln Pro Pro Gly Ala Thr
    690                 695                 700

Asn Met Ile Gly Tyr Met Gly Gln Thr Ala Tyr Gln Tyr Pro Pro Pro
705                 710                 715                 720

Pro Pro Pro Pro Pro Pro Ser Arg Lys
                725

<210>  8
<211>  387

<212> PRT
<213> Bombyx mori

<400> 8

```
Gly Glu Asn Pro Pro Arg Pro Ile Glu Ser Phe Glu Thr Ala Asn Leu
1               5                   10                  15

Arg Lys Tyr Val Leu Asp Asn Val Leu Lys Ala Gly Tyr Arg Lys Pro
            20                  25                  30

Thr Pro Ile Gln Lys Asn Ala Ile Pro Ile Ile Met Ser Gly Arg Asp
        35                  40                  45

Leu Met Gly Cys Ala Gln Thr Gly Ser Gly Lys Thr Ala Ala Phe Leu
    50                  55                  60

Val Pro Ile Ile Asn Met Leu Leu Gln Asp Pro Lys Asp Leu Ile Ser
65                  70                  75                  80

Glu Asn Gly Cys Ala Gln Pro Gln Val Ile Ile Val Ser Pro Thr Arg
                85                  90                  95

Glu Leu Thr Leu Gln Ile Phe Asn Glu Ala Arg Lys Phe Ser Tyr Gly
            100                 105                 110

Ser Val Leu Lys Val Ala Val Ala Tyr Gly Gly Thr Ala Val Arg His
        115                 120                 125

Gln Gly Asp Asn Ile Ala Arg Gly Cys His Ile Leu Val Ala Thr Pro
    130                 135                 140

Gly Arg Leu His Asp Phe Val Glu Arg Asn Arg Val Ser Phe Gly Ser
145                 150                 155                 160

Val Arg Phe Val Val Leu Asp Gln Ala Asp Cys Met Leu Asp Met Gly
                165                 170                 175

Phe Met Pro Ser Ile Glu Lys Met Met Leu His Pro Thr Met Val Glu
            180                 185                 190

Thr Thr Lys Arg Gln Thr Leu Met Phe Ser Ala Thr Phe Pro Glu Asp
        195                 200                 205

Ile Gln His Leu Ala Gly Arg Phe Leu Asn Asn Tyr Leu Phe Val Ala
    210                 215                 220

Val Gly Ile Val Gly Gly Ala Ser Thr Asp Val Glu Gln Ile Phe Ile
225                 230                 235                 240
```

Glu Val Thr Lys Tyr Glu Lys Arg Asn Ser Leu Lys Gln Leu Ile Glu
245 250 255

Glu Asn Asp Gly Lys Arg Ile Leu Val Phe Val Glu Thr Lys Arg Asn
260 265 270

Ala Asp Phe Ile Ala Ala Met Leu Ser Glu Gln Gln Leu Leu Thr Ser
275 280 285

Ser Ile His Gly Asp Arg Met Gln Arg Glu Arg Glu Glu Ala Leu Gln
290 295 300

Asn Phe Lys Ser Gly Lys His Cys Ile Leu Val Ala Thr Ala Val Ala
305 310 315 320

Ala Arg Gly Leu Asp Ile Lys Asn Val Asp Ile Val Val Asn Tyr Asp
325 330 335

Leu Pro Lys Ser Ile Asp Glu Tyr Val His Arg Ile Gly Arg Thr Gly
340 345 350

Arg Val Gly Asn Arg Gly Lys Ala Val Ser Phe Tyr Asp Ser Asp Gln
355 360 365

Asp Leu Ala Leu Val Ala Asp Leu Ser Lys Ile Leu Arg Gln Ala Asp
370 375 380

Gln Ser Val
385

<210> 9
<211> 783
<212> PRT
<213> Homo sapiens

<400> 9

Met Pro Gly Lys Leu Arg Ser Asp Ala Gly Leu Glu Ser Asp Thr Ala
1 5 10 15

Met Lys Lys Gly Glu Thr Leu Arg Lys Gln Thr Glu Glu Lys Glu Lys
20 25 30

Lys Glu Lys Pro Lys Ser Asp Lys Thr Glu Glu Ile Ala Glu Glu Glu
35 40 45

Glu Thr Val Phe Pro Lys Ala Lys Gln Val Lys Lys Ala Glu Pro
50 55 60

Ser Glu Val Asp Met Asn Ser Pro Lys Ser Lys Lys Ala Lys Lys Lys
65 70 75 80

Glu Glu Pro Ser Gln Asn Asp Ile Ser Pro Lys Thr Lys Ser Leu Arg
85 90 95

Lys Lys Lys Glu Pro Ile Glu Lys Lys Val Val Ser Ser Lys Thr Lys
100 105 110

Lys Val Thr Lys Asn Glu Glu Pro Ser Glu Glu Glu Ile Asp Ala Pro
115 120 125

Lys Pro Lys Lys Met Lys Lys Glu Lys Glu Met Asn Gly Glu Thr Arg
130 135 140

Glu Lys Ser Pro Lys Leu Lys Asn Gly Phe Pro His Pro Glu Pro Asp
145 150 155 160

Cys Asn Pro Ser Glu Ala Ala Ser Glu Glu Ser Asn Ser Glu Ile Glu
165 170 175

Gln Glu Ile Pro Val Glu Gln Lys Glu Gly Ala Phe Ser Asn Phe Pro
180 185 190

Ile Ser Glu Glu Thr Ile Lys Leu Leu Lys Gly Arg Gly Val Thr Phe
195 200 205

Leu Phe Pro Ile Gln Ala Lys Thr Phe His His Val Tyr Ser Gly Lys
210 215 220

Asp Leu Ile Ala Gln Ala Arg Thr Gly Thr Gly Lys Thr Phe Ser Phe
225 230 235 240

Ala Ile Pro Leu Ile Glu Lys Leu His Gly Glu Leu Gln Asp Arg Lys
245 250 255

Arg Gly Arg Ala Pro Gln Val Leu Val Leu Ala Pro Thr Arg Glu Leu
260 265 270

Ala Asn Gln Val Ser Lys Asp Phe Ser Asp Ile Thr Lys Lys Leu Ser
275 280 285

Val Ala Cys Phe Tyr Gly Gly Thr Pro Tyr Gly Gly Gln Phe Glu Arg
290 295 300

Met Arg Asn Gly Ile Asp Ile Leu Val Gly Thr Pro Gly Arg Ile Lys

305 310 315 320

Asp His Ile Gln Asn Gly Lys Leu Asp Leu Thr Lys Leu Lys His Val
325 330 335

Val Leu Asp Glu Val Asp Gln Met Leu Asp Met Gly Phe Ala Asp Gln
340 345 350

Val Glu Glu Ile Leu Ser Val Ala Tyr Lys Lys Asp Ser Glu Asp Asn
355 360 365

Pro Gln Thr Leu Leu Phe Ser Ala Thr Cys Pro His Trp Val Phe Asn
370 375 380

Val Ala Lys Lys Tyr Met Lys Ser Thr Tyr Glu Gln Val Asp Leu Ile
385 390 395 400

Gly Lys Lys Thr Gln Lys Thr Ala Ile Thr Val Glu His Leu Ala Ile
405 410 415

Lys Cys His Trp Thr Gln Arg Ala Ala Val Ile Gly Asp Val Ile Arg
420 425 430

Val Tyr Ser Gly His Gln Gly Arg Thr Ile Ile Phe Cys Glu Thr Lys
435 440 445

Lys Glu Ala Gln Glu Leu Ser Gln Asn Ser Ala Ile Lys Gln Asp Ala
450 455 460

Gln Ser Leu His Gly Asp Ile Pro Gln Lys Gln Arg Glu Ile Thr Leu
465 470 475 480

Lys Gly Phe Arg Asn Gly Ser Phe Gly Val Leu Val Ala Thr Asn Val
485 490 495

Ala Ala Arg Gly Leu Asp Ile Pro Glu Val Asp Leu Val Ile Gln Ser
500 505 510

Ser Pro Pro Lys Asp Val Glu Ser Tyr Ile His Arg Ser Gly Arg Thr
515 520 525

Gly Arg Ala Gly Arg Thr Gly Val Cys Ile Cys Phe Tyr Gln His Lys
530 535 540

Glu Glu Tyr Gln Leu Val Gln Val Glu Gln Lys Ala Gly Ile Lys Phe
545 550 555 560

Lys Arg Ile Gly Val Pro Ser Ala Thr Glu Ile Ile Lys Ala Ser Ser
565 570 575

Lys Asp Ala Ile Arg Leu Leu Asp Ser Val Pro Pro Thr Ala Ile Ser
580 585 590

His Phe Lys Gln Ser Ala Glu Lys Leu Ile Glu Glu Lys Gly Ala Val
595 600 605

Glu Ala Leu Ala Ala Ala Leu Ala His Ile Ser Gly Ala Thr Ser Val
610 615 620

Asp Gln Arg Ser Leu Ile Asn Ser Asn Val Gly Phe Val Thr Met Ile
625 630 635 640

Leu Gln Cys Ser Ile Glu Met Pro Asn Ile Ser Tyr Ala Trp Lys Glu
645 650 655

Leu Lys Glu Gln Leu Gly Glu Glu Ile Asp Ser Lys Val Lys Gly Met
660 665 670

Val Phe Leu Lys Gly Lys Leu Gly Val Cys Phe Asp Val Pro Thr Ala
675 680 685

Ser Val Thr Glu Ile Gln Glu Lys Trp His Asp Ser Arg Arg Trp Gln
690 695 700

Leu Ser Val Ala Thr Glu Gln Pro Glu Leu Glu Gly Pro Arg Glu Gly
705 710 715 720

Tyr Gly Gly Phe Arg Gly Gln Arg Glu Gly Ser Arg Gly Phe Arg Gly
725 730 735

Gln Arg Asp Gly Asn Arg Arg Phe Arg Gly Gln Arg Glu Gly Ser Arg
740 745 750

Gly Pro Arg Gly Gln Arg Ser Gly Gly Gly Asn Lys Ser Asn Arg Ser
755 760 765

Gln Asn Lys Gly Gln Lys Arg Ser Phe Ser Lys Ala Phe Gly Gln
770 775 780

<210> 10
<211> 376
<212> PRT
<213> Bombyx mori

<400> 10

```
Pro Ile Glu Ser Phe Glu Thr Ala Asn Leu Arg Lys Tyr Val Leu Asp
1               5                   10                  15

Asn Val Leu Lys Ala Gly Tyr Arg Lys Pro Thr Pro Ile Gln Lys Asn
            20                  25                  30

Ala Ile Pro Ile Ile Met Ser Gly Arg Asp Leu Met Gly Cys Ala Gln
        35                  40                  45

Thr Gly Ser Gly Lys Thr Ala Ala Phe Leu Val Pro Ile Ile Asn Met
    50                  55                  60

Leu Leu Gln Asp Pro Lys Asp Leu Ile Ser Glu Asn Gly Cys Ala Gln
65                  70                  75                  80

Pro Gln Val Ile Ile Val Ser Pro Thr Arg Glu Leu Thr Leu Gln Ile
                85                  90                  95

Phe Asn Glu Ala Arg Lys Phe Ser Tyr Gly Ser Val Leu Lys Val Ala
            100                 105                 110

Val Ala Tyr Gly Gly Thr Ala Val Arg His Gln Gly Asp Asn Ile Ala
        115                 120                 125

Arg Gly Cys His Ile Leu Val Ala Thr Pro Gly Arg Leu His Asp Phe
    130                 135                 140

Val Glu Arg Asn Arg Val Ser Phe Gly Ser Val Arg Phe Val Val Leu
145                 150                 155                 160

Asp Gln Ala Asp Cys Met Leu Asp Met Gly Phe Met Pro Ser Ile Glu
                165                 170                 175

Lys Met Met Leu His Pro Thr Met Val Glu Thr Thr Lys Arg Gln Thr
            180                 185                 190

Leu Met Phe Ser Ala Thr Phe Pro Glu Asp Ile Gln His Leu Ala Gly
        195                 200                 205

Arg Phe Leu Asn Asn Tyr Leu Phe Val Ala Val Gly Ile Val Gly Gly
    210                 215                 220

Ala Ser Thr Asp Val Glu Gln Ile Phe Ile Glu Val Thr Lys Tyr Glu
225                 230                 235                 240

Lys Arg Asn Ser Leu Lys Gln Leu Ile Glu Glu Asn Asp Gly Lys Arg
                245                 250                 255
```

Ile Leu Val Phe Val Glu Thr Lys Arg Asn Ala Asp Phe Ile Ala Ala
260 265 270

Met Leu Ser Glu Gln Gln Leu Leu Thr Ser Ser Ile His Gly Asp Arg
275 280 285

Met Gln Arg Glu Arg Glu Glu Ala Leu Gln Asn Phe Lys Ser Gly Lys
290 295 300

His Cys Ile Leu Val Ala Thr Ala Val Ala Ala Arg Gly Leu Asp Ile
305 310 315 320

Lys Asn Val Asp Ile Val Val Asn Tyr Asp Leu Pro Lys Ser Ile Asp
325 330 335

Glu Tyr Val His Arg Ile Gly Arg Thr Gly Arg Val Gly Asn Arg Gly
340 345 350

Lys Ala Val Ser Phe Tyr Asp Ser Asp Gln Asp Leu Ala Leu Val Ala
355 360 365

Asp Leu Ser Lys Ile Leu Arg Gln
370 375

<210> 11
<211> 820
<212> PRT
<213> Homo sapiens

<400> 11

Met Ala Gly Glu Leu Ala Asp Lys Lys Asp Arg Asp Ala Ser Pro Ser
1 5 10 15

Lys Glu Glu Arg Lys Arg Ser Arg Thr Pro Asp Arg Glu Arg Asp Arg
20 25 30

Asp Arg Asp Arg Lys Ser Ser Pro Ser Lys Asp Arg Lys Arg His Arg
35 40 45

Ser Arg Asp Arg Arg Arg Gly Gly Ser Arg Ser Arg Ser Arg Ser Arg
50 55 60

Ser Lys Ser Ala Glu Arg Glu Arg Arg His Lys Glu Arg Glu Arg Asp
65 70 75 80

Lys Glu Arg Asp Arg Asn Lys Lys Asp Arg Asp Arg Asp Lys Asp Gly
85 90 95

```
His Arg Arg Asp Lys Asp Arg Lys Arg Ser Ser Leu Ser Pro Gly Arg
            100                 105                 110

Gly Lys Asp Phe Lys Ser Arg Lys Asp Arg Asp Ser Lys Lys Asp Glu
        115                 120                 125

Glu Asp Glu His Gly Asp Lys Lys Pro Lys Ala Gln Pro Leu Ser Leu
    130                 135                 140

Glu Glu Leu Leu Ala Lys Lys Lys Ala Glu Glu Glu Ala Glu Ala Lys
145                 150                 155                 160

Pro Lys Phe Leu Ser Lys Ala Glu Arg Glu Ala Glu Ala Leu Lys Arg
                165                 170                 175

Arg Gln Gln Glu Val Glu Glu Arg Gln Arg Met Leu Glu Glu Glu Arg
            180                 185                 190

Lys Lys Arg Lys Gln Phe Gln Asp Leu Gly Arg Lys Met Leu Glu Asp
        195                 200                 205

Pro Gln Glu Arg Glu Arg Arg Glu Arg Arg Glu Arg Met Glu Arg Glu
    210                 215                 220

Thr Asn Gly Asn Glu Asp Glu Glu Gly Arg Gln Lys Ile Arg Glu Glu
225                 230                 235                 240

Lys Asp Lys Ser Lys Glu Leu His Ala Ile Lys Glu Arg Tyr Leu Gly
                245                 250                 255

Gly Ile Lys Lys Arg Arg Arg Thr Arg His Leu Asn Asp Arg Lys Phe
            260                 265                 270

Val Phe Glu Trp Asp Ala Ser Glu Asp Thr Ser Ile Asp Tyr Asn Pro
        275                 280                 285

Leu Tyr Lys Glu Arg His Gln Val Gln Leu Leu Gly Arg Gly Phe Ile
    290                 295                 300

Ala Gly Ile Asp Leu Lys Gln Gln Lys Arg Glu Gln Ser Arg Phe Tyr
305                 310                 315                 320

Gly Asp Leu Met Glu Lys Arg Arg Thr Leu Glu Glu Lys Glu Gln Glu
                325                 330                 335

Glu Ala Arg Leu Arg Lys Leu Arg Lys Lys Glu Ala Lys Gln Arg Trp
            340                 345                 350
```

```
Asp Asp Arg His Trp Ser Gln Lys Lys Leu Asp Glu Met Thr Asp Arg
        355                 360                 365

Asp Trp Arg Ile Phe Arg Glu Asp Tyr Ser Ile Thr Thr Lys Gly Gly
    370                 375                 380

Lys Ile Pro Asn Pro Ile Arg Ser Trp Lys Asp Ser Ser Leu Pro Pro
385                 390                 395                 400

His Ile Leu Glu Val Ile Asp Lys Cys Gly Tyr Lys Glu Pro Thr Pro
                405                 410                 415

Ile Gln Arg Gln Ala Ile Pro Ile Gly Leu Gln Asn Arg Asp Ile Ile
            420                 425                 430

Gly Val Ala Glu Thr Gly Ser Gly Lys Thr Ala Ala Phe Leu Ile Pro
        435                 440                 445

Leu Leu Val Trp Ile Thr Thr Leu Pro Lys Ile Asp Arg Ile Glu Glu
    450                 455                 460

Ser Asp Gln Gly Pro Tyr Ala Ile Ile Leu Ala Pro Thr Arg Glu Leu
465                 470                 475                 480

Ala Gln Gln Ile Glu Glu Glu Thr Ile Lys Phe Gly Lys Pro Leu Gly
                485                 490                 495

Ile Arg Thr Val Ala Val Ile Gly Gly Ile Ser Arg Glu Asp Gln Gly
            500                 505                 510

Phe Arg Leu Arg Met Gly Cys Glu Ile Val Ile Ala Thr Pro Gly Arg
        515                 520                 525

Leu Ile Asp Val Leu Glu Asn Arg Tyr Leu Val Leu Ser Arg Cys Thr
    530                 535                 540

Tyr Val Val Leu Asp Glu Ala Asp Arg Met Ile Asp Met Gly Phe Glu
545                 550                 555                 560

Pro Asp Val Gln Lys Ile Leu Glu His Met Pro Val Ser Asn Gln Lys
                565                 570                 575

Pro Asp Thr Asp Glu Ala Glu Asp Pro Glu Lys Met Leu Ala Asn Phe
            580                 585                 590

Glu Ser Gly Lys His Lys Tyr Arg Gln Thr Val Met Phe Thr Ala Thr
```

595 600 605

Met Pro Pro Ala Val Glu Arg Leu Ala Arg Ser Tyr Leu Arg Arg Pro
610 615 620

Ala Val Val Tyr Ile Gly Ser Ala Gly Lys Pro His Glu Arg Val Glu
625 630 635 640

Gln Lys Val Phe Leu Met Ser Glu Ser Glu Lys Arg Lys Lys Leu Leu
645 650 655

Ala Ile Leu Glu Gln Gly Phe Asp Pro Pro Ile Ile Ile Phe Val Asn
660 665 670

Gln Lys Lys Gly Cys Asp Val Leu Ala Lys Ser Leu Glu Lys Met Gly
675 680 685

Tyr Asn Ala Cys Thr Leu His Gly Gly Lys Gly Gln Glu Gln Arg Glu
690 695 700

Phe Ala Leu Ser Asn Leu Lys Ala Gly Ala Lys Asp Ile Leu Val Ala
705 710 715 720

Thr Asp Val Ala Gly Arg Gly Ile Asp Ile Gln Asp Val Ser Met Val
725 730 735

Val Asn Tyr Asp Met Ala Lys Asn Ile Glu Asp Tyr Ile His Arg Ile
740 745 750

Gly Arg Thr Gly Arg Ala Gly Lys Ser Gly Val Ala Ile Thr Phe Leu
755 760 765

Thr Lys Glu Asp Ser Ala Val Phe Tyr Glu Leu Lys Gln Ala Ile Leu
770 775 780

Glu Ser Pro Val Ser Ser Cys Pro Pro Glu Leu Ala Asn His Pro Asp
785 790 795 800

Ala Gln His Lys Pro Gly Thr Ile Leu Thr Lys Lys Arg Arg Glu Glu
805 810 815

Thr Ile Phe Ala
820

<210> 12
<211> 385
<212> PRT
<213> Drosophila melanogaster

<400> 12

Val Lys Val Thr Gly Ser Asp Val Pro Gln Pro Ile Gln His Phe Thr
1 5 10 15

Ser Ala Asp Leu Arg Asp Ile Ile Ile Asp Asn Val Asn Lys Ser Gly
20 25 30

Tyr Lys Ile Pro Thr Pro Ile Gln Lys Cys Ser Ile Pro Val Ile Ser
35 40 45

Ser Gly Arg Asp Leu Met Ala Cys Ala Gln Thr Gly Ser Gly Lys Thr
50 55 60

Ala Ala Phe Leu Leu Pro Ile Leu Ser Lys Leu Leu Glu Asp Pro His
65 70 75 80

Glu Leu Glu Leu Gly Arg Pro Gln Val Val Ile Val Ser Pro Thr Arg
85 90 95

Glu Leu Ala Ile Gln Ile Phe Asn Glu Ala Arg Lys Phe Ala Phe Glu
100 105 110

Ser Tyr Leu Lys Ile Gly Ile Val Tyr Gly Gly Thr Ser Phe Arg His
115 120 125

Gln Asn Glu Cys Ile Thr Arg Gly Cys His Val Val Ile Ala Thr Pro
130 135 140

Gly Arg Leu Leu Asp Phe Val Asp Arg Thr Phe Ile Thr Phe Glu Asp
145 150 155 160

Thr Arg Phe Val Val Leu Asp Glu Ala Asp Arg Met Leu Asp Met Gly
165 170 175

Phe Ser Glu Asp Met Arg Arg Ile Met Thr His Val Thr Met Arg Pro
180 185 190

Glu His Gln Thr Leu Met Phe Ser Ala Thr Phe Pro Glu Glu Ile Gln
195 200 205

Arg Met Ala Gly Glu Phe Leu Lys Asn Tyr Val Phe Val Ala Ile Gly
210 215 220

Ile Val Gly Gly Ala Cys Ser Asp Val Lys Gln Thr Ile Tyr Glu Val
225 230 235 240

```
Asn Lys Tyr Ala Lys Arg Ser Lys Leu Ile Glu Ile Leu Ser Glu Gln
                245                 250                 255

Ala Asp Gly Thr Ile Val Phe Val Glu Thr Lys Arg Gly Ala Asp Phe
            260                 265                 270

Leu Ala Ser Phe Leu Ser Glu Lys Glu Phe Pro Thr Thr Ser Ile His
        275                 280                 285

Gly Asp Arg Leu Gln Ser Gln Arg Glu Gln Ala Leu Arg Asp Phe Lys
    290                 295                 300

Asn Gly Ser Met Lys Val Leu Ile Ala Thr Ser Val Ala Ser Arg Gly
305                 310                 315                 320

Leu Asp Ile Lys Asn Ile Lys His Val Ile Asn Tyr Asp Met Pro Ser
                325                 330                 335

Lys Ile Asp Asp Tyr Val His Arg Ile Gly Arg Thr Gly Arg Val Gly
            340                 345                 350

Asn Asn Gly Arg Ala Thr Ser Phe Phe Asp Pro Glu Lys Asp Arg Ala
        355                 360                 365

Ile Ala Ala Asp Leu Val Lys Ile Leu Glu Gly Ser Gly Gln Thr Val
    370                 375                 380

Pro
385
```